# EUROPEAN PATENT APPLICATION

(11) **EP 1 982 768 A2**
(43) Date of publication of application: **22.10.2008**
(21) Application number: 08251104.9
(22) Date of filing: 27.03.2008
(51) Int. Cl.: B01L 3/00, G01N 30/00, G01N 33/543

(54) **Methods for pathogen detection**

(30) Priority: 27.03.2007 US 729301; 27.03.2007 US 729274; 27.03.2007 US 729276; 27.03.2007 US 729275
(71) Applicant: Searete LLC, Bellevue, WA 98004 (US)
(72) Inventor: Jung, Edward K. Y., Bellevue WA 98005-1403 (US); Leuthardt, Eric C., St. Louis, MO 63130 (US); Levien, Royce A., Lexington, MA 02420 (US); Lord, Robert W., Seattle, WA 98112 (US); Malamud, Mark A., Seattle, WA 98119 (US); Rinaldo, John D. Jr., Bellevue, WA 98005 (US); Wood, Lowell L. Jr., Bellevue, WA 98004 (US)
(74) Representative: Harris, Ian Richard

(57) **Abstract**

The present disclosure relates to methods, systems, devices, and microfluidic chips that may be used for the detection of pathogens.

## Description

### Related Applications:

The present application is related to the following United States Patent Applications:
United States Patent Application No.Unknown, entitled SYSTEMS FOR PATHOGEN DETECTION, naming Edward K.Y. Jung, Eric C. Leuthardt, Royce A. Levien, Robert W. Lord, Mark A. Malamud, John D. Rinaldo, Jr., and Lowell L. Wood, Jr. as inventors, filed 27 March 2007.
United States Patent Application No.Unknown, entitled DEVICES FOR PATHOGEN DETECTION, naming Edward K.Y. Jung, Eric C. Leuthardt, Royce A. Levien, Robert W. Lord, Mark A. Malamud, John D. Rinaldo, Jr., and Lowell L. Wood, Jr. as inventors, filed 27 March 2007.
United States Patent Application No.Unknown, entitled MICROFLUIDIC CHIPS FOR PATHOGEN DETECTION, naming Edward K.Y. Jung, Eric C. Leuthardt, Royce A. Levien, Robert W. Lord, Mark A. Malamud, John D. Rinaldo, Jr., and Lowell L. Wood, Jr. as inventors, filed 27 March 2007.
All subject matter of the Related Applications and of any and all parent, grandparent, great-grandparent, etc. applications of the Related Applications is incorporated herein by reference to the extent such subject matter is not inconsistent herewith.

### TECHNICAL FIELD

The present disclosure relates to methods, systems, devices, and microfluidic chips that may be used for detection of one or more pathogens.

### SUMMARY

In some embodiments one or more methods are provided that include accepting one or more samples with one or more microfluidic chips and processing the one or more samples with the one or more microfluidic chips to facilitate analysis of one or more pathogen indicators associated with the one or more samples. The method may optionally include analyzing the one or more pathogen indicators with one or more analysis units that are configured to operably associate with the one or more microfluidic chips. The method may optionally include identifying one or more pathogens present within the one or more samples. In addition to the foregoing, other aspects are described in the claims, drawings, and text forming a part of the present disclosure.

In some embodiments one or more methods are provided that include processing one or more samples with one or more microfluidic chips to facilitate analysis of one or more pathogen indicators associated with the one or more samples and analyzing the one or more samples with one or more analysis units that are configured to operably associate with the one or more microfluidic chips. The method may optionally include identifying one or more pathogens present within the one or more samples. In addition to the foregoing, other aspects are described in the claims, drawings, and text forming a part of the present disclosure.

In some embodiments one or more methods are provided that include combining one or more samples with one or more magnetically active pathogen indicator binding agents that can bind to one or more pathogen indicators associated with the one or more samples to form one or more magnetically active pathogen indicator complexes and separating the one or more magnetically active pathogen indicator complexes from the one or more samples through use of one or more magnetic fields and one or more separation fluids that are in substantially parallel flow with the one or more samples. The method may optionally include analyzing the one or more samples with one or more analysis units. The method may optionally include identifying one or more pathogens present within the one or more samples. In addition to the foregoing, other aspects are described in the claims, drawings, and text forming a part of the present disclosure.

In some embodiments one or more methods are provided that include combining one or more samples with one or more magnetically active pathogen indicator binding agents that can bind to one or more pathogen indicators associated with the one or more samples to form one or more magnetically active pathogen indicator complexes and separating the one or more magnetically active pathogen indicator complexes from the one or more samples through use of one or more magnetic fields and one or more separation fluids that are in substantially antiparallel flow with the one or more samples. The method may optionally include analyzing the one or more samples with one or more analysis units. The method may optionally include identifying one or more pathogens present within the one or more samples. In addition to the foregoing, other aspects are described in the claims, drawings, and text forming a part of the present disclosure.

In some embodiments one or more methods are provided that include accepting one or more samples that include one or more magnetically active pathogen indicator binding agents that can bind to one or more pathogen indicators associated with the one or more samples to form one or more magnetically active pathogen indicator complexes and separating the one or more magnetically active pathogen indicator complexes from the one or more samples through use of one or more magnetic fields and one or more separation fluids that are in substantially parallel flow with the one or more samples. The method may optionally include analyzing the one or more samples with one or more analysis units. The method may optionally include identifying one or more pathogens present within the one or more samples. In addition to the foregoing, other aspects are described in the claims, drawings, and text forming a part of the present disclosure.

In some embodiments one or more methods are provided that include accepting one or more samples that include one or more magnetically active pathogen indicator binding agents that can bind to one or more pathogen indicators associated with the one or more samples to form one or more magnetically active pathogen indicator complexes and separating the one or more magnetically active pathogen indicator complexes from the one or more samples through use of one or more magnetic fields and one or more separation fluids that are in substantially antiparallel flow with the one or more samples. The method may optionally include analyzing the one or more samples with one or more analysis units. The method may optionally include identifying one or more pathogens present within the one or more samples. In addition to the foregoing, other aspects are described in the claims, drawings, and text forming a part of the present disclosure.

In some embodiments one or more methods are provided that include separating one or more magnetically active pathogen indicator complexes from one or more samples through use of one or more magnetic fields and one or more separation fluids that are in substantially parallel flow with the one or more samples. The method may optionally include detecting one or more pathogen indicators with one or more detection units. The method may optionally include identifying one or more pathogens present within the one or more samples. In addition to the foregoing, other aspects are described in the claims, drawings, and text forming a part of the present disclosure.

In some embodiments one or more methods are provided that include separating one or more magnetically active pathogen indicator complexes from one or more samples through use of one or more magnetic fields and one or more separation fluids that are in substantially antiparallel flow with the one or more samples. The method may optionally include detecting one or more pathogen indicators with one or more detection units. The method may optionally include identifying one or more pathogens present within the one or more samples. In addition to the foregoing, other aspects are described in the claims, drawings, and text forming a part of the present disclosure.

In some embodiments one or more systems are provided that include one or more micro fluidic chips configured to facilitate detection of one or more pathogen indicators associated with one or more samples and one or more detection units configured to detect the one or more pathogen indicators. The system may optionally include one or more display units operably associated with the one or more detection units. The system may optionally include one or more reagent delivery units configured to deliver one or more reagents to the one or more microfluidic chips. The system may optionally include one or more centrifugation units. The system may optionally include one or more reservoir units. In addition to the foregoing, other aspects are described in the claims, drawings, and text forming a part of the present disclosure.

In some embodiments one or more systems are provided that include one or more microfluidic chips that are configured to allow one or more magnetically active pathogen indicator binding agents to bind to one or more pathogen indicators associated with one or more samples to form one or more magnetically active pathogen indicator complexes and separate the one or more magnetically active pathogen indicator complexes from the one or more samples through use of one or more magnetic fields and one or more separation fluids that are in substantially parallel flow with the one or more samples. The system may optionally include one or more detection units configured to detect the one or more pathogen indicators associated with the one or more samples. The system may optionally include one or more display units operably associated with the one or more detection units. The system may optionally include one or more reagent delivery units configured to deliver one or more reagents to the one or more microfluidic chips. The system may optionally include one or more centrifugation units. The system may optionally include one or more reservoir units. In addition to the foregoing, other aspects are described in the claims, drawings, and text forming a part of the present disclosure.

In some embodiments one or more systems are provided that include one or more microfluidic chips that are configured to allow one or more magnetically active pathogen indicator binding agents to bind to one or more pathogen indicators associated with one or more samples to form one or more magnetically active pathogen indicator complexes and separate the one or more magnetically active pathogen indicator complexes from the one or more samples through use of one or more magnetic fields and one or more separation fluids that are in substantially antiparallel flow with the one or more samples. The system may optionally include one or more detection units configured to detect the one or more pathogen indicators associated with the one or more samples. The system may optionally include one or more display units operably associated with the one or more detection units. The system may optionally include one or more reagent delivery units configured to deliver one or more reagents to the one or more microfluidic chips. The system may optionally include one or more centrifugation units. The system may optionally include one or more reservoir units. In addition to the foregoing, other aspects are described in the claims, drawings, and text forming a part of the present disclosure.

In some embodiments one or more devices are provided that include one or more detection units configured to detachably connect to one or more microfluidic chips and configured to detect one or more pathogen indicators that are associated with one or more samples. The device may optionally include one or more reagent delivery units that are configured to deliver one or more reagents to the one or more microfluidic chips. The device may optionally include one or more controllable magnets that are configured to facilitate movement of a magnetically active plug that is included within the one or more microfluidic chips. In addition to the foregoing, other aspects are described in the claims, drawings, and text forming a part of the present disclosure.

In some embodiments one or more devices are provided that include one or more fasteners adapted to detachably associate with one or more microfluidic chips that include one or more separation channels that are configured to allow one or more samples that include one or more magnetically active pathogen indicator complexes to flow in a substantially parallel manner with one or more separation fluids and one or more magnets that facilitate movement of the one or more magnetically active pathogen indicator complexes associated with the one or more samples into the one or more separation fluids. In addition to the foregoing, other aspects are described in the claims, drawings, and text forming a part of the present disclosure.

In some embodiments one or more devices are provided that include one or more fasteners adapted to detachably associate with one or more microfluidic chips that include one or more separation channels that are configured to allow one or more samples that include one or more magnetically active pathogen indicator complexes to flow in a substantially antiparallel manner with one or more separation fluids and one or more magnets that facilitate movement of the one or more magnetically active pathogen indicator complexes associated with the one or more samples into the one or more separation fluids. In addition to the foregoing, other aspects are described in the claims, drawings, and text forming a part of the present disclosure.

In some embodiments one or more microfluidic chips are provided that include one or more accepting units configured to accept one or more samples and one or more processing units configured to process the one or more samples for one or more pathogen indicators associated with the one or more samples. The microfluidic chips may optionally include one or more analysis units configured for analysis of the one or more pathogen indicators associated with the one or more samples. The microfluidic chips may optionally include one or more detection chambers configured to facilitate detection of the one or more pathogen indicators associated with the one or more samples. In addition to the foregoing, other aspects are described in the claims, drawings, and text forming a part of the present disclosure.

In some embodiments one or more microfluidic chips are provided that include one or more separation channels that are configured to allow one or more samples that include one or more magnetically active pathogen indicator complexes to flow in a substantially parallel manner with one or more separation fluids and one or more magnetic fields that facilitate movement of the one or more magnetically active pathogen indicator complexes associated with the one or more samples into the one or more separation fluids. The microfluidic chips may optionally include one or more mixing chambers that are configured to allow one or more magnetically active pathogen indicator binding agents to bind to one or more pathogen indicators associated with the one or more samples to form one or more magnetically active pathogen indicator complexes. The microfluidic chips may optionally include one or more detection chambers configured to facilitate detection of the one or more pathogen indicators associated with the one or more samples. In addition to the foregoing, other aspects are described in the claims, drawings, and text forming a part of the present disclosure.

In some embodiments one or more microfluidic chips are provided that include one or more separation channels that are configured to allow one or more samples that include one or more magnetically active pathogen indicator complexes to flow in a substantially antiparallel manner with one or more separation fluids and one or more magnetic fields that facilitate movement of the one or more magnetically active pathogen indicator complexes associated with the one or more samples into the one or more separation fluids. The microfluidic chips may optionally include one or more mixing chambers that are configured to allow one or more magnetically active pathogen indicator binding agents to bind to one or more pathogen indicators associated with the one or more samples to form the one or more magnetically active pathogen indicator complexes. The microfluidic chips may optionally include one or more detection chambers configured to facilitate detection of the one or more pathogen indicators associated with the one or more samples. In addition to the foregoing, other aspects are described in the claims, drawings, and text forming a part of the present disclosure.

In some embodiments, means include but are not limited to circuitry and/or programming for effecting the herein referenced functional aspects; the circuitry and/or programming can be virtually any combination of hardware, software, and/or firmware configured to effect the herein referenced functional aspects depending upon the design choices of the system designer. In addition to the foregoing, other system aspects means are described in the claims, drawings, and/or text forming a part of the present disclosure.

In some embodiments, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer. In addition to the foregoing, other system aspects are described in the claims, drawings, and/or text forming a part of the present application.

The foregoing summary is illustrative only and is not intended to be in any way limiting. In addition to the illustrative aspects, embodiments, and features described above, further aspects, embodiments, and features will become apparent by reference to the drawings, claims, and the following detailed description.

### BRIEF DESCRIPTION OF THE FIGURES

FIG. 1 illustrates an example system 100 in which embodiments may be implemented.
FIG. 2 illustrates an operational flow representing example operations related to methods and systems for analysis of pathogens.
FIG. 3 illustrates alternate embodiments of the example operational flow of FIG. 2.
FIG. 4 illustrates alternate embodiments of the example operational flow of FIG. 2.
FIG. 5 illustrates alternate embodiments of the example operational flow of FIG. 2.
FIG. 6 illustrates alternate embodiments of the example operational flow of FIG. 2.
FIG. 7 illustrates an operational flow representing example operations related to methods and systems for analysis of pathogens.
FIG. 8 illustrates alternate embodiments of the example operational flow of FIG. 7.
FIG. 9 illustrates alternate embodiments of the example operational flow of FIG. 7.
FIG. 10 illustrates alternate embodiments of the example operational flow of FIG. 7.
FIG. 11 illustrates an operational flow representing example operations related to methods and systems for analysis of pathogens.
FIG. 12 illustrates alternate embodiments of the example operational flow of FIG. 11.
FIG. 13 illustrates alternate embodiments of the example operational flow of FIG. 11.
FIG. 14 illustrates alternate embodiments of the example operational flow of FIG. 11.
FIG. 15 illustrates alternate embodiments of the example operational flow of FIG. 11.
FIG. 16 illustrates an operational flow representing example operations related to methods and systems for analysis of pathogens.
FIG. 17 illustrates alternate embodiments of the example operational flow of FIG. 16.
FIG. 18 illustrates alternate embodiments of the example operational flow of FIG. 16.
FIG. 19 illustrates alternate embodiments of the example operational flow of FIG. 16.
FIG. 20 illustrates alternate embodiments of the example operational flow of FIG. 16.
**FIG.** 21 illustrates an operational flow representing example operations related to methods and systems for analysis of pathogens.

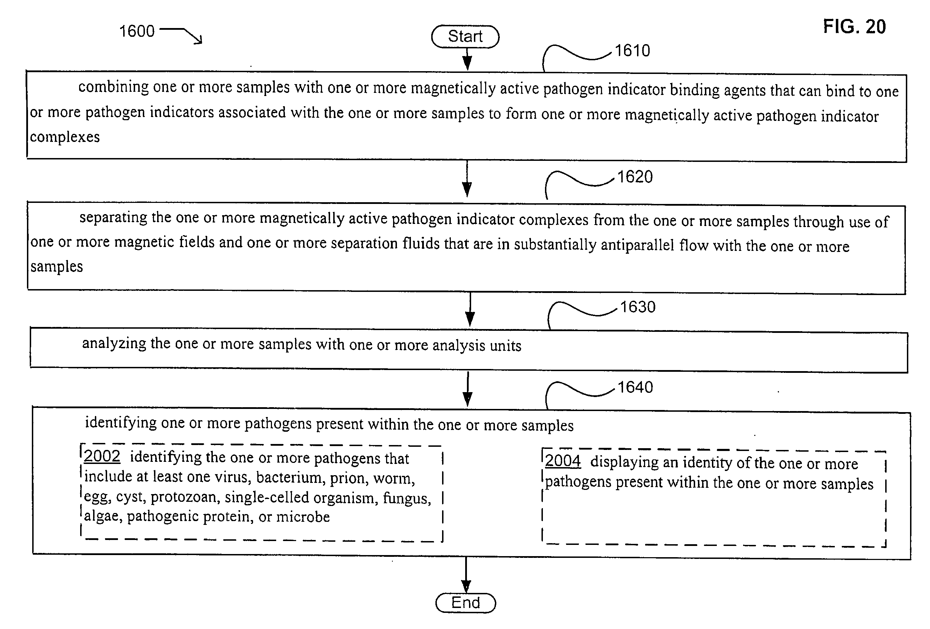

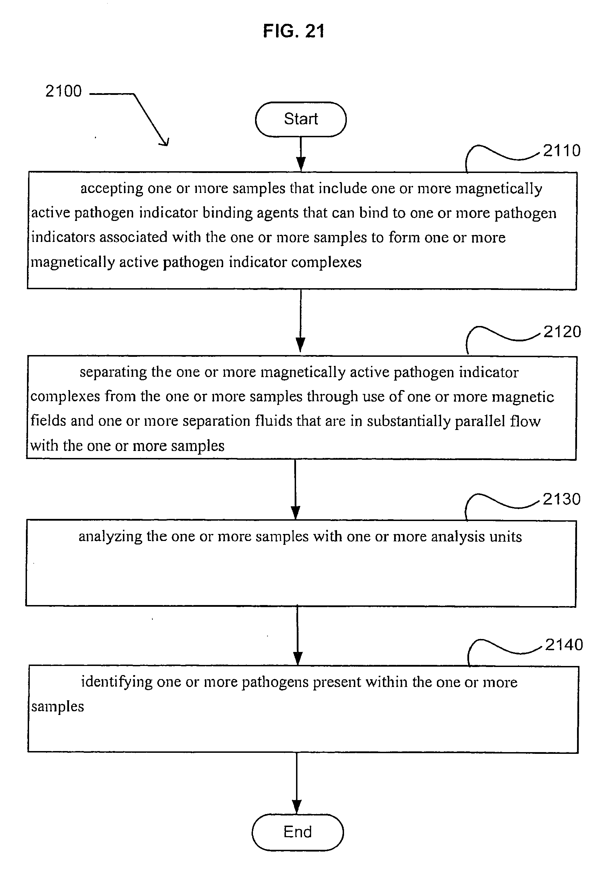

FIG. 20 illustrates alternate embodiments of the example operational flow of FIG. 21.
FIG. 21 illustrates alternate embodiments of the example operational flow of FIG. 21.

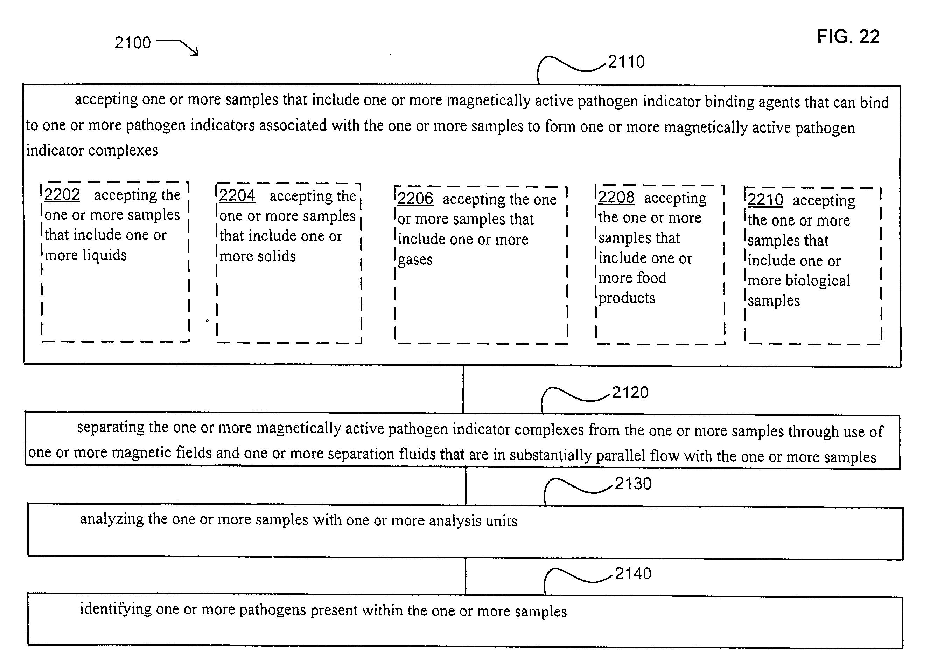

FIG. 22 illustrates alternate embodiments of the example operational flow of FIG. 21.

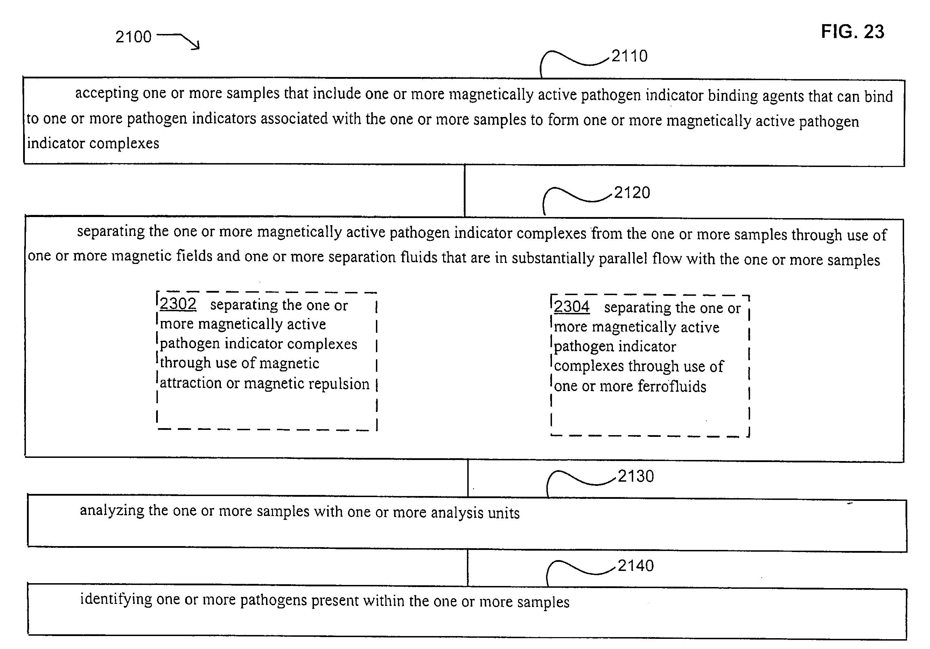

FIG. 23 illustrates alternate embodiments of the example operational flow of FIG. 21.

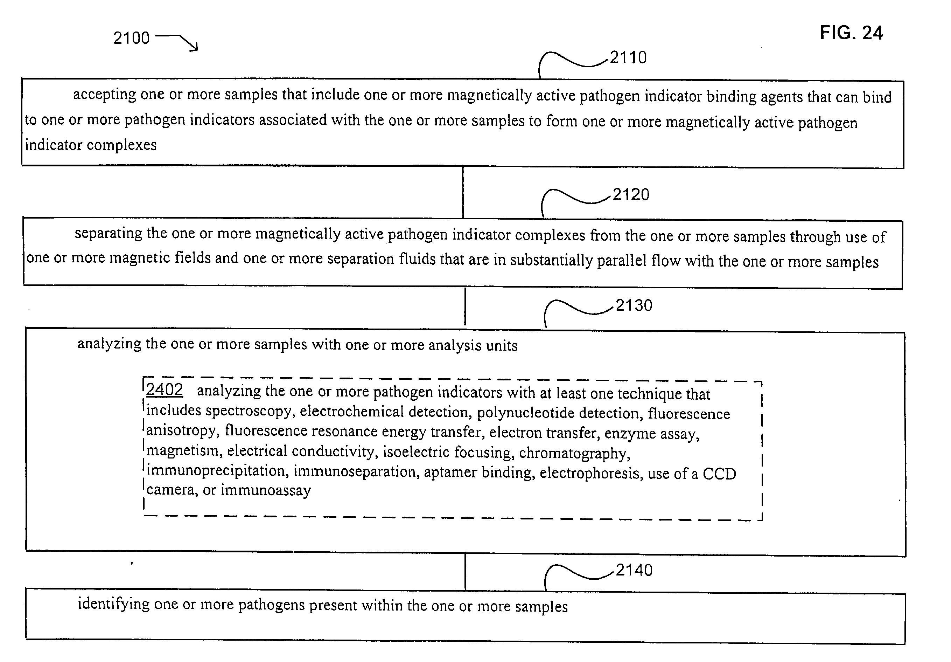

FIG. 24 illustrates alternate embodiments of the example operational flow of FIG. 21.

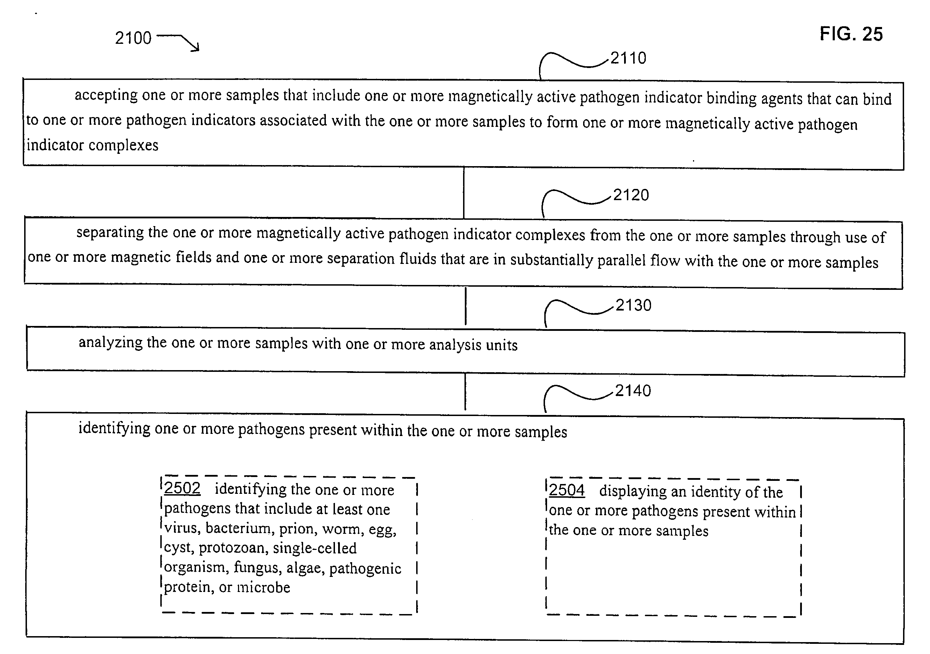

FIG. 25 illustrates alternate embodiments of the example operational flow of FIG. 21.

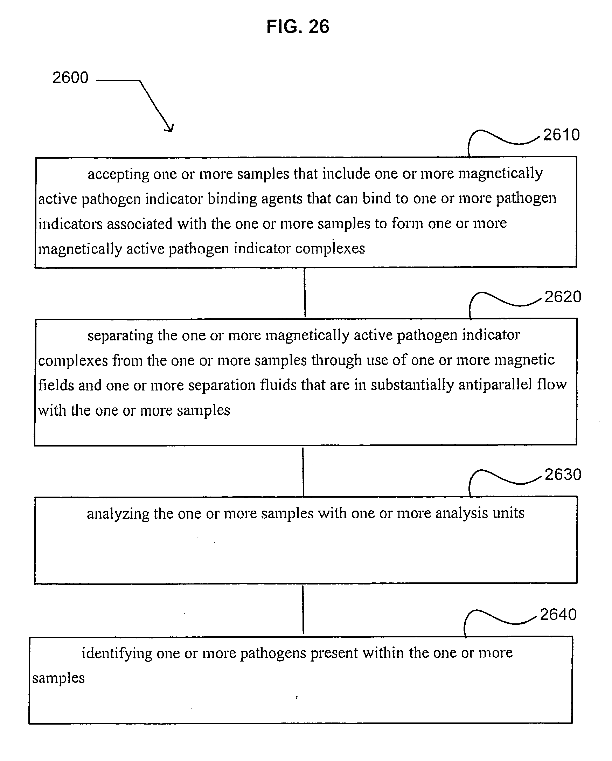

FIG. 26 illustrates an operational flow representing example operations related to methods and systems for analysis of pathogens.

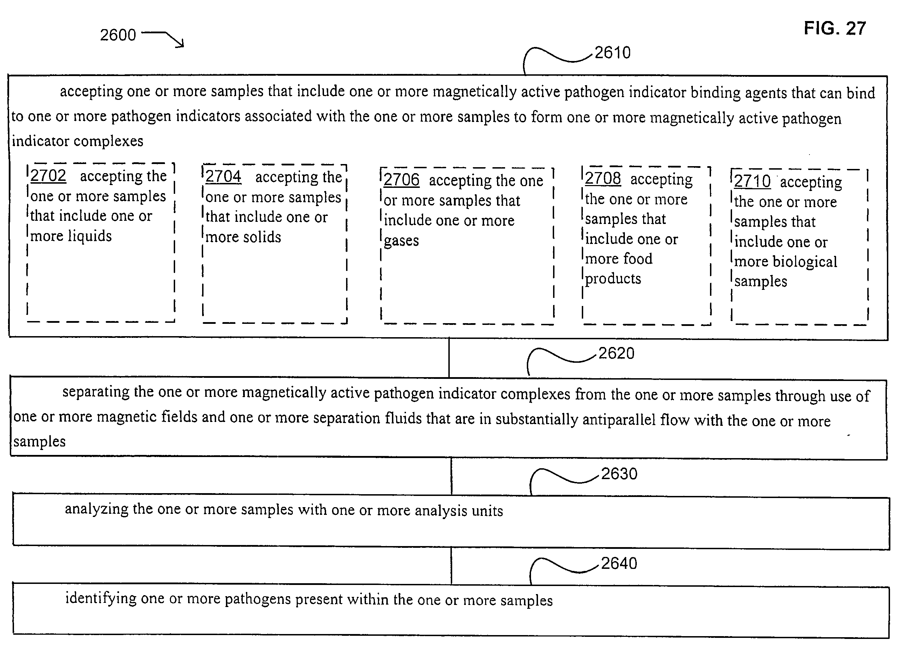

FIG. 27 illustrates alternate embodiments of the example operational flow of FIG. 26.

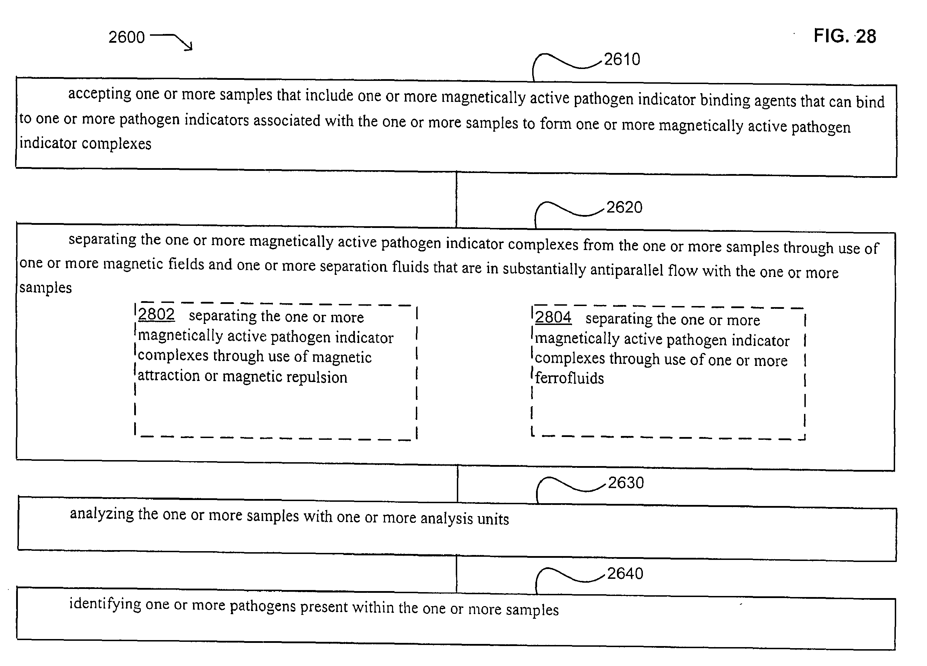

FIG. 28 illustrates alternate embodiments of the example operational flow of FIG. 26.

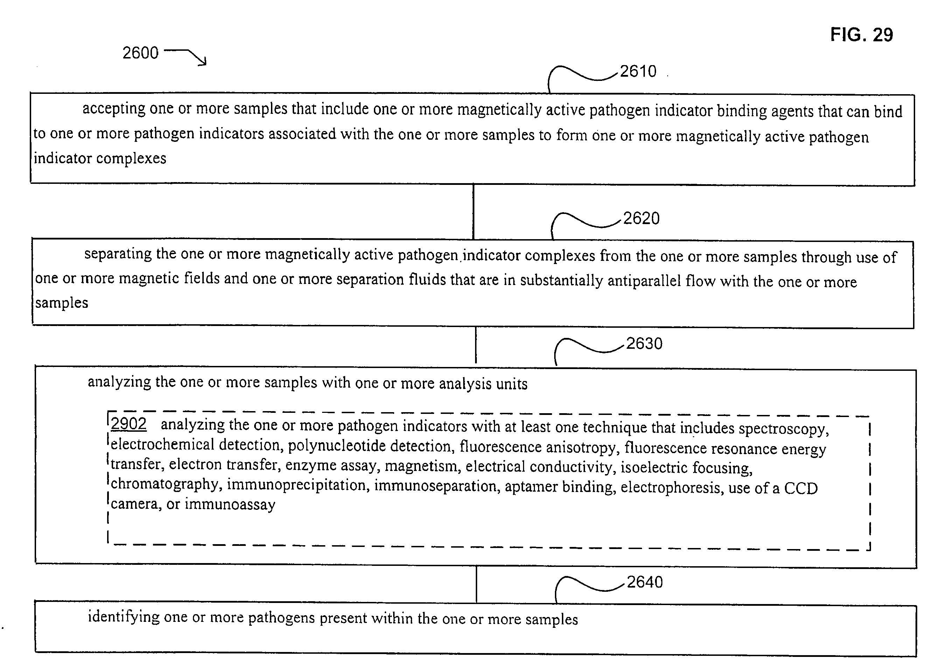

**FIG.** 29 illustrates alternate embodiments of the example operational flow of FIG. 26.

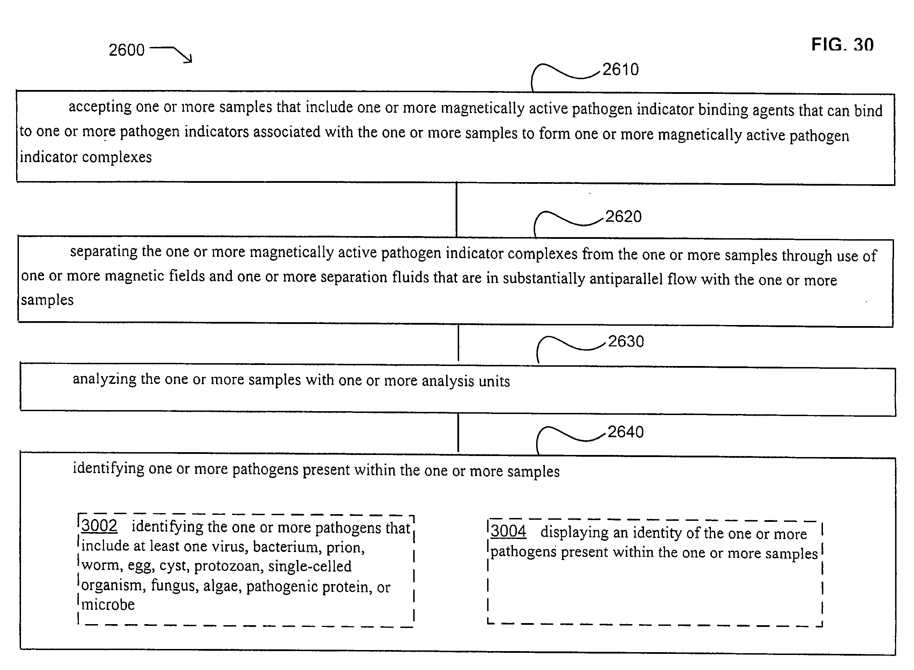

FIG. 30 illustrates alternate embodiments of the example operational flow of FIG. 26.

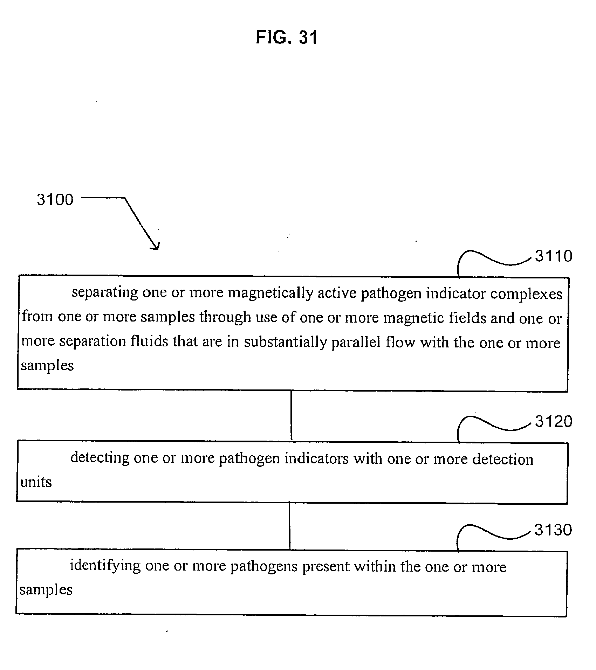

FIG. 31 illustrates an operational flow representing example operations related to methods and systems for analysis of pathogens.

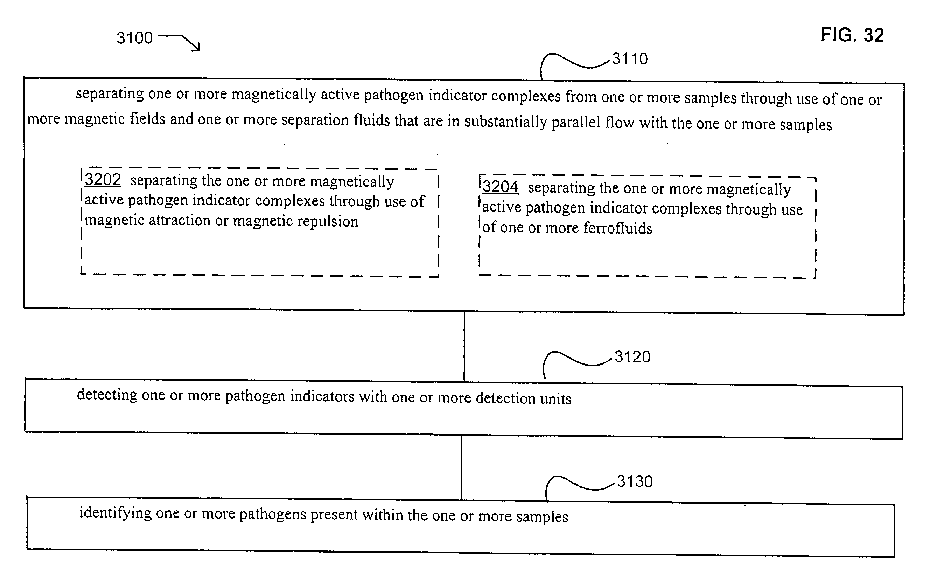

FIG. 32 illustrates alternate embodiments of the example operational flow of FIG. 31.

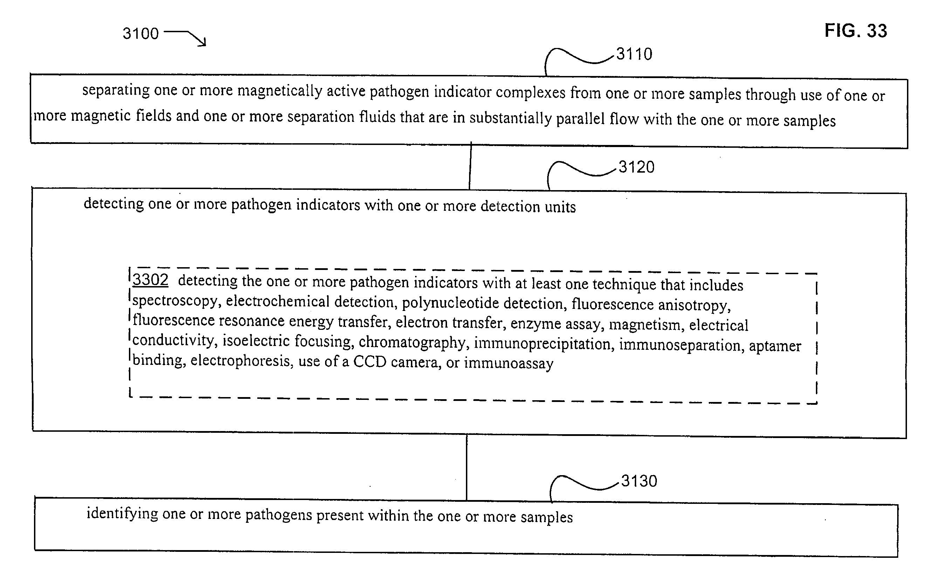

FIG. 33 illustrates alternate embodiments of the example operational flow of FIG. 31.

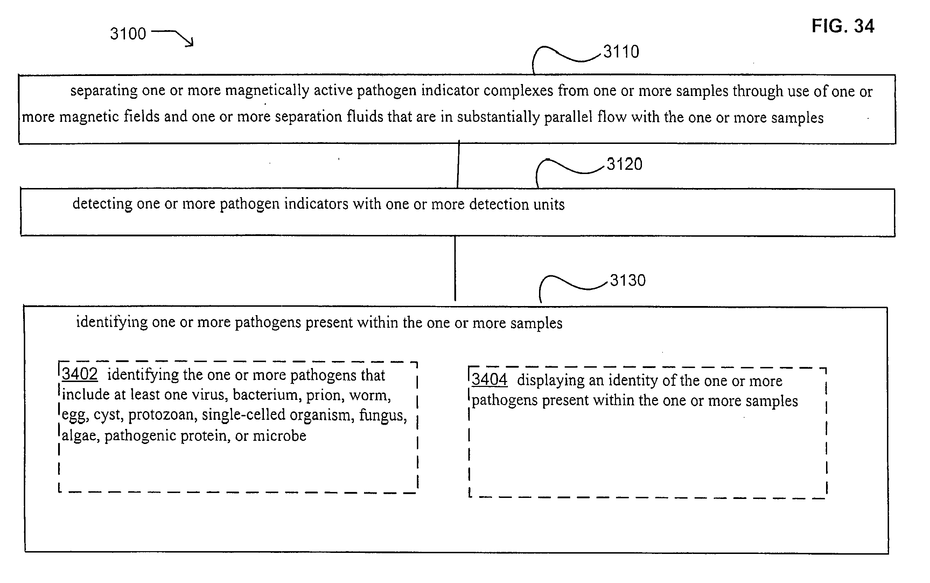

FIG. 34 illustrates alternate embodiments of the example operational flow of FIG. 31.

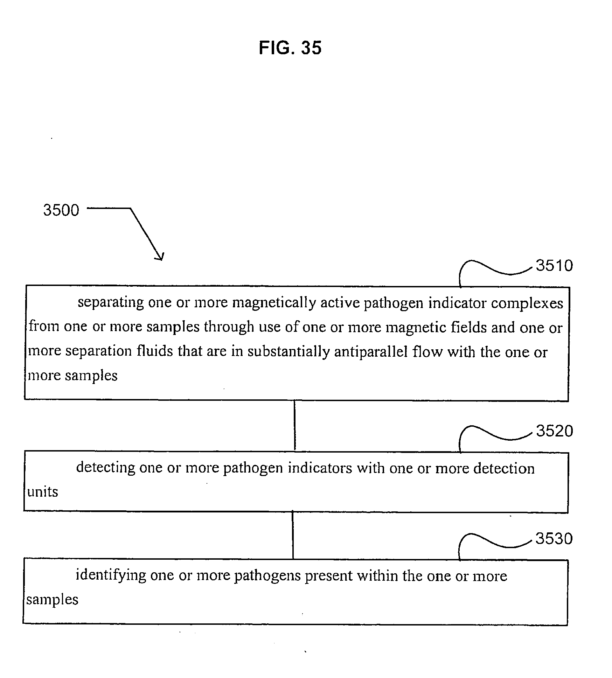

FIG. 35 illustrates an operational flow representing example operations related to methods and systems for analysis of pathogens.

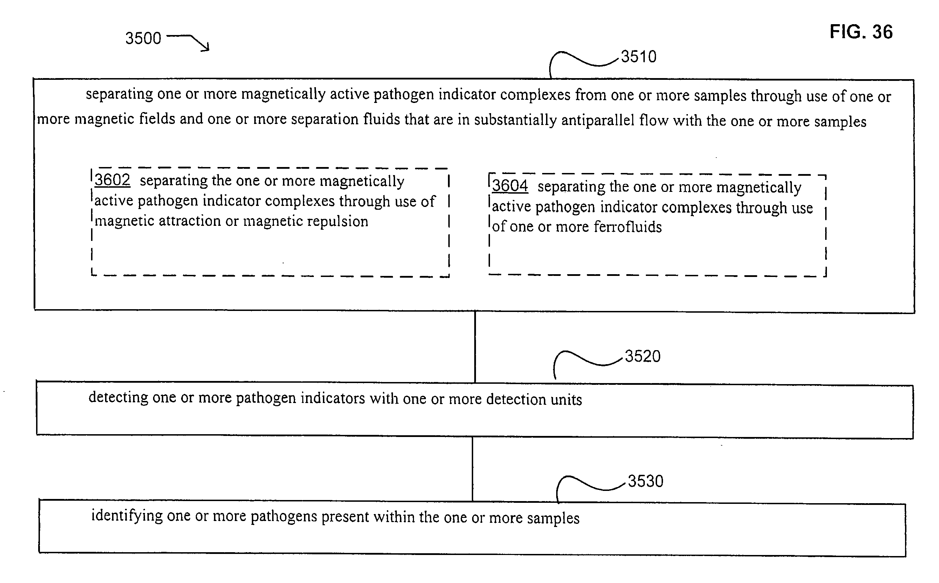

FIG. 36 illustrates alternate embodiments of the example operational flow of FIG. 35.

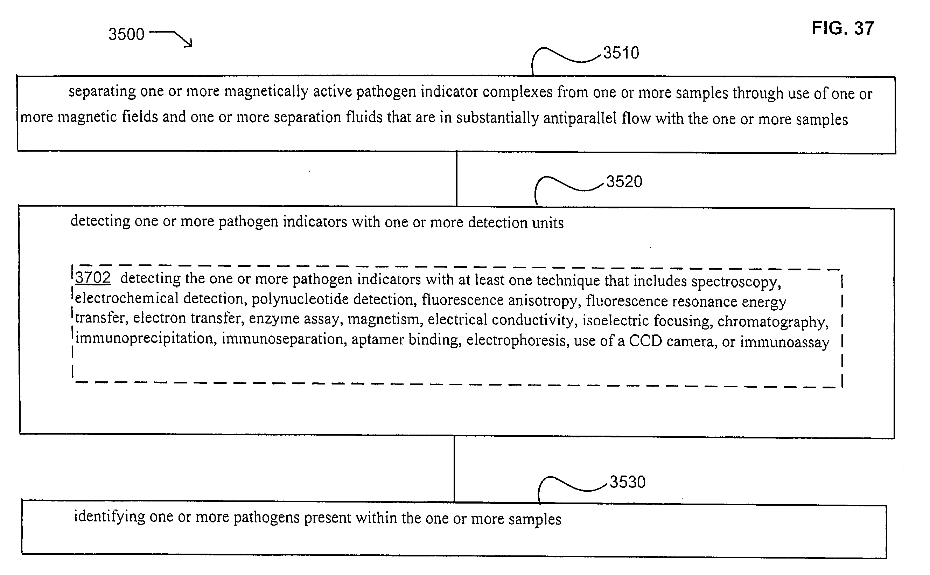

FIG. 37 illustrates alternate embodiments of the example operational flow of FIG. 35.

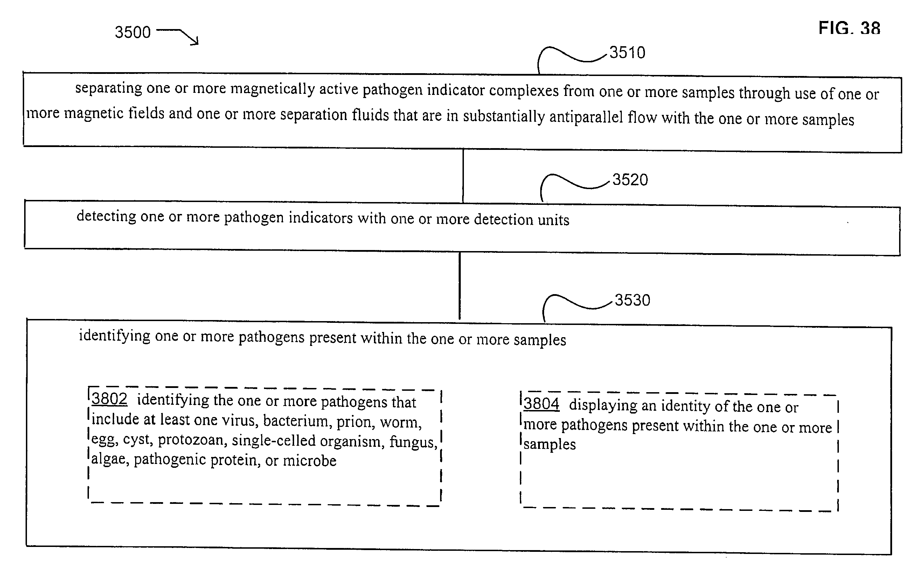

FIG. 38 illustrates alternate embodiments of the example operational flow of FIG. 35.

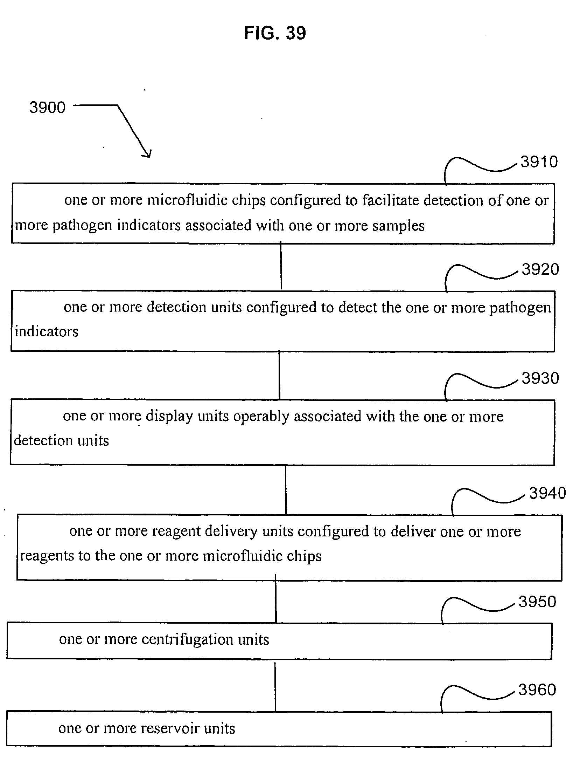

FIG. 39 illustrates an example system 3900 in which embodiments may be implemented.

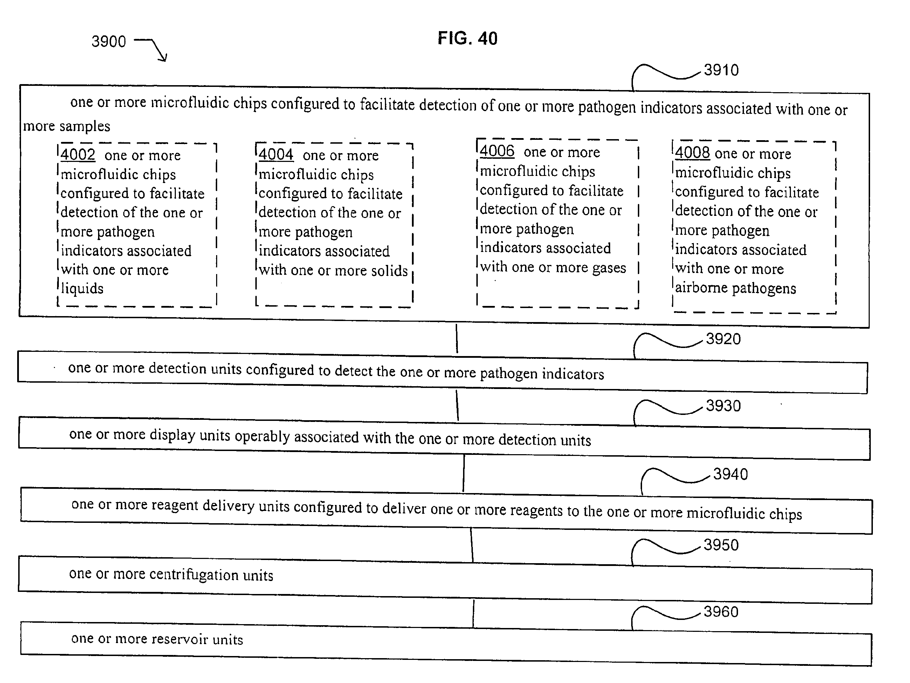

FIG. 40 illustrates alternate embodiments of the system of FIG. 39.

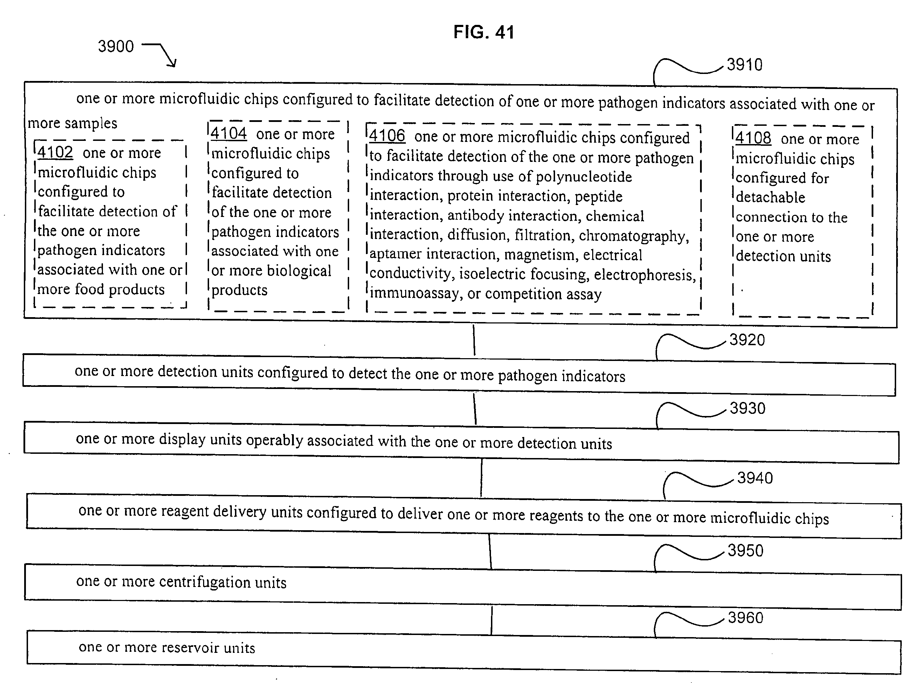

FIG. 41 illustrates alternate embodiments of the system of FIG. 39.

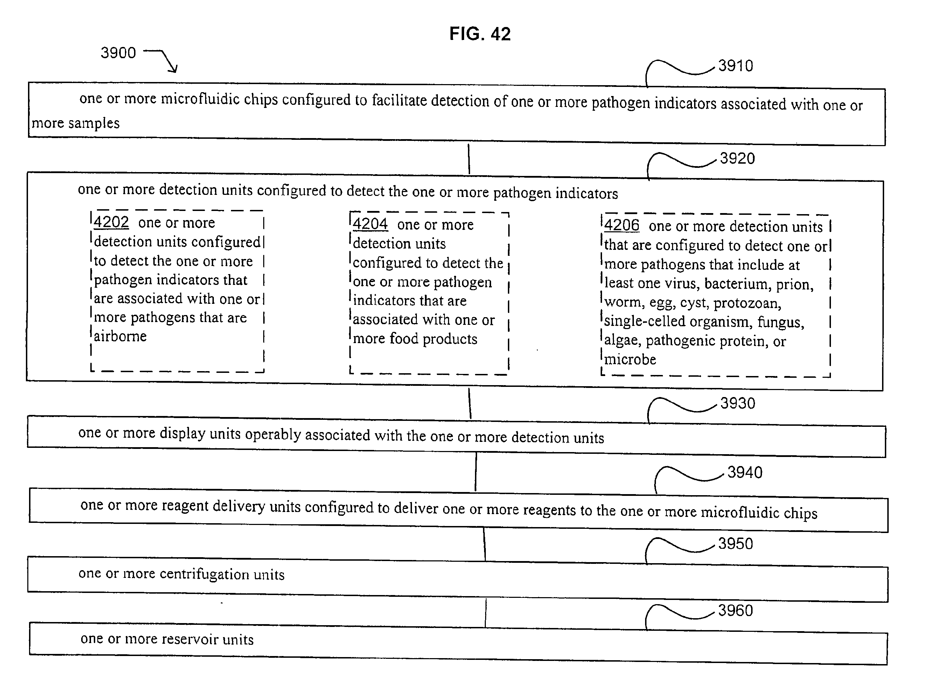

FIG. 42 illustrates alternate embodiments of the system of FIG. 39.

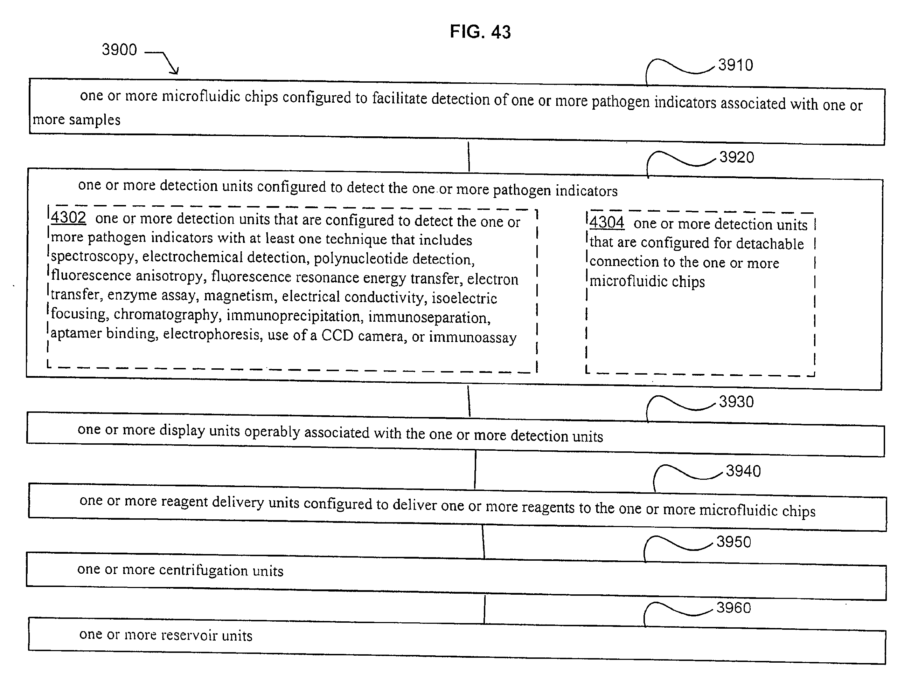

FIG. 43 illustrates alternate embodiments of the system of FIG. 39.

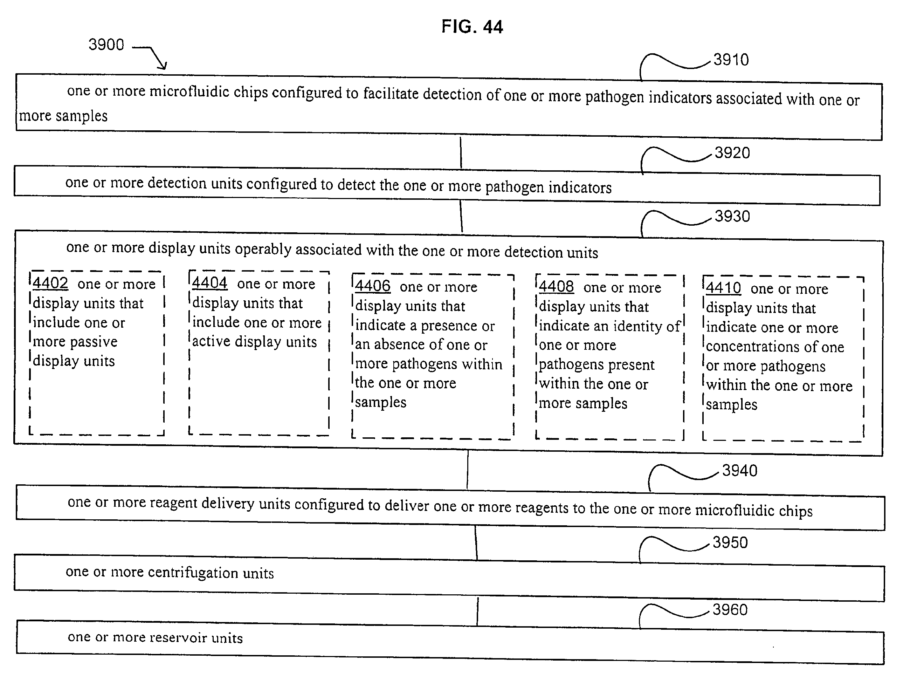

FIG. 44 illustrates alternate embodiments of the system of FIG. 39.

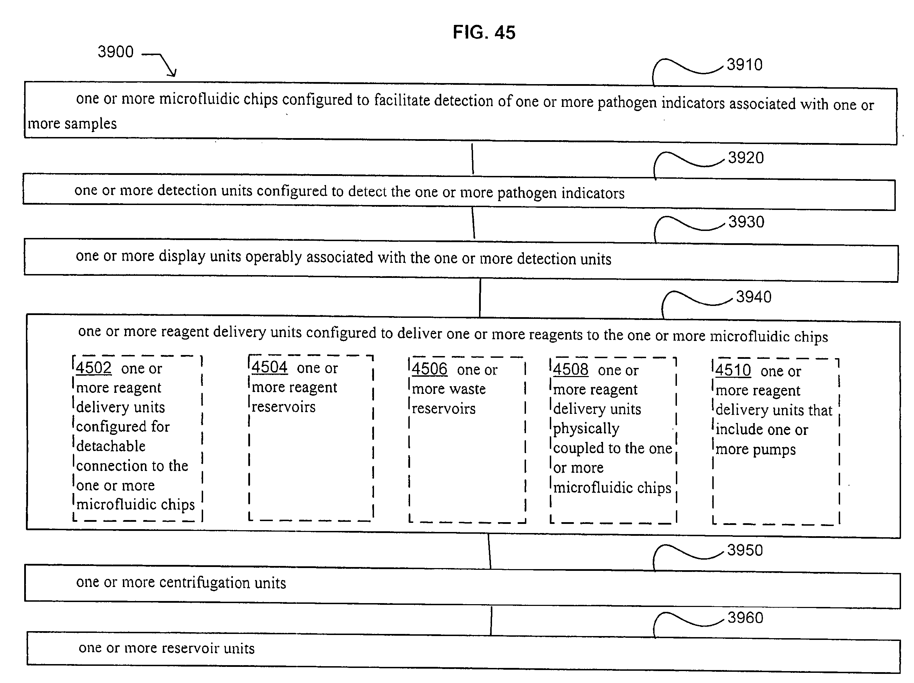

FIG. 45 illustrates alternate embodiments of the system of FIG. 39.

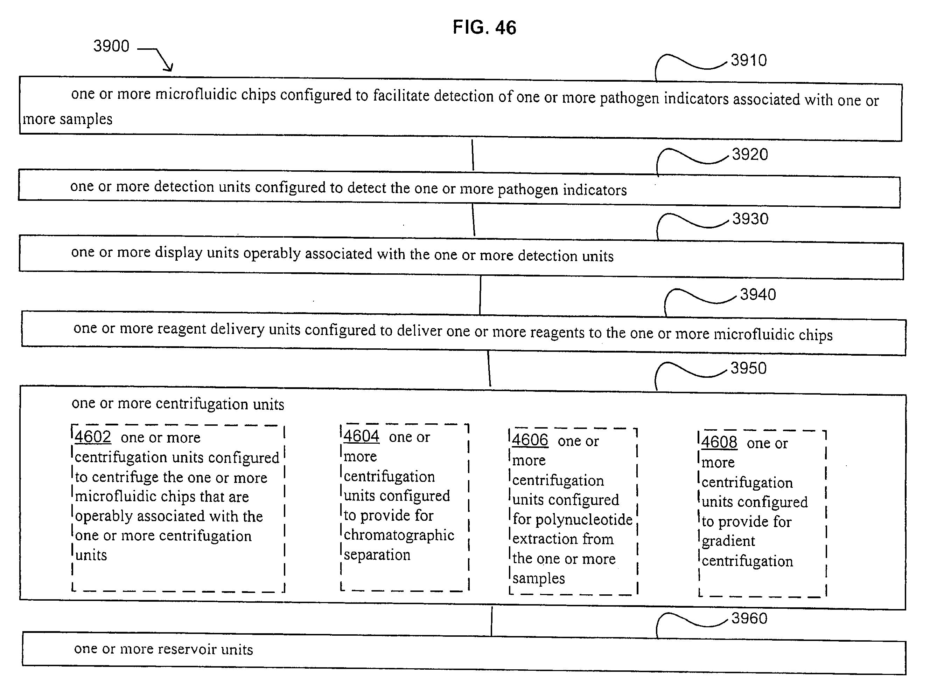

FIG. 46 illustrates alternate embodiments of the system of FIG. 39.

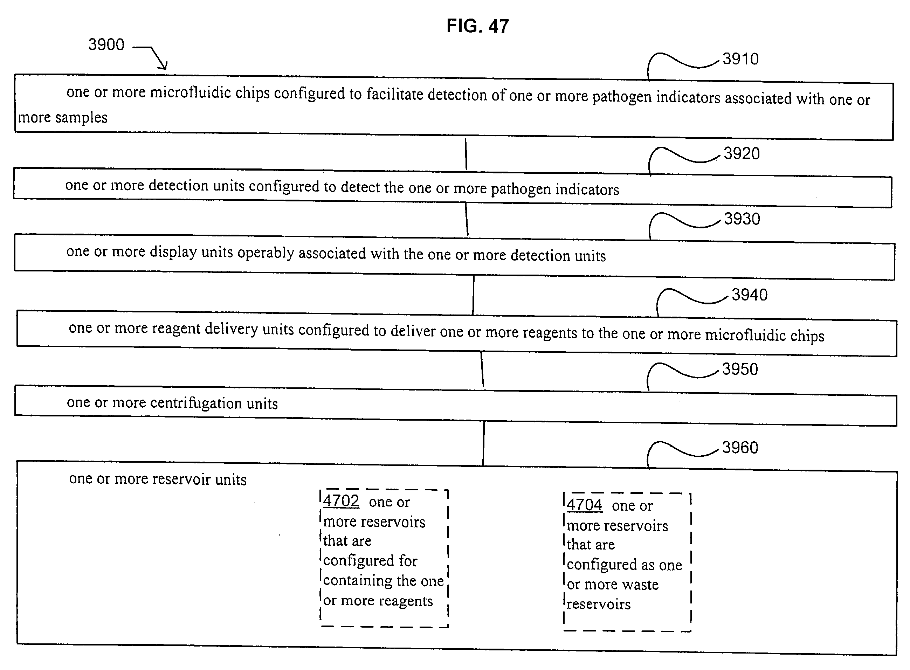

FIG. 47 illustrates alternate embodiments of the system of FIG. 39.

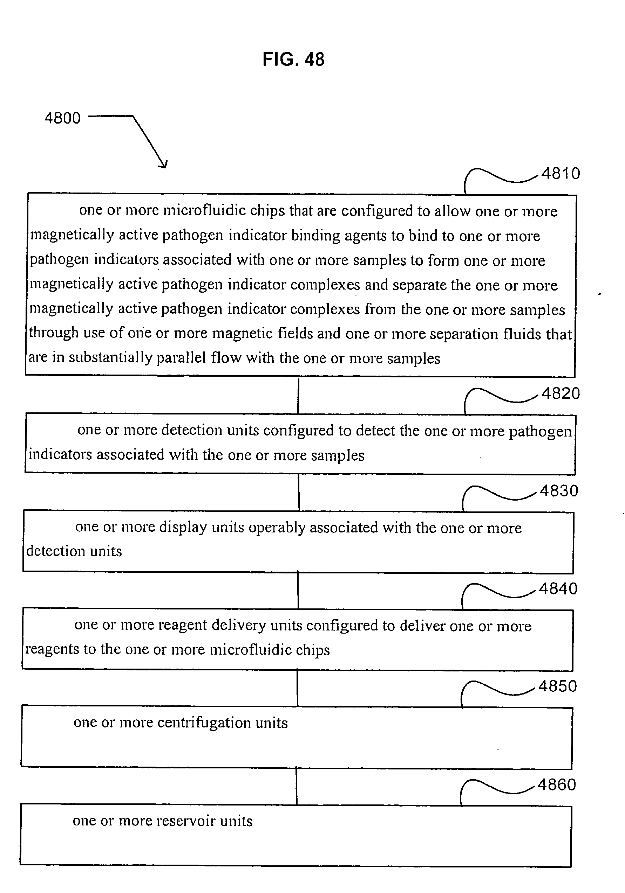

FIG. 48 illustrates an example system 4800 in which embodiments may be implemented.

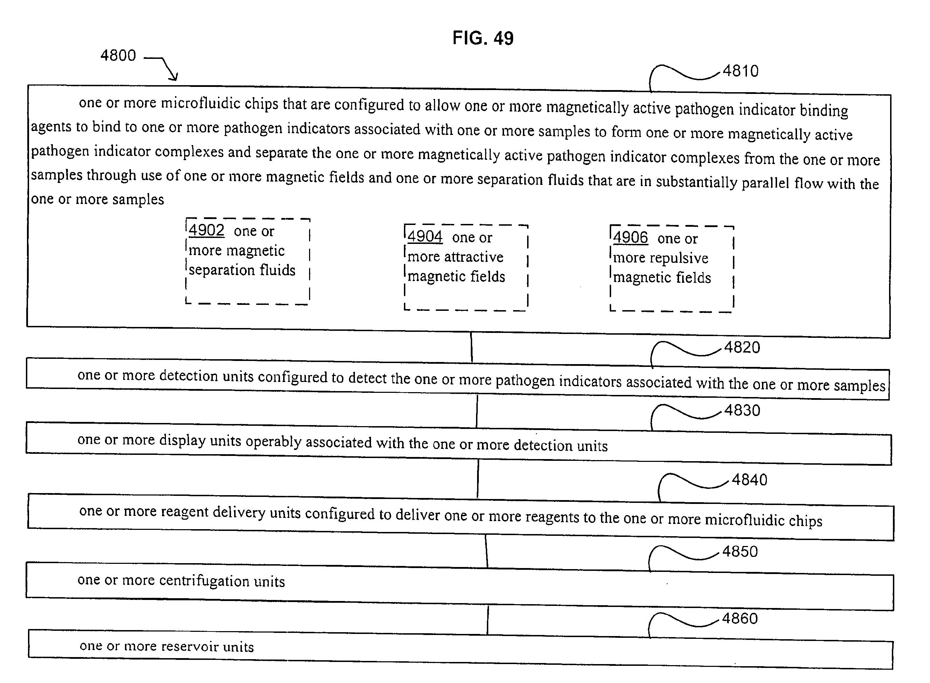

FIG. 49 illustrates alternate embodiments of the system of FIG. 48.

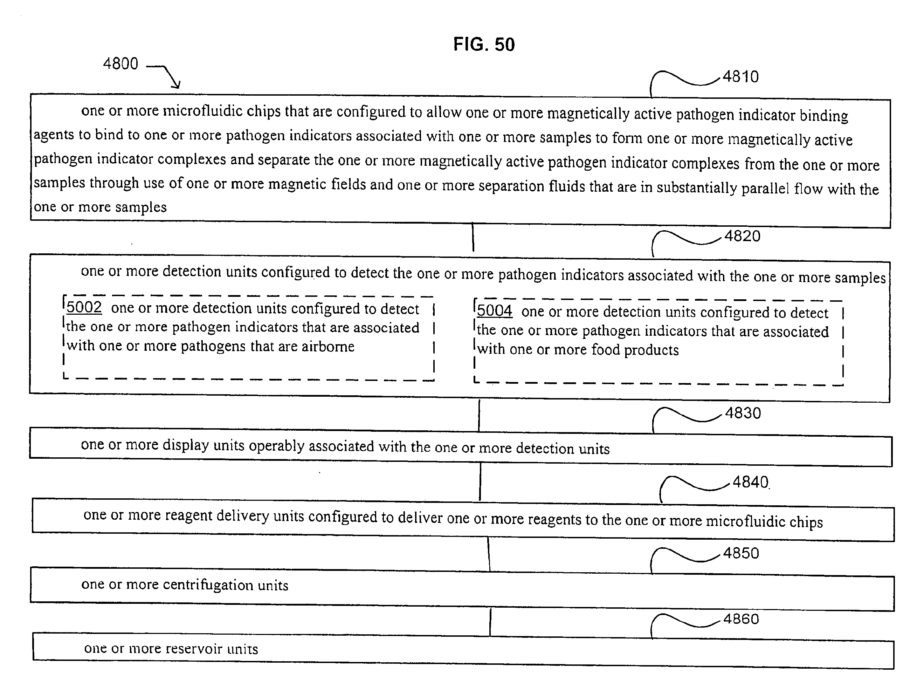

FIG. 50 illustrates alternate embodiments of the system of FIG. 48.

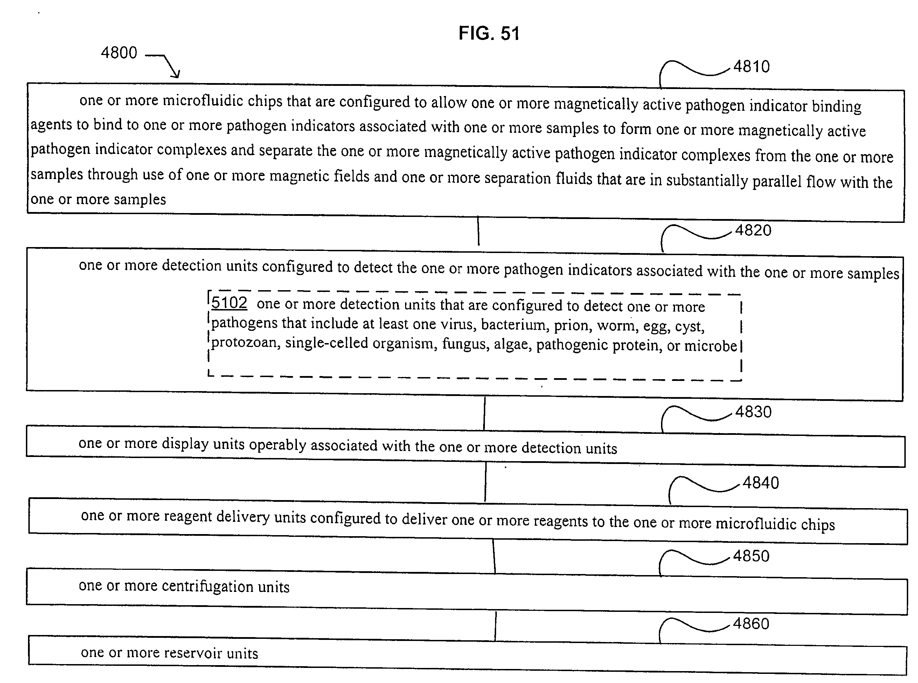

FIG. 51 illustrates alternate embodiments of the system of FIG. 48.

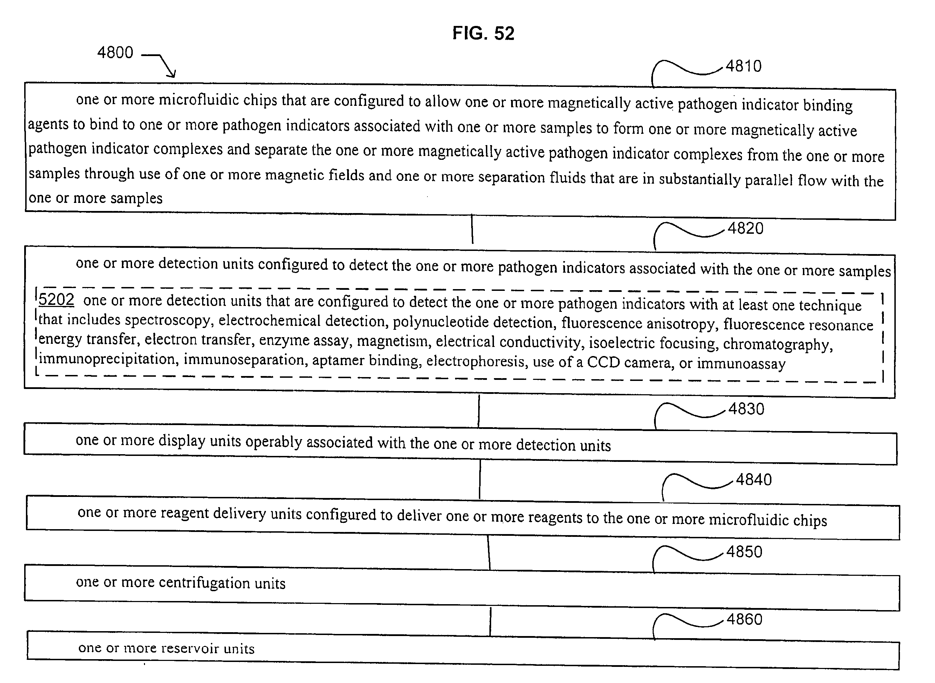

FIG. 52 illustrates alternate embodiments of the system of FIG. 48.

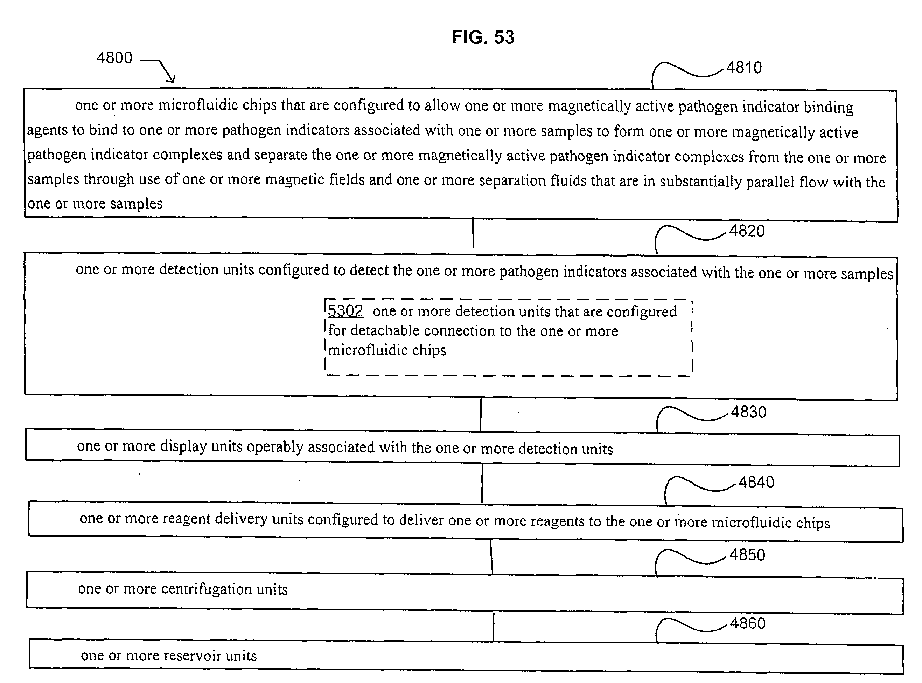

FIG. 53 illustrates alternate embodiments of the system of FIG. 48.

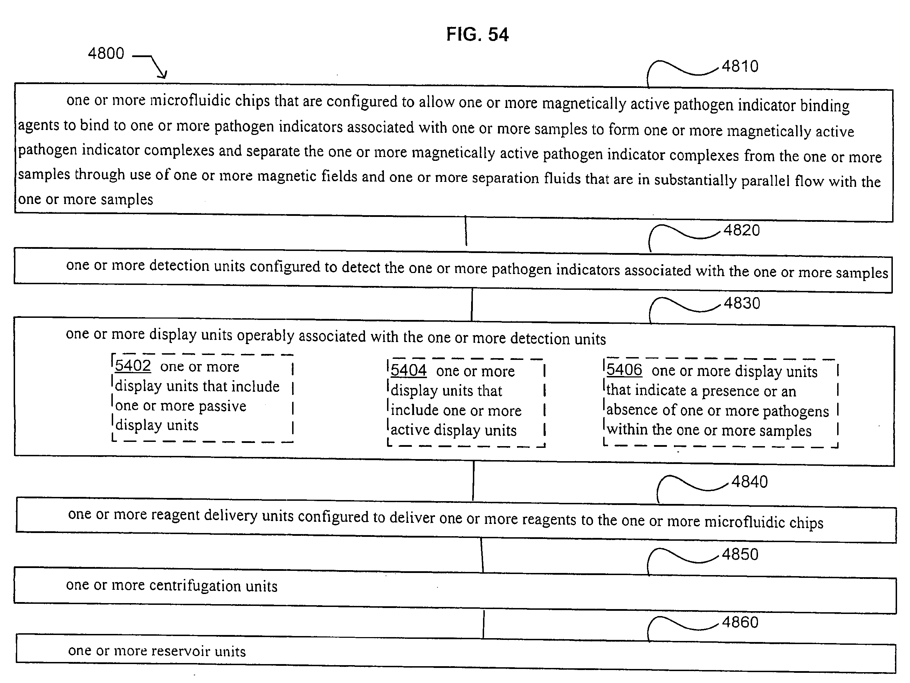

FIG. 54 illustrates alternate embodiments of the system of FIG. 48.

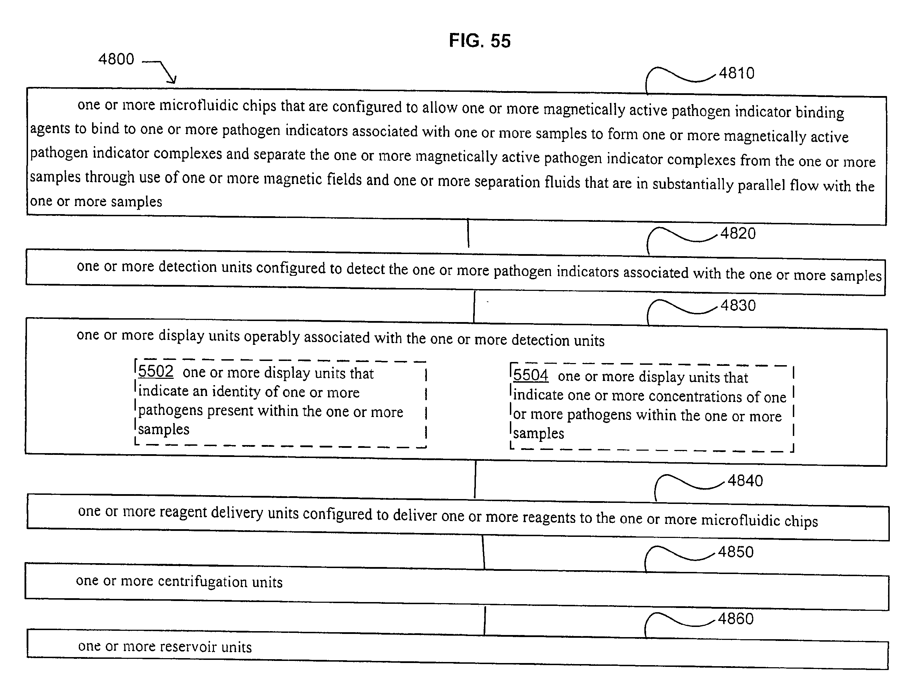

FIG. 55 illustrates alternate embodiments of the system of FIG. 48.

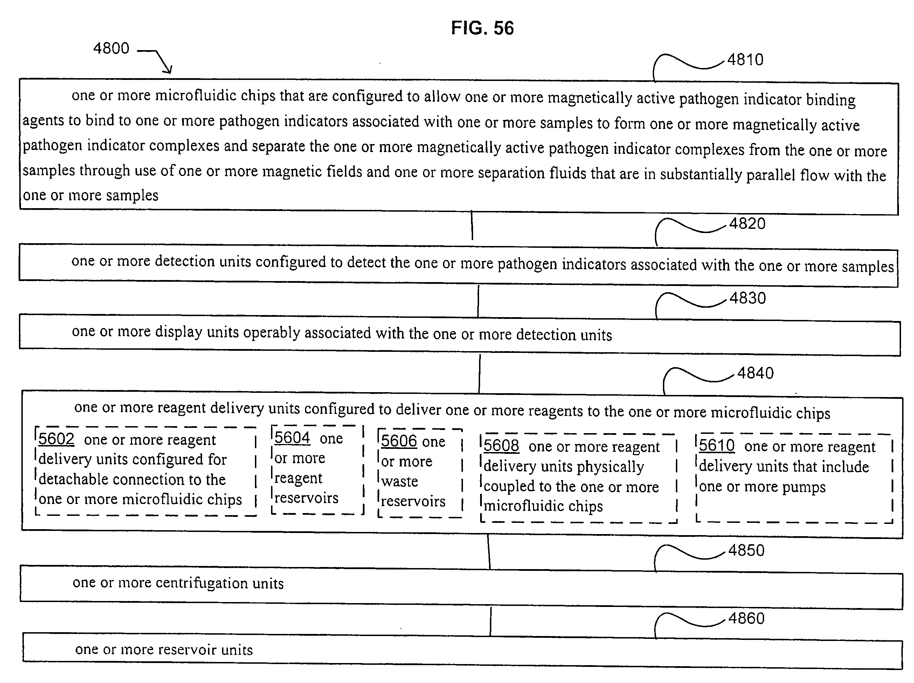

FIG. 56 illustrates alternate embodiments of the system of FIG. 48.

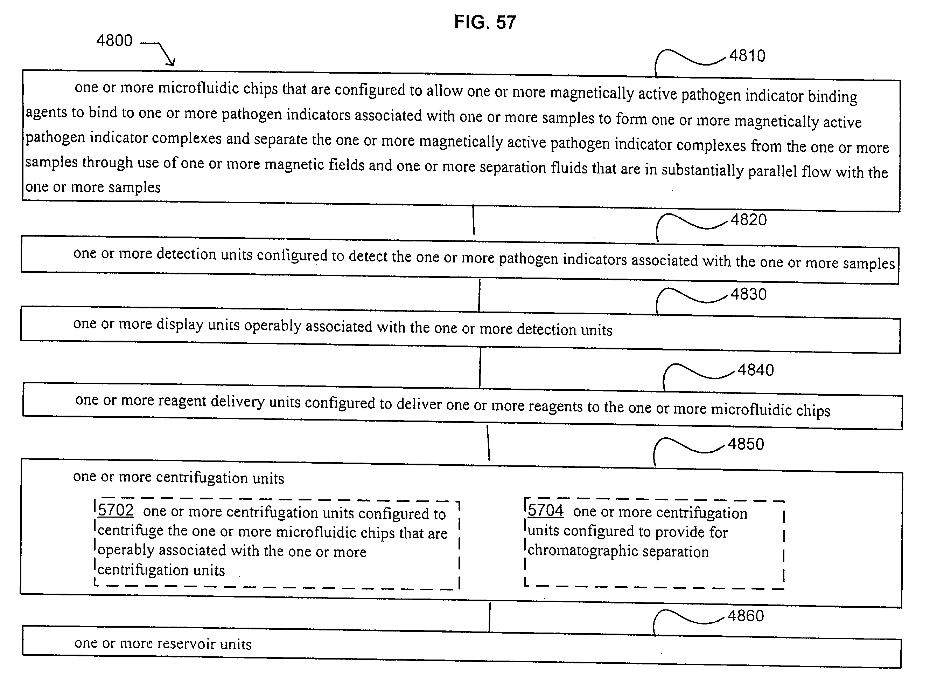

FIG. 57 illustrates alternate embodiments of the system of FIG. 48.

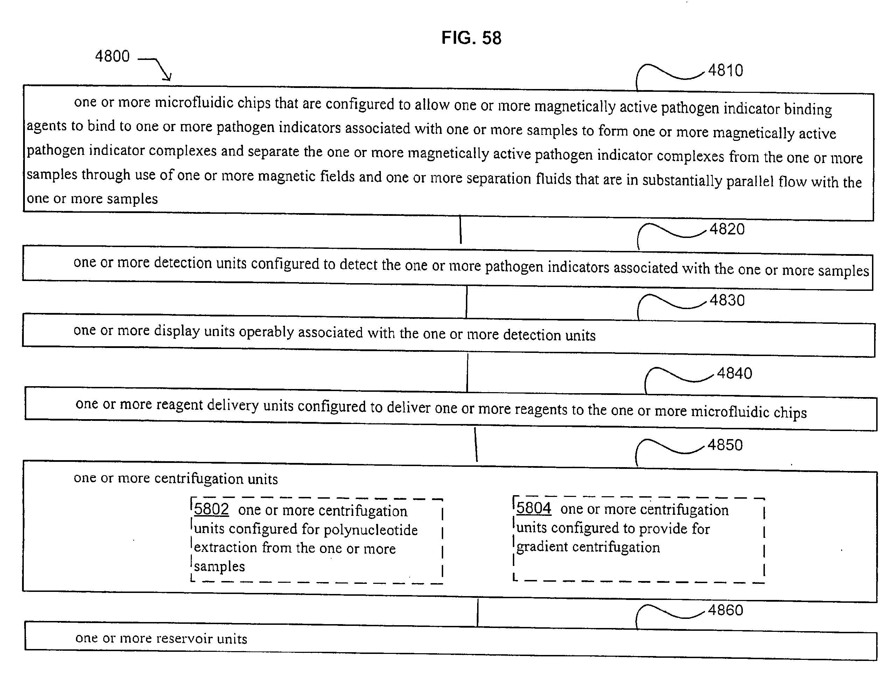

FIG. 58 illustrates alternate embodiments of the system of FIG. 48.

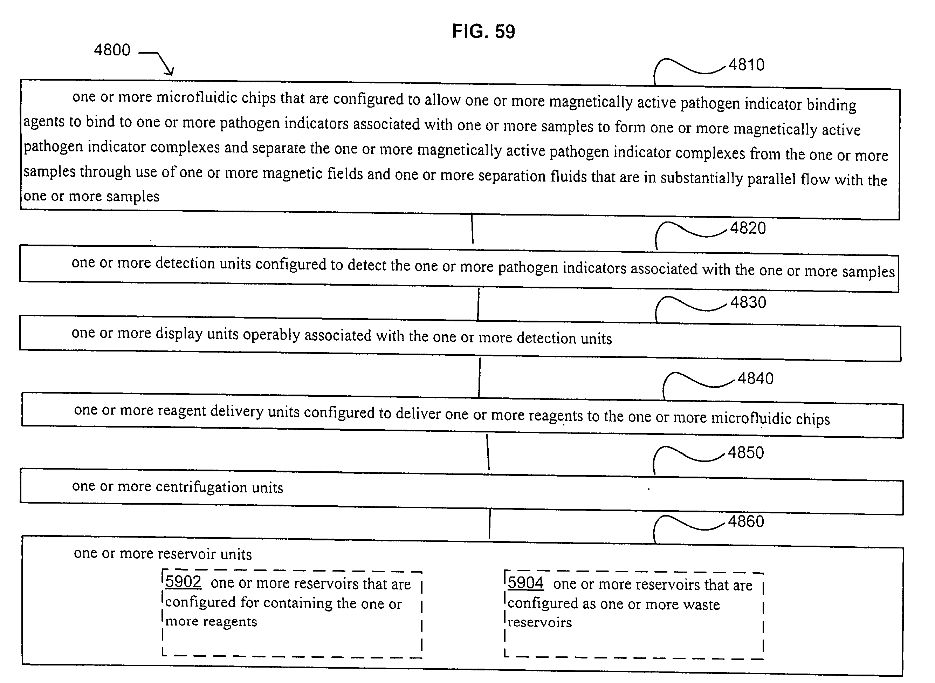

FIG. 59 illustrates alternate embodiments of the system of FIG. 48.

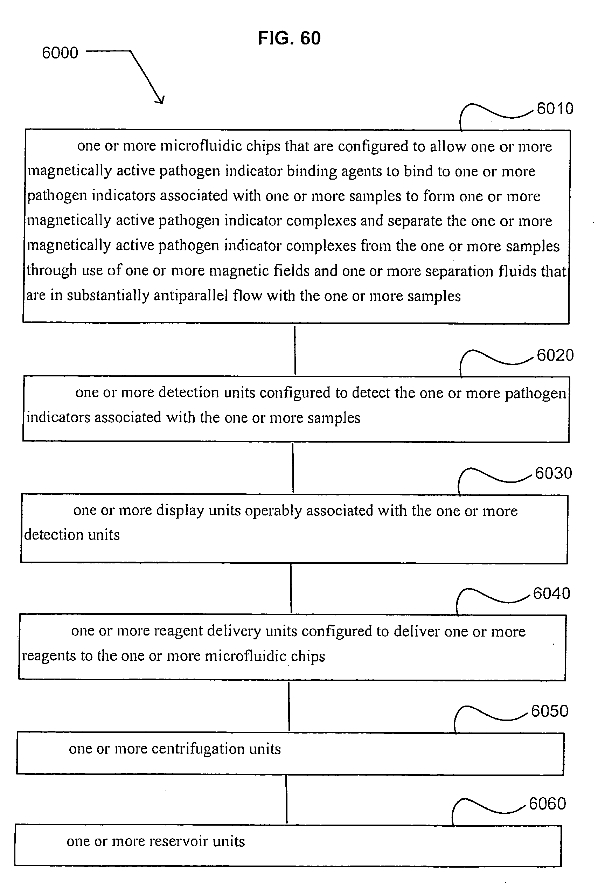

FIG. 60 illustrates an example system 6000 in which embodiments may be implemented.

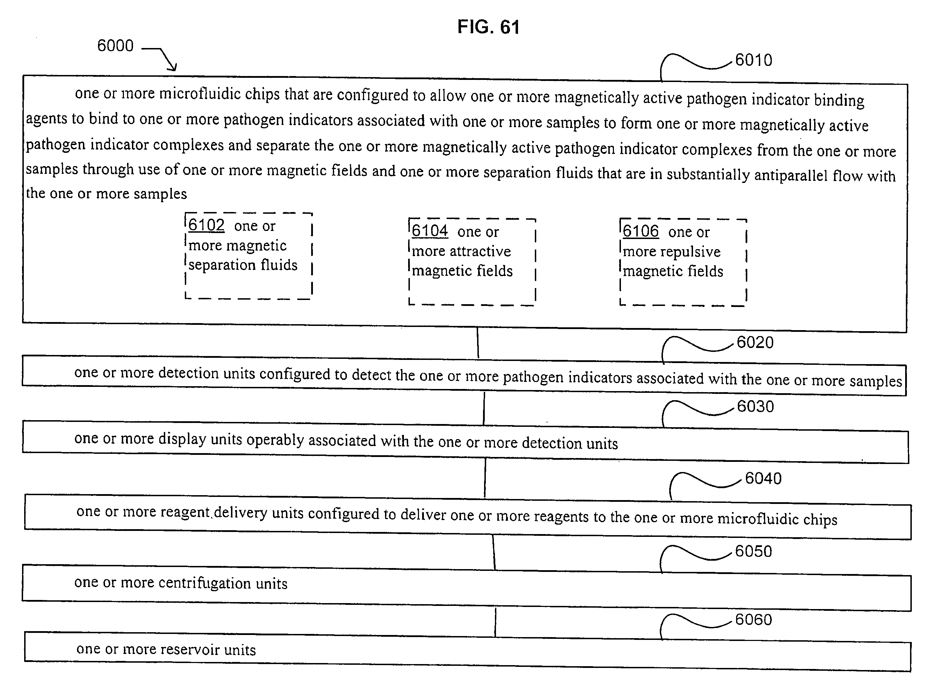

FIG. 61 illustrates alternate embodiments of the system of FIG. 60.

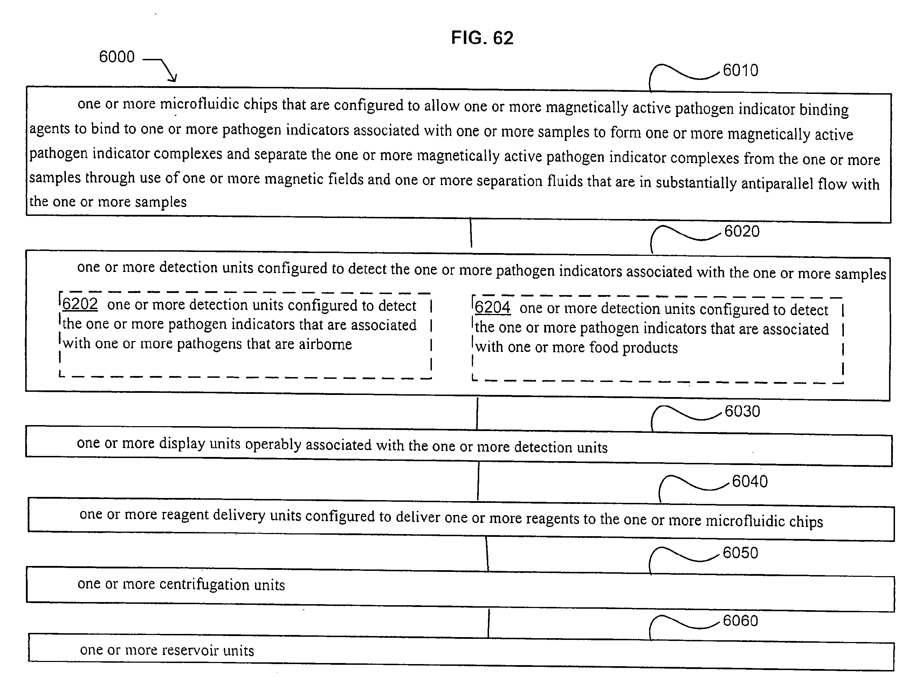

FIG. 62 illustrates alternate embodiments of the system of FIG. 60.

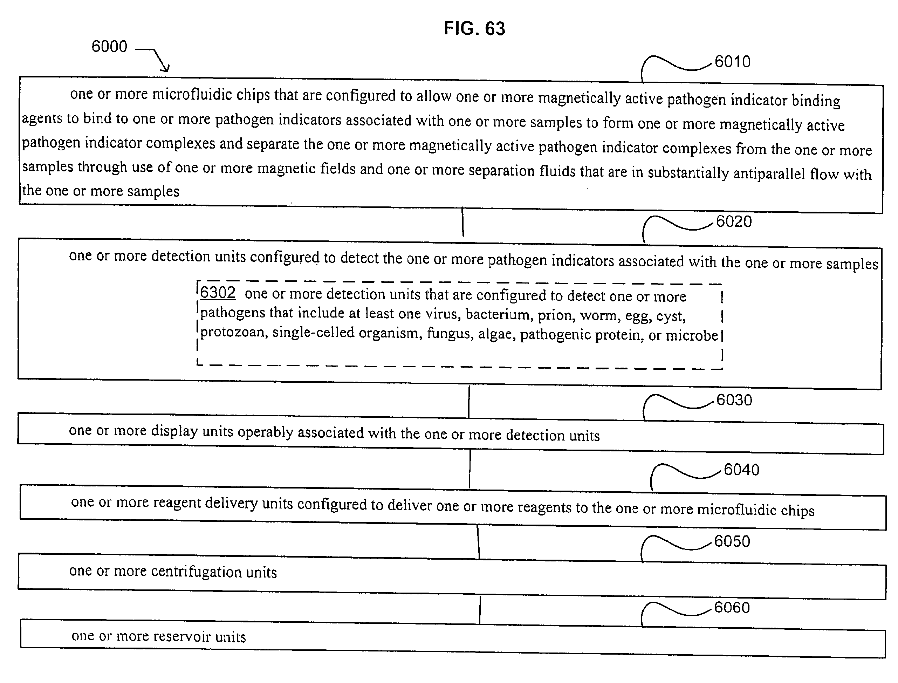

FIG. 63 illustrates alternate embodiments of the system of FIG. 60.

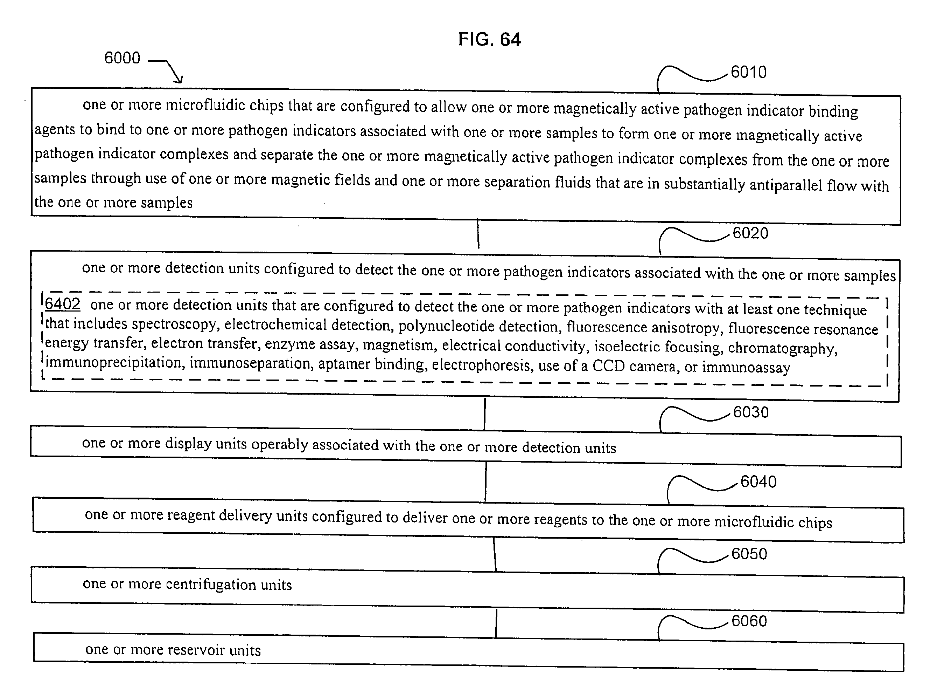

FIG. 64 illustrates alternate embodiments of the system of FIG. 60.

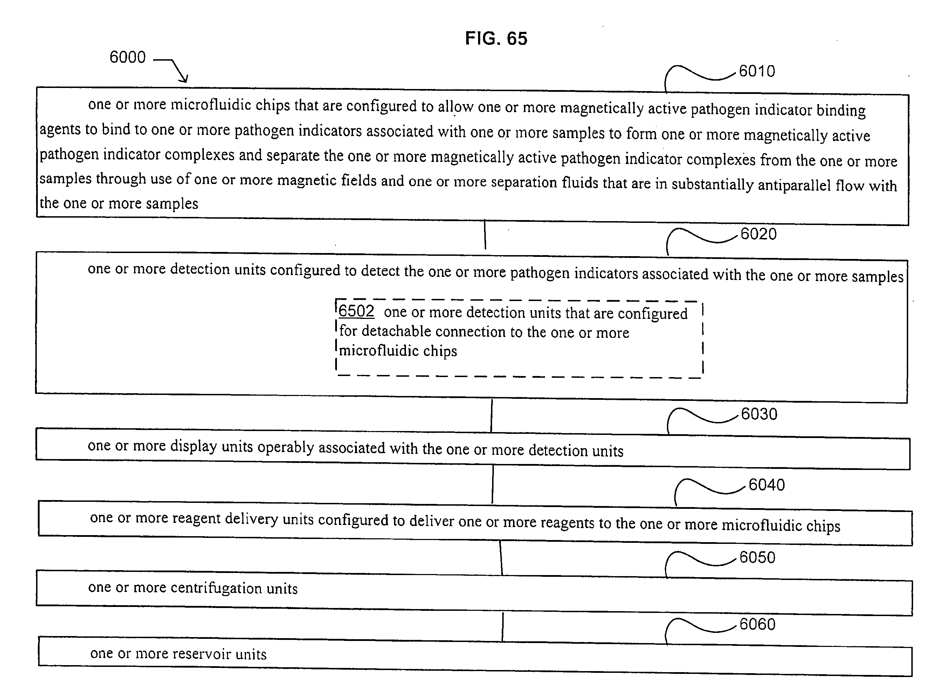

FIG. 65 illustrates alternate embodiments of the system of FIG. 60.

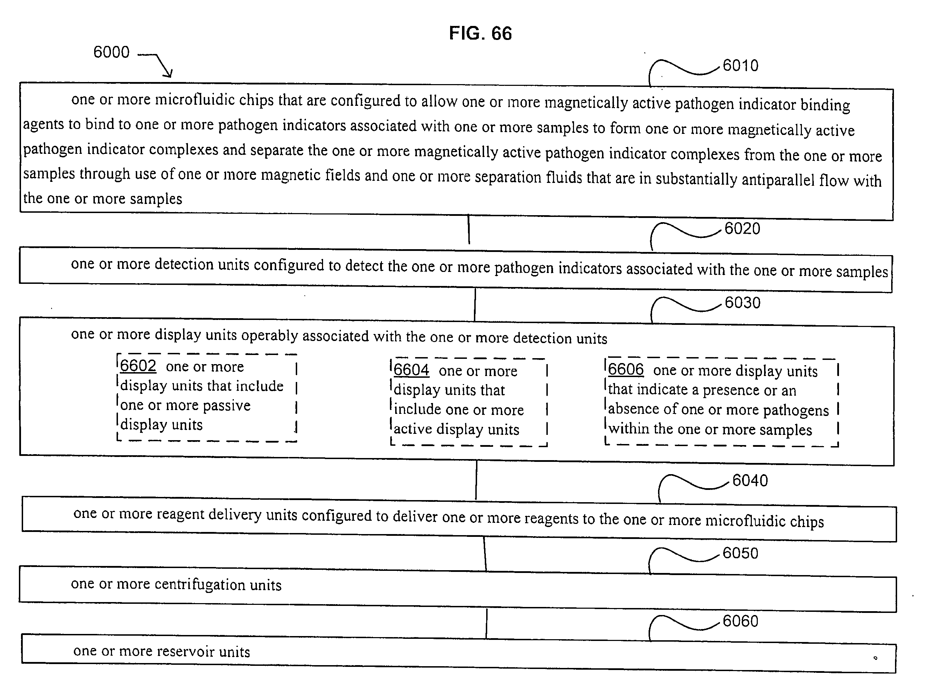

FIG. 66 illustrates alternate embodiments of the system of FIG. 60.

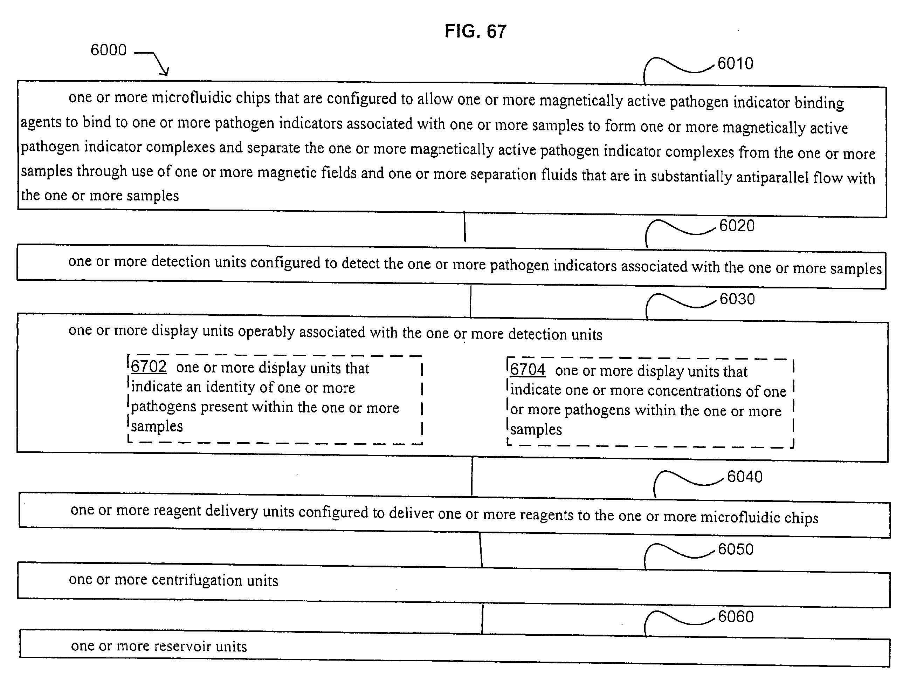

FIG. 67 illustrates alternate embodiments of the system of FIG. 60.

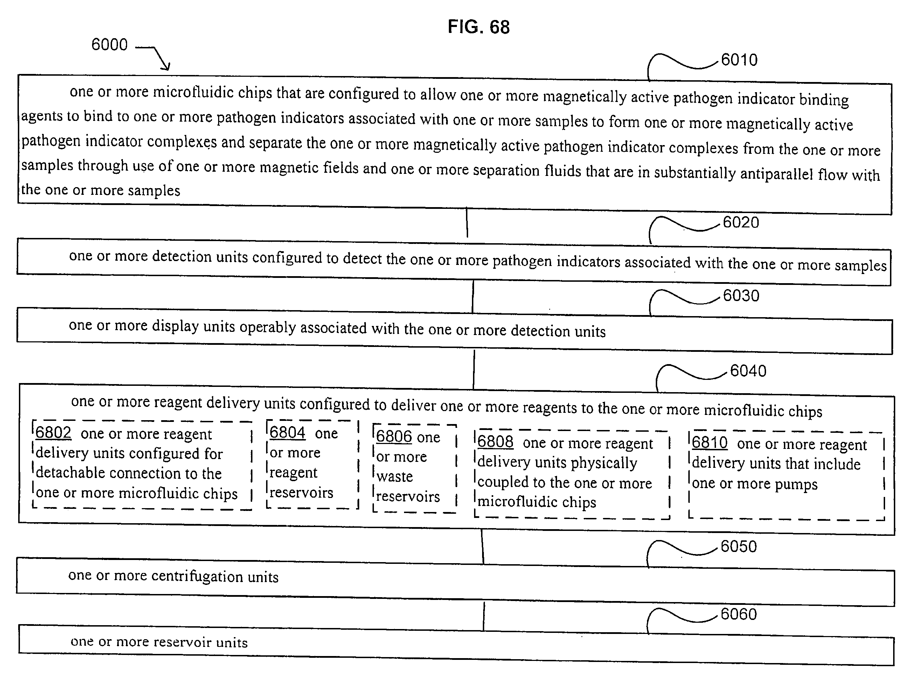

FIG. 68 illustrates alternate embodiments of the system of FIG. 60.

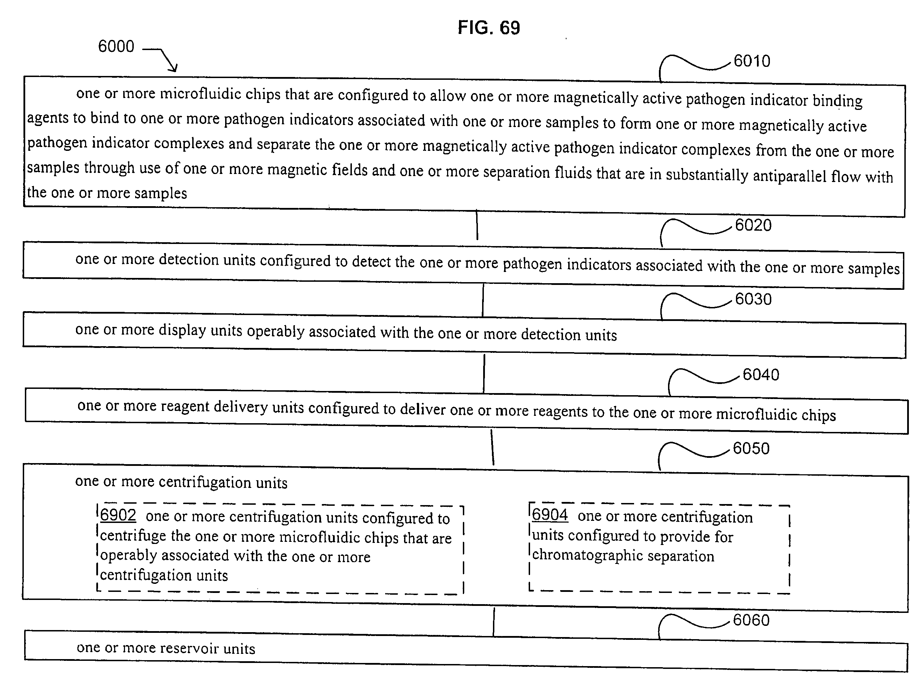

FIG. 69 illustrates alternate embodiments of the system of FIG. 60.

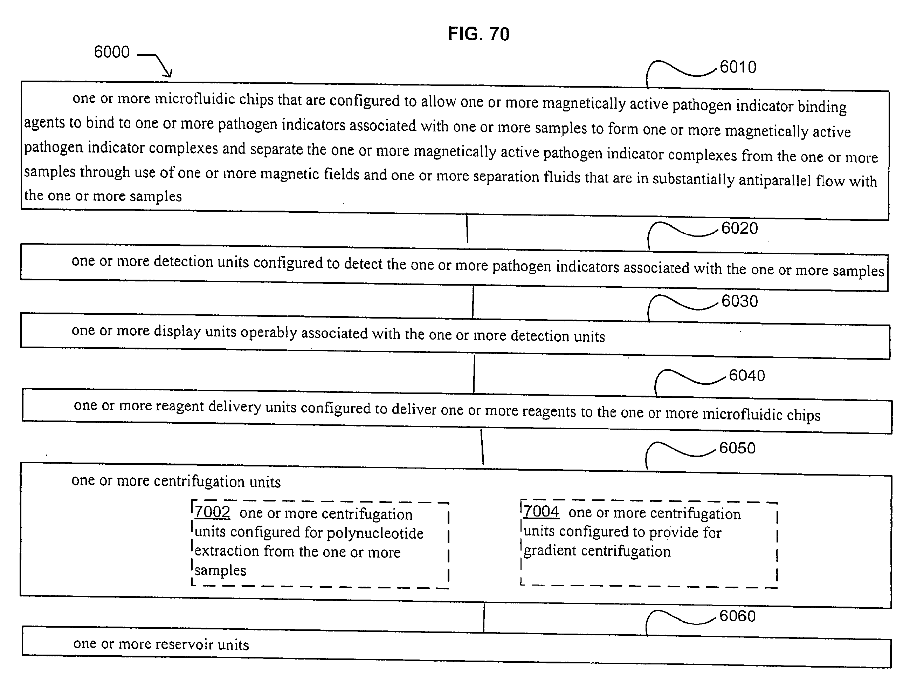

FIG. 70 illustrates alternate embodiments of the system of FIG. 60.

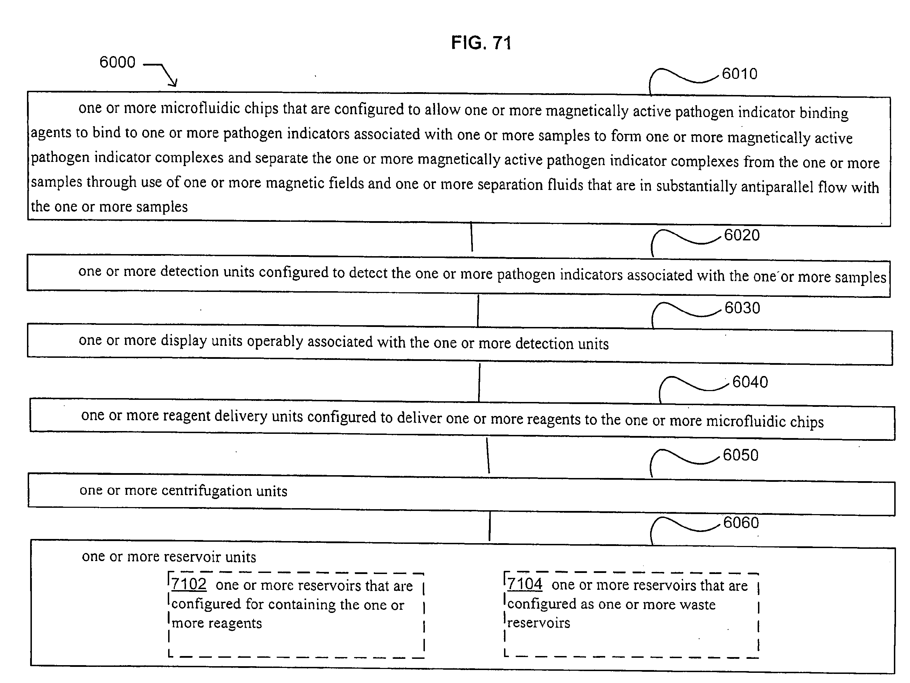

FIG. 71 illustrates alternate embodiments of the system of FIG. 60.

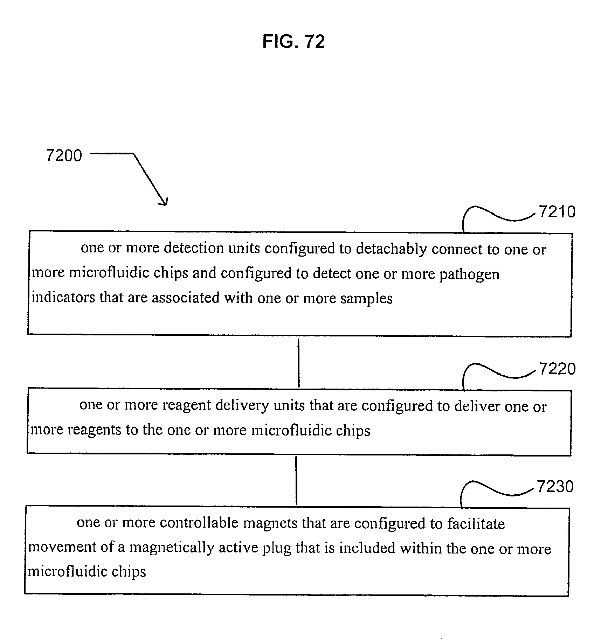

FIG. 72 illustrates an example device 7200 in which embodiments may be implemented.

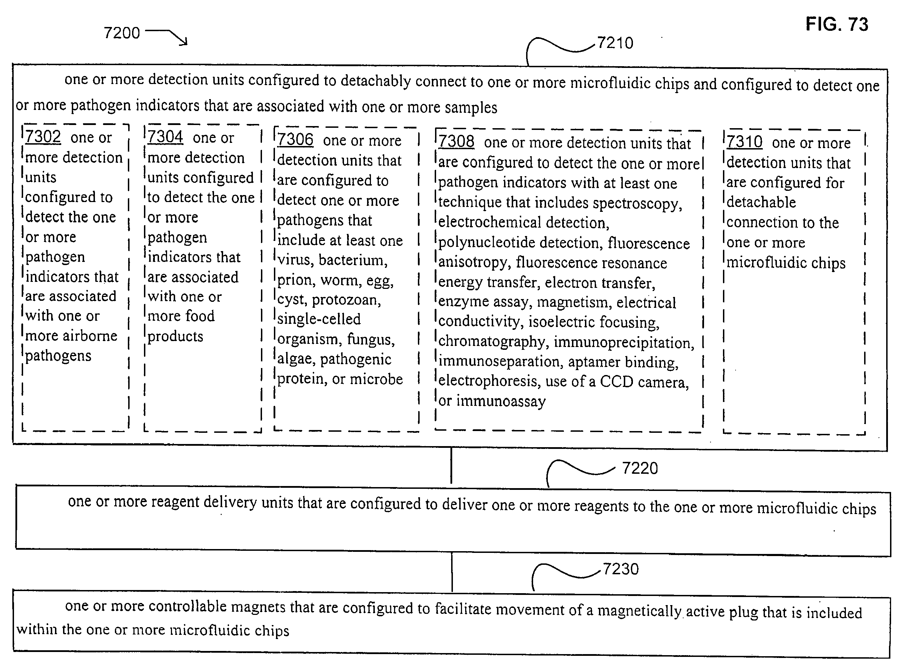

FIG. 73 illustrates alternate embodiments of the device of FIG. 72.

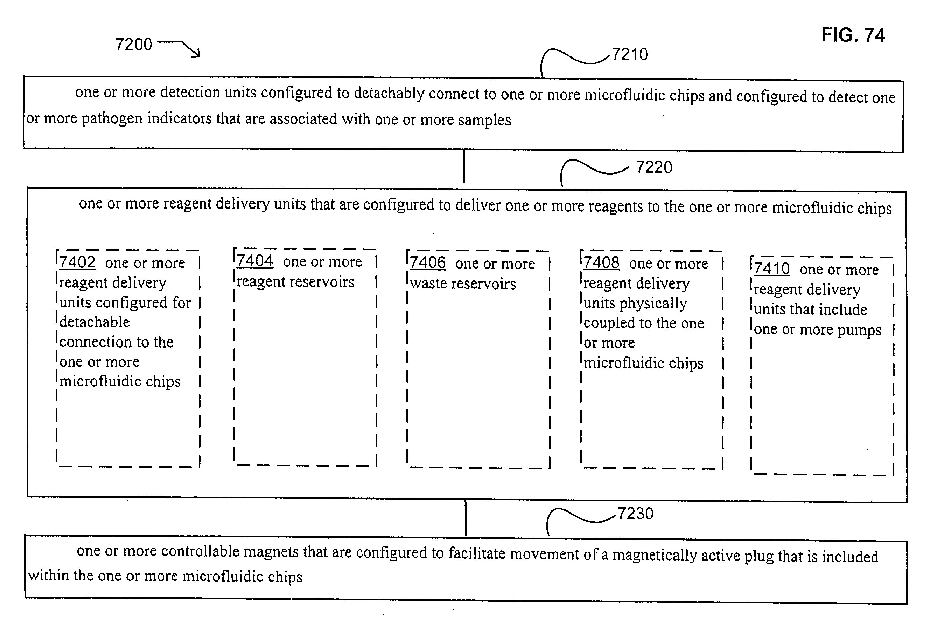

FIG. 74 illustrates alternate embodiments of the device of FIG. 72.

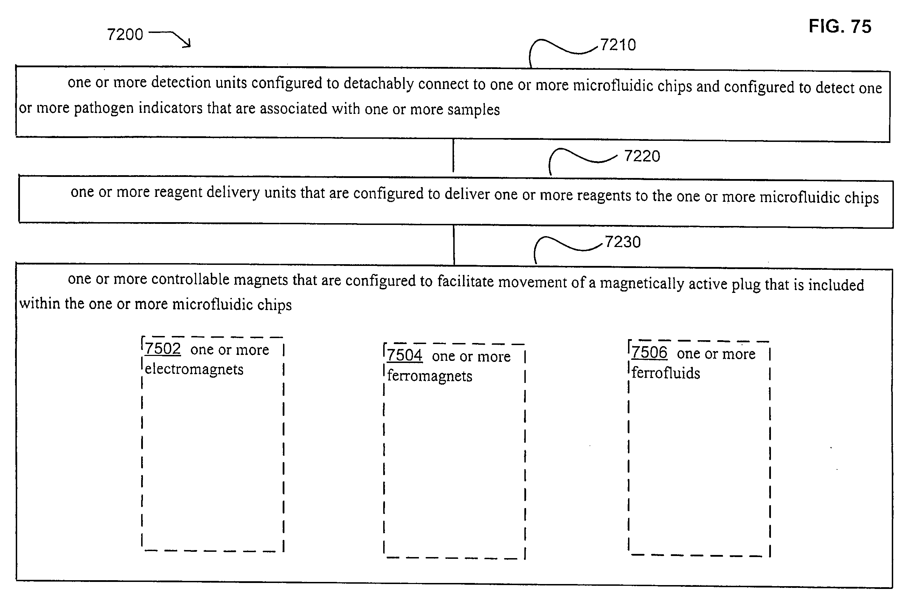

FIG. 75 illustrates alternate embodiments of the device of FIG. 72.

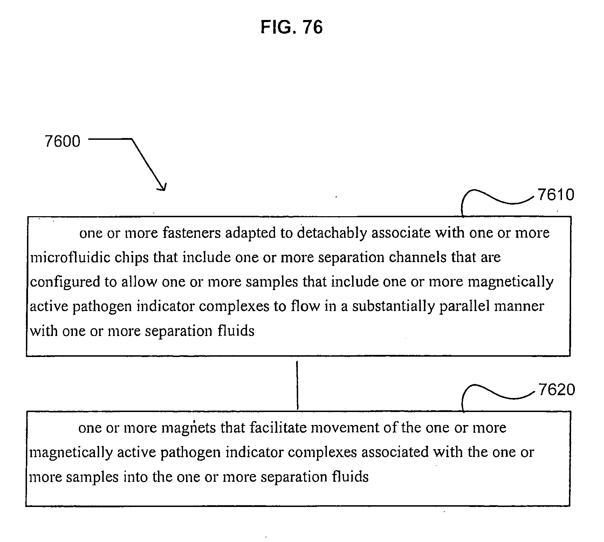

FIG. 76 illustrates an example device 7600 in which embodiments may be implemented.

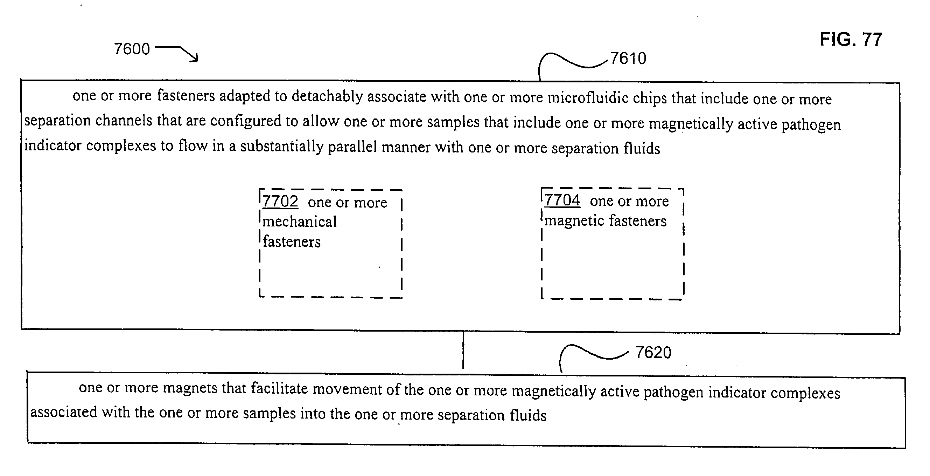

FIG. 77 illustrates alternate embodiments of the device of FIG. 76.

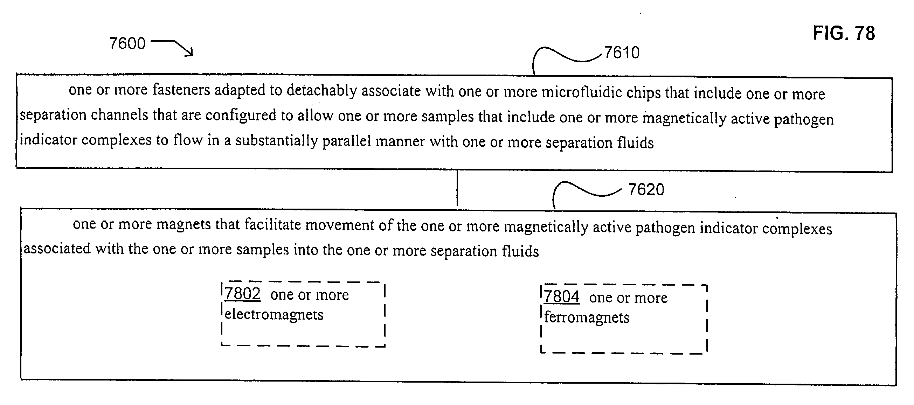

FIG. 78 illustrates alternate embodiments of the device of FIG. 76.

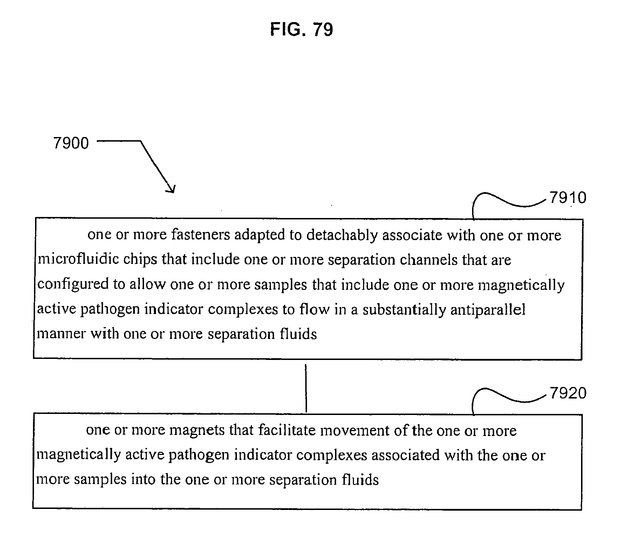

FIG. 79 illustrates an example device 7900 in which embodiments may be implemented.

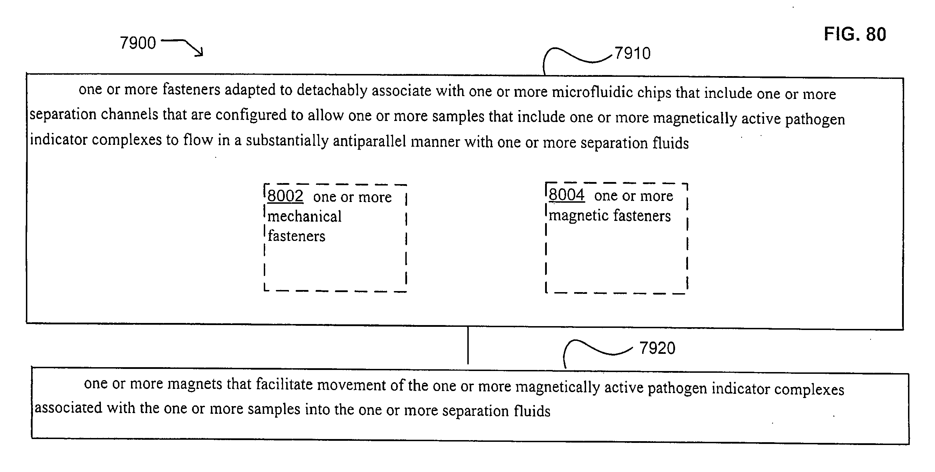

FIG. 80 illustrates alternate embodiments of the device of FIG. 79.

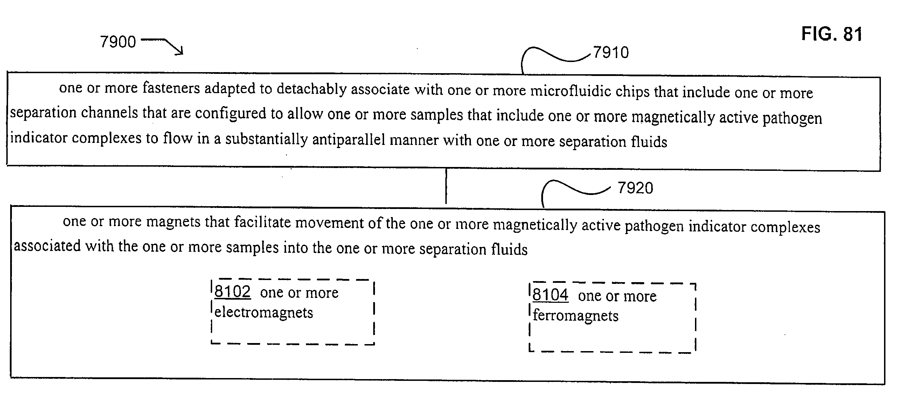

FIG. 81 illustrates alternate embodiments of the device of FIG. 79.

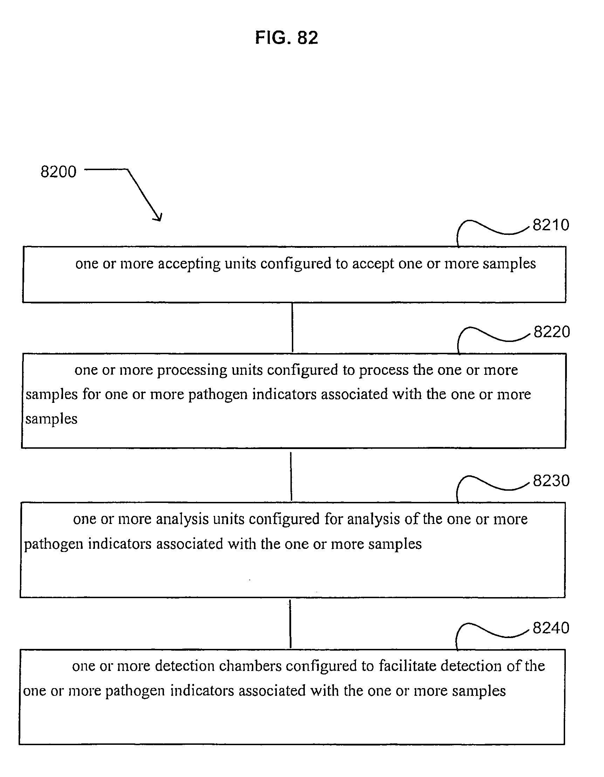

FIG. 82 illustrates an example microfluidic chip 8200 in which embodiments may be implemented.

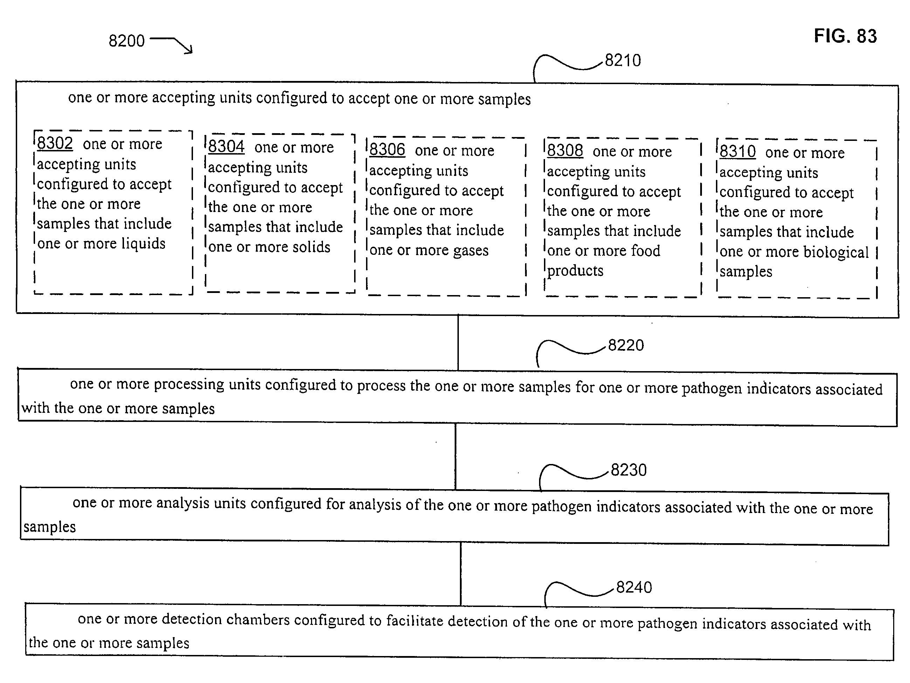

FIG. 83 illustrates alternate embodiments of the microfluidic chip of FIG. 82.

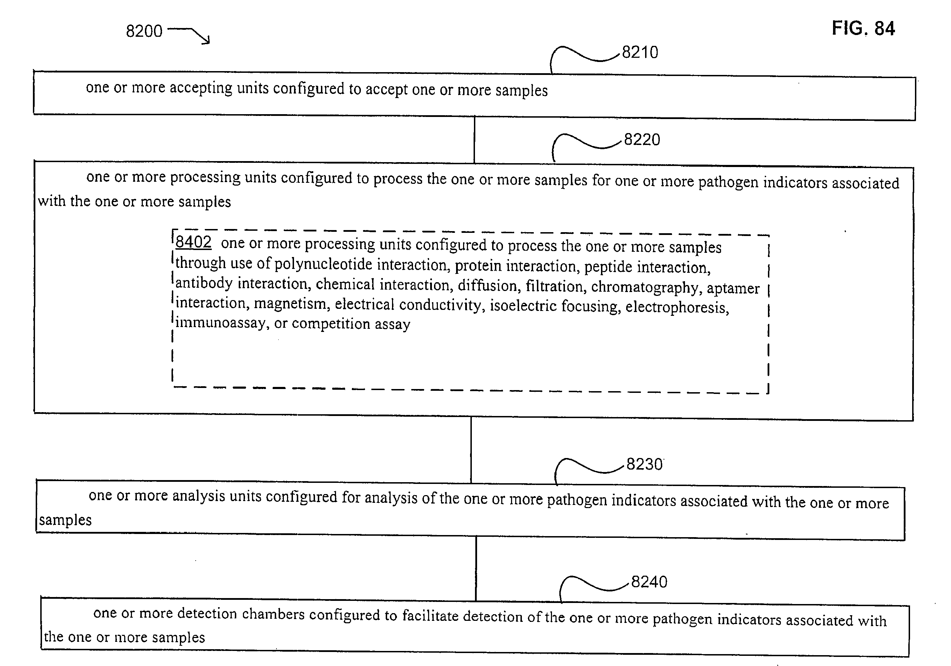

FIG. 84 illustrates alternate embodiments of the microfluidic chip of FIG. 82.

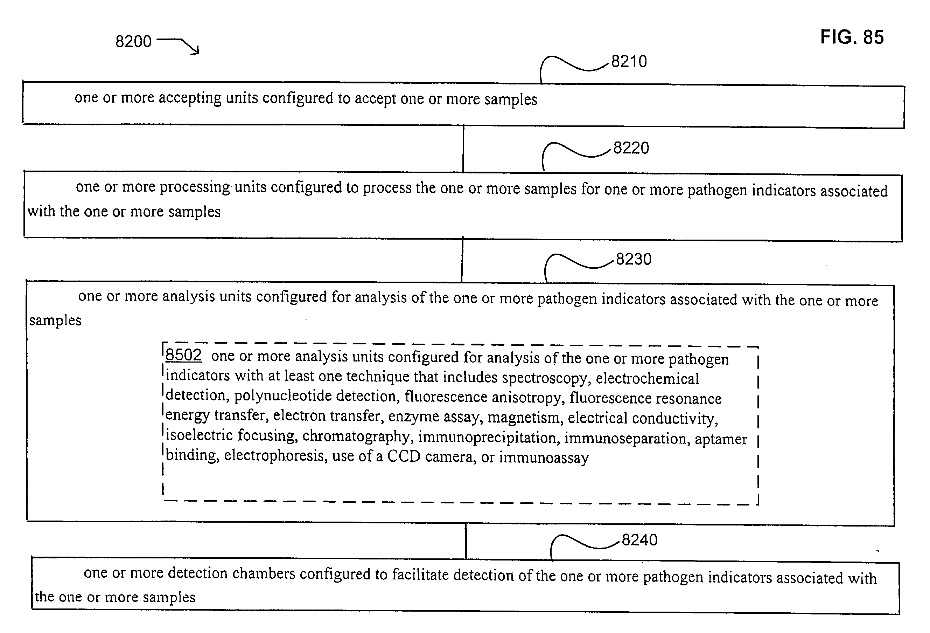

FIG. 85 illustrates alternate embodiments of the microfluidic chip of FIG. 82.

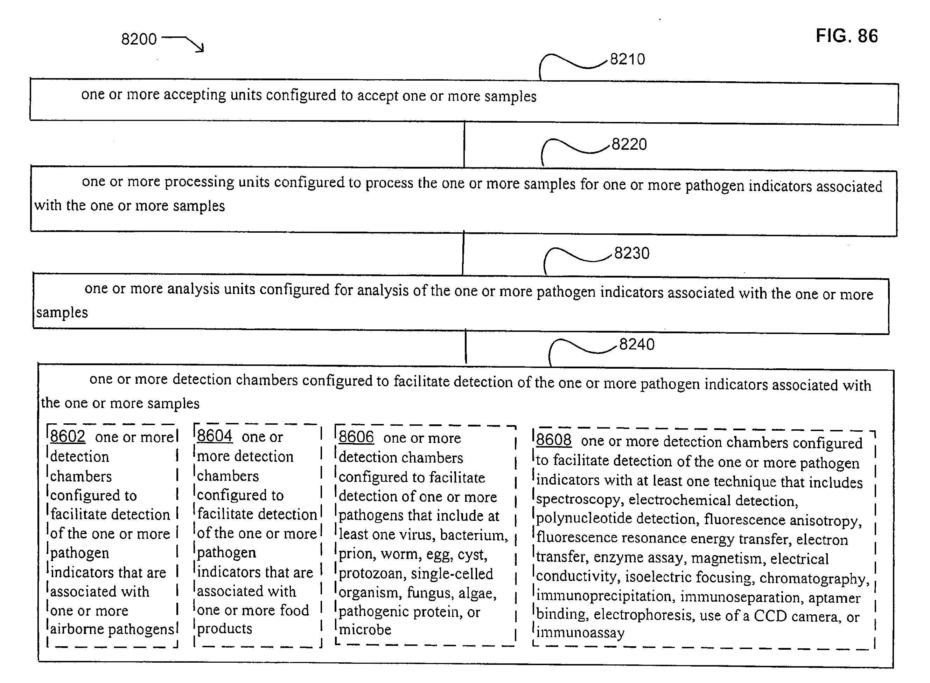

FIG. 86 illustrates alternate embodiments of the microfluidic chip of FIG. 82.

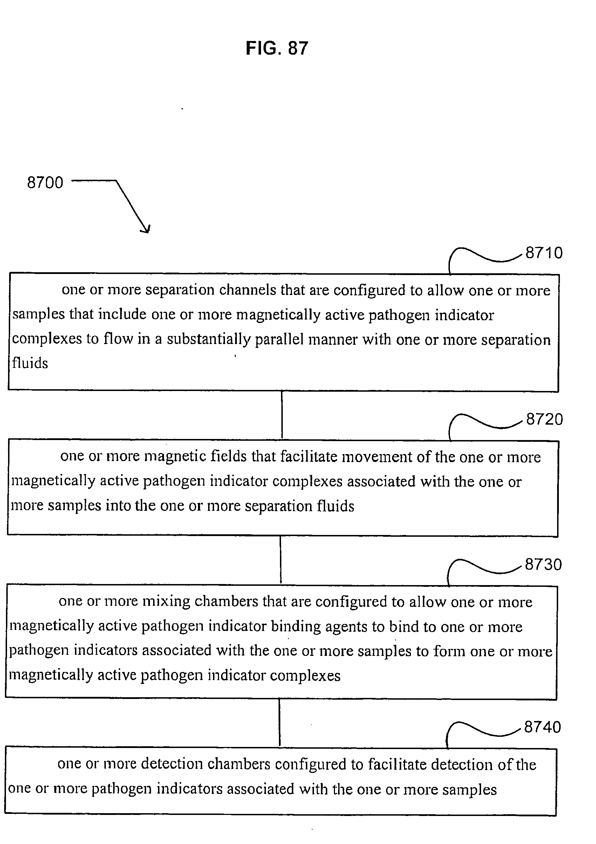

FIG. 87 illustrates an example microfluidic chip 8700 in which embodiments may be implemented.

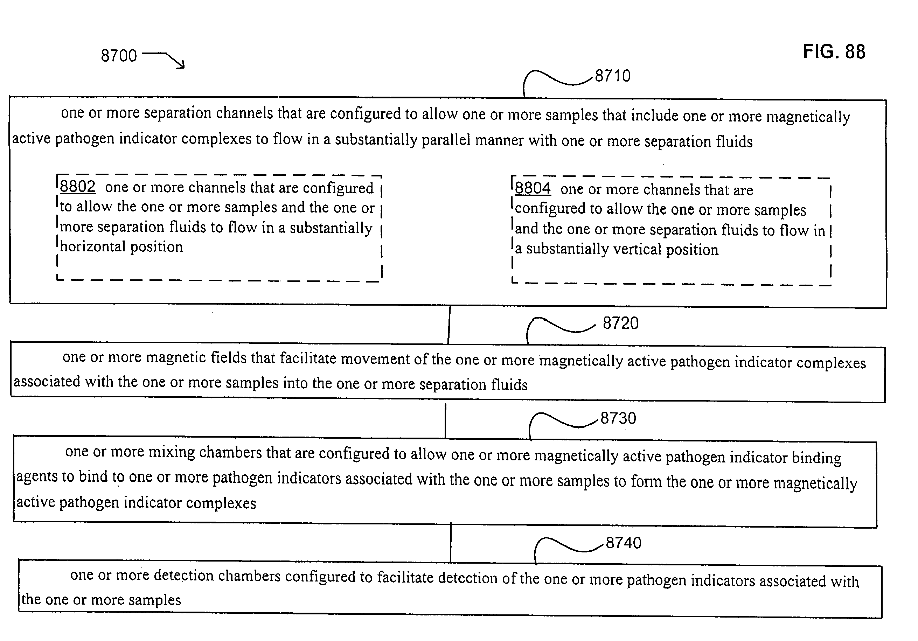

FIG. 88 illustrates alternate embodiments of the microfluidic chip of FIG. 87.

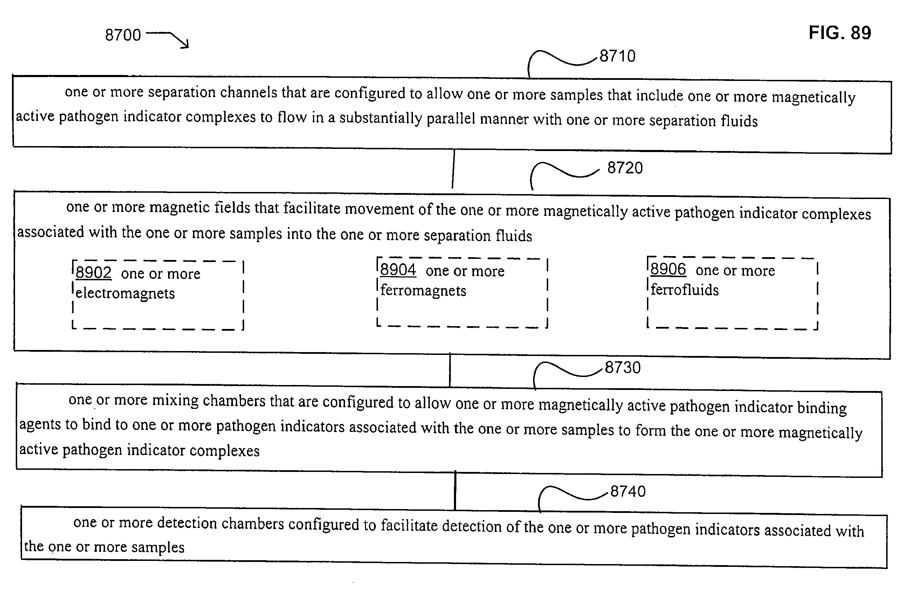

FIG. 89 illustrates alternate embodiments of the microfluidic chip of FIG. 87.

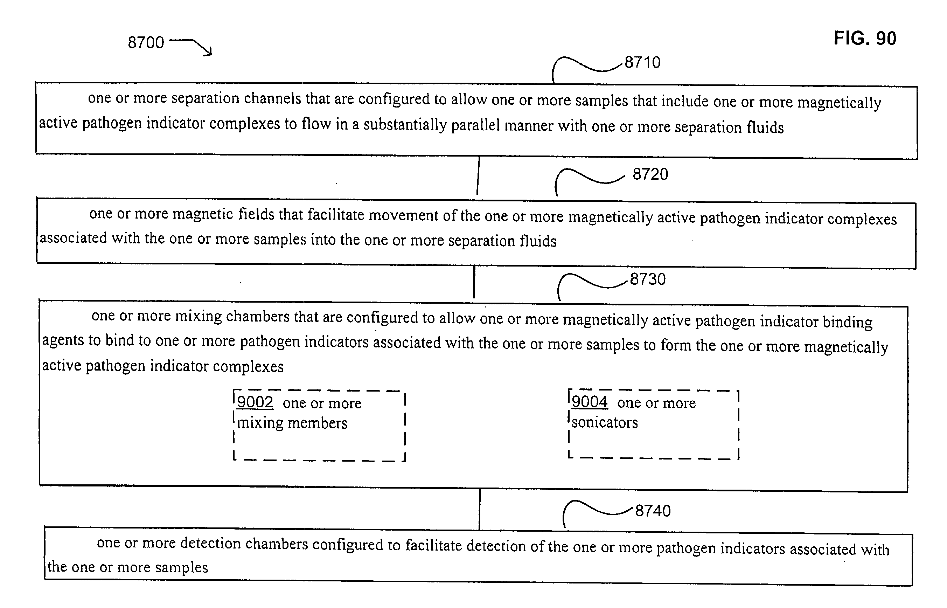

FIG. 90 illustrates alternate embodiments of the microfluidic chip of FIG. 87.

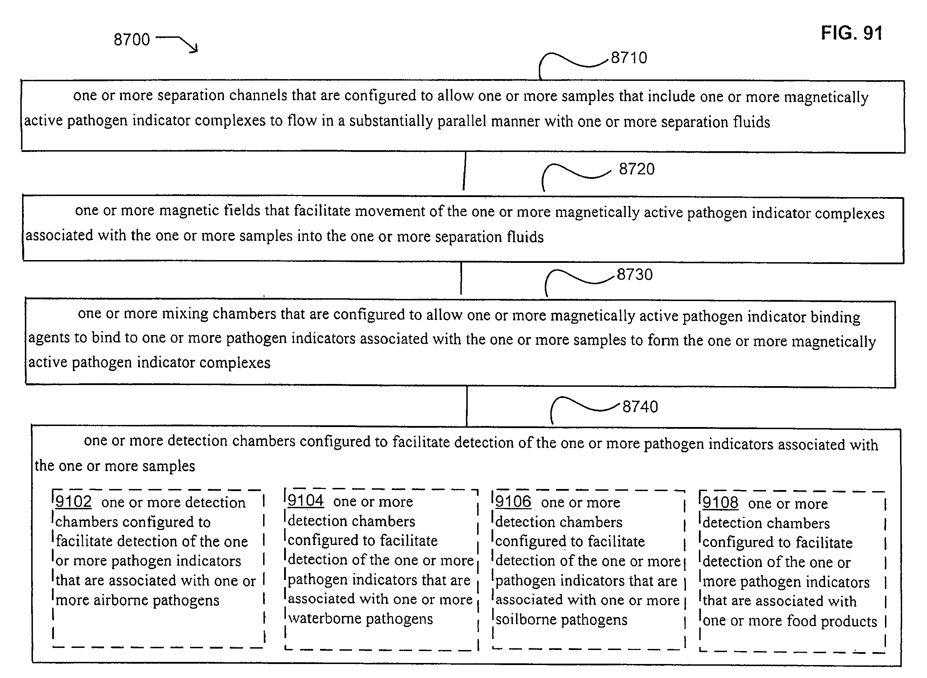

FIG. 91 illustrates alternate embodiments of the microfluidic chip of FIG. 87.

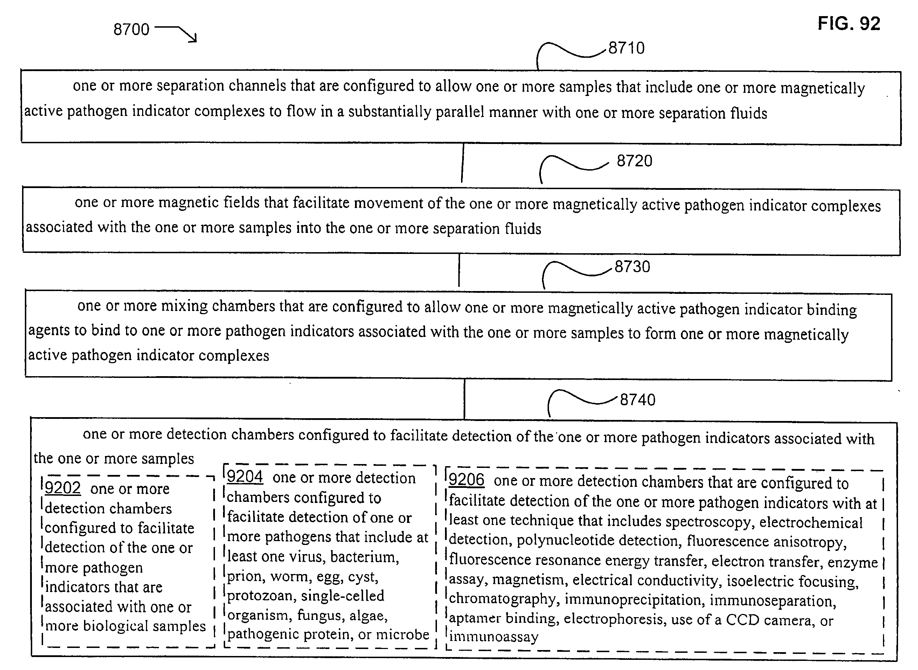

FIG. 92 illustrates alternate embodiments of the microfluidic chip of FIG. 87.

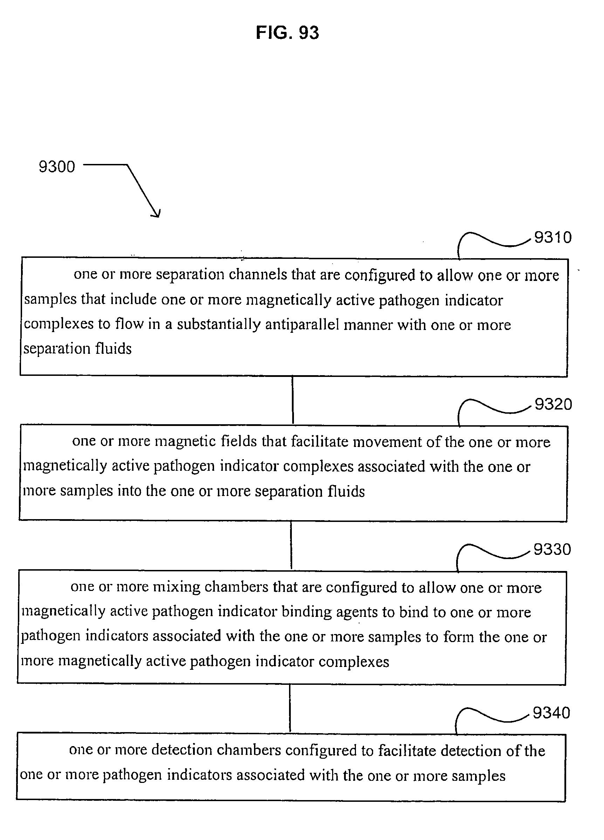

FIG. 93 illustrates an example microfluidic chip 9300 in which embodiments may be implemented.

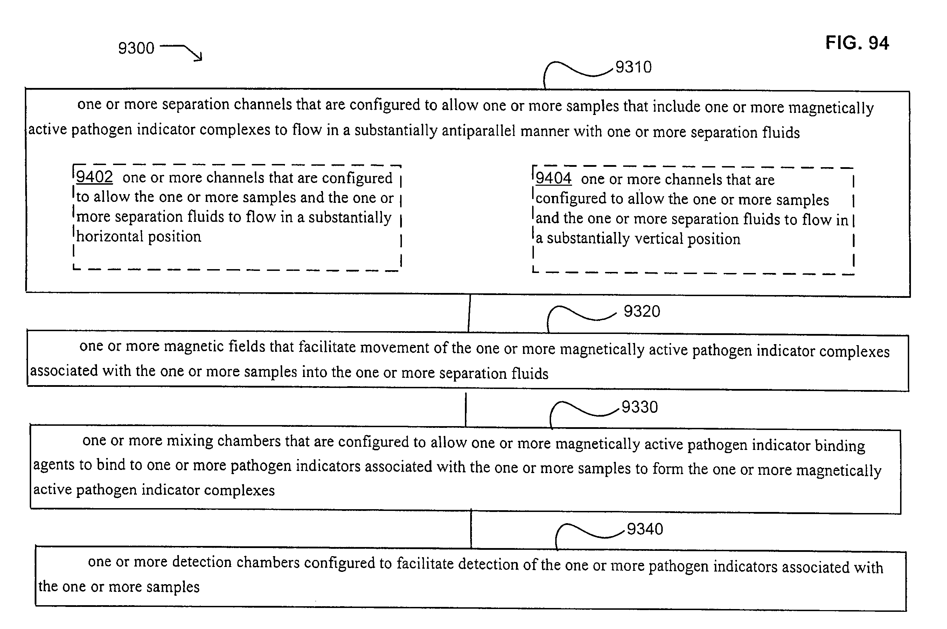

FIG. 94 illustrates alternate embodiments of the microfluidic chip of FIG. 93.

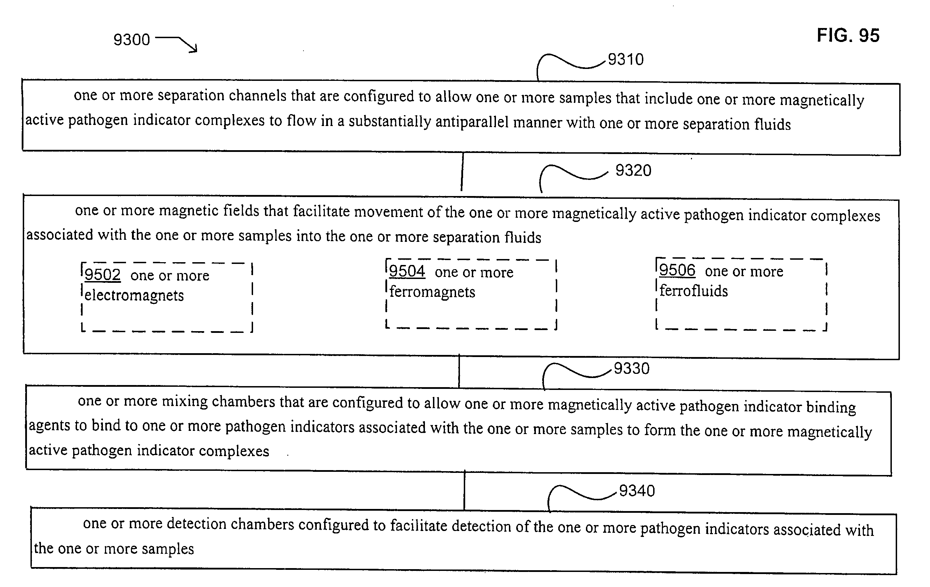

FIG. 95 illustrates alternate embodiments of the microfluidic chip of FIG. 93.

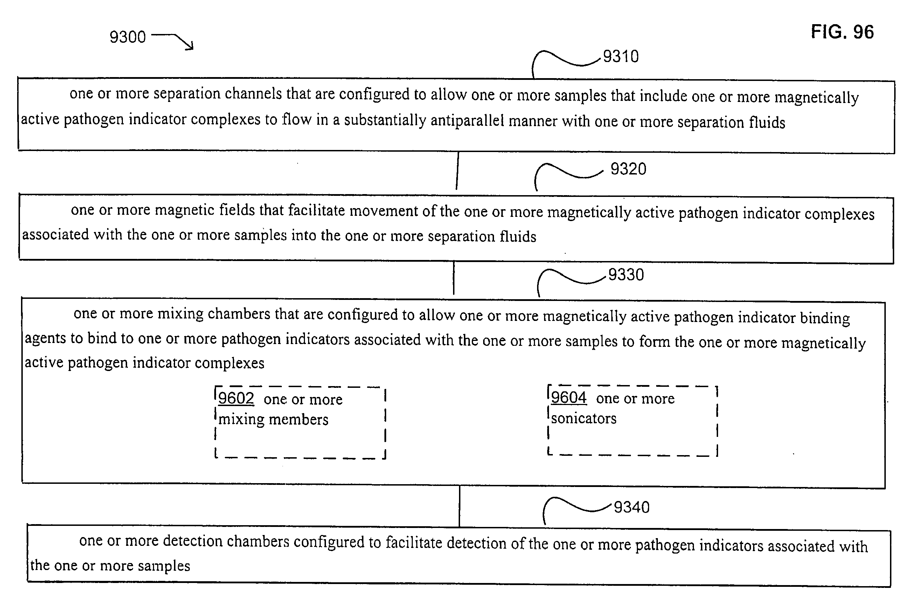

FIG. 96 illustrates alternate embodiments of the microfluidic chip of FIG. 93.

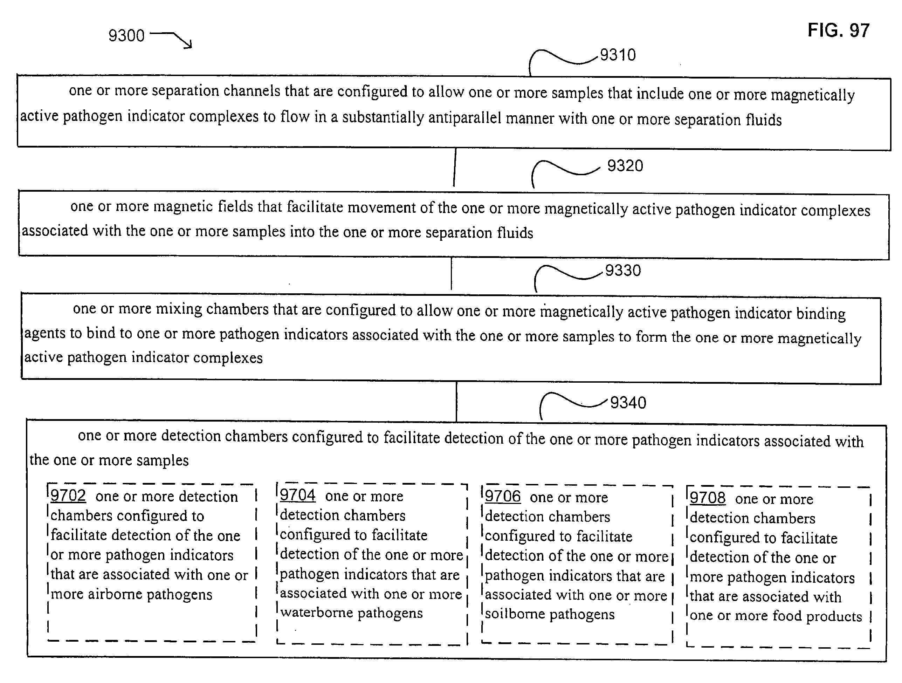

FIG. 97 illustrates alternate embodiments of the microfluidic chip of FIG. 93.

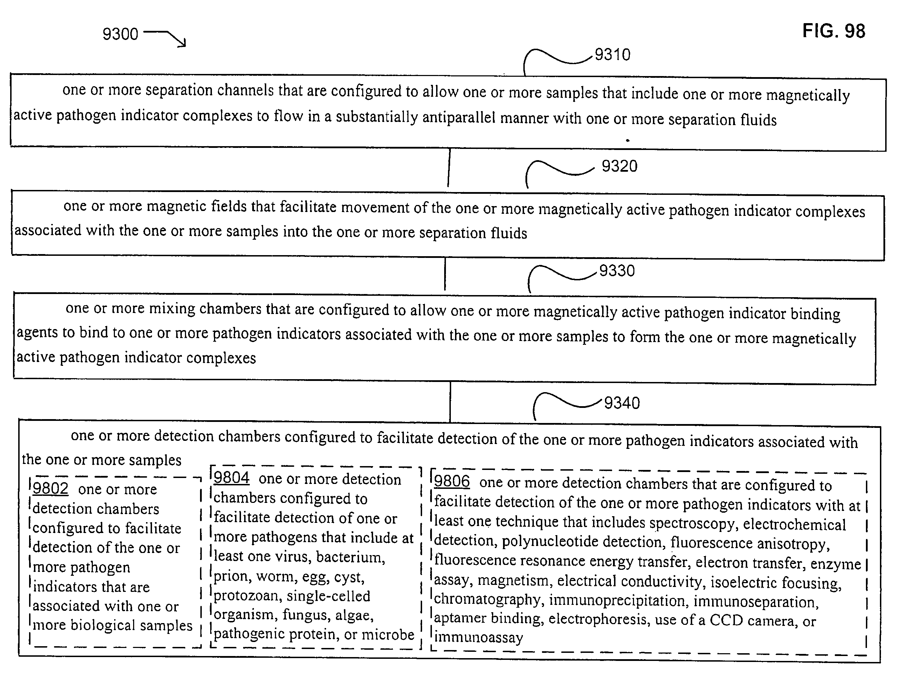

FIG. 98 illustrates alternate embodiments of the microfluidic chip of FIG. 93.

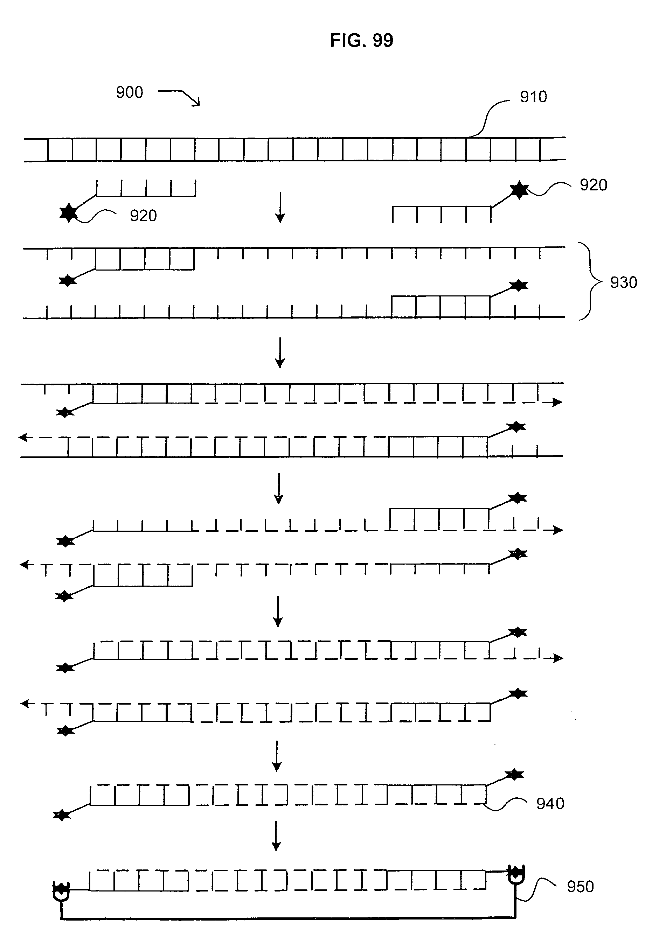

FIG. 99 illustrates a procedure to facilitate detection of a pathogen indicator that includes a polynucleotide.

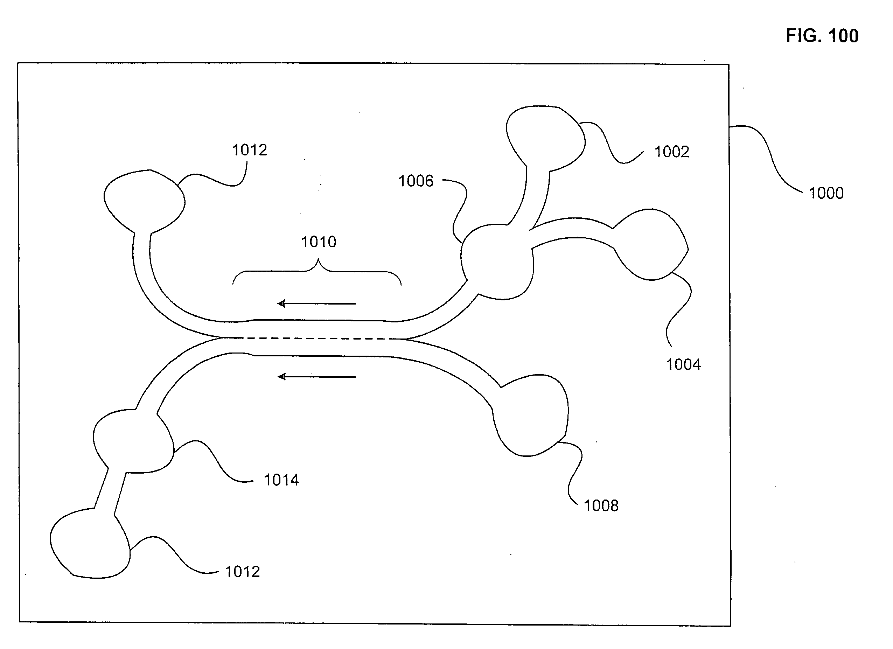

FIG. 100 illustrates an example microfluidic chip 1000.

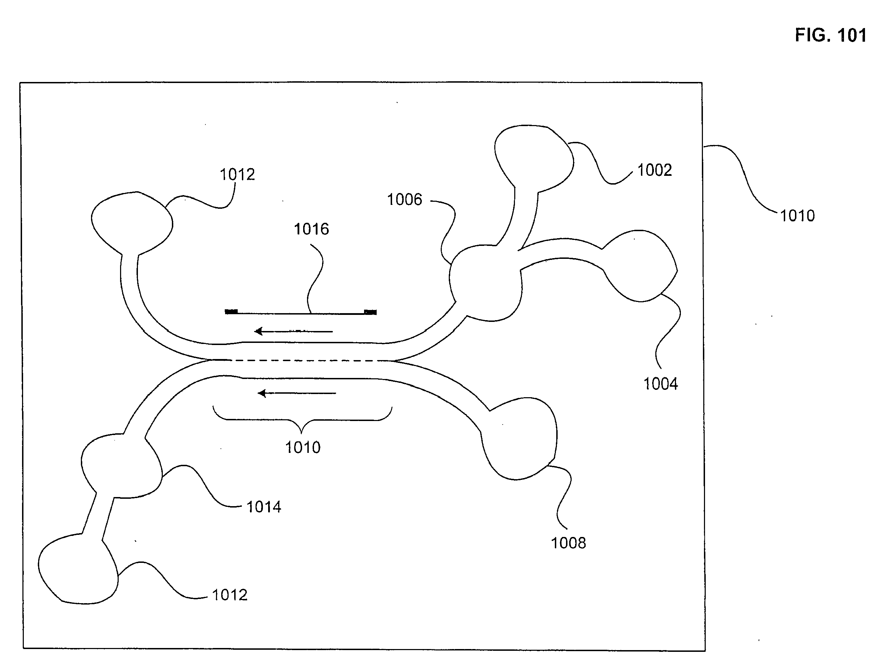

FIG. 101 illustrates an example microfluidic chip 1010.

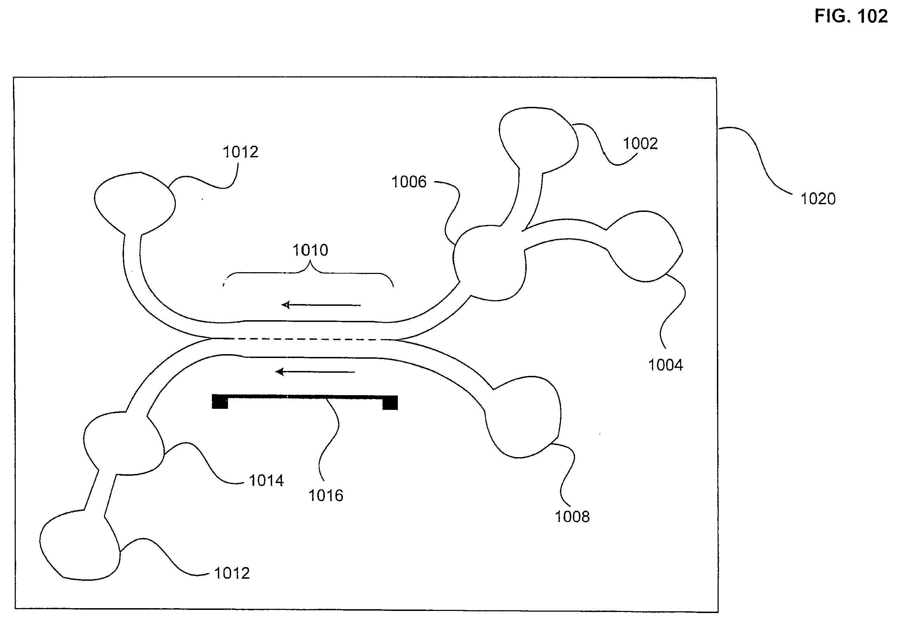

FIG. 102 illustrates an example microfluidic chip 1020.

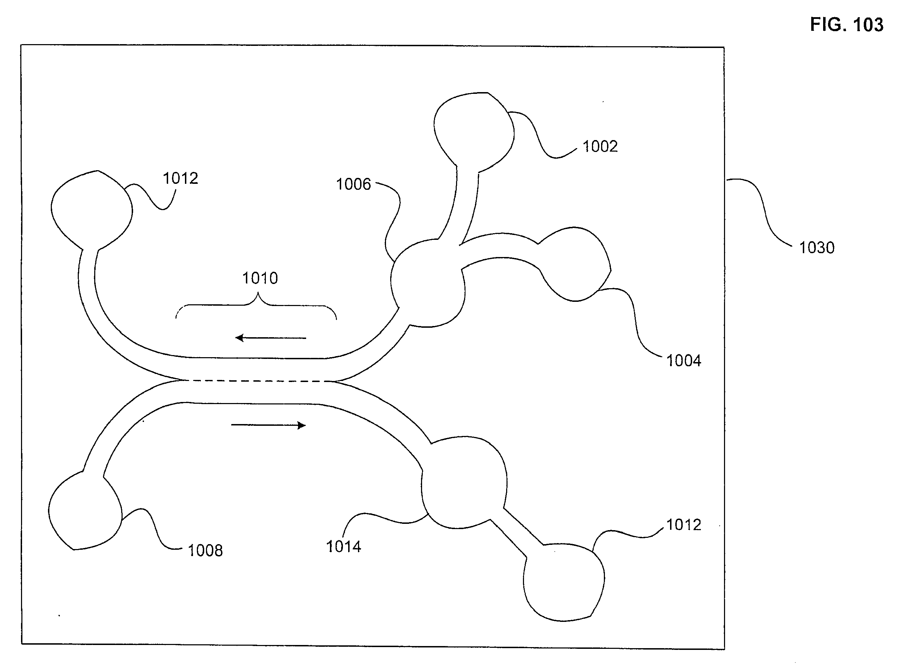

FIG. 103 illustrates an example microfluidic chip 1030.

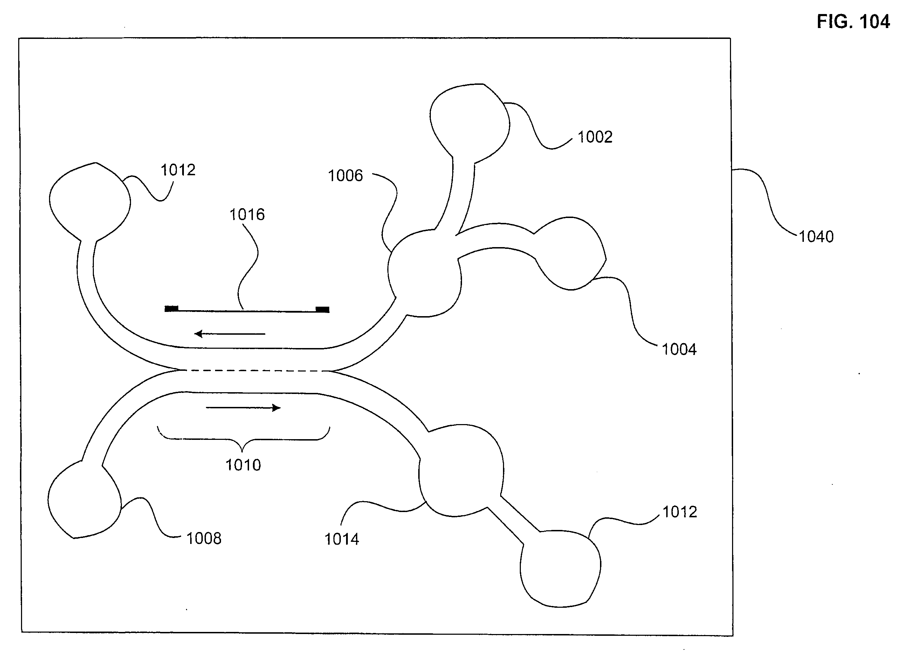

FIG. 104 illustrates an example microfluidic chip 1040.

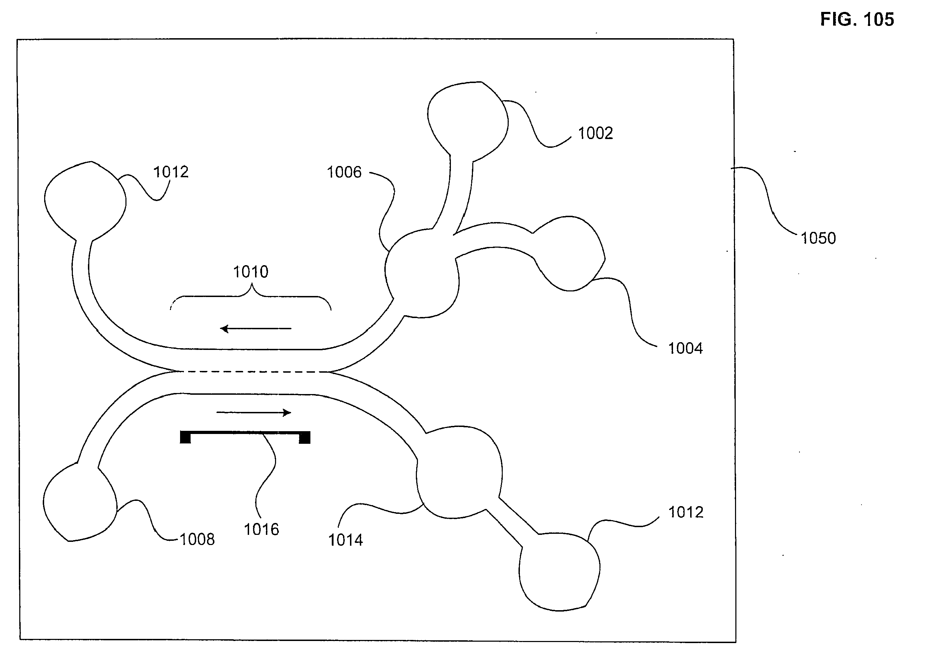

FIG. 105 illustrates an example microfluidic chip 1050.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

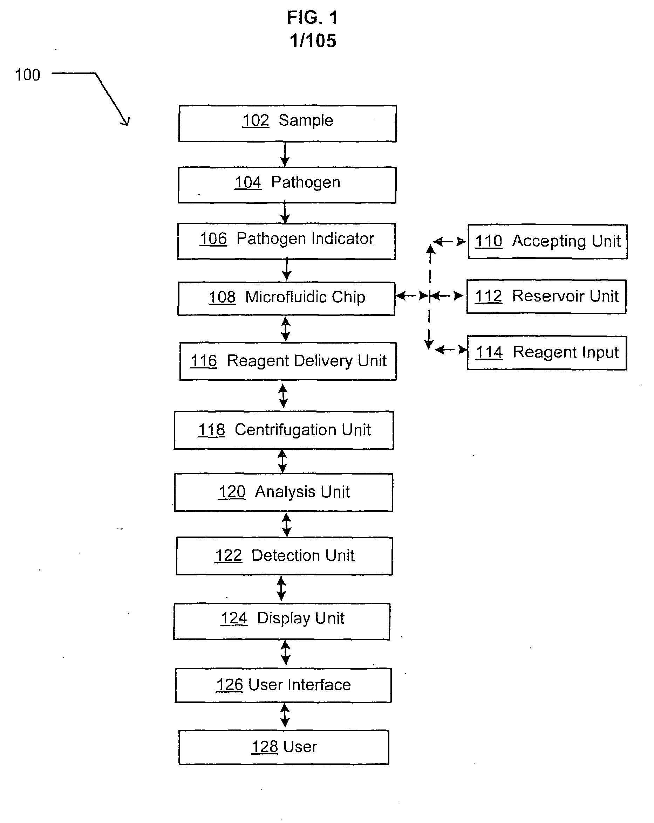

Fig. 1 illustrates an example system 100 in which embodiments may be implemented. In some embodiments, the system 100 is operable to provide a method that may be used to analyze one or more pathogens 104. In some embodiments, one or more samples 102 may be processed with one or more microfluidic chips 108 that are configured to process one or more pathogens 104. In some embodiments, one or more samples 102 associated with an individual may be processed. In some embodiments, one sample 102 associated with an individual may be processed. In some embodiments, one or more microfluidic chips 108 may be used to process one or more samples 102. In some embodiments, one microfluidic chip 108 may be used to process one or more samples 102. In some embodiments, one or more microfluidic chips 108 may be used to process one or more pathogens 104. In some embodiments, one or more microfluidic chips 108 may be used to process one pathogen 104. In some embodiments, one or more microfluidic chips 108 may include one or more accepting units 110. In some embodiments, one or more microfluidic chips 108 may include one or more reservoir units 112. In some embodiments, one or more microfluidic chips 108 may include one or more reagent inputs 114. In some embodiments, one or more microfluidic chips 108 may be configured to operably associate with one or more reagent delivery units 116. In some embodiments, one or more microfluidic chips 108 may be configured to operably associate with one or more centrifugation units 118. In some embodiments, one or more microfluidic chips 108 may be configured to operably associate with one or more analysis units 120. In some embodiments, one or more microfluidic chips 108 may be configured to operably associate with one or more detection units 122. In some embodiments, one or more microfluidic chips 108 may be configured to operably associate with one or more display units 124. In some embodiments, one or more detection units 122 may be used to detect one or more pathogens 104. In some embodiments, one detection unit 122 may be used to detect one or more pathogens 104. In some embodiments, one or more detection units 122 may be used to detect one or more pathogen indicators 106. In some embodiments, one or more detection units 122 may be portable detection units 122. In some embodiments, one or more detection units 122 may be non-portable detection units 122. In some embodiments, one or more detection units 122 may be hand-held detection units 122. In some embodiments, one or more detection units 122 may include one or more user interfaces 126. In some embodiments, one or more detection units 122 may include one user interface 126. In some embodiments, one or more detection units 122 may include one or more user interfaces 126 that are directly coupled with the one or more detection units 122. In some embodiments, one or more detection units 122 may include one or more user interfaces 126 that are remotely coupled with one or more detection units 122. For example, in some embodiments, a user 128 may interact with the one or more detection units 122 through direct physical interaction with the one or more detection units 122. In other embodiments, a user 128 may interact with one or more detection units 122 through remote interaction. In some embodiments, one or more detection units 122 may include one or more display units 124. In some embodiments, one or more detection units 122 may be directly coupled to one or more display units 124. In some embodiments, one or more detection units 122 may be remotely coupled to one or more display units 124. In some embodiments, one or more display units 124 may include one or more user interfaces 126. In some embodiments, one or more display units 124 may include one user interface 126.

### SAMPLE

Numerous types of samples 102 may be analyzed through use of system 100. In some embodiments, one or more samples 102 may be associated with an individual. For example, in some embodiments, system 100 may be used to diagnose an individual for infection with one or more pathogens 104. In some embodiments, one or more samples 102 may include a liquid. In some embodiments, one or more samples 102 may include a solid. In some embodiments, one or more samples 102 may include a vapor. In some embodiments, one or more samples 102 may include a semi-solid. In some embodiments, one or more samples 102 may include a gas. Examples of such samples 102 include, but are not limited to, air, water, food, food products, solids, samples 102 obtained from animals, samples 102 obtained from humans, samples 102 that are associated with, but not limited to, one or more toxins, viruses, bacteria, protozoans, single-celled organisms, fungus, algae, prions, microbes, cyst, eggs, pathogenic proteins, or substantially any combination thereof.

### PATHOGEN INDICATOR

Numerous pathogen indicators 106 may be processed, analyzed and/or detected through use of system 100. In some embodiments, pathogen indicators 106 include pathogens 104 and components of pathogens 104. For example, in some embodiments, pathogen indicators 106 may include polynucleotides and/or polypeptides that are associated with a pathogen 104. In some embodiments, pathogen indicators 106 may include one or more products of a pathogen 104. In some embodiments, pathogen indicators 106 may include products and/or substrates that are associated with the activity of one or more pathogen 104 associated enzymes. In some embodiments, pathogen indicators 106 may include compounds and/or particles that exhibit an adjuvant effect with regard to one or more pathogens 104. Examples of pathogen indicators 106 that may be processed, analyzed and/or detected through use of system 100 include, but are not limited to, pathogen indicators 106 associated with plant pathogens 104, animal pathogens 104, human pathogens 104, fish pathogens 104, bird pathogens 104, and the like. Examples of such pathogens 104 include, but are not limited to, viruses, bacteria, prions, protozoans, single-celled organisms, algae, eggs of pathogenic organisms, microbes, cysts, molds, fungus, worms, amoeba, pathogenic proteins, or substantially any combination thereof. Numerous pathogens 104 are known and have been described (e.g., Foodborne Pathogens: Microbiology and Molecular Biology, Caister Academic Press, eds. Fratamico, Bhunia, and Smith (2005); Maizels et al., Parasite Antigens Parasite Genes: A Laboratory Manual for Molecular Parasitology, Cambridge University Press (1991); National Library of Medicine).

### MICROFLUIDIC CHIP

Numerous types of microfluidic chips 108 may be utilized within system 100. Methods to construct and utilize microfluidic chips 108 have been described (e.g., U.S. Statutory Invention Registration No. H201; U.S. Patent Nos.: 6,454,945; 6,818,435; 6,812,458; 6,794,196; 6,709,869; 6,582,987; 6,482,306; 5,726,404; 7,118,910; 7,081,192; herein incorporated by reference).

In some embodiments, a microfluidic chip 108 may be configured to utilize microfluidic principles. Accordingly, in some embodiments, a microfluidic chip 108 may be configured to include one or more channels with at least one dimension that is less than 1 millimeter. However, in some embodiments, microfluidic chips 108 may be configured such that they do not utilize microfluidic principles. Accordingly, in some embodiments, microfluidic chips 108 may be configured such that there are not any components that have a dimension that is less than 1 millimeter. Accordingly, in some embodiments, microfluidic chips 108 may be configured that include components having a dimension that is less than 1 millimeter, while in other embodiments, microfluidic chips 108 may be configured with components having dimensions that are greater than 1 millimeter. In some embodiments, a microfluidic chip 108 may include at least one component that has at least one dimension that is less than 1 millimeter and at least one component having at least one dimension that is greater than 1 millimeter.

For example, microfluidic chips 108 may be configured to utilize a variety of methods to process one or more pathogens 104. Examples of such methods include, but are not limited to, nucleic acid (polynucleotide) hybridization based methods, immunological based methods, chromatographic based methods, affinity based methods, extraction based methods, separation based methods, isolation based methods, filtration based methods, enzyme based methods, isoelectric focusing methods, or substantially any combination thereof.

Microfluidic chips 108 may utilize numerous methods for analysis of one or more pathogen indicators 106. For example, in some embodiments, one or more microfluidic chips 108 may be configured to utilize: chemiluminescent methods (e.g., U.S. Patent Nos.: 6,090,545 and 5,093,268; herein incorporated by reference), plasmon resonance sensors (e.g., U.S. Patent No.: 7,030,989; herein incorporated by reference), nuclear magnetic resonance detectors (e.g., U.S. Patent No.: 6,194,900; herein incorporated by reference), gradient-based assays (e.g., U.S. Patent No.: 7,112,444; herein incorporated by reference), reporter beads (e.g., U.S. Patent No.: 5,747,349; herein incorporated by reference), transverse electrophoresis (e.g., Macounova et al., Analytical Chemistry, 73:1627-1633 (2001)); isoelectric focusing (e.g., Macounova et al., Analytical Chemistry, 72:3745-3751 (2000); Xu et al., Isoelectric focusing of green fluorescent proteins in plastic microfluidic channels. Abstracts of Papers of the American Chemical Society, 219:9-ANYL (2000); Macounova et al., Analytical Chemistry, 73:1627-1633 (2001)), diffusion based systems (e.g., Kamholz et al., Biophysical Journal, 80:1967-1972 (2001); Hatch et al., Nature Biotechnology, 19:461-465 (2001); U.S. Patent Nos.: 6,221,677; 5,972,710; herein incorporated by reference), high performance liquid chromatography (e.g., U.S. Patent No.: 6,923,907; herein incorporated by reference), polynucleotide analysis (e.g., Belgrader et al., Biosensors & Bioelectronics, 14:849-852 (2000); Buchholz et al., Analytical Chemistry, 73:157-164 (2001); Fan et al., Analytical Chemistry, 71:4851-4859 (1999); Koutny et al., Analytical Chemistry, 72:3388-3391 (2000); Lee et al., Microfabricated plastic chips by hot embossing methods and their applications for DNA separation and detection. Sensors and Actuators B-Chemical, 75:142-148 (2001); U.S. Patent No.: 6,958,216; herein incorporated by reference), capillary electrophoresis (e.g., Kameoka et al., Analytical Chemistry, 73:1935-1941 (2001)), immunoassays (e.g., Hatch et al., Nature Biotechnology, 19:461-465 (2001); Eteshola and Leckband, D. Development and characterization of an ELISA assay in PDMS microfluidic channels. Sensors and Actuators B-Chemical 72:129-133 (2001); Cheng et al., Analytical Chemistry, 73:1472-1479 (2001); Yang et al., Analytical Chemistry, 73:165-169 (2001)), flow cytometry (e.g., Sohn et al., Proc. Natl. Acad. Sci., 97:10687-10690 (2000)), PCR amplification (e.g., Belgrader et al., Biosensors & Bioelectronics, 14:849-852 (2000); Khandurina et al., Analytical Chemistry, 72:2995-3000 (2000); Lagally et al., Analytical Chemistry, 73:565-570 (2001)), cell manipulation (e.g., Glasgow et al., IEEE Transactions On Biomedical Engineering, 48:570-578 (2001)), cell separation (e.g., Yang et al., Analytical Chemistry, 71:911-918 (1999)), cell patterning (e.g., Chiu et al., Proc. Natl. Acad. Sci., 97:2408-2413 (2000); Folch et al., Journal of Biomedical Materials Research, 52:346-353 (2000)), chemical gradient formation (e.g., Dertinger et al., Analytical Chemistry, 73:1240-1246 (2001); Jeon et al., Langmuir, 16:8311-8316 (2000)), microcantilevers (e.g., U.S. Patent Nos.: 7,141,385; 6,935,165; 6,926,864; 6,763,705; 6,523,392; 6,325,904; herein incorporated by reference), or substantially any combination thereof.

In some embodiments, one or more microfluidic chips 108 may be configured to utilize one or more magnets that may be used during processing and/or analysis of one or more samples 102. For example, in some embodiments, ferrous metallic particles may be associated with one or more pathogen indicators 106 that are associated with one or more samples 102 (e.g., use of antibodies, aptamers, peptides, polynucleotides, and the like that bind to one or more pathogen indicators 106 and that are coupled to a ferrous metallic particle). The one or more pathogen indicators 106 may be separated from the remainder of the one or more samples 102 through use of one or more magnets. In some embodiments, one or more magnets may be used to create eddy currents that may be used to process and/or analyze one or more samples 102. For example, in some embodiments, non-ferrous metallic particles may be associated with one or more pathogen indicators 106 that are associated with one or more samples 102 (e.g., use of antibodies, aptamers, peptides, polynucleotides, and the like that bind to one or more pathogen indicators 106 and that are coupled to a non-ferrous metallic particle). One or more microfluidic chips 108 may be configured such that passage of a non-ferrous metallic particle through a magnetic field will cause an eddy current to impart kinetic energy to the non-ferrous metallic particle and provide for separation of the associated pathogen indicators 106 from the remainder of the one or more samples 102. In some embodiments, such methods may be combined with additional methods to provide for separation of one or more pathogen indicators 106 from one or more samples 102. For example, magnetic separation may be used in combination with one or more methods that may include, but are not limited to, diffusion (e.g., use of an H-filter), filtration, precipitation, immunoassay, immunodiffusion, and the like.

In some embodiments, one or more microfluidic chips 108 may be configured to utilize ferrofluids to separate one or more pathogen indicators 106 from one or more samples 102. For example, in some embodiments, a microfluidic chip 108 may include an H-filter where a sample fluid and a ferrofluid flow substantially in parallel (e.g., the sample fluid and the ferrofluid flow side-by-side through the H-filter (horizontal) and/or above and below (vertical)). In some embodiments, one or more microfluidic chips 108 may include a ferrofluid having magnetic particles such that ferrous materials contained within the sample fluid are attracted to the ferrofluid and thereby separated from the sample fluid. Accordingly, such microfluidic chips 108 may be configured to separate one or more pathogen indicators 106 from one or more samples 102. In some embodiments, one or more microfluidic chips 108 may include a ferrofluid having ferrous particles such that magnetic materials contained within the sample fluid are attracted to the ferrofluid and thereby separated from the sample fluid. Accordingly, in such embodiments, one or more microfluidic chips 108 may be configured to utilize ferrofluids to separate one or more pathogen indicators 106 from one or more samples 102.

Microfluidic chips 108 may be configured to process numerous types of samples 102. For example, in some embodiments, a microfluidic chip 108 may be configured to sonicate one or more samples 102. In some embodiments, a microfluidic chip 108 may include one or more ultrasonic electronic generators that produce a signal (e.g., 20 kilohertz) that can be used to drive a piezoelectric converter/transducer. This electrical signal may be converted by the transducer to a mechanical vibration due to the characteristics of the internal piezoelectric crystals. This vibration can be amplified and transmitted to one or more probes having tips that expand and contract to provide for sonication of one or more samples 102. In some embodiments, a microfluidic chip 108 may include one or more sonication probes. Such probes may be configured such that are able to operably associate with one or more vibration sources in a detachable manner. Accordingly, in some embodiments, one or more microfluidic chips 108 that include one or more probes may be configured to detachably connect with one or more vibration sources that produce a vibration that can be coupled to the one or more probes. In some embodiments, one or more detection units 122 may include one or more vibration sources.

In some embodiments, a microfluidic chip 108 may be configured to mix one or more samples 102. For example, in some embodiments, a microfluidic chip 108 may include a mixing chamber which includes one or more ferrous mixing members and electromagnets which are configured such that motion may be imparted to the one or more ferrous mixing members. In some embodiments, a microfluidic chip 108 may include one or more mixing chambers that include two or more electromagnets positioned around the one or more mixing chambers and one or more ferrous members positioned within the one or more mixing chambers and between the electromagnets. Accordingly, mixing of one or more materials within the one or more mixing chambers may be facilitated by alternating current between the electromagnets positioned around the mixing chamber. In some embodiments, a mixing chamber may include an elastomeric material that includes a ferrous material (e.g., an elastomeric-ferrous material) such that movement of the elastomeric-ferrous material may be facilitated through use of one or more magnets, such as electromagnets.

In some embodiments, elastomeric-ferrous materials may be utilized to fabricate pumps that are associated with microfluidic chips 108. For example, in some embodiments, a tube may include an elastomeric material that includes ferrous material such that movement of the elastomeric material may be facilitated through use of one or more magnets. Accordingly, valves and ferrous materials may be associated with the elastomeric tube such that expansion of a portion of the elastomeric tube through the action of a magnet, such as an electromagnetic, will act like a vacuum pump to draw fluids into the expanded portion of the elastomeric tube. In some embodiments, release of the elastomeric material from the magnetic field will cause the expanded portion of the tube to contract and will act to push the fluid from the formerly expanded portion of the elastomeric tubing. In some embodiments, valves may be positioned within the tube to provide for directional flow of fluid through the elastomeric tube. Accordingly, such pumps may be configured as vacuum pumps, propulsion type pumps, and/or both vacuum and propulsion type pumps.

In some embodiments, microfluidic chips 108 may be configured to utilize magnetically actuated fluid handling. In some embodiments, a microfluidic chip 108 may utilize magnetic fluid (e.g., ferrofluid, ferrogel, and the like) to move one or more gases and/or liquids through flow channels. For example, magnetically actuated slugs of magnetic fluid may be moved within channels of a microfluidic chip 108 to facilitate valving and/or pumping of one or more gases and/or liquids. In some embodiments, the magnets used to control gas and/or liquid movement may be individual magnets that are moved along the flow channels and/or one or more arrays of magnets that may be individually controlled to hold or move one or more magnetic slugs. In some embodiments, an array of electromagnets may be positioned along a flow channel which may be turned on and off in a predetermined pattern to move magnetic fluid slugs in desired paths in one or more flow channels. Methods to construct magnetically actuated fluid handling devices have been described (e.g., U.S. Patent Nos.: 6,408,884 and 7,110,646; herein incorporated by reference).

Accordingly, microfluidic chips 108 may be configured for analysis of numerous types of pathogen indicators 106.

### REAGENT DELIVERY UNIT

System 100 may include one or more reagent delivery units 116. In some embodiments, one or more reagent delivery units 116 may be configured to operably associate with one or more microfluidic chips 108. Accordingly, in some embodiments, one or more reagent delivery units 116 may be configured to contain one or more reagents that may be used within one or more microfluidic chips 108 to analyze and/or detect one or more pathogens 104 and/or one or more pathogen indicators 106. In some embodiments, one or more reagent delivery units 116 may include one or more pumps to facilitate delivery of one or more reagents. Numerous types of pumps may be used within a reagent delivery unit 116. In some embodiments, one or more reagent delivery units 116 may be configured to operably associate with one or more centrifugation units 118. Accordingly, reagents may be delivered through use of centrifugal force. Reagent delivery units 116 may be configured in numerous ways. For example, in some embodiments, reagent delivery units 116 may include one or more reagent reservoirs, one or more waste reservoirs or substantially any combination thereof. Reagent delivery units 116 may be configured to contain and/or deliver numerous types of reagents. Examples of such reagents include, but are not limited to, phenol, chloroform, alcohol, salt solutions, detergent solutions, solvents, reagents used for polynucleotide precipitation, reagents used for polypeptide precipitation, reagents used for polynucleotide extraction, reagents used for polypeptide extraction, reagents used for chemical extractions, and the like. Accordingly, reagent delivery units 116 may be configured to contain and/or deliver virtually any reagent that may be used for the analysis of one or more pathogens 104 and/or pathogen indicators 106.

### CENTRIFUGATION UNIT

System 100 may include one or more centrifugation units 118. In some embodiments, one or more centrifugation units 118 may be configured to operably associate with one or more microfluidic chips 108. Accordingly, in some embodiments, one or more centrifugation units 118 may be used to facilitate analysis and/or detection of one or more pathogens 104 and/or one or more pathogen indicators 106. Methods to fabricate devices that may be used to drive fluid movement through centripetal acceleration in a microfluidics system have been described (e.g., U.S. Patent No: 6,709,869; herein incorporated by reference).

For example, in some embodiments, one or more centrifugation units 118 may be used to facilitate the analysis of one or more polynucleotides from one or more samples 102 that are applied to one or more microfluidic chips 108 (e.g., U.S. Patent Application Serial Nos.: 11/699,770; 11/699,920; 11/699,747; and 11/699,774; herein incorporated by reference).

In some embodiments, one or more centrifugation units 118 may be configured to centrifuge one or more microfluidic chips 108 to facilitate movement of one or more samples 102, one or more reagents, one or more fluids, and the like through the one or more microfluidic chips 108.

In some embodiments, one or more centrifugation units 118 may be configured to centrifuge one or more microfluidic chips 108 to create a gradient. In some embodiments, velocity gradients may be created to facilitate analysis of one or more samples 102. For example, glycerol gradients may be used to separate polypeptides from one or more samples 102. In other embodiments, density gradients may be created to facilitate analysis of one or more samples 102. For example, cesium chloride may be used to create a density gradient to facilitate the analysis of one or more polynucleotides. In some embodiments, gradient centrifugation may be used to analyze one or more viral particles.

In some embodiments, one or more centrifugation units 118 may be configured to centrifuge one or more microfluidic chips 108 to facilitate chromatographic separations of components within one or more samples 102. For example, chromatographic media may be packed within a microfluidic chip 108 to facilitate the separation of components, such as pathogens 104 and/or pathogen indicators 106, from one or more samples 102. Such chromatographic media is commercially available (e.g., Qiagen Sciences, Germantown, MD and Pfizer, New York, NY).

### ANALYSIS UNIT

System 100 may include one or more analysis units 120. Analysis units 120 may be configured for analysis of numerous types of pathogens 104 and/or pathogen indicators 106. In some embodiments, one or more analysis units 120 may be configured for analysis of one or more polynucleotides, polypeptides, polysaccharides, enzyme activities, and the like. In some embodiments, one or more polynucleotides, polypeptides, polysaccharides, enzyme activities, and the like that are associated with one or more pathogens may be analyzed. In some embodiments, one or more polynucleotides, polypeptides, polysaccharides, enzyme activities, and the like that are associated with pathogen activity may be analyzed.

For example, in some embodiments, one or more analysis units 120 may be configured for analysis of one or more polypeptides through use of numerous techniques that include, but are not limited to, competition assays, immunological methods (e.g., sandwich assays), and the like.

In other embodiments, one or more analysis units 120 may be configured for analysis of one or more polynucleotides through use of numerous techniques that include, but are not limited to, competition assays, electron transfer assays, electrical conductivity assays, and the like.

### DETECTION UNIT

Numerous types of detection units 122 may be used within system 100. Accordingly, numerous types of detection methods may be used within system 100. Examples of such detection methods include, but are not limited to, colorimetric methods, spectroscopic methods, resonance based methods, electron transfer based methods (redox), conductivity based methods, gravimetric based assays, turbidity based methods, ion-specific based methods, refractive index based methods, radiological based methods, or substantially any combination thereof. In some embodiments, a detection unit 122 may be stationary. For example, in some embodiments, a detection unit 122 may be a laboratory instrument. In some embodiments, a detection unit 122 may be portable. For example, in some embodiments, a detection unit 122 may be hand-held device.

### DISPLAY UNIT

The system 100 may include one or more display units 124. Numerous types of display units 124 may be used in association with system 100. Examples of such display units 124 include, but are not limited to, liquid crystal displays, printers, audible displays, cathode ray displays, plasma display panels, Braille displays, passive displays, chemical displays, active displays, and the like. In some embodiments, display units 124 may display information in numerous languages. Examples of such languages include, but are not limited to, English, Spanish, German, Japanese, Chinese, Italian, and the like. In some embodiments, display units 124 may display information pictographically, colorometrically, and/or physically, such as displaying information in Braille.

In some embodiments, one or more display units 124 may be physically coupled to one or more microfluidic chips 108. In some embodiments, one or more display units 124 may be remotely coupled to one or more microfluidic chips 108. In some embodiments, one or more display units 124 may be physically coupled to one or more analysis units 120. In some embodiments, one or more display units 124 may be remotely coupled to one or more analysis units 120. In some embodiments, one or more display units 124 may be physically coupled to one or more detection units 122. In some embodiments, one or more display units 124 may be remotely coupled to one or more detection units 122. Accordingly, one or more display units 124 may be positioned in one or more locations that are remote from the position where analysis of one or more pathogens 104 takes place. Examples of such remote locations include, but are not limited to, the offices of physicians, nurses, pharmacists, and the like.

### USER INTERFACE / USER

Numerous types of users 128 may interact with system 100. In some embodiments, a user 128 may be human. In some embodiments, a user 128 may be non-human. In some embodiments, a user 128 may interact with one or more microfluidic chips 108, one or more reagent delivery units 116, one or more centrifugation units 118, one or more analysis units 120, one or more detection units 122, one or more display units 124, one or more user interfaces 126, or substantially any combination thereof. The user 128 can interact through use of numerous types of user interfaces 126. For example, one or more users 128 may interact through use of numerous user interfaces 126 that utilize hardwired methods, such as through use of a keyboard, use of wireless methods, use of the internet, and the like. In some embodiments, a user 128 may be a health-care worker. Examples of such health-care workers include, but are not limited to, physicians, nurses, pharmacists, and the like. In some embodiments, a user 128 may be a hiker, a farmer, a food inspector, a cook, a traveler, and the like.

### I. METHODS FOR ANALYSIS OF ONE OR MORE PATHOGENS

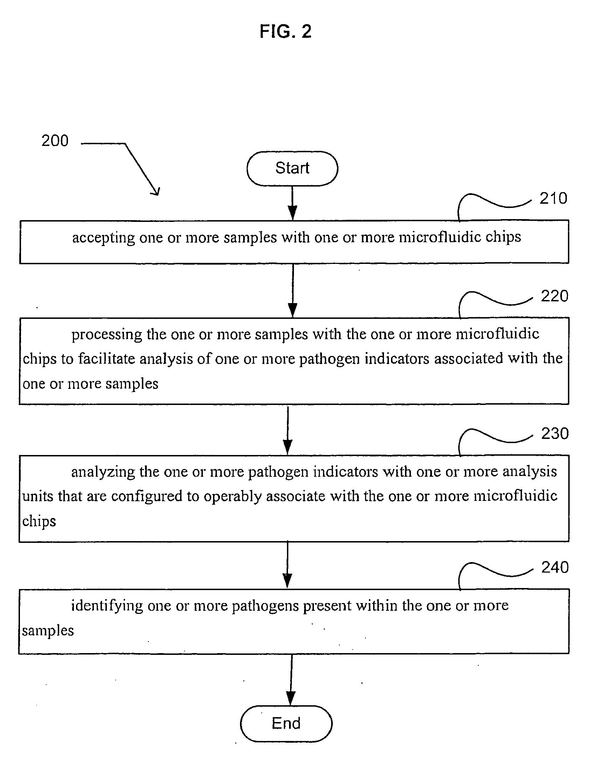

FIG. 2 illustrates an operational flow 200 representing examples of operations that are related to the performance of a method for analysis of one or more pathogens 104. In FIG. 2 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 200 includes an accepting operation 210 involving accepting one or more samples with one or more microfluidic chips. In some embodiments, accepting operation 210 may include accepting the one or more samples that include one or more liquids. In some embodiments, accepting operation 210 may include accepting the one or more samples that include one or more solids. In some embodiments, accepting operation 210 may include accepting the one or more samples that include one or more gases. In some embodiments, accepting operation 210 may include accepting the one or more samples that include one or more food products. In some embodiments, accepting operation 210 may include accepting the one or more samples that include one or more biological samples.

After a start operation, the operational flow 200 includes a processing operation 220 involving processing the one or more samples with the one or more microfluidic chips to facilitate analysis of one or more pathogen indicators associated with the one or more samples. In some embodiments, processing operation 220 may include processing the one or more samples through use of polynucleotide interaction, protein interaction, peptide interaction, antibody interaction, chemical interaction, diffusion, filtration, chromatography, aptamer interaction, electrical conductivity, isoelectric focusing, electrophoresis, immunoassay, or competition assay.

The operational flow 200 may optionally include an analyzing operation 230 involving analyzing the one or more pathogen indicators with one or more analysis units that are configured to operably associate with the one or more microfluidic chips. In some embodiments, analyzing operation 230 may include analyzing the one or more pathogen indicators with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, electrophoresis, use of a CCD camera, or immunoassay.

The operational flow 200 may optionally include an identifying operation 240 involving identifying one or more pathogens present within the one or more samples. In some embodiments, identifying operation 240 may include identifying the one or more pathogens that include at least one virus, bacterium, prion, worm, egg, cyst, protozoan, single-celled organism, fungus, algae, pathogenic protein or microbe. In some embodiments, identifying operation 240 may include displaying an identity of the one or more pathogens present within the one or more samples.

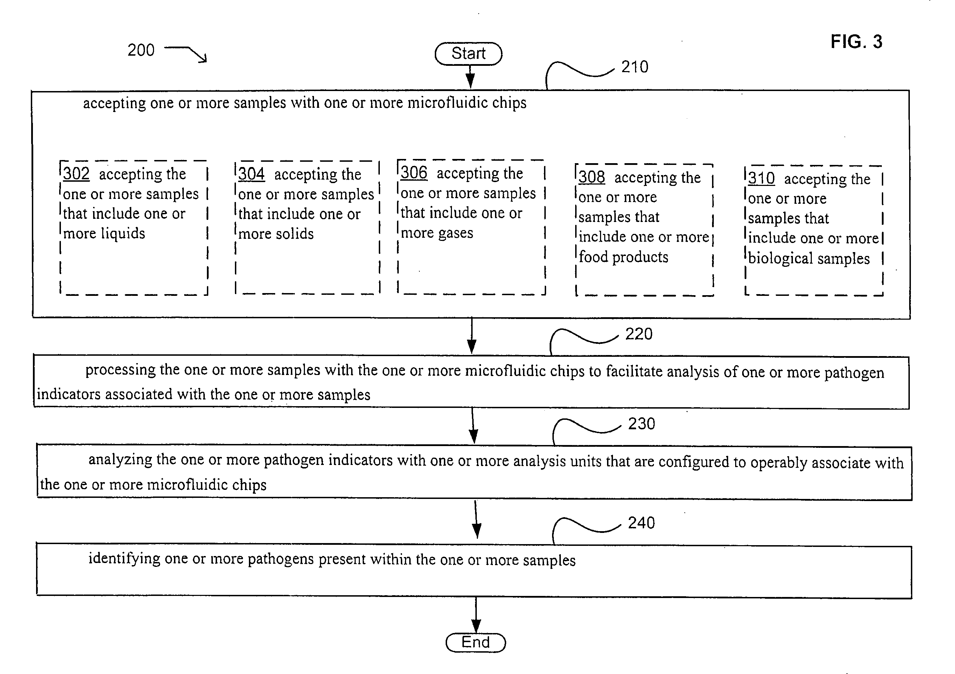

FIG. 3 illustrates alternative embodiments of the example operational flow 200 of FIG. 2. FIG. 3 illustrates example embodiments where the accepting operation 210 may include at least one additional operation. Additional operations may include an operation 302, an operation 304, an operation 306, an operation 308, and/or an operation 310.

At operation 302, the accepting operation 210 may include accepting the one or more samples that include one or more liquids. In some embodiments, one or more microfluidic chips 108 may accept one or more samples 102 that include one or more liquids. In some embodiments, one or more microfluidic chips 108 may include one or more lancets. Such lancets may be configured to provide for collection of one or more samples 102 that include a fluid. For example, in some embodiments, a lancet may be used to collect one or more samples 102 from a food product to facilitate analysis of the food product for the presence of one or more pathogens 104. In some embodiments, a microfluidic chip 108 may include one or more septa through which a needle may be passed to deliver a fluid sample 102 to the microfluidic chip 108. In some embodiments, a microfluidic chip 108 may include one or more leur lock connectors to which one or more syringes may be coupled to deliver one or more fluid samples 102 to the microfluidic chip 108. In some embodiments, a microfluidic chip 108 may be configured to operably associate with one or more devices that are configured to deliver one or more liquid samples 102 to the microfluidic chip 108. In some embodiments, a microfluidic chip 108 may include one or more sonicators that facilitate release of the liquid portion from a sample 102 to make it available to the microfluidic chip 108. Microfluidic chips 108 may be configured to accept numerous types of liquids. Examples of such liquids include, but are not limited to, beverages, water, food products, solvents, and the like. In some embodiments, microfluidic chips 108 may be configured for use by travelers to determine if a consumable item contains one or more pathogens 104. Accordingly, microfluidic chips 108 may be configured in numerous ways such that they may accept one or more samples 102 that include a liquid.

At operation 304, the accepting operation 210 may include accepting the one or more samples that include one or more solids. In some embodiments, one or more microfluidic chips 108 may accept one or more samples 102 that include one or more solids. Examples of such solid samples 102 include, but are not limited to, food products, soil samples 102, and the like. In some embodiments, microfluidic chips 108 may be configured to suspend a solid sample 102 in a fluid. In some embodiments, microfluidic chips 108 may be configured to crush a sample 102 into smaller particles. For example, in some embodiments, a microfluidic chip 108 may accept a solid sample 102. The sample 102 may be ground into smaller particles to facilitate detection of one or more pathogen indicators 106 that may be present within the sample 102. In some embodiments, a microfluidic chip 108 may include one or more sonicators that break the sample 102 into smaller particles to facilitate detection of one or more pathogen indicators 106 that may be present within the sample 102. For example, in some embodiments, viral particles may be broken into smaller particles to provide for detection of one or more polynucleotides that are associated with the viral particles. Accordingly, microfluidic chips 108 may be configured in numerous ways such that they may accept one or more samples 102 that include a solid.

At operation 306, the accepting operation 210 may include accepting the one or more samples that include one or more gases. In some embodiments, one or more microfluidic chips 108 may accept one or more samples 102 that include one or more gases. For example, in some embodiments, a microfluidic chip 108 may include one or more fans that blow and/or draw gas into the microfluidic chip 108. In some embodiments, a microfluidic chip 108 may include one or more bubble chambers through which one or more gases pass. In some embodiments, such bubble chambers may be configured to include one or more fluids (e.g., solvents) that may be used to selectively retain (e.g., extract) one or more pathogen indicators 106 from one or more gas samples 102. In some embodiments, a microfluidic chip 108 may include one or more electrostatic filters through which one or more gases pass. Such electrostatic filters (e.g., air ionizers) may be configured to capture numerous types of pathogen indicators 106. In some embodiments, a microfluidic chip 108 may include one or more filters through which one or more gases pass. In some embodiments, such microfluidic chips 108 may be used to detect and/or identify airborne pathogens 104, such as viruses, spores, and the like.

At operation 308, the accepting operation 210 may include accepting the one or more samples that include one or more food products. In some embodiments, one or more microfluidic chips 108 may accept one or more samples 102 that include one or more food products. For example, in some embodiments, one or more microfluidic chips 108 may include one or more lancets that may be inserted into the food product to withdraw one or more samples 102. In some embodiments, one or more microfluidic chips 108 may include one or more septa that may be configured to operably associate with a syringe or the like. In some embodiments, one or more microfluidic chips 108 may be configured to accept one or more food samples 102 that are solids, such as meats, cheeses, nuts, vegetables, fruits, and the like, and/or liquids, such as water, juice, milk, and the like. In some embodiments, one or more microfluidic chips 108 may include one or more mechanisms that can facilitate processing of the one or more samples 102. Examples of such mechanisms include, but are not limited to, grinders, sonicators, treatment of the one or more samples 102 with degredative enzymes (e.g., protease, nuclease, lipase, collagenase, and the like), strainers, filters, centrifugation chambers, and the like. Accordingly, such microfluidic chips 108 may be used to detect one or more pathogen indicators 106 in one or more food products. Examples of such pathogen indicators 106 include, but are not limited to: Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

At operation 310, the accepting operation 210 may include accepting the one or more samples that include one or more biological samples. In some embodiments, one or more microfluidic chips 108 may accept one or more samples 102 that include one or more biological samples 102. Examples of biological samples 102 include, but are not limited to, blood, cerebrospinal fluid, mucus, breath, urine, fecal material, skin, tissue, tears, hair, and the like.

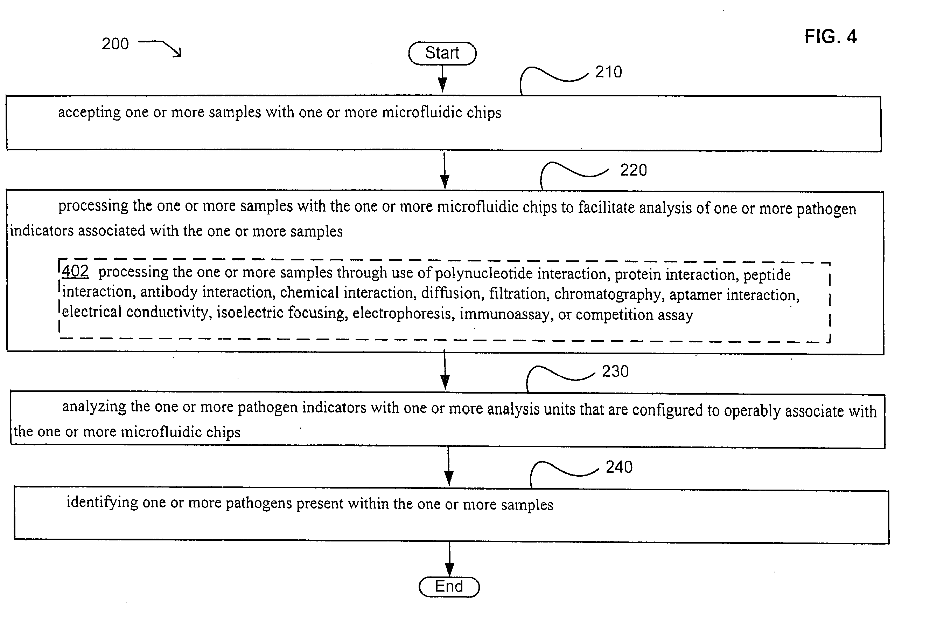

FIG. 4 illustrates alternative embodiments of the example operational flow 200 of FIG. 2. FIG. 4 illustrates example embodiments where the processing operation 220 may include at least one additional operation. Additional operations may include an operation 402.

At operation 402, the processing operation 220 may include processing the one or more samples through use of polynucleotide interaction, protein interaction, peptide interaction, antibody interaction, chemical interaction, diffusion, filtration, chromatography, aptamer interaction, electrical conductivity, isoelectric focusing, electrophoresis, immunoassay, or competition assay. In some embodiments, one or more microfluidic chips 108 may process one or more pathogen indicators 106 through use of polynucleotide interaction, protein interaction, peptide interaction, antibody interaction, chemical interaction, diffusion, filtration, chromatography, aptamer interaction, electrical conductivity, isoelectric focusing, electrophoresis, immunoassay, competition assay, or substantially any combination thereof.

In some embodiments, one or more microfluidic chips 108 may process one or more samples 102 through use of polynucleotide interaction. Numerous methods based on polynucleotide interaction may be used. Examples of such methods include, but are not limited to, those based on polynucleotide hybridization, polynucleotide ligation, polynucleotide amplification, polynucleotide degradation, and the like. Methods that utilize intercalation dyes, FRET analysis, capacitive DNA detection, and nucleic acid amplification have been described (e.g., U.S. Patent Nos.: 7,118,910 and 6,960,437; herein incorporated by reference). In some embodiments, fluorescence resonance energy transfer, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube are combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Methods to utilize carbon nanotubes as probes for nucleic acid interaction have been described (e.g., U.S. Patent No.: 6,821,730; herein incorporated by reference).

In some embodiments, one or more microfluidic chips 108 may be configured to process one or more samples 102 through use of protein interaction. Numerous methods based on protein interaction may be used. In some embodiments, protein interaction may be used to immobilize one or more pathogen indicators 106. In some embodiments, protein interaction may be used to separate one or more pathogen indicators 106 from one or more samples 102. Examples of such methods include, but are not limited to, those based on ligand binding, protein-protein binding, protein cross-linking, use of green fluorescent protein, phage display, the two-hybrid system, protein arrays, fiber optic evanescent wave sensors, chromatographic techniques, fluorescence resonance energy transfer, regulation of pH to control protein assembly and/or oligomerization, and the like. Methods that may be used to construct protein arrays have been described (e.g., Warren et al., Anal. Chem., 76:4082-4092 (2004) and Walter et al., Trends Mol. Med., 8:250-253 (2002), U.S. Patent No.: 6,780,582; herein incorporated by reference).

In some embodiments, one or more microfluidic chips 108 may process one or more samples 102 through use of peptide interaction. Peptides are generally described as being polypeptides that include less than one hundred amino acids. For example, peptides include dipeptides, tripeptides, and the like. In some embodiments, peptides may include from two to one hundred amino acids. In some embodiments, peptides may include from two to fifty amino acids. In some embodiments, peptides may include from two to one twenty amino acids. In some embodiments, peptides may include from ten to one hundred amino acids. In some embodiments, peptides may include from ten to fifty amino acids. Accordingly, peptides can include numerous numbers of amino acids. Numerous methods based on peptide interaction may be used. In some embodiments, peptide interaction may be used to immobilize one or more pathogen indicators 106. In some embodiments, peptide interaction may be used to separate one or more pathogen indicators 106 from one or more samples 102. Examples of such methods include, but are not limited to, those based on ligand binding, peptide-protein binding, peptide-peptide binding, peptide-polynucleotide binding, peptide cross-linking, use of a fluorescent protein, phage display, the two-hybrid system, protein arrays, peptide arrays, fiber optic evanescent wave sensors, chromatographic techniques, fluorescence resonance energy transfer, regulation of pH to control peptide and/or protein assembly and/or oligomerization, and the like. Accordingly, virtually any technique that may be used to analyze proteins may be utilized for the analysis of peptides. In some embodiments, high-speed capillary electrophoresis may be used to detect one or more pathogen indicators 106 through use of fluorescently labeled phosphopeptides as affinity probes (Yang et al., Anal. Chem., 10.1021/ac061936e (2006)). Methods to immobilize proteins and peptides have been reported (Taylor, Protein Immobilization: Fundamentals and Applications, Marcel Dekker, Inc., New York (1991)).

In some embodiments, one or more microfluidic chips 108 may be configured to process one or more samples 102 through use of antibody interaction. Antibodies may be raised that will bind to numerous pathogen indicators 106 through use of known methods (e.g., Harlow and Lane, Antibodies: A Laboratory Manual, Cold Spring Harbor Laboratory Press, Cold Spring Harbor, New York (1988)). Antibodies may be configured in numerous ways within one or more microfluidic chips 108 to process one or more pathogen indicators 106. For example, in some embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. A labeled detector antibody that binds to the pathogen indicator 106 (or the antibody-pathogen indicator 106 complex) may then be passed over the one or more antibody-pathogen indicator 106 complexes such that the labeled detector antibody will label the pathogen indicator 106 (or the antibody-pathogen indicator 106 complex). Numerous labels may be used that include, but are not limited to, enzymes, fluorescent molecules, radioactive labels, spin labels, redox labels, and the like. In other embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. Such binding provides for detection of the antibody-pathogen indicator 106 complex through use of methods that include, but are not limited to, surface plasmon resonance, conductivity, and the like (e.g., U.S. Patent No.: 7,030,989; herein incorporated by reference). In some embodiments, antibodies may be coupled to a substrate within a microfluidic chip 108 to provide for a competition assay. One or more samples 102 may be mixed with one or more reagent mixtures that include one or more labeled pathogen indicators 106. The mixture may then be passed over the antibodies to facilitate binding of pathogen indicators 106 in the sample 102 and labeled pathogen indicators 106 in the reagent mixture to the antibodies. The unlabeled pathogen indicators 106 in the sample 102 will compete with the labeled pathogen indicators 106 in the reagent mixture for binding to the antibodies. Accordingly, the amount of label bound to the antibodies will vary in accordance with the concentration of unlabeled pathogen indicator 106 in the sample 102. In some embodiments, antibody interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more antibodies may be used in conjunction with one or more aptamers to process one or more samples 102. Accordingly, in some embodiments, aptamers and antibodies may be used interchangeably to process one or more samples 102.

In some embodiments, one or more microfluidic chips 108 may be configured to process one or more samples 102 through use of chemical interaction. In some embodiments, one or more microfluidic chips 108 may be configured to utilize chemical extraction to process one or more samples 102. For example, in some embodiments, one or more samples 102 may be mixed with a reagent mixture that includes one or more solvents in which the one or more pathogen indicators 106 are soluble. Accordingly, the solvent phase containing the one or more pathogen indicators 106 may be separated from the sample phase to provide for detection of the one or more pathogen indicators 106. In some embodiments, one or more samples 102 may be mixed with a reagent mixture that includes one or more chemicals that cause precipitation of one or more pathogen indicators 106. Accordingly, the sample phase may be washed away from the one or more precipitated pathogen indicators 106 to provide for detection of the one or more pathogen indicators 106. Accordingly, reagent mixtures that include numerous types of chemicals that interact with one or more pathogen indicators 106 may be used.

In some embodiments, one or more microfluidic chips 108 may be configured to process one or more samples 102 through use of diffusion. In some embodiments, one or more microfluidic chips 108 may be configured to process one or more fluid samples 102 through use of an H-filter. For example, a microfluidic chip 108 may be configured to include a channel through which a fluid sample 102 and a second fluid flow such that the fluid sample 102 and the second fluid undergo substantially parallel flow through the channel without significant mixing of the sample fluid and the second fluid. As the fluid sample 102 and the second fluid flow through the channel, one or more pathogen indicators 106 in the fluid sample 102 may diffuse through the fluid sample 102 into the second fluid. Accordingly, such diffusion provides for the separation of the one or more pathogen indicators 106 from the sample 102. Methods to construct H-filters have been described (e.g., U.S. Patent Nos.: 6,742,661; 6,409,832; 6,007,775; 5,974,867; 5,971,158; 5,948,684; 5,932,100; 5,716,852; herein incorporated by reference). In some embodiments, diffusion based methods may be combined with immunoassay based methods to process and detect one or more pathogen indicators 106. Methods to conduct microscale diffusion immunoassays have been described (e.g., U.S. Patent No.: 6,541,213; herein incorporated by reference). Accordingly, micro fluidic chips 108 may be configured in numerous ways to process one or more pathogen indicators 106 through use of diffusion.

In some embodiments, one or more microfluidic chips 108 may be configured to process one or more samples 102 through use of filtration. In some embodiments, one or more microfluidic chips 108 may be configured to include one or more filters that have a molecular weight cut-off. For example, a filter may allow molecules of low molecular weight to pass through the filter while disallowing molecules of high molecular weight to pass through the filter. Accordingly, one or more pathogen indicators 106 that are contained within a sample 102 may be allowed to pass through a filter while larger molecules contained within the sample 102 are disallowed from passing through the filter. Accordingly, in some embodiments, a microfluidic chip 108 may include two or more filters that selectively retain, or allow passage, of one or more pathogen indicators 106 through the filters. Such configurations provide for selective separation of one or more pathogen indicators 106 from one or more samples 102. Membranes and filters having numerous molecular weight cut-offs are commercially available (e.g., Millipore, Billerica, MA). In some embodiments, one or more microfluidic chips 108 may be configured to provide for dialysis of one or more samples 102. For example, in some embodiments, a microfluidic chip 108 may be configured to contain one or more samples 102 in one or more sample chambers that are separated from one or more dialysis chambers by a semi-permeable membrane. Accordingly, in some embodiments, one or more pathogen indicators 106 that are able to pass through the semi-permeable membrane may be collected in the dialysis chamber. In other embodiments, one or more pathogen indicators 106 may be retained in the one or more sample chambers while other sample 102 components may be separated from the one or more pathogen indicators 106 by their passage through the semi-permeable membrane into the dialysis chamber. Accordingly, one or more microfluidic chips 108 may be configured to include two or more dialysis chambers for selective separation of one or more pathogen indicators 106 from one or more samples 102. Semi-permeable membranes and dialysis tubing is available from numerous commercial sources (e.g., Millipore, Billerica, MA; Pierce, Rockford, IL; Sigma-Aldrich, St. Louis, MO). Methods that may be used for microfiltration have been described (e.g., U.S. Patent No.: 5,922,210; herein incorporated by reference).

In some embodiments, one or more microfluidic chips 108 may be configured to process one or more samples 102 through use of chromatography. Numerous chromatographic methods may be used to process one or more samples 102. Examples of such chromatographic methods include, but are not limited to, ionexchange chromatography, affinity chromatography, gel filtration chromatography, hydroxyapatite chromatography, gas chromatography, reverse phase chromatography, thin layer chromatography, capillary chromatography, size exclusion chromatography, hydrophobic interaction media, and the like. In some embodiments, a microfluidic chip 108 may be configured to process one or more samples 102 through use of one or more chromatographic methods. In some embodiments, chromatographic methods may be used to process one or more samples 102 for one or more pathogen indicators 106 that include one or more polynucleotides. For example, in some embodiments, one or more samples 102 may be applied to a chromatographic media to which the one or more polynucleotides bind. The remaining components of the sample 102 may be washed from the chromatographic media. The one or more polynucleotides may then be eluted from chromatographic media in a more purified state. Similar methods may be used to process one or more samples 102 for one or more pathogen indicators 106 that include one or more proteins or polypeptides (e.g., Mondal and Gupta, Biomol. Eng., 23:59-76 (2006)). Chromatography media able to separate numerous types of molecules is commercially available (e.g., Bio-Rad, Hercules, CA; Qiagen, Valencia, CA; Pfizer, New York, NY; Millipore, Billerica, MA; GE Healthcare BioSciences Corp., Piscataway, NJ).

In some embodiments, one or more microfluidic chips 108 may be configured to process one or more samples 102 through use of aptamer interaction. In some embodiments, one or more aptamers may include polynucleotides (e.g., deoxyribonucleic acid; ribonucleic acid; and derivatives of polynucleotides that may include polynucleotides that include modified bases, polynucleotides in which the phosphodiester bond is replaced by a different type of bond, or many other types of modified polynucleotides). In some embodiments, one or more aptamers may include peptide aptamers. Methods to prepare and use aptamers have been described (e.g., Collett et al., Methods, 37:4-15 (2005); Collet et al., Anal. Biochem., 338:113-123 (2005); Cox et al., Nucleic Acids Res., 30:20 e108 (2002); Kirby et al., Anal. Chem., 76:4066-4075 (2004); Ulrich, Handb. Exp. Pharmacol., 173:305-326 (2006); Baines and Colas, Drug Discovery Today, 11:334-341 (2006); Guthrie et al., Methods, 38:324-330 (2006); Geyer et al., Chapter 13: Selection of Genetic Agents from Random Peptide Aptamer Expression Libraries, Methods in Enzymology, Academic Press, pg. 171-208 (2000); U.S. Patent No.: 6,569,630; herein incorporated by reference). Aptamers may be configured in numerous ways within one or more micro fluidic chips 108 to process one or more pathogen indicators 106. For example, in some embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Labeled detector antibodies and/or aptamers that bind to the pathogen indicator 106 (or the aptamer-pathogen indicator 106 complex) may then be passed over the one or more aptamer-pathogen indicator 106 complexes such that the labeled detector antibodies and/or aptamers will label the pathogen indicator 106 (or the aptamer-pathogen indicator 106 complex). Numerous labels may be used that include, but are not limited to, enzymes, fluorescent molecules, radioactive labels, spin labels, redox labels, and the like. In other embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Such binding provides for detection of the aptamer-pathogen indicator 106 complex through use of methods that include, but are not limited to, surface plasmon resonance, conductivity, and the like (e.g., U.S. Patent No.: 7,030,989; herein incorporated by reference). In some embodiments, aptamers may be coupled to a substrate within a microfluidic chip 108 to provide for a competition assay. One or more samples 102 may be mixed with one or more reagent mixtures that include one or more labeled pathogen indicators 106. The mixture may then be passed over the aptamers to facilitate binding of pathogen indicators 106 in the sample 102 and labeled pathogen indicators 106 in the reagent mixture to the aptamers. The unlabeled pathogen indicators 106 in the sample 102 will compete with the labeled pathogen indicators 106 in the reagent mixture for binding to the aptamers. Accordingly, the amount of label bound to the aptamers will vary in accordance with the concentration of unlabeled pathogen indicators 106 in the sample 102. In some embodiments, aptamer interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more aptamers may be used in conjunction with one or more antibodies to process one or more samples 102. In some embodiments, aptamers and antibodies may be used interchangeably to process one or more samples 102. Accordingly, in some embodiments, methods and/or systems for processing and/or detecting pathogen indicators 106 may utilize antibodies and aptamers interchangeably and/or in combination.

In some embodiments, one or more microfluidic chips 108 may be configured to process one or more samples 102 through use of electrical conductivity. In some embodiments, one or more samples 102 may be processed through use of magnetism. For example, in some embodiments, one or more samples 102 may be combined with one or more tagged polynucleotides that are tagged with a ferrous material, such as a ferrous bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed over an electromagnet to immobilize the hybridized complexes. Other components in the sample 102 may then be washed away from the hybridized complexes. In some embodiments, a chamber containing the magnetically immobilized hybridized complexes may be heated to release the sample polynucleotides from the magnetically immobilized tagged polynucleotides. The sample polynucleotides may then be collected in a more purified state. In other embodiments, similar methods may be used in conjunction with antibodies, aptamers, peptides, ligands, and the like. Accordingly, one or more microfluidic chips 108 may be configured in numerous ways to utilize magnetism to process one or more samples 102. In some embodiments, one or more samples 102 may be processed through use of eddy currents. Eddy current separation uses electromagnetic induction in conducting materials to separate non-ferrous metals by their different electric conductivities. An electrical charge is induced into a conductor by changes in magnetic flux cutting through it. Moving permanent magnets passing a conductor generates the change in magnetic flux. Accordingly, in some embodiments, one or more microfluidic chips 108 may be configured to include a magnetic rotor such that when conducting particles move through the changing flux of the magnetic rotor, a spiraling current and resulting magnetic field are induced. The magnetic field of the conducting particles may interact with the magnetic field of the magnetic rotor to impart kinetic energy to the conducting particles. The kinetic energy imparted to the conducting particles may then be used to direct movement of the conducting particles. Accordingly, non-ferrous particles, such as metallic beads, may be utilized to process one or more samples 102. For example, in some embodiments, one or more samples 102 may be combined with one or more tagged polynucleotides that are tagged with a non-ferrous material, such as an aluminum bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed through a magnetic field to impart kinetic energy to the non-ferrous bead. This kinetic energy may then be used to separate the hybridized complex. In other embodiments, similar methods may be used in conjunction with antibodies, aptamers, peptides, ligands, and the like. Accordingly, one or more microfluidic chips 108 may be configured in numerous ways to utilize eddy currents to process one or more samples 102. One or more microfluidic chips 108 may be configured in numerous ways to utilize electrical conductivity to process one or more samples 102.

In some embodiments, one or more microfluidic chips 108 may be configured to process one or more samples 102 through use of isoelectric focusing. Methods have been described that may be used to construct capillary isoelectric focusing systems (e.g., Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. PatentNos.: 7,150,813; 7,070,682; 6,730,516; herein incorporated by reference). Such systems may be modified to provide for the processing of one or more samples 102.

In some embodiments, one or more microfluidic chips 108 may be configured to process one or more samples 102 through use of electrophoresis. In some embodiments, one or more microfluidic chips 108 may be configured to process one or more samples 102 through use of one-dimensional electrophoresis. In some embodiments, one or more microfluidic chips 108 may be configured to process one or more samples 102 through use of two-dimensional electrophoresis. In some embodiments, one or more microfluidic chips 108 may be configured to process one or more samples 102 through use of gradient gel electrophoresis. In some embodiments, one or more microfluidic chips 108 may be configured to process one or more samples 102 through use of electrophoresis under denaturing conditions. In some embodiments, one or more microfluidic chips 108 may be configured to process one or more samples 102 through use of electrophoresis under native conditions. One or more microfluidic chips 108 may be configured to utilize numerous electrophoretic methods.

In some embodiments, one or more microfluidic chips 108 may be configured to process one or more samples 102 through use of immunoassay. In some embodiments, one or more microfluidic chips 108 may be configured to process one or more samples 102 through use of enzyme linked immunosorbant assay (ELISA). In some embodiments, one or more microfluidic chips 108 may be configured to process one or more samples 102 through use of radioimmuno assay (RIA). In some embodiments, one or more microfluidic chips 108 may be configured to process one or more samples 102 through use of enzyme immunoassay (EIA). In some embodiments, such methods may utilize antibodies (e.g., monoclonal antibodies, polyclonal antibodies, antibody fragments, single-chain antibodies, and the like), aptamers, or substantially any combination thereof. In some embodiments, a labeled antibody and/or aptamer may be used within an immunoassay. Numerous types of labels may be utilized in association with immunoassays. Examples of such labels include, but are not limited to, radioactive labels, fluorescent labels, enzyme labels, spin labels, magnetic labels, gold labels, colorimetric labels, redox labels, and the like. Numerous immunoassays are known and may be configured for processing one or more samples 102.

In some embodiments, one or more microfluidic chips 108 may be configured to process one or more samples 102 through use of one or more competition assays. In some embodiments, one or more microfluidic chips 108 may be configured to process one or more samples 102 through use of one or more polynucleotide based competition assays. One or more microfluidic chips 108 may be configured to include one or more polynucleotides coupled to a substrate, such as a polynucleotide array. The one or more microfluidic chips 108 may be further configured so that a sample 102 and/or substantially purified polynucleotides obtained from one or more samples 102, may be mixed with one or more reagent mixtures that include one or more labeled polynucleotides to form an analysis mixture. This analysis mixture is then passed over the substrate such that the labeled polynucleotides and the sample polynucleotides are allowed to hybridize to the polynucleotides that are immobilized on the substrate. The sample polynucleotides and the labeled polynucleotides will compete for binding to the polynucleotides that are coupled on the substrate. Accordingly, the presence and/or concentration of the polynucleotides in the sample 102 can be determined through detection of the label (e.g., the concentration of the polynucleotides in the sample 102 will be inversely related to the amount of label that is bound to the substrate). Numerous labels may be used that include, but are not limited to, enzymes, fluorescent molecules, radioactive labels, spin labels, redox labels, and the like. In some embodiments, one or more microfluidic chips 108 may be configured to include one or more antibodies, proteins, peptides, and/or aptamers that are coupled to a substrate. The one or more microfluidic chips 108 may be further configured so that a sample 102 and/or substantially purified sample polypeptides and/or sample peptides obtained from one or more samples 102, may be mixed with one or more reagent mixtures that include one or more labeled polypeptides and/or labeled peptides to form an analysis mixture. This analysis mixture can then be passed over the substrate such that the labeled polypeptides and/or labeled peptides and the sample polypeptides and/or sample peptides are allowed to bind to the antibodies, proteins, peptides, and/or aptamers that are immobilized on the substrate. The sample polypeptides and/or sample peptides and the labeled polypeptides and/or sample peptides will compete for binding to the antibodies, proteins, peptides, and/or aptamers that are coupled on the substrate. Accordingly, the presence and/or concentration of the sample polypeptides and/or sample peptides in the sample 102 can be determined through detection of the label (e.g., the concentration of the sample polypeptides and/or sample peptides in the sample 102 will be inversely related to the amount of label that is bound to the substrate). Numerous labels may be used that include, but are not limited to, enzymes, fluorescent molecules, radioactive labels, spin labels, redox labels, and the like. Microfluidic chips 108 may be configured to utilize numerous types of competition assays.

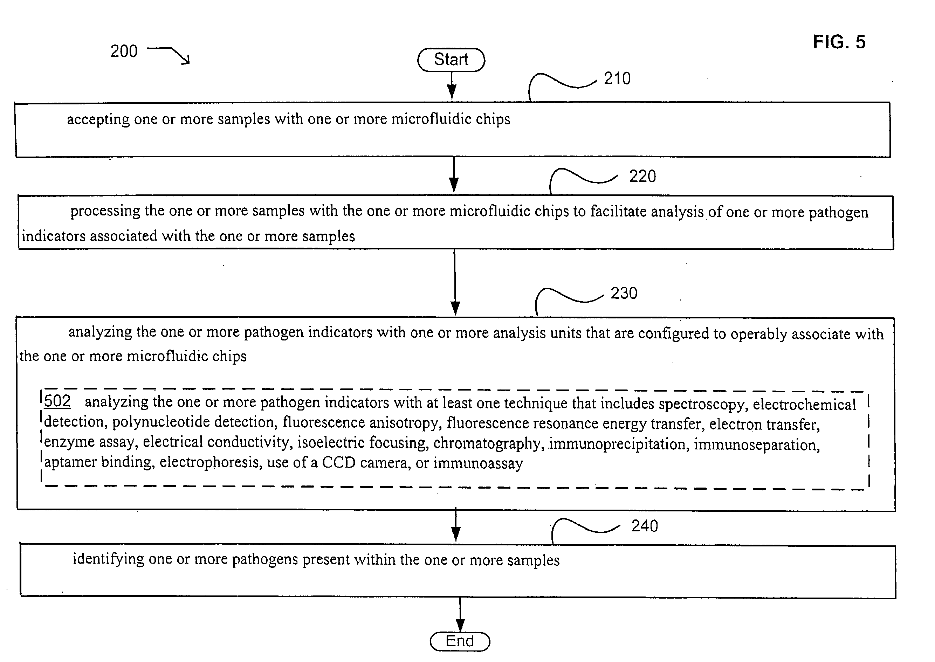

FIG. 5 illustrates alternative embodiments of the example operational flow 200 of FIG. 2. FIG. 5 illustrates example embodiments where the optional analyzing operation 230 may include at least one additional operation. Additional operations may include an operation 502.

At operation 502, the analyzing operation 230 may include analyzing the one or more pathogen indicators with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens 104 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a protozoan, a single-celled organism, a fungus, an algae, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present within the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units 120 and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102 that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

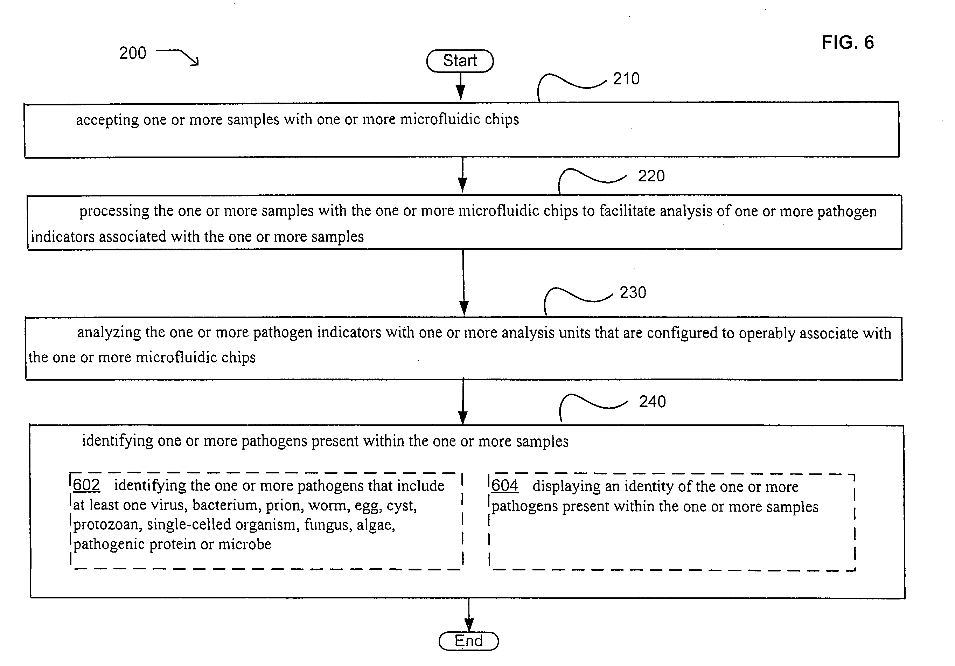

FIG. 6 illustrates alternative embodiments of the example operational flow 200 of FIG. 2. FIG. 6 illustrates example embodiments where the optional identifying operation 240 may include at least one additional operation. Additional operations may include an operation 602, and/or an operation 604.

At operation 602, the identifying operation 240 may include identifying the one or more pathogens that include at least one virus, bacterium, prion, worm, egg, cyst, protozoan, single-celled organism, fungus, algae, pathogenic protein or microbe. In some embodiments, one or more display units 124 may indicate an identity of one or more pathogens that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 604, the identifying operation 240 may include displaying an identity of the one or more pathogens present within the one or more samples. In some embodiments, one or more display units 124 may indicate an identity of one or more pathogens 104 that correspond to the one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples may be biological samples 102. Examples of such biological samples 102 include, but are not limited to, blood samples 102, fecal samples 102, urine samples 102, and the like.

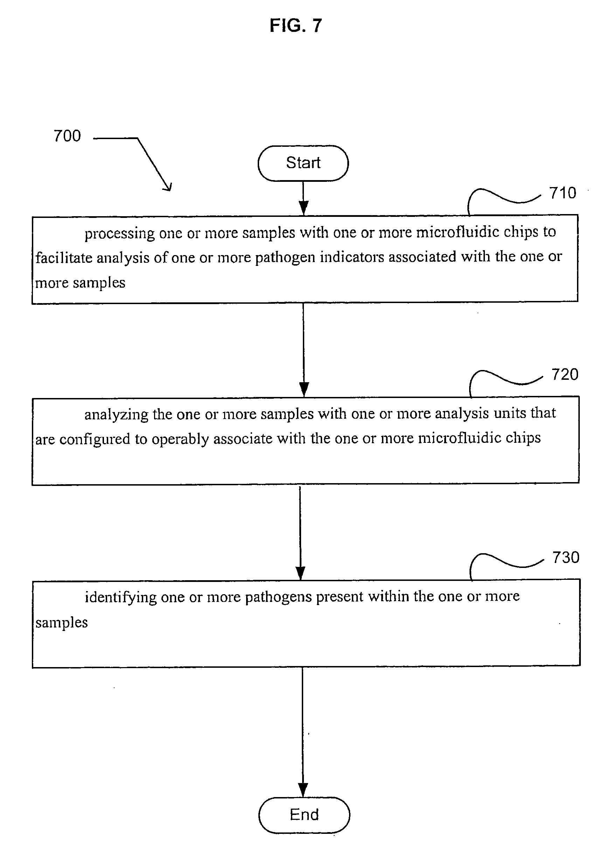

FIG. 7 illustrates an operational flow 700 representing examples of operations that are related to the performance of a method for analysis of one or more pathogens 104. In FIG. 7 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 700 includes a processing operation 710 involving processing one or more samples with one or more microfluidic chips to facilitate analysis of one or more pathogen indicators associated with the one or more samples. In some embodiments, processing operation 710 may include processing the one or more samples through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay.

After a start operation, the operational flow 700 includes an analyzing operation 720 involving analyzing the one or more samples with one or more analysis units that are configured to operably associate with the one or more microfluidic chips. In some embodiments, analyzing operation 720 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 700 may optionally include an identifying operation 730 involving identifying one or more pathogens present within the one or more samples. In some embodiments, identifying operation 730 may include identifying the one or more pathogens that include at least one virus, bacterium, prion, worm, egg, cyst, protozoan, single-celled organism, fungus, algae, pathogenic protein, or microbe. In some embodiments, identifying operation 730 may include displaying an identity of the one or more pathogens present within the one or more samples.

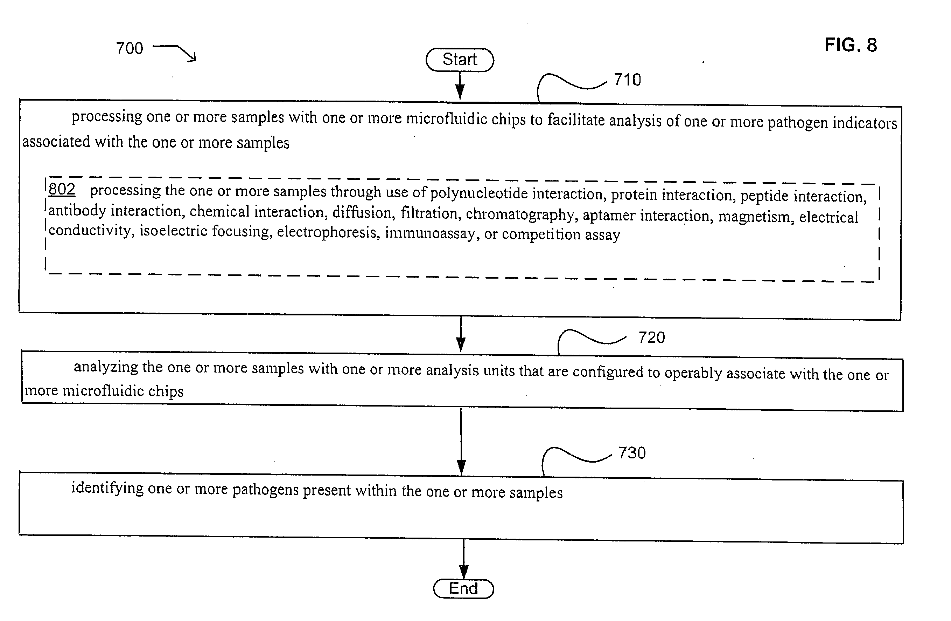

FIG. 8 illustrates alternative embodiments of the example operational flow 700 of FIG. 7. FIG. 8 illustrates example embodiments where the processing operation 710 may include at least one additional operation. Additional operations may include an operation 802.

At operation 802, the processing operation 710 may include processing the one or more samples through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay. In some embodiments, one or more samples 102 may be processed with one or more microfluidic chips 108 that are configured for processing the one or more pathogen indicators 106 through use of polynucleotide interaction, protein interaction, peptide interaction, antibody interaction, chemical interaction, diffusion, filtration, chromatography, aptamer interaction, electrical conductivity, isoelectric focusing, electrophoresis, immunoassay, competition assay, or substantially any combination thereof. In some embodiments, pathogen indicators 106 may be separated from other materials included within one or more samples 102 through processing. In some embodiments, pathogen indicators 106 may be immobilized through one or more processing procedures to facilitate analysis, detection and/or identification of the one or more pathogen indicators 106.

In some embodiments, one or more microfluidic chips 108 may be configured to process one or more samples 102 through use of polynucleotide interaction. Numerous methods based on polynucleotide interaction may be used. Examples of such methods include, but are not limited to, those based on polynucleotide hybridization, polynucleotide ligation, polynucleotide amplification, polynucleotide degradation, and the like. Methods that utilize intercalation dyes, FRET analysis, capacitive DNA detection, and nucleic acid amplification have been described (e.g., U.S. Patent Nos.: 7,118,910 and 6,960,437; herein incorporated by reference). In some embodiments, fluorescence resonance energy transfer, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube are combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Methods to utilize carbon nanotubes as probes for nucleic acid interaction have been described (e.g., U.S. Patent No.: 6,821,730; herein incorporated by reference). In some embodiments, one or more microfluidic chips 108 may be configured to process one or more samples 102 through use of protein interaction. Numerous methods based on protein interaction may be used. In some embodiments, protein interaction may be used to immobilize one or more pathogen indicators 106. In some embodiments, protein interaction may be used to separate one or more pathogen indicators 106 from one or more samples 102. Examples of such methods include, but are not limited to, those based on ligand binding, protein-protein binding, protein cross-linking, use of green fluorescent protein, phage display, the two-hybrid system, protein arrays, fiber optic evanescent wave sensors, chromatographic techniques, fluorescence resonance energy transfer, regulation of pH to control protein assembly and/or oligomerization, and the like. Methods that may be used to construct protein arrays have been described (e.g., Warren et al., Anal. Chem., 76:4082-4092 (2004) and Walter et al., Trends Mol. Med., 8:250-253 (2002), U.S. Patent No.: 6,780,582; herein incorporated by reference).

In some embodiments, one or more microfluidic chips 108 may be configured to process one or more samples 102 through use of peptide interaction. Peptides are generally described as being polypeptides that include less than one hundred amino acids. For example, peptides include dipeptides, tripeptides, and the like. In some embodiments, peptides may include from two to one hundred amino acids. In some embodiments, peptides may include from two to fifty amino acids. In some embodiments, peptides may include from two to one twenty amino acids. In some embodiments, peptides may include from ten to one hundred amino acids. In some embodiments, peptides may include from ten to fifty amino acids. Accordingly, peptides can include numerous numbers of amino acids. Numerous methods based on peptide interaction may be used. In some embodiments, peptide interaction may be used to immobilize one or more pathogen indicators 106. In some embodiments, peptide interaction may be used to separate one or more pathogen indicators 106 from one or more samples 102. Examples of such methods include, but are not limited to, those based on ligand binding, peptide-protein binding, peptide-peptide binding, peptide-polynucleotide binding, peptide cross-linking, use of green fluorescent protein, phage display, the two-hybrid system, protein arrays, peptide arrays, fiber optic evanescent wave sensors, chromatographic techniques, fluorescence resonance energy transfer, regulation of pH to control peptide and/or protein assembly and/or oligomerization, and the like. In some embodiments, one or more samples 102 may be treated with one or more proteases and/or chemical agents to cleave polypeptides within the one or more samples 102 to produce pathogen associated peptides that may be analyzed and/or detected. Accordingly, nearly any technique that may be used to analyze proteins may be utilized for the analysis of peptides. In some embodiments, high-speed capillary electrophoresis may be used to detect binding through use of fluorescently labeled phosphopeptides as affinity probes (Yang et al., Anal. Chem., 10.1021/ac061936e (2006)). Methods to immobilize proteins and peptides have been reported (Taylor, Protein Immobilization: Fundamentals and Applications, Marcel Dekker, Inc., New York (1991)).

In some embodiments, one or more microfluidic chips 108 may be configured to process one or more samples 102 through use of antibody interaction. Antibodies may be raised that will bind to numerous pathogen indicators 106 through use of known methods (e.g., Harlow and Lane, Antibodies: A Laboratory Manual, Cold Spring Harbor Laboratory Press, Cold Spring Harbor, New York (1988)). Antibodies may be configured in numerous ways within one or more microfluidic chips 108 to process one or more pathogen indicators 106. For example, in some embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. One or more labeled detector antibodies that bind to the pathogen indicator 106 (or the antibody-pathogen indicator 106 complex) may then be passed over the one or more antibody-pathogen indicator 106 complexes such that the one or more labeled detector antibodies will label the pathogen indicator 106 (or the antibody-pathogen indicator 106 complex). Numerous labels may be used that include, but are not limited to, enzymes, fluorescent molecules (e.g., quantum dots), radioactive labels, spin labels, redox labels, and the like. In other embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. Such binding provides for detection of the antibody-pathogen indicator 106 complex through use of methods that include, but are not limited to, surface plasmon resonance, conductivity, and the like (e.g., U.S. Patent No.: 7,030,989; herein incorporated by reference). In some embodiments, antibodies may be coupled to a substrate within a microfluidic chip 108 to provide for a competition assay. One or more samples 102 may be mixed with one or more reagent mixtures that include one or more labeled pathogen indicators 106. The mixture may then be passed over the antibodies to facilitate binding of pathogen indicators 106 in the sample 102 and labeled pathogen indicators 106 in the reagent mixture to the antibodies. The unlabeled pathogen indicators 106 in the sample 102 will compete with the labeled pathogen indicators 106 in the reagent mixture for binding to the antibodies. Accordingly, the amount of label bound to the antibodies will vary in accordance with the concentration of unlabeled pathogen indicators 106 in the sample 102. In some embodiments, antibody interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more antibodies may be used in conjunction with one or more aptamers to process one or more samples 102. Accordingly, in some embodiments, aptamers and antibodies may be used interchangeably to process one or more samples 102.

In some embodiments, one or more microfluidic chips 108 may be configured to process one or more samples 102 through use of chemical interaction. In some embodiments, one or more microfluidic chips 108 may be configured to utilize chemical extraction to process one or more samples 102. For example, in some embodiments, one or more samples 102 may be mixed with a reagent mixture that includes one or more solvents in which the one or more pathogen indicators 106 are soluble. Accordingly, the solvent phase containing the one or more pathogen indicators 106 may be separated from the sample phase to provide for detection of the one or more pathogen indicators 106. In some embodiments, one or more samples 102 may be mixed with a reagent mixture that includes one or more chemicals that cause precipitation of one or more pathogen indicators 106. Accordingly, the sample phase may be washed away from the one or more precipitated pathogen indicators 106 to provide for detection of the one or more pathogen indicators 106. Accordingly, reagent mixtures that include numerous types of chemicals that interact with one or more pathogen indicators 106 may be used. In some embodiments, pathogen associated polynucleotides may be extracted from one or more samples 102 through use of chemical extraction.

In some embodiments, one or more microfluidic chips 108 may be configured to process one or more samples 102 through use of diffusion. In some embodiments, one or more microfluidic chips 108 may be configured to process one or more fluid samples 102 through use of an H-filter. For example, a microfluidic chip 108 may be configured to include a channel through which a sample fluid and an extraction fluid flow such that the sample fluid and the extraction fluid undergo substantially parallel or antiparallel flow through the channel without significant mixing of the sample fluid and the extraction fluid. As the sample fluid and the extraction fluid flow through the channel, one or more pathogen indicators 106 in the sample fluid may diffuse through the sample fluid into the extraction fluid. Accordingly, such diffusion provides for the separation of the one or more pathogen indicators 106 from the sample 102. Methods to construct H-filters have been described (e.g., U.S. Patent Nos.: 6,742,661; 6,409,832; 6,007,775; 5,974,867; 5,971,158; 5,948,684; 5,932,100; 5,716,852; herein incorporated by reference). In some embodiments, diffusion based methods may be combined with immunoassay based methods to process, analyze, and/or detect one or more pathogen indicators 106. Methods to conduct microscale diffusion immunoassays have been described (e.g., U.S. Patent No.: 6,541,213; herein incorporated by reference). Accordingly, microfluidic chips 108 may be configured in numerous ways to process one or more pathogen indicators 106 through use of diffusion.

In some embodiments, one or more microfluidic chips 108 may be configured to process one or more samples 102 through use of filtration. In some embodiments, one or more microfluidic chips 108 may be configured to include one or more filters that have a molecular weight cut-off. For example, a filter may allow molecules of low molecular weight to pass through the filter while disallowing molecules of high molecular weight to pass through the filter. Accordingly, one or more pathogen indicators 106 that are contained within a sample 102 may be allowed to pass through a filter while larger molecules contained within the sample 102 are disallowed from passing through the filter. Accordingly, in some embodiments, a microfluidic chip 108 may include two or more filters that selectively retain, or allow passage, of one or more pathogen indicators 106 through the filters. Such configurations provide for selective separation of one or more pathogen indicators 106 from one or more samples 102. Membranes and filters having numerous molecular weight cut-offs are commercially available (e.g., Millipore, Billerica, MA). In some embodiments, one or more microfluidic chips 108 may be configured to provide for dialysis of one or more samples 102. For example, in some embodiments, a microfluidic chip 108 may be configured to contain one or more samples 102 in one or more sample chambers that are separated from one or more dialysis chambers by a semi-permeable membrane. Accordingly, in some embodiments, one or more pathogen indicators 106 that are able to pass through the semi-permeable membrane may be collected in the dialysis chamber. In other embodiments, one or more pathogen indicators 106 may be retained in the one or more sample chambers while other sample 102 components may be separated from the one or more pathogen indicators 106 by their passage through the semi-permeable membrane into the dialysis chamber. Accordingly, one or more microfluidic chips 108 may be configured to include two or more dialysis chambers for selective separation of one or more pathogen indicators 106 from one or more samples 102. Semi-permeable membranes and dialysis tubing is available from numerous commercial sources (e.g., Millipore, Billerica, MA; Pierce, Rockford, IL; Sigma-Aldrich, St. Louis, MO). Methods that may be used for microfiltration have been described (e.g., U.S. Patent No.: 5,922,210; herein incorporated by reference).

In some embodiments, one or more microfluidic chips 108 may be configured to process one or more samples 102 through use of chromatography. Numerous chromatographic methods may be used to process one or more samples 102. Examples of such chromatographic methods include, but are not limited to, ionexchange chromatography, affinity chromatography, gel filtration chromatography, hydroxyapatite chromatography, gas chromatography, reverse phase chromatography, thin layer chromatography, capillary chromatography, size exclusion chromatography, hydrophobic interaction media, and the like. In some embodiments, a microfluidic chip 108 may be configured to process one or more samples 102 through use of one or more chromatographic methods. In some embodiments, chromatographic methods may be used to process one or more samples 102 for one or more pathogen indicators 106 that include one or more polynucleotides. For example, in some embodiments, one or more samples 102 may be applied to a chromatographic media to which the one or more polynucleotides bind. The remaining components of the sample 102 may be washed from the chromatographic media. The one or more polynucleotides may then be eluted from chromatographic media in a more purified state. Similar methods may be used to process one or more samples 102 for one or more pathogen indicators 106 that include one or more proteins or polypeptides (e.g., Mondal and Gupta, Biomol. Eng., 23:59-76 (2006)). Chromatography media able to separate numerous types of molecules is commercially available (e.g., Bio-Rad, Hercules, CA; Qiagen, Valencia, CA; Pfizer, New York, NY; Millipore, Billerica, MA; GE Healthcare BioSciences Corp., Piscataway, NJ).

In some embodiments, one or more microfluidic chips 108 may be configured to process one or more samples 102 through use of aptamer interaction. In some embodiments, one or more aptamers may include polynucleotides (e.g., deoxyribonucleic acid; ribonucleic acid; and derivatives of polynucleotides that may include polynucleotides that include modified bases, polynucleotides in which the phosphodiester bond is replaced by a different type of bond, or many other types of modified polynucleotides). In some embodiments, one or more aptamers may include peptide aptamers. Methods to prepare and use aptamers have been described (e.g., Collett et al., Methods, 37:4-15 (2005); Collet et al., Anal. Biochem., 338:113-123 (2005); Cox et al., Nucleic Acids Res., 30:20 e108 (2002); Kirby et al., Anal. Chem., 76:4066-4075 (2004); Ulrich, Handb. Exp. Pharmacol., 173:305-326 (2006); Baines and Colas, Drug Discovery Today, 11:334-341 (2006); Guthrie et al., Methods, 38:324-330 (2006); Geyer et al., Chapter 13: Selection of Genetic Agents from Random Peptide Aptamer Expression Libraries, Methods in Enzymology, Academic Press, pg. 171-208 (2000); U.S. Patent No.: 6,569,630; herein incorporated by reference). Aptamers may be configured in numerous ways within one or more microfluidic chips 108 to process one or more pathogen indicators 106. For example, in some embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Labeled detector antibodies and/or aptamers that bind to the pathogen indicator 106 (or the aptamer-pathogen indicator 106 complex) may then be passed over the one or more aptamer-pathogen indicator 106 complexes such that the labeled detector antibodies and/or aptamers will label the pathogen indicator 106 (or the aptamer-pathogen indicator 106 complex). Numerous labels may be used that include, but are not limited to, enzymes, fluorescent molecules, radioactive labels, spin labels, redox labels, and the like. In other embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Such binding provides for detection of the aptamer-pathogen indicator 106 complex through use of methods that include, but are not limited to, surface plasmon resonance, conductivity, and the like (e.g., U.S. Patent No.: 7,030,989; herein incorporated by reference). In some embodiments, aptamers may be coupled to a substrate within a microfluidic chip 108 to provide for a competition assay. One or more samples 102 may be mixed with one or more reagent mixtures that include one or more labeled pathogen indicators 106. The mixture may then be passed over the aptamers to facilitate binding of pathogen indicators 106 in the sample 102 and labeled pathogen indicators 106 in the reagent mixture to the aptamers. The unlabeled pathogen indicators 106 in the sample 102 will compete with the labeled pathogen indicators 106 in the reagent mixture for binding to the aptamers. Accordingly, the amount of label bound to the aptamers will vary in accordance with the concentration of unlabeled pathogen indicators 106 in the sample 102. In some embodiments, aptamer interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more aptamers may be used in conjunction with one or more antibodies to process one or more samples 102. In some embodiments, aptamers and antibodies may be used interchangeably to process one or more samples 102. Accordingly, in some embodiments, methods and/or systems for processing and/or detecting pathogen indicators 106 may utilize antibodies and aptamers interchangeably and/or in combination.

In some embodiments, one or more microfluidic chips 108 may be configured to process one or more samples 102 through use of magnetism and/or electrical conductivity. In some embodiments, one or more samples 102 may be processed through use of magnetism. For example, in some embodiments, one or more samples 102 may be combined with one or more tagged polynucleotides that are tagged with a ferrous material, such as a ferrous bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed over a magnet to immobilize the hybridized complexes. In some embodiments, the magnet may be an electromagnet. Other components in the sample 102 may then be washed away from the hybridized complexes. In some embodiments, a chamber containing the magnetically immobilized hybridized complexes may be heated to release the sample polynucleotides from the magnetically immobilized tagged polynucleotides. The sample polynucleotides may then be collected in a more purified state. In other embodiments, similar methods may be used in conjunction with antibodies, aptamers, peptides, ligands, and the like. Accordingly, one or more microfluidic chips 108 may be configured in numerous ways to utilize magnetism to process one or more samples 102. In some embodiments, one or more samples 102 may be processed through use of eddy currents. Eddy current separation uses the principles of electromagnetic induction in conducting materials to separate non-ferrous metals by their different electric conductivities. An electrical charge is induced into a conductor by changes in magnetic flux cutting through it. Moving permanent magnets passing a conductor generates the change in magnetic flux. Accordingly, in some embodiments, one or more microfluidic chips 108 may be configured to include a magnetic rotor such that when conducting particles move through the changing flux of the magnetic rotor, a spiraling current and resulting magnetic field are induced. The magnetic field of the conducting particles may interact with the magnetic field of the magnetic rotor to impart kinetic energy to the conducting particles. The kinetic energy imparted to the conducting particles may then be used to direct movement of the conducting particles. Accordingly, non-ferrous particles, such as metallic beads, may be utilized to process one or more samples 102. For example, in some embodiments, one or more samples 102 may be combined with one or more tagged polynucleotides that are tagged with a non-ferrous material, such as an aluminum bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed through a magnetic field to impart kinetic energy to the non-ferrous bead. This kinetic energy may then be used to separate the hybridized complex. In other embodiments, similar methods may be used in conjunction with antibodies, aptamers, peptides, ligands, and the like. Accordingly, one or more microfluidic chips 108 may be configured in numerous ways to utilize eddy currents to process one or more samples 102. One or more microfluidic chips 108 may be configured in numerous ways to utilize magnetism and/or electrical conductivity to process one or more samples 102.

In some embodiments, one or more microfluidic chips 108 may be configured to process one or more samples 102 through use of isoelectric focusing. Methods have been described that may be used to construct capillary isoelectric focusing systems (e.g., Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). Such systems may be modified to provide for the processing of one or more samples 102.

In some embodiments, one or more microfluidic chips 108 may be configured to process one or more samples 102 through use of electrophoresis. In some embodiments, one or more microfluidic chips 108 may be configured to process one or more samples 102 through use of one-dimensional electrophoresis. In some embodiments, one or more microfluidic chips 108 may be configured to process one or more samples 102 through use of two-dimensional electrophoresis. In some embodiments, one or more microfluidic chips 108 may be configured to process one or more samples 102 through use of gradient gel electrophoresis. In some embodiments, one or more microfluidic chips 108 may be configured to process one or more samples 102 through use of electrophoresis under denaturing conditions. In some embodiments, one or more microfluidic chips 108 may be configured to process one or more samples 102 through use of electrophoresis under native conditions. One or more microfluidic chips 108 may be configured to utilize numerous electrophoretic methods.

In some embodiments, one or more microfluidic chips 108 may be configured to process one or more samples 102 through use of immunoassay. In some embodiments, one or more microfluidic chips 108 may be configured to process one or more samples 102 through use of enzyme linked immunosorbant assay (ELISA). In some embodiments, one or more microfluidic chips 108 may be configured to process one or more samples 102 through use of radioimmuno assay (RIA). In some embodiments, one or more microfluidic chips 108 may be configured to process one or more samples 102 through use of enzyme immunoassay (EIA). In some embodiments, such methods may utilize antibodies (e.g., monoclonal antibodies, polyclonal antibodies, antibody fragments, single-chain antibodies, and the like), aptamers, or substantially any combination thereof. In some embodiments, a labeled antibody and/or aptamer may be used within an immunoassay. In some embodiments, a labeled ligand to which the antibody and/or aptamer binds may be used within an immunoassay. Numerous types of labels may be utilized. Examples of such labels include, but are not limited to, radioactive labels, fluorescent labels, enzyme labels, spin labels, magnetic labels, gold labels, colorimetric labels, redox labels, and the like. Numerous immunoassays are known and may be configured for processing one or more samples 102. In some embodiments, one or more microfluidic chips 108 may be configured to process one or more samples 102 through use of one or more competition assays. In some embodiments, one or more microfluidic chips 108 may be configured to process one or more samples 102 through use of one or more polynucleotide based competition assays. One or more microfluidic chips 108 may be configured to include one or more polynucleotides coupled to a substrate, such as a polynucleotide array. The one or more microfluidic chips 108 may be further configured so that a sample 102 and/or substantially purified polynucleotides obtained from one or more samples 102, may be mixed with one or more reagent mixtures that include one or more labeled polynucleotides to form an analysis mixture. This analysis mixture is then passed over the substrate such that the labeled polynucleotides and the sample polynucleotides are allowed to hybridize to the polynucleotides that are immobilized on the substrate. The sample polynucleotides and the labeled polynucleotides will compete for binding to the polynucleotides that are coupled on the substrate. Accordingly, the presence and/or concentration of the polynucleotides in the sample 102 can be determined through detection of the label (e.g., the concentration of the polynucleotides in the sample 102 will be inversely related to the amount of label that is bound to the substrate). Numerous labels may be used that include, but are not limited to, enzymes, fluorescent molecules, radioactive labels, spin labels, redox labels, and the like. In some embodiments, one or more microfluidic chips 108 may be configured to include one or more antibodies, proteins, peptides, and/or aptamers that are coupled to a substrate. The one or more microfluidic chips 108 may be further configured so that a sample 102 and/or substantially purified sample polynucleotides and/or sample peptides obtained from one or more samples 102, may be mixed with one or more reagent mixtures that include one or more labeled polypeptides and/or labeled peptides to form an analysis mixture. This analysis mixture can then be passed over the substrate such that the labeled polypeptides and/or labeled peptides and the sample polynucleotides and/or sample peptides are allowed to bind to the antibodies, proteins, peptides, and/or aptamers that are immobilized on the substrate. The sample polypeptides and/or sample peptides and the labeled polypeptides and/or sample peptides will compete for binding to the antibodies, proteins, peptides, and/or aptamers that are coupled on the substrate. Accordingly, the presence and/or concentration of the sample polypeptides and/or sample peptides in the sample 102 can be determined through detection of the label (e.g., the concentration of the sample polypeptides and/or sample peptides in the sample 102 will be inversely related to the amount of label that is bound to the substrate). Numerous labels may be used that include, but are not limited to, enzymes, fluorescent molecules, radioactive labels, spin labels, redox labels, and the like. Microfluidic chips 108 may be configured to utilize numerous types of competition assays.

In some embodiments, one or more microfluidic chips 108 may be configured to utilize numerous processing methods. For example, in some embodiments, one or more pathogen indicators 106 may be precipitated with salt, dialyzed, and then applied to a chromatographic column.

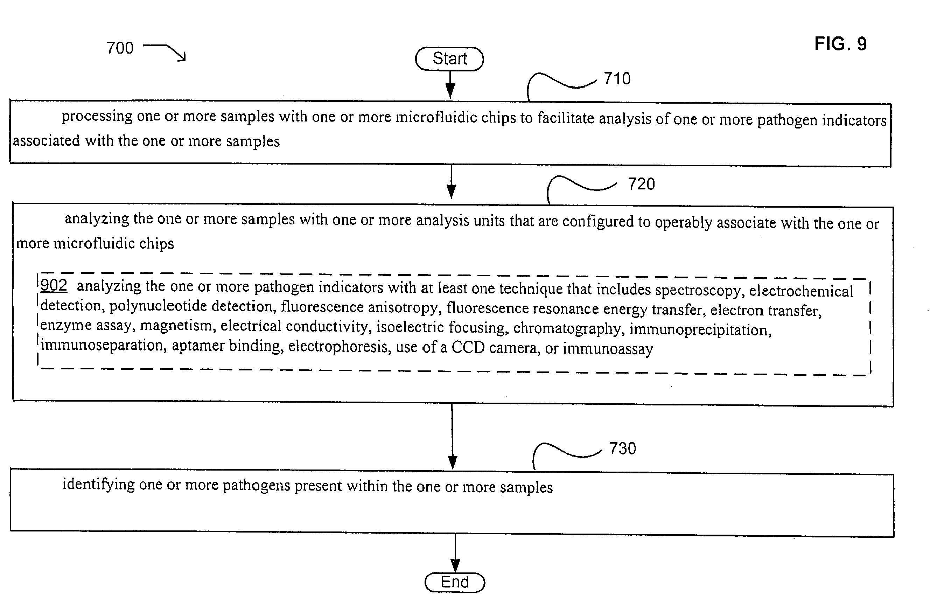

FIG. 9 illustrates alternative embodiments of the example operational flow 700 of FIG. 7. FIG. 9 illustrates example embodiments where the processing operation 720 may include at least one additional operation. Additional operations may include an operation 902.

At operation 902, the analyzing operation 720 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens 104 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, a protozoan, a single-celled organism, a fungus, an algae, a protein, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units 120 and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

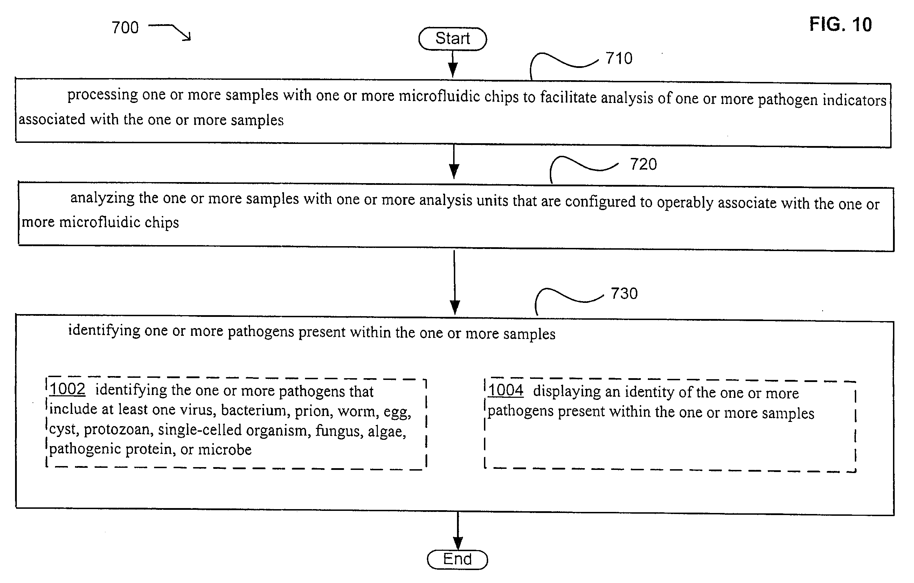

FIG. 10 illustrates alternative embodiments of the example operational flow 700 of FIG. 7. FIG. 10 illustrates example embodiments where the optional identifying operation 730 may include at least one additional operation. Additional operations may include an operation 1002 and/or operation 1004.

At operation 1002, the identifying operation 730 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 1004, the identifying operation 730 may include displaying an identity of the one or more pathogens present within the one or more samples. In some embodiments, one or more display units 124 may indicate an identity of one or more pathogens 104 that correspond to the one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples 102 may be biological samples 102. Examples of such biological samples 102 include, but are not limited to, blood samples 102, fecal samples 102, urine samples 102, and the like.

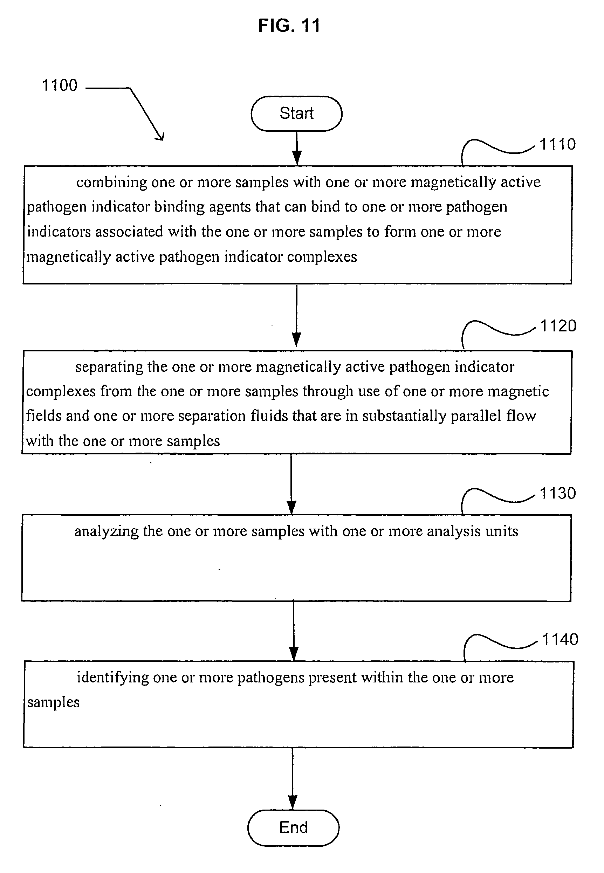

FIG. 11 illustrates an operational flow 1100 representing examples of operations that are related to the performance of a method for analysis of one or more pathogens 104. In FIG. 11 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 1100 includes a combining operation 1110 involving combining one or more samples with one or more magnetically active pathogen indicator binding agents that can bind to one or more pathogen indicators associated with the one or more samples to form one or more magnetically active pathogen indicator complexes. In some embodiments, combining operation 1110 may include combining the one or more samples with at least one magnetically active antibody, aptamer, polynucleotide, or polypeptide.

After a start operation, the operational flow 1100 includes a separating operation 1120 involving separating the one or more magnetically active pathogen indicator complexes from the one or more samples through use of one or more magnetic fields and one or more separation fluids that are in substantially parallel flow with the one or more samples. In some embodiments, separating operation 1120 may include separating the one or more magnetically active pathogen indicator complexes through use of magnetic attraction or magnetic repulsion. In some embodiments, separating operation 1120 may include separating the one or more magnetically active pathogen indicator complexes through use of one or more ferrofluids.

After a start operation, the operational flow 1100 may optionally include an analyzing operation 1130 involving analyzing the one or more samples with one or more analysis units. In some embodiments, analyzing operation 1130 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 1100 may optionally include an identifying operation 1140 involving identifying one or more pathogens present within the one or more samples. In some embodiments, identifying operation 1140 may include identifying the one or more pathogens that include at least one virus, bacterium, prion, worm, egg, cyst, protozoan, single-celled organism, fungus, algae, pathogenic protein, or microbe. In some embodiments, identifying operation 1140 may include displaying an identity of the one or more pathogens present within the one or more samples.

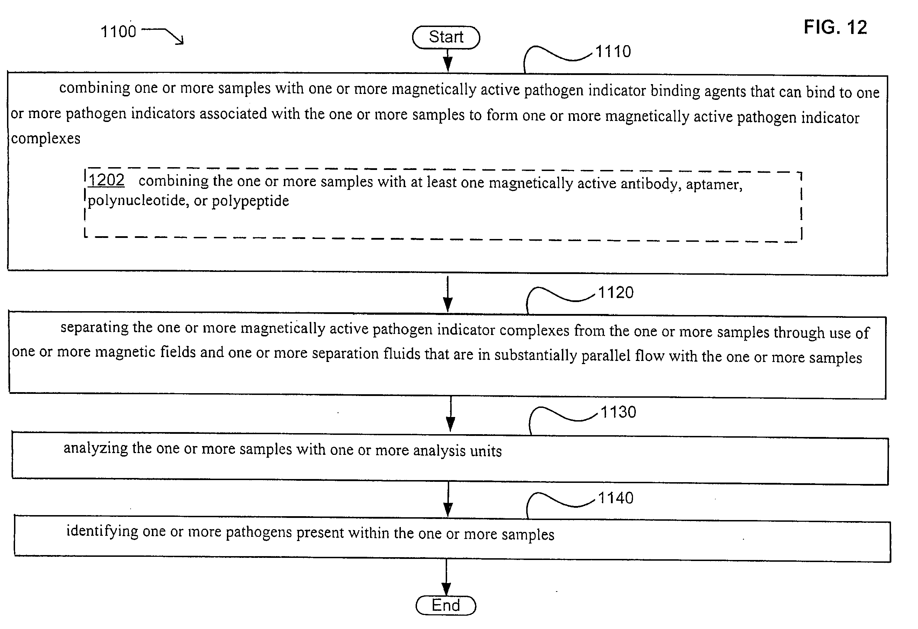

FIG. 12 illustrates alternative embodiments of the example operational flow 1100 of FIG. 11. FIG. 12 illustrates example embodiments where the combining operation 1110 may include at least one additional operation. Additional operations may include an operation 1202.

At operation 1202, the combining operation 1110 may include combining the one or more samples with at least one magnetically active antibody, aptamer, polynucleotide, or polypeptide. In some embodiments, one or more samples 102 may be combined with at least one magnetically active antibody, aptamer, polynucleotide, polypeptide, or substantially any combination thereof. In some embodiments, such mixing may occur within one or more mixing chambers. In some embodiments, such mixing may occur within one or more mixing chambers that are configured to allow one or more magnetically active pathogen indicator binding agents to bind to one or more pathogen indicators 106 associated with the one or more samples 102 to form one or more magnetically active pathogen indicator complexes. In some embodiments, magnetically active pathogen indicator binding agents may be repelled by a magnetic field. In some embodiments, magnetically active pathogen indicator binding agents may be attracted to a magnetic field.

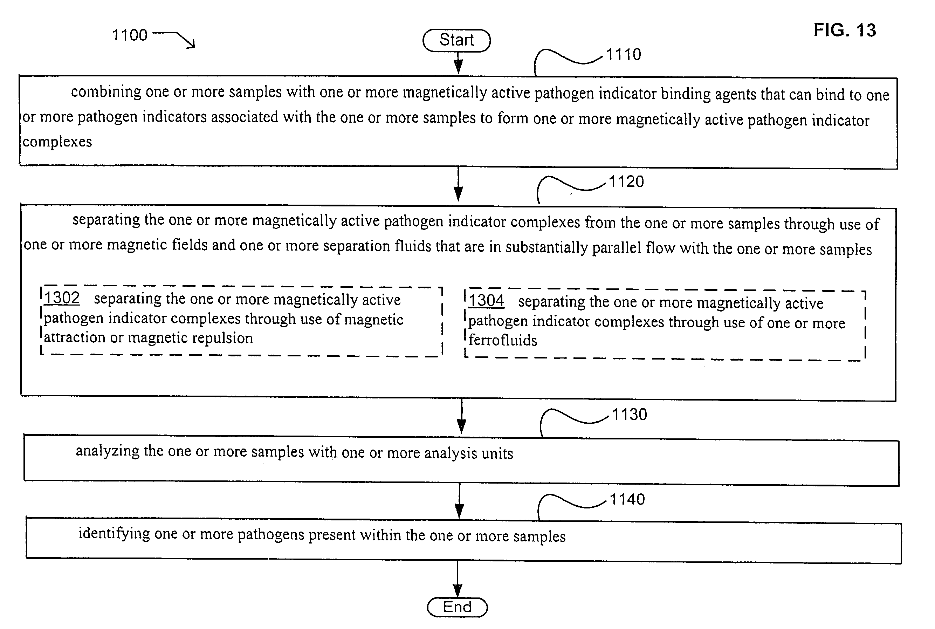

FIG. 13 illustrates alternative embodiments of the example operational flow 1100 of FIG. 11. FIG. 13 illustrates example embodiments where the separating operation 1120 may include at least one additional operation. Additional operations may include an operation 1302, and/or 1304.

At operation 1302, the separating operation 1120 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples 102 to flow in a substantially parallel manner with one or more separation fluids (e.g., an H-filter) and using one or more magnets to cause translocation of the one or more magnetically active pathogen indicator complexes from the one or more samples 120 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12, CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples 102. In some embodiments, magnetically active pathogen indicator complexes may be attracted to magnetically active particles within the one or more separation fluids and thereby separated from the one or more samples 102.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples 102 to flow in a substantially parallel manner with one or more separation fluids and using one or more magnets to cause translocation of the one or more magnetically active pathogen indicator complexes from the one or more samples 102 into the one or more separation fluids.

At operation 1304, the separating operation 1120 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

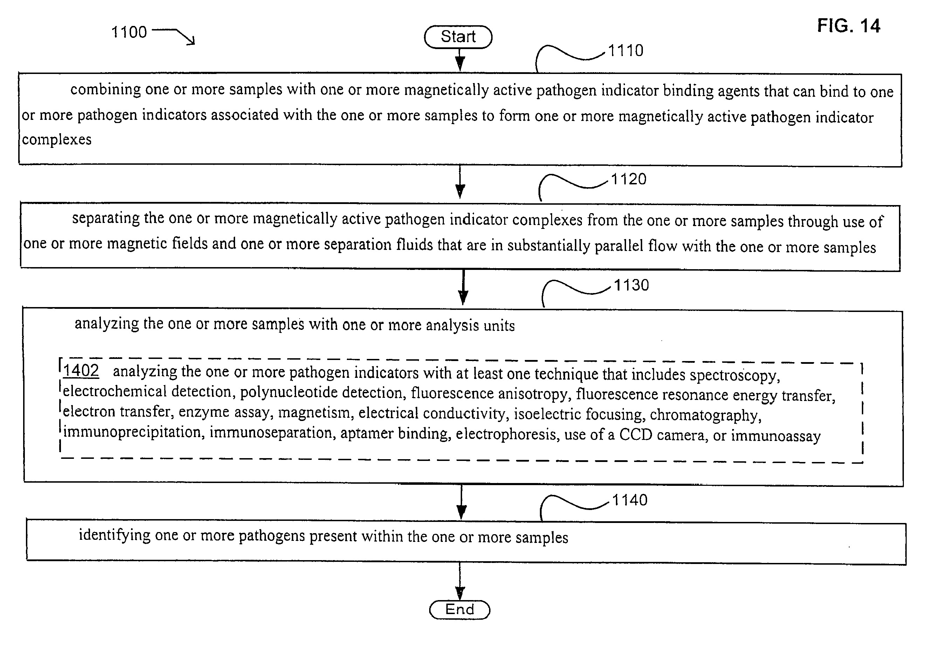

FIG. 14 illustrates alternative embodiments of the example operational flow 1100 of FIG. 11. FIG. 14 illustrates example embodiments where the analyzing operation 1130 may include at least one additional operation. Additional operations may include an operation 1402.

At operation 1402, the analyzing operation 1130 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens indicators 106 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units 120 and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

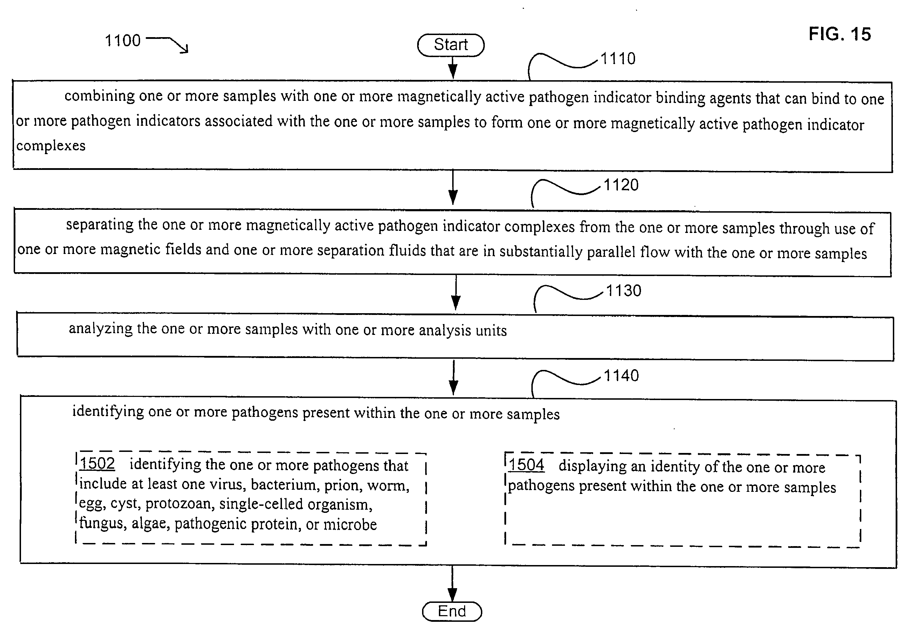

FIG. 15 illustrates alternative embodiments of the example operational flow 1100 of FIG. 11. FIG. 15 illustrates example embodiments where the identifying operation 1140 may include at least one additional operation. Additional operations may include an operation 1502, and/or 1504.

At operation 1502, the identifying operation 1140 may include identifying the one or more pathogens that include at least one virus, bacterium, prion, worm, egg, cyst, protozoan, single-celled organism, fungus, algae, pathogenic protein, or microbe. In some embodiments, one or more detection units 122 may identify the one or more pathogens that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 1504, the identifying operation 1140 may include displaying an identity of the one or more pathogens present within the one or more samples. In some embodiments, one or more display units 124 may indicate an identity of one or more pathogens 104 that correspond to the one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples may be biological samples. Examples of such biological samples 102 include, but are not limited to, blood samples 102, fecal samples 102, urine samples 102, and the like.

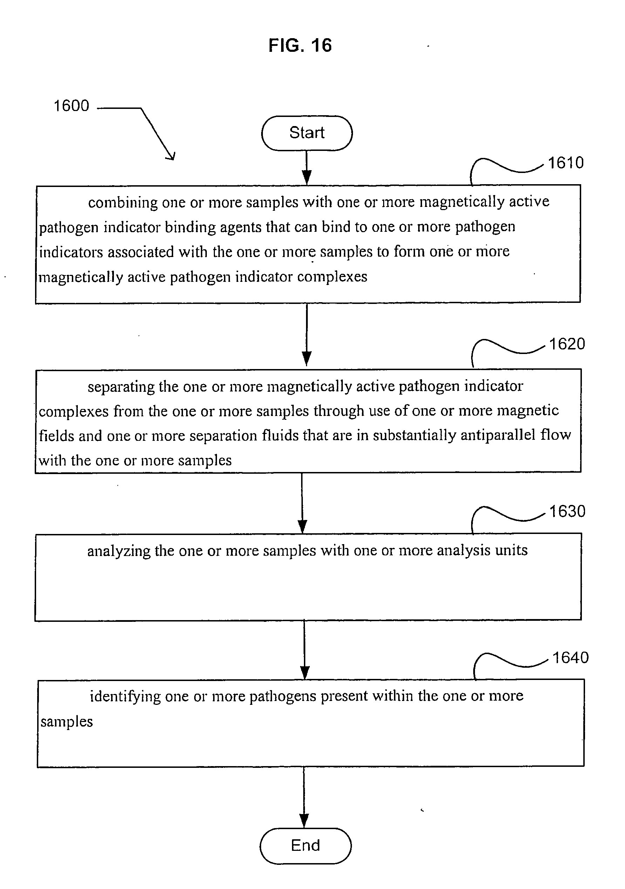

FIG. 16 illustrates an operational flow 1600 representing examples of operations that are related to the performance of a method for analysis of one or more pathogens 104. In FIG. 16 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 1600 includes a combining operation 1610 involving combining one or more samples with one or more magnetically active pathogen indicator binding agents that can bind to one or more pathogen indicators associated with the one or more samples to form one or more magnetically active pathogen indicator complexes. In some embodiments, combining operation 1610 may include combining the one or more samples with at least one magnetically active antibody, aptamer, polynucleotide, or polypeptide.

After a start operation, the operational flow 1600 includes a separating operation 1620 involving separating the one or more magnetically active pathogen indicator complexes from the one or more samples through use of one or more magnetic fields and one or more separation fluids that are in substantially antiparallel flow with the one or more samples. In some embodiments, separating operation 1620 may include separating the one or more magnetically active pathogen indicator complexes through use of magnetic attraction or magnetic repulsion. In some embodiments, separating operation 1620 may include separating the one or more magnetically active pathogen indicator complexes through use of one or more ferrofluids.

After a start operation, the operational flow 1600 may optionally include an analyzing operation 1630 involving analyzing the one or more samples with one or more analysis units. In some embodiments, analyzing operation 1630 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 1600 may optionally include an identifying operation 1640 involving identifying one or more pathogens present within the one or more samples. In some embodiments, identifying operation 1640 may include identifying the one or more pathogens that include at least one virus, bacterium, prion, worm, egg, cyst, protozoan, single-celled organism, fungus, algae, pathogenic protein, or microbe. In some embodiments, identifying operation 1640 may include displaying an identity of the one or more pathogens present within the one or more samples.

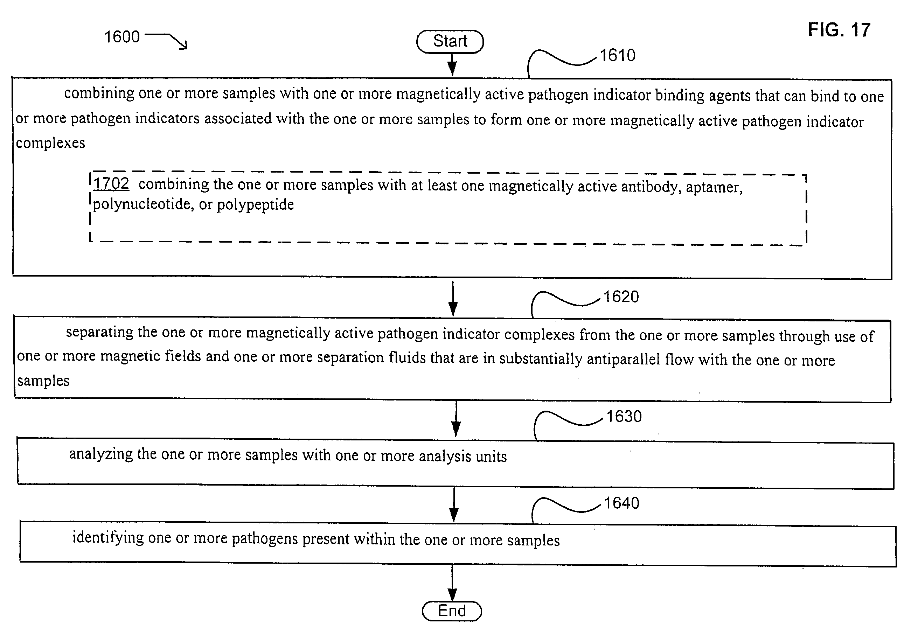

FIG. 17 illustrates alternative embodiments of the example operational flow 1600 of FIG. 16. FIG. 17 illustrates example embodiments where the combining operation 1610 may include at least one additional operation. Additional operations may include an operation 1702.

At operation 1702, the combining operation 1610 may include combining the one or more samples with at least one magnetically active antibody, aptamer, polynucleotide, or polypeptide. In some embodiments, one or more samples 102 may be combined with at least one magnetically active antibody, aptamer, polynucleotide, polypeptide, or substantially any combination thereof. In some embodiments, such mixing may occur within one or more mixing chambers. In some embodiments, such mixing may occur within one or more mixing chambers that are configured to allow one or more magnetically active pathogen indicator binding agents to bind to one or more pathogen indicators associated with the one or more samples 102 to form one or more magnetically active pathogen indicator complexes. In some embodiments, magnetically active pathogen indicator binding agents may be repelled by a magnetic field. In some embodiments, magnetically active pathogen indicator binding agents may be attracted to a magnetic field.

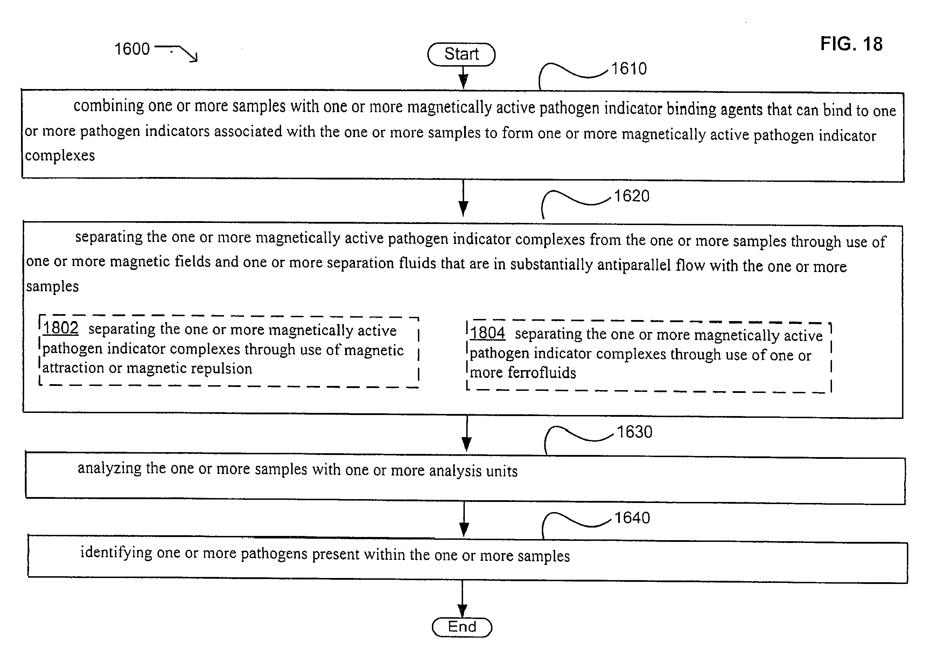

FIG. 18 illustrates alternative embodiments of the example operational flow 1600 of FIG. 16. FIG. 18 illustrates example embodiments where the separating operation 1620 may include at least one additional operation. Additional operations may include an operation 1802, and/or 1804.

At operation 1802, the separating operation 1620 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples 102 to flow in a substantially parallel manner with one or more separation fluids (e.g., an H-filter) and using one or more magnets to cause translocation of the one or more magnetically active pathogen indicator complexes from the one or more samples 102 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12, CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples. In some embodiments, magnetically active pathogen indicator complexes may be attracted to magnetically active particles within the one or more separation fluids and thereby separated from the one or more samples.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples to flow in a substantially parallel manner with one or more separation fluids and using one or more magnets to cause translocation of the one or more magnetically active pathogen indicator complexes from the one or more samples 102 into the one or more separation fluids.

At operation 1804, the separating operation 1620 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

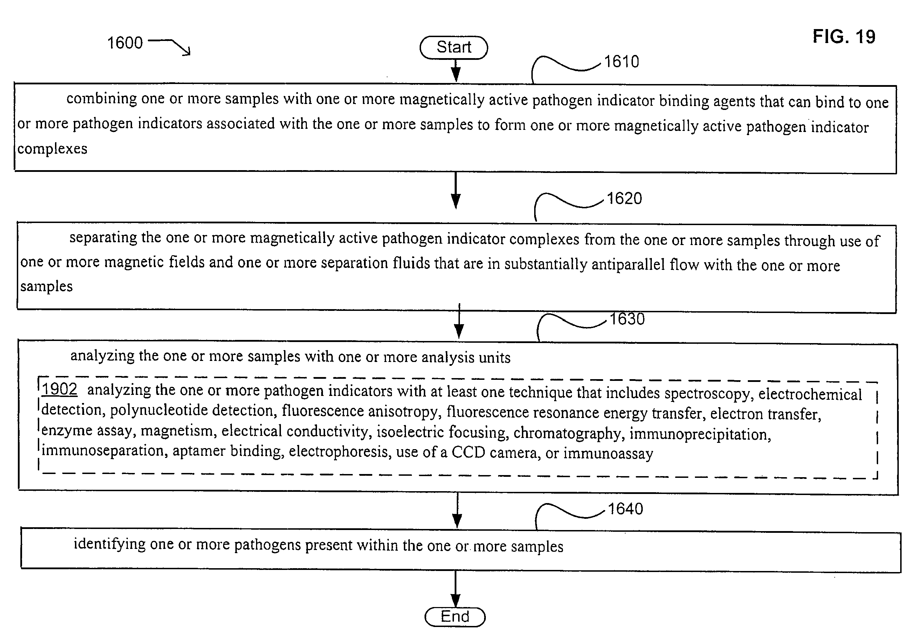

FIG. 19 illustrates alternative embodiments of the example operational flow 1600 of FIG. 16. FIG. 19 illustrates example embodiments where the analyzing operation 1630 may include at least one additional operation. Additional operations may include an operation 1902.

At operation 1902, the analyzing operation 1630 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens indicators 106 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 120, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, a protozoan, a single-celled organism, a fungus, an algae, a protein, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 20 illustrates alternative embodiments of the example operational flow 1600 of FIG. 16. FIG. 20 illustrates example embodiments where the identifying operation 1640 may include at least one additional operation. Additional operations may include an operation 2002, and/or 2004.

At operation 2002, the identifying operation 1640 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 2004, the identifying operation 1640 may include displaying an identity of the one or more pathogens present within the one or more samples. In some embodiments, one or more display units 124 may indicate an identity of the one or more pathogens 104 that correspond to one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples 102 may be biological samples 102. Examples of such biological samples 102 include, but are not limited to, blood samples 102, fecal samples 102, urine samples 102, and the like.

FIG. 21 illustrates an operational flow 2100 representing examples of operations that are related to the performance of a method for analysis of one or more pathogens 104. In FIG. 21 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 2100 includes an accepting operation 2110 involving accepting one or more samples that include one or more magnetically active pathogen indicator binding agents that can bind to one or more pathogen indicators associated with the one or more samples to form one or more magnetically active pathogen indicator complexes. In some embodiments, accepting operation 2110 may include accepting the one or more samples that include one or more liquids. In some embodiments, accepting operation 2110 may include accepting the one or more samples that include one or more solids. In some embodiments, accepting operation 2110 may include accepting the one or more samples that include one or more gases. In some embodiments, accepting operation 2110 may include accepting the one or more samples that include one or more food products. In some embodiments, accepting operation 2110 may include accepting the one or more samples that include one or more biological samples.

After a start operation, the operational flow 2100 includes a separating operation 2120 involving separating the one or more magnetically active pathogen indicator complexes from the one or more samples through use of one or more magnetic fields and one or more separation fluids that are in substantially parallel flow with the one or more samples. In some embodiments, separating operation 2120 may include separating the one or more magnetically active pathogen indicator complexes through use of magnetic attraction or magnetic repulsion. In some embodiments, separating operation 2120 may include separating the one or more magnetically active pathogen indicator complexes through use of one or more ferrofluids.

After a start operation, the operational flow 2100 may optionally include an analyzing operation 2130 involving analyzing the one or more samples with one or more analysis units. In some embodiments, analyzing operation 2130 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 2100 may optionally include an identifying operation 2140 involving identifying one or more pathogens present within the one or more samples. In some embodiments, identifying operation 2140 may include identifying the one or more pathogens that include at least one virus, bacterium, prion, worm, egg, cyst, protozoan, single-celled organism, fungus, algae, pathogenic protein, or microbe. In some embodiments, identifying operation 2140 may include displaying an identity of the one or more pathogens present within the one or more samples.

FIG. 22 illustrates alternative embodiments of the example operational flow 2100 of FIG. 21. FIG. 22 illustrates example embodiments where the accepting operation 2110 may include at least one additional operation. Additional operations may include an operation 2202, an operation 2204, an operation 2206, an operation 2208, and/or an operation 2210.

At operation 2202, the accepting operation 2110 may include accepting the one or more samples that include one or more liquids. In some embodiments, one or more microfluidic chips 108 may accept one or more samples 102 that include one or more liquids. In some embodiments, one or more microfluidic chips 108 may include one or more lancets. Such lancets may be configured to provide for collection of one or more samples 102 that include a fluid. For example, in some embodiments, a lancet may be used to collect one or more samples 102 from a food product to facilitate analysis of the food product for the presence of one or more pathogens 104. In some embodiments, a microfluidic chip 108 may include one or more septa through which a needle may be passed to deliver a fluid sample 102 to the microfluidic chip 108. In some embodiments, a microfluidic chip 108 may include one or more leur lock connectors to which one or more syringes may be coupled to deliver one or more fluid samples 102 to the microfluidic chip 108. In some embodiments, a microfluidic chip 108 may be configured to operably associate with one or more devices that are configured to deliver one or more liquid samples 102 to the microfluidic chip 108. In some embodiments, a microfluidic chip 108 may include one or more sonicators that facilitate release of the liquid portion from a sample 102 to make it available to the microfluidic chip 108. Microfluidic chips 108 may be configured to accept numerous types of liquids. Examples of such liquids include, but are not limited to, beverages, water, food products, solvents, and the like. In some embodiments, microfluidic chips 108 may be configured for use by travelers to determine if a consumable item contains one or more pathogens 104. Accordingly, microfluidic chips 108 may be configured in numerous ways such that they may accept one or more samples 102 that include a liquid.

At operation 2204, the accepting operation 2110 may include accepting the one or more samples that include one or more solids. In some embodiments, one or more microfluidic chips 108 may accept one or more samples 102 that include one or more solids. Examples of such solid samples include, but are not limited to, food products, soil samples 102, and the like. In some embodiments, microfluidic chips 108 may be configured to suspend a solid sample 102 in a fluid. In some embodiments, microfluidic chips 108 may be configured to crush a sample 102 into smaller particles. For example, in some embodiments, a microfluidic chip 108 may accept a solid sample 102. The sample 102 may be ground into smaller particles to facilitate detection of one or more pathogen indicators 106 that may be present within the sample 102. In some embodiments, a microfluidic chip 108 may include one or more sonicators that break the sample 102 into smaller particles to facilitate detection of one or more pathogen indicators 106 that may be present within the sample 102. For example, in some embodiments, viral particles may be broken into smaller particles to provide for detection of one or more polynucleotides that are associated with the viral particles. Accordingly, microfluidic chips 108 may be configured in numerous ways such that they may accept one or more samples 102 that include a solid.

At operation 2206, the accepting operation 2110 may include accepting the one or more samples that include one or more gases. In some embodiments, one or more microfluidic chips 108 may accept one or more samples 102 that include one or more gases. For example, in some embodiments, a microfluidic chip 108 may include one or more fans that blow and/or draw gas into the microfluidic chip 108. In some embodiments, a microfluidic chip 108 may include one or more bubble chambers through which one or more gases pass. In some embodiments, such bubble chambers may be configured to include one or more fluids (e.g., solvents) that may be used to selectively retain (e.g., extract) one or more pathogen indicators 106 from one or more gas samples 102. In some embodiments, a microfluidic chip 108 may include one or more electrostatic filters through which one or more gases pass. Such electrostatic filters (e.g., air ionizers) may be configured to capture numerous types of pathogen indicators 106. In some embodiments, a microfluidic chip 108 may include one or more filters through which one or more gases pass. In some embodiments, such microfluidic chips 108 may be used to detect and/or identify airborne pathogens 104, such as viruses, spores, and the like.

At operation 2208, the accepting operation 2110 may include accepting the one or more samples that include one or more food products. In some embodiments, one or more microfluidic chips 108 may accept one or more samples 102 that include one or more food products. For example, in some embodiments, one or more microfluidic chips 108 may include one or more lancets that may be inserted into the food product to withdraw one or more samples 102. In some embodiments, one or more microfluidic chips 108 may include one or more septa that may be configured to operably associate with a syringe or the like. In some embodiments, one or more microfluidic chips 108 may be configured to accept one or more food samples 102 that are solids, such as meats, cheeses, nuts, vegetables, fruits, and the like, and/or liquids, such as water, juice, milk, and the like. In some embodiments, one or more microfluidic chips 108 may include one or more mechanisms that can facilitate processing of the one or more samples 102. Examples of such mechanisms include, but are not limited to, grinders, sonicators, treatment of the one or more samples 102 with degredative enzymes (e.g., protease, nuclease, lipase, collagenase, and the like), strainers, filters, centrifugation chambers, and the like. Accordingly, such microfluidic chips 108 may be used to detect one or more pathogen indicators 106 in one or more food products. Examples of such pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

At operation 2210, the accepting operation 2110 may include accepting the one or more samples that include one or more biological samples. In some embodiments, one or more microfluidic chips 108 may accept one or more samples 102 that include one or more biological samples 102. Examples of biological samples 102 include, but are not limited to, blood, cerebrospinal fluid, mucus, breath, urine, fecal material, skin, tissue, tears, hair, and the like.

FIG. 23 illustrates alternative embodiments of the example operational flow 2100 of FIG. 21. FIG. 23 illustrates example embodiments where the separating operation 2120 may include at least one additional operation. Additional operations may include an operation 2302, and/or an operation 2304.

At operation 2302, the separating operation 2120 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples 102 to flow in a substantially parallel manner with one or more separation fluids (e.g., an H-filter) and using one or more magnets to cause translocation of the one or more magnetically active pathogen indicator complexes from the one or more samples 102 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12, CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples 102. In some embodiments, magnetically active pathogen indicator complexes may be attracted to magnetically active particles within the one or more separation fluids and thereby separated from the one or more samples 102.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples 102 to flow in a substantially parallel manner with one or more separation fluids and using one or more magnets to cause translocation of the one or more magnetically active pathogen indicator complexes from the one or more samples 102 into the one or more separation fluids.

At operation 2304, the separating operation 2120 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

FIG. 24 illustrates alternative embodiments of the example operational flow 2100 of FIG. 21. FIG. 24 illustrates example embodiments where the analyzing operation 2130 may include at least one additional operation. Additional operations may include an operation 2402.

At operation 2402, the analyzing operation 2130 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens indicators 106 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units 120 and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble-form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102 that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

**FIG.** 25 illustrates alternative embodiments of the example operational flow 2100 of FIG. 21. FIG. 25 illustrates example embodiments where the identifying operation 2140 may include at least one additional operation. Additional operations may include an operation 2502, and/or an operation 2504.

At operation 2502, the identifying operation 2140 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 2504, the identifying operation 2140 may include displaying an identity of the one or more pathogens present within the one or more samples. In some embodiments, one or more display units 124 may indicate an identity of one or more pathogens 104 that correspond to the one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples 102 may be biological samples 102. Examples of such biological samples 102 include, but are not limited to, blood samples 102, fecal samples 102, urine samples 102, and the like.

FIG. 26 illustrates an operational flow 2600 representing examples of operations that are related to the performance of a method for analysis of one or more pathogens 104. In FIG. 26 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 2600 includes an accepting operation 2610 involving accepting one or more samples that include one or more magnetically active pathogen indicator binding agents that can bind to one or more pathogen indicators associated with the one or more samples to form one or more magnetically active pathogen indicator complexes. In some embodiments, accepting operation 2610 may include accepting the one or more samples that include one or more liquids. In some embodiments, accepting operation 2610 may include accepting the one or more samples that include one or more solids. In some embodiments, accepting operation 2610 may include accepting the one or more samples that include one or more gases. In some embodiments, accepting operation 2610 may include accepting the one or more samples that include one or more food products. In some embodiments, accepting operation 2610 may include accepting the one or more samples that include one or more biological samples.

After a start operation, the operational flow 2600 includes a separating operation 2620 involving separating the one or more magnetically active pathogen indicator complexes from the one or more samples through use of one or more magnetic fields and one or more separation fluids that are in substantially antiparallel flow with the one or more samples. In some embodiments, separating operation 2620 may include separating the one or more magnetically active pathogen indicator complexes through use of magnetic attraction or magnetic repulsion. In some embodiments, separating operation 2620 may include separating the one or more magnetically active pathogen indicator complexes through use of one or more ferrofluids.

After a start operation, the operational flow 2600 may optionally include an analyzing operation 2630 involving analyzing the one or more samples with one or more analysis units. In some embodiments, analyzing operation 2630 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 2600 may optionally include an identifying operation 2640 involving identifying one or more pathogens present within the one or more samples. In some embodiments, identifying operation 2640 may include identifying the one or more pathogens that include at least one virus, bacterium, prion, worm, egg, cyst, protozoan, single-celled organism, fungus, algae, pathogenic protein, or microbe. In some embodiments, identifying operation 2640 may include displaying an identity of the one or more pathogens present within the one or more samples.

FIG. 27 illustrates alternative embodiments of the example operational flow 2600 of FIG. 26. FIG. 27 illustrates example embodiments where the accepting operation 2610 may include at least one additional operation. Additional operations may include an operation 2702, an operation 2704, an operation 2706, an operation 2708, and/or an operation 2710.

At operation 2702, the accepting operation 2610 may include accepting the one or more samples that include one or more liquids. In some embodiments, one or more microfluidic chips 108 may accept one or more samples 102 that include one or more liquids. In some embodiments, one or more microfluidic chips 108 may include one or more lancets. Such lancets may be configured to provide for collection of one or more samples 102 that include a fluid. For example, in some embodiments, a lancet may be used to collect one or more samples 102 from a food product to facilitate analysis of the food product for the presence of one or more pathogens 104. In some embodiments, a microfluidic chip 108 may include one or more septa through which a needle may be passed to deliver a fluid sample 102 to the microfluidic chip 108. In some embodiments, a microfluidic chip 108 may include one or more leur lock connectors to which one or more syringes may be coupled to deliver one or more fluid samples 102 to the microfluidic chip 108. In some embodiments, a microfluidic chip 108 may be configured to operably associate with one or more devices that are configured to deliver one or more liquid samples 102 to the microfluidic chip 108. In some embodiments, a microfluidic chip 108 may include one or more sonicators that facilitate release of the liquid portion from a sample 102 to make it available to the microfluidic chip 108. Microfluidic chips 108 may be configured to accept numerous types of liquids. Examples of such liquids include, but are not limited to, beverages, water, food products, solvents, and the like. In some embodiments, microfluidic chips may be configured for use by travelers to determine if a consumable item contains one or more pathogens 104. Accordingly, microfluidic chips 108 may be configured in numerous ways such that they may accept one or more samples 102 that include a liquid.

At operation 2704, accepting operation 2610 may include accepting the one or more samples that include one or more solids. In some embodiments, one or more microfluidic chips 108 may accept one or more samples 102 that include one or more solids. Examples of such solid samples include, but are not limited to, food products, soil samples 102, and the like. In some embodiments, microfluidic chips 108 may be configured to suspend a solid sample 102 in a fluid. In some embodiments, microfluidic chips 108 may be configured to crush a sample 102 into smaller particles. For example, in some embodiments, a microfluidic chip 108 may accept a solid sample 102. The sample 102 may be ground into smaller particles to facilitate detection of one or more pathogen indicators 106 that may be present within the sample 102. In some embodiments, a microfluidic chip 108 may include one or more sonicators that break the sample 102 into smaller particles to facilitate detection of one or more pathogen indicators 106 that may be present within the sample 102. For example, in some embodiments, viral particles may be broken into smaller particles to provide for detection of one or more polynucleotides that are associated with the viral particles. Accordingly, microfluidic chips 108 may be configured in numerous ways such that they may accept one or more samples 102 that include a solid.

At operation 2706, accepting operation 2610 may include accepting the one or more samples that include one or more gases. In some embodiments, one or more microfluidic chips 108 may accept one or more samples 102 that include one or more gases. For example, in some embodiments, a microfluidic chip 108 may include one or more fans that blow and/or draw gas into the microfluidic chip 108. In some embodiments, a microfluidic chip 108 may include one or more bubble chambers through which one or more gases pass. In some embodiments, such bubble chambers may be configured to include one or more fluids (e.g., solvents) that may be used to selectively retain (e.g., extract) one or more pathogen indicators 106 from one or more gas samples 102. In some embodiments, a microfluidic chip 108 may include one or more electrostatic filters through which one or more gases pass. Such electrostatic filters (e.g., air ionizers) may be configured to capture numerous types of pathogen indicators 106. In some embodiments, a microfluidic chip 108 may include one or more filters through which one or more gases pass. In some embodiments, such microfluidic chips 108 may be used to detect and/or identify airborne pathogens 104, such as viruses, spores, and the like.

At operation 2708, accepting operation 2610 may include accepting the one or more samples that include one or more food products. In some embodiments, one or more microfluidic chips 108 may accept one or more samples 102 that include one or more food products. For example, in some embodiments, one or more microfluidic chips 108 may include one or more lancets that may be inserted into the food product to withdraw one or more samples 102. In some embodiments, one or more microfluidic chips 108 may include one or more septa that may be configured to operably associate with a syringe or the like. In some embodiments, one or more microfluidic chips 108 may be configured to accept one or more food samples 102 that are solids, such as meats, cheeses, nuts, vegetables, fruits, and the like, and/or liquids, such as water, juice, milk, and the like. In some embodiments, one or more microfluidic chips 108 may include one or more mechanisms that can facilitate processing of the one or more samples 102. Examples of such mechanisms include, but are not limited to, grinders, sonicators, treatment of the one or more samples 102 with degredative enzymes (e.g., protease, nuclease, lipase, collagenase, and the like), strainers, filters, centrifugation chambers, and the like. Accordingly, such microfluidic chips 108 may be used to detect one or more pathogen indicators in one or more food products. Examples of such pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

At operation 2710, accepting operation 2610 may include accepting the one or more samples that include one or more biological samples. In some embodiments, one or more microfluidic chips 108 may accept one or more samples 102 that include one or more biological samples. Examples of biological samples 102 include, but are not limited to, blood, cerebrospinal fluid, mucus, breath, urine, fecal material, skin, tissue, tears, hair, and the like.

FIG. 28 illustrates alternative embodiments of the example operational flow 2600 of FIG. 26. FIG. 28 illustrates example embodiments where the separating operation 2620 may include at least one additional operation. Additional operations may include an operation 2802, and/or an operation 2804.

At operation 2802, the separating operation 2620 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples to flow in a substantially parallel manner with one or more separation fluids (e.g., an H-filter) and using one or more magnets to cause translocation of the one or more magnetically active pathogen indicator complexes from the one or more samples 102 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12, CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples 102. In some embodiments, magnetically active pathogen indicator complexes may be attracted to magnetically active particles within the one or more separation fluids and thereby separated from the one or more samples 102.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples to flow in a substantially parallel manner with one or more separation fluids and using one or more magnets to cause translocation of the one or more magnetically active pathogen indicator complexes from the one or more samples 102 into the one or more separation fluids.

At operation 2804, separating operation 2620 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

FIG. 29 illustrates alternative embodiments of the example operational flow 2600 of FIG. 26. FIG. 29 illustrates example embodiments where the analyzing operation 2630 may include at least one additional operation. Additional operations may include an operation 2902.

At operation 2902, the analyzing operation 2630 may include analyzing the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more analysis units 120 may be configured to analyze one or more pathogens indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more analysis units 120 may be included within one or more microfluidic chips 108. In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122.

In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, Analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, a prion, a protozoan, a single-celled organism, a fungus, an algae, a protein, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Micro column Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, fluorescent labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples 102) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators 106 through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator complex. One or more insoluble pathogen indicator binding constituents, such as a sepharose bead that includes an antibody or aptamer that binds to the one or more pathogen indicators 106, may be bound to the fluorescently labeled antibody-pathogen indicator complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 30 illustrates alternative embodiments of the example operational flow 2600 of FIG. 26. FIG. 30 illustrates example embodiments where the identifying operation 2640 may include at least one additional operation. Additional operations may include an operation 3002, and/or an operation 3004.

At operation 3002, the identifying operation 2640 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens 104 that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 3004, the identifying operation 2640 may include displaying an identity of the one or more pathogens present within the one or more samples. In some embodiments, one or more display units 124 may indicate an identity of the one or more pathogens 104 that correspond to one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples may be biological samples. Examples of such biological samples 102 include, but are not limited to, blood samples 102, fecal samples 102, urine samples 102, and the like.

**FIG.** 31 illustrates an operational flow 3100 representing examples of operations that are related to the performance of a method for analysis of one or more pathogens 104. In FIG. 31 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 3100 includes a separating operation 3110 involving separating one or more magnetically active pathogen indicator complexes from one or more samples through use of one or more magnetic fields and one or more separation fluids that are in substantially parallel flow with the one or more samples. In some embodiments, separating operation 3110 may include separating the one or more magnetically active pathogen indicator complexes through use of magnetic attraction or magnetic repulsion. In some embodiments, separating operation 3110 may include separating the one or more magnetically active pathogen indicator complexes through use of one or more ferrofluids.

After a start operation, the operational flow 3100 may optionally include a detecting operation 3120 involving detecting one or more pathogen indicators with one or more detection units. In some embodiments, detecting operation 3120 may include detecting the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 3100 may optionally include an identifying operation 3130 involving identifying one or more pathogens present within the one or more samples. In some embodiments, identifying operation 3130 may include identifying the one or more pathogens that include at least one virus, bacterium, prion, worm, egg, cyst, protozoan, single-celled organism, fungus, algae, pathogenic protein, or microbe. In some embodiments, identifying operation 3130 may include displaying an identity of the one or more pathogens present within the one or more samples.

FIG. 32 illustrates alternative embodiments of the example operational flow 3100 of FIG. 31. FIG. 32 illustrates example embodiments where the separating operation 3110 may include at least one additional operation. Additional operations may include an operation 3202, and/or an operation 3204.

At operation 3202, the separating operation 3110 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples to flow in a substantially parallel manner with one or more separation fluids (e.g., an H-filter) and using one or more magnets to cause translocation of the one or more magnetically active pathogen indicator complexes from the one or more samples 102 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12, CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples 102. In some embodiments, magnetically active pathogen indicator complexes may be attracted to magnetically active particles within the one or more separation fluids and thereby separated from the one or more samples 102.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples 102 to flow in a substantially parallel manner with one or more separation fluids and using one or more magnets to cause translocation of the one or more magnetically active pathogen indicator complexes from the one or more samples 102 into the one or more separation fluids.

At operation 3204, the separating operation 3110 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

FIG. 33 illustrates alternative embodiments of the example operational flow 3100 of FIG. 31. FIG. 33 illustrates example embodiments where the detecting operation 3120 may include at least one additional operation. Additional operations may include an operation 3302.

At operation 3302, the detecting operation 3120 may include detecting the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more detection units 122 may be used to detect one or more pathogen indicators 106 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more detection units 122 may be configured to detect one or more pathogen indicators 106 that have been processed by one or more microfluidic chips 108. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect the one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108. In some embodiments, one or more microfluidic chips 108 may be configured to process one or more samples 102 through use of surface plasmon resonance. In some embodiments, the one or more microfluidic chips 108 may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more microfluidic chips 108 may include an exposed substrate surface that is configured to operably associate with one or more prisms that are included within one or more detection units 122. In some embodiments, one or more microfluidic chips 108 may include a nuclear magnetic resonance (NMR) probe. In such embodiments, the microfluidic chips 108 may be configured to associate with one or more detection units 122 that accept the NMR probe and are configured to detect one or more pathogen indicators 106 through use of NMR spectroscopy. Accordingly, microfluidic chips 108 and detection units 122 may be configured in numerous ways to associate with each other to provide for detection of one or more pathogen indicators 106.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 1.06. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more microfluidic chips 108 may be configured to process one or more samples 102 and provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a pollen particle, a dander particle, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors are configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to process one or more samples 102 and provide for detection of one or more pathogen indicators 106 through use of isoelectric focusing. In some embodiments, native isoelectric focusing may be utilized to process and/or detect one or more pathogen indicators 106. In some embodiments, denaturing isoelectric focusing may be utilized to process and/or detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more microfluidic chips 108 may be configured to process one or more samples 102 through use of methods that include isoelectric focusing. In some embodiments, one or more detection units 122 may be configured to operably associate with one or more such microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding molecules and/or binding complexes that bind to one or more pathogen indicators 106 that may be present with the one or more samples 102 to form a pathogen indicator-binding molecule/binding complex. Examples of such binding molecules and/or binding complexes that bind to one or more pathogen indicators 106 include, but are not limited to, antibodies, aptamers, peptides, proteins, polynucleotides, and the like. In some embodiments, a pathogen indicator-binding molecule/binding complex may be processed through use of isoelectric focusing and then detected with one or more detection units 122. In some embodiments, one or more binding molecules and/or one or more binding complexes may include a label. Numerous labels may be used and include, but are not limited to, radioactive labels, fluorescent labels, colorimetric labels, spin labels, fluorescent labels, and the like. Accordingly, in some embodiments, a pathogen indicator-binding molecule (labeled)/binding complex (labeled) may be processed through use of isoelectric focusing and then detected with one or more detection units 122 that are configured to detect the one or more labels. Microfluidic chips 108 and detection units 122 may be configured in numerous ways to process one or more samples 102 and detect one or more pathogen indicators 106 through use of isoelectric focusing.

In some embodiments, one or more pathogen indicators 106 may be detected through use of chromatographic methodology alone or in combination with additional processing and/or detection methods. In some embodiments, one or more microfluidic chips 108 may be configured to process one or more samples 102 and provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106 that were processed through use of chromatographic methods. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are processed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. In some embodiments, one or more detection units 122 may be configured to utilize one or more ion-specific electrodes to detect one or more pathogen indicators 106. For example, such electrodes may be used to detect pathogen indicators 106 that include, but are not limited to, metals (e.g., tin, silver, nickel, cobalt, chromate), nitrates, nitrites, sulfites, and the like. Such pathogen indicators 106 are often associated with food, beverages, clothing, jewelry, and the like. Accordingly, chromatographic methods may be used in combination with additional methods and in combination with numerous types of detection methods.

In some embodiments, one or more pathogen indicators 106 may be detected through use of immunoprecipitation. In some embodiments, one or more detection units 122 may be configured to detect one or more pathogen indicators 106 through use of immunoprecipitation. In some embodiments, immunoprecipitation may be utilized in combination with additional processing and/or detection methods to detect one or more pathogen indicators 106. In some embodiments, one or more microfluidic chips 108 may be configured to process one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be detected through use of immunoseparation. In some embodiments, one or more detection units 122 may be configured to detect one or more pathogen indicators 106 through use of immunoseparation. In some embodiments, immunoseparation may be utilized in combination with additional processing and/or detection methods to detect one or more pathogen indicators 106. In some embodiments, one or more microfluidic chips 108 may be configured to process one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex.

Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more pathogen indicators 106 may be detected through use of aptamer binding. In some embodiments, one or more detection units 122 may be configured to detect one or more pathogen indicators 106 through use of aptamer binding. In some embodiments, aptamer binding may be utilized in combination with additional processing and/or detection methods to detect one or more pathogen indicators 106. In some embodiments, one or more microfluidic chips 108 may be configured to process one or more samples 102 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to process one or more samples 102 through use of electrophoresis. In some embodiments, such microfluidic chips 108 may be configured to operably associate with one or more detection units 122. Accordingly, in some embodiments, one or more detection units 122 may be configured to operably associate with one or more microfluidic chips 108 and detect one or more pathogen indicators 106 that were processed through use of electrophoresis. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to process and detect one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous processing methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to process one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to process one or more samples 102 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 34 illustrates alternative embodiments of the example operational flow 3100 of FIG. 31. FIG. 34 illustrates example embodiments where the identifying operation 3130 may include at least one additional operation. Additional operations may include an operation 3402, and/or an operation 3404.

At operation 3402, the identifying operation 3130 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens 104 that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 3404, the identifying operation 3130 may include displaying an identity of the one or more pathogens present within the one or more samples. In some embodiments, one or more display units 124 may indicate an identity of the one or more pathogens 104 that correspond to one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples may be biological samples. Examples of such biological samples 102 include, but are not limited to, blood samples 102, fecal samples 102, urine samples 102, and the like.

FIG. 35 illustrates an operational flow 3500 representing examples of operations that are related to the performance of a method for analysis of one or more pathogens 104. In FIG. 35 and in following figures that include various examples of operations used during performance of the method, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various operations are presented in the sequence(s) illustrated, it should be understood that the various operations may be performed in other orders than those which are illustrated, or may be performed concurrently.

After a start operation, the operational flow 3500 includes a separating operation 3510 involving separating one or more magnetically active pathogen indicator complexes from one or more samples through use of one or more magnetic fields and one or more separation fluids that are in substantially antiparallel flow with the one or more samples. In some embodiments, separating operation 3510 may include separating the one or more magnetically active pathogen indicator complexes through use of magnetic attraction or magnetic repulsion. In some embodiments, separating operation 3510 may include separating the one or more magnetically active pathogen indicator complexes through use of one or more ferrofluids.

After a start operation, the operational flow 3500 may optionally include a detecting operation 3520 involving detecting one or more pathogen indicators with one or more detection units. In some embodiments, detecting operation 3520 may include detecting the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

After a start operation, the operational flow 3500 may optionally include an identifying operation 3530 involving identifying one or more pathogens present within the one or more samples. In some embodiments, identifying operation 3530 may include identifying the one or more pathogens that include at least one virus, bacterium, prion, worm, egg, cyst, protozoan, single-celled organism, fungus, algae, pathogenic protein, or microbe. In some embodiments, identifying operation 3530 may include displaying an identity of the one or more pathogens present within the one or more samples.

FIG. 36 illustrates alternative embodiments of the example operational flow 3500 of FIG. 35. FIG. 36 illustrates example embodiments where the separating operation 3510 may include at least one additional operation. Additional operations may include an operation 3602, and/or an operation 3604.

At operation 3602, the separating operation 3510 may include separating the one or more magnetically active pathogen indicator complexes through use of magnetic attraction or magnetic repulsion. In some embodiments, one or more magnetically active pathogen indicator complexes may be separated from one or more samples 102 through use of magnetic attraction. For example, in some embodiments, one or more magnetically active pathogen indicator complexes may include a magnetically active material that is attracted to one or more magnets. Accordingly, magnetically active pathogen indicator complexes may be separated from one or more samples 102 by causing the one or more samples 102 to flow in a substantially parallel manner with one or more separation fluids (e.g., an H-filter) and using one or more magnets to cause translocation of the one or more magnetically active pathogen indicator complexes from the one or more samples 102 into the one or more separation fluids. Examples of such magnets include, but are not limited to, electromagnets, permanent magnets, and magnets made from ferromagnetic materials (e.g., Co, Fe, FeOFe2O3, NiOFe2O3, CuOFe2O3, MgOFe2O3, MnBi, Ni, MnSb, MnOFe2O3, Y3Fe5O12 CrO2, MnAs, Gd, Dy, and EuO). In some embodiments, magnetic particles may be included within the one or more separation fluids. Accordingly, magnetically active pathogen indicator complexes may be attracted to the magnetic separation fluid and thereby separated from the one or more samples. In some embodiments, magnetically active pathogen indicator complexes may be attracted to magnetically active particles within the one or more separation fluids and thereby separated from the one or more samples 102.

In some embodiments, one or more magnetically active pathogen indicator complexes may be separated from one or more samples 102 through use of magnetic repulsion (e.g., through use of an eddy current). For example, in some embodiments, one or more magnetically active pathogen indicator complexes may include a magnetically active material that is repelled by one or more magnets. In some embodiments, the magnetically active material that is repelled by one or more magnets may include a non-ferrous metallic material, such as aluminum and/or copper. Accordingly, magnetically active pathogen indicator complexes may be separated from one or more samples 102 by causing the one or more samples to flow in a substantially parallel manner with one or more separation fluids and using one or more magnets to cause translocation of the one or more magnetically active pathogen indicator complexes from the one or more samples 102 into the one or more separation fluids.

At operation 3604, the separating operation 3510 may include separating the one or more magnetically active pathogen indicator complexes through use of one or more ferrofluids. In some embodiments, one or more magnetically active pathogen indicator complexes may be separated from one or more samples 102 through use of one or more ferrofluids. For example, in some embodiments, one or more ferrofluids may be used as separation fluids. In some embodiments, such separation fluids may be aqueous solutions. In some embodiments, such separation fluids may be nonaqueous solutions. In some embodiments, such separation fluids may be solvent solutions. For example, in some embodiments, such separation fluids may include organic solvents. In some embodiments, such separation fluids may be immiscible with water. Accordingly, in some embodiments, mixing of one or more sample fluids and one or more separation fluids may be avoided through use of immiscible fluids.

FIG. 37 illustrates alternative embodiments of the example operational flow 3500 of FIG. 35. FIG. 37 illustrates example embodiments where the detecting operation 3520 may include at least one additional operation. Additional operations may include an operation 3702.

At operation 3702, the detecting operation 3520 may include detecting the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more detection units 122 may be used to detect one or more pathogen indicators 106 with at least one technique that includes spectroscopy, electrochemical detection, polynucleotide detection, fluorescence anisotropy, fluorescence resonance energy transfer, electron transfer, enzyme assay, electrical conductivity, isoelectric focusing, chromatography, immunoprecipitation, immunoseparation, aptamer binding, filtration, electrophoresis, use of a CCD camera, immunoassay, or substantially any combination thereof. In some embodiments, one or more detection units 122 may be configured to detect one or more pathogen indicators 106 that have been processed by one or more microfluidic chips 108. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect the one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108. In some embodiments, one or more microfluidic chips 108 may be configured to process one or more samples 102 through use of surface plasmon resonance. In some embodiments, the one or more microfluidic chips 108 may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more microfluidic chips 108 may include an exposed substrate surface that is configured to operably associate with one or more prisms that are included within one or more detection units 122. In some embodiments, one or more microfluidic chips 108 may include a nuclear magnetic resonance (NMR) probe. In such embodiments, the microfluidic chips 108 may be configured to associate with one or more detection units 122 that accept the NMR probe and are configured to detect one or more pathogen indicators 106 through use of NMR spectroscopy. Accordingly, microfluidic chips 108 and detection units 122 may be configured in numerous ways to associate with each other to provide for detection of one or more pathogen indicators 106.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more microfluidic chips 108 may be configured to process one or more samples 102 and provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a pollen particle, a dander particle, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors are configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to process one or more samples 102 and provide for detection of one or more pathogen indicators 106 through use of isoelectric focusing. In some embodiments, native isoelectric focusing may be utilized to process and/or detect one or more pathogen indicators 106. In some embodiments, denaturing isoelectric focusing may be utilized to process and/or detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (clEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more microfluidic chips 108 may be configured to process one or more samples 102 through use of methods that include isoelectric focusing. In some embodiments, one or more detection units 122 may be configured to operably associate with one or more such microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding molecules and/or binding complexes that bind to one or more pathogen indicators 106 that may be present with the one or more samples 102 to form a pathogen indicator-binding molecule/binding complex. Examples of such binding molecules and/or binding complexes that bind to one or more pathogen indicators 106 include, but are not limited to, antibodies, aptamers, peptides, proteins, polynucleotides, and the like. In some embodiments, a pathogen indicator-binding molecule/binding complex may be processed through use of isoelectric focusing and then detected with one or more detection units 122. In some embodiments, one or more binding molecules and/or one or more binding complexes may include a label. Numerous labels may be used and include, but are not limited to, radioactive labels, fluorescent labels, colorimetric labels, spin labels, and the like. Accordingly, in some embodiments, a pathogen indicator-binding molecule (labeled)/binding complex (labeled) may be processed through use of isoelectric focusing and then detected with one or more detection units 122 that are configured to detect the one or more labels. Microfluidic chips 108 and detection units 122 may be configured in numerous ways to process one or more samples 102 and detect one or more pathogen indicators 106 through use of isoelectric focusing.

In some embodiments, one or more pathogen indicators 106 may be detected through use of chromatographic methodology alone or in combination with additional processing and/or detection methods. In some embodiments, one or more microfluidic chips 108 may be configured to process one or more samples 102 and provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106 that were processed through use of chromatographic methods. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are processed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. In some embodiments, one or more detection units 122 may be configured to utilize one or more ion-specific electrodes to detect one or more pathogen indicators 106. For example, such electrodes may be used to detect pathogen indicators 106 that include, but are not limited to, metals (e.g., tin, silver, nickel, cobalt, chromate), nitrates, nitrites, sulfites, and the like. Such pathogen indicators 106 are often associated with food, beverages, clothing, jewelry, and the like. Accordingly, chromatographic methods may be used in combination with additional methods and in combination with numerous types of detection methods.

In some embodiments, one or more pathogen indicators 106 may be detected through use of immunoprecipitation. In some embodiments, one or more detection units 122 may be configured to detect one or more pathogen indicators 106 through use of immunoprecipitation. In some embodiments, immunoprecipitation may be utilized in combination with additional processing and/or detection methods to detect one or more pathogen indicators 106. In some embodiments, one or more microfluidic chips 108 may be configured to process one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be detected through use of immunoseparation. In some embodiments, one or more detection units 122 may be configured to detect one or more pathogen indicators 106 through use of immunoseparation. In some embodiments, immunoseparation may be utilized in combination with additional processing and/or detection methods to detect one or more pathogen indicators 106. In some embodiments, one or more microfluidic chips 108 may be configured to process one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex.

Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more pathogen indicators 106 may be detected through use of aptamer binding. In some embodiments, one or more detection units 122 may be configured to detect one or more pathogen indicators 106 through use of aptamer binding. In some embodiments, aptamer binding may be utilized in combination with additional processing and/or detection methods to detect one or more pathogen indicators 106. In some embodiments, one or more microfluidic chips 108 may be configured to process one or more samples 102 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to process one or more samples 102 through use of electrophoresis. In some embodiments, such microfluidic chips 108 may be configured to operably associate with one or more detection units 122. Accordingly, in some embodiments, one or more detection units 122 may be configured to operably associate with one or more microfluidic chips 108 and detect one or more pathogen indicators 106 that were processed through use of electrophoresis. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to process and detect one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous processing methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to process one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to process one or more samples 102 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 38 illustrates alternative embodiments of the example operational flow 3500 of FIG. 35. FIG. 38 illustrates example embodiments where the identifying operation 3530 may include at least one additional operation. Additional operations may include an operation 3802, and/or an operation 3804.

At operation 3802, the identifying operation 3530 may include identifying the one or more pathogens that include at least one virus, bacterium, prion, worm, egg, cyst, protozoan, single-celled organism, fungus, algae, pathogenic protein, or microbe. In some embodiments, one or more display units 124 may indicate an identity of one or more pathogens 104 that include at least one virus, bacterium, prion, worm, egg, cyst, protozoan, single-celled organism, fungus, algae, pathogenic protein, microbe, or substantially any combination thereof.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g.,.Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At operation 3804, the identifying operation 3530 may include displaying an identity of the one or more pathogens present within the one or more samples. In some embodiments, one or more display units 124 may indicate an identity of the one or more pathogens 104 that correspond to one or more pathogen indicators 106 present within the one or more samples 102. In some embodiments, such display units 124 may include one or more active display units 124. In some embodiments, such display units 124 may include one or more passive display units 124. In some embodiments, one or more display units 124 may be operably associated with one or more microfluidic chips 108 that are configured to process one or more samples 102. In some embodiments, one or more display units 124 may be operably associated with one or more analysis units 120. In some embodiments, one or more display units 124 may be operably associated with one or more detection units 122. Accordingly, in some embodiments, one or more display units 124 may be configured to display the identity of one or more pathogens 104 that are present and/or absent from one or more samples 102. In some embodiments, one or more display units 124 may be configured to display the concentration of one or more pathogens 104 that are present and/or absent from one or more samples 102. In some embodiments, the one or more samples may be biological samples. Examples of such biological samples 102 include, but are not limited to, blood samples 102, fecal samples 102, urine samples 102, and the like.

### II. SYSTEMS FOR ANALYSIS OF ONE OR MORE PATHOGENS

FIG. 39 illustrates a system 3900 representing examples of modules that may be used to perform a method for analysis of one or more pathogens 104. In FIG. 39, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The system 3900 includes module 3910 that includes one or more microfluidic chips configured to facilitate detection of one or more pathogen indicators associated with one or more samples. In some embodiments, module 3910 may include one or more microfluidic chips configured to facilitate detection of the one or more pathogen indicators associated with one or more liquids. In some embodiments, module 3910 may include one or more microfluidic chips configured to facilitate detection of the one or more pathogen indicators associated with one or more solids. In some embodiments, module 3910 may include one or more microfluidic chips configured to facilitate detection of the one or more pathogen indicators associated with one or more gases. In some embodiments, module 3910 may include one or more microfluidic chips configured to facilitate detection of the one or more pathogen indicators associated with one or more airborne pathogens. In some embodiments, module 3910 may include one or more microfluidic chips configured to facilitate detection of the one or more pathogen indicators associated with one or more food products. In some embodiments, module 3910 may include one or more microfluidic chips configured to facilitate detection of the one or more pathogen indicators associated with one or more biological products. In some embodiments, module 3910 may include one or more microfluidic chips configured to facilitate detection of the one or more pathogen indicators through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay. In some embodiments, module 3910 may include one or more microfluidic chips configured for detachable connection to the one or more detection units.

The system 3900 includes module 3920 that includes one or more detection units configured to detect the one or more pathogen indicators. In some embodiments, module 3920 may include one or more detection units configured to detect the one or more pathogen indicators that are associated with one or more pathogens that are airborne. In some embodiments, module 3920 may include one or more detection units configured to detect the one or more pathogen indicators that are associated with one or more food products. In some embodiments, module 3920 may include one or more detection units that are configured to detect one or more pathogens that include at least one virus, bacterium, prion, worm, egg, cyst, protozoan, single-celled organism, fungus, algae, pathogenic protein, or microbe. In some embodiments, module 3920 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, module 3920 may include one or more detection units that are configured for detachable connection to the one or more microfluidic chips.

The system 3900 may optionally include module 3930 that includes one or more display units operably associated with the one or more detection units. In some embodiments, module 3930 may optionally include one or more display units that include one or more passive display units. In some embodiments, module 3930 may optionally include one or more display units that include one or more active display units. In some embodiments, module 3930 may optionally include one or more display units that indicate a presence or an absence of one or more pathogens within the one or more samples. In some embodiments, module 3930 may optionally include one or more display units that indicate an identity of one or more pathogens present within the one or more samples. In some embodiments, module 3930 may optionally include one or more display units that indicate one or more concentrations of one or more pathogens within the one or more samples.

The system 3900 may optionally include module 3940 that includes one or more reagent delivery units configured to deliver one or more reagents to the one or more microfluidic chips. In some embodiments, module 3940 may optionally include one or more reagent delivery units configured for detachable connection to the one or more microfluidic chips. In some embodiments, module 3940 may optionally include one or more reagent reservoirs. In some embodiments, module 3940 may optionally include one or more waste reservoirs. In some embodiments, module 3940 may optionally include one or more reagent delivery units physically coupled to the one or more microfluidic chips. In some embodiments, module 3940 may optionally include one or more reagent delivery units that include one or more pumps.

The system 3900 may optionally include module 3950 that includes one or more centrifugation units. In some embodiments, module 3950 may optionally include one or more centrifugation units configured to centrifuge the one or more microfluidic chips that are operably associated with the one or more centrifugation units. In some embodiments, module 3950 may optionally include one or more centrifugation units configured to provide for chromatographic separation. In some embodiments, module 3950 may optionally include one or more centrifugation units configured for polynucleotide extraction from the one or more samples. In some embodiments, module 3950 may optionally include one or more centrifugation units configured to provide for gradient centrifugation.

The system 3900 may optionally include module 3960 that includes one or more reservoir units. In some embodiments, module 3960 may optionally include one or more reservoirs that are configured for containing the one or more reagents. In some embodiments, module 3960 may optionally include one or more reservoirs that are configured as one or more waste reservoirs.

FIG. 40 illustrates alternative embodiments of system 3900 of FIG. 39. FIG. 40 illustrates example embodiments of module 3910. Additional embodiments may include an embodiment 4002, an embodiment 4004, an embodiment 4006, and/or an embodiment 4008.

At embodiment 4002, module 3910 includes one or more microfluidic chips configured to facilitate detection of the one or more pathogen indicators associated with one or more liquids. In some embodiments, a system may include one or more microfluidic chips 108 configured to facilitate detection of the one or more pathogen indicators 106 associated with one or more liquids. Examples of such liquids include, but are not limited to, beverages, water, food products, solvents, biological fluids, and the like. In some embodiments, the one or more liquids may be directly analyzed for a presence or an absence of one or more pathogen indicators 106. In some embodiments, the one or more liquids may be extracted to facilitate detection of the one or more pathogen indicators 106 associated with one or more liquids. For example, in some embodiments, a microfluidic chip 108 may be configured to accept a water sample and facilitate detection of one or more pathogens 104 that are associated with water.

At embodiment 4004, module 3910 includes one or more microfluidic chips configured to facilitate detection of the one or more pathogen indicators associated with one or more solids. In some embodiments, a system may include one or more microfluidic chips 108 configured to facilitate detection of the one or more pathogen indicators 106 associated with one or more solids. In some embodiments, a microfluidic chip 108 may be configured to suspend a solid sample 102 in a fluid. In some embodiments, a microfluidic chip 108 may be configured to extract one or more solid samples 102 with one or more solvents. In some embodiments, such microfluidic chips 108 may be configured to crush a sample 102 into smaller particles. For example, in some embodiments, a microfluidic chip 108 may crush a solid sample 102. In some embodiments, a microfluidic chip 108 may include one or more sonicators that break a sample 102 into smaller particles to facilitate detection of one or more pathogen indicators 106 that may be present within the sample 102. For example, in some embodiments, viral particles may be broken into smaller particles to provide for detection of one or more polynucleotides that are associated with the viral particles. Accordingly, microfluidic chips 108 may be configured in numerous ways such that they may analyze one or more samples 102 that include a solid.

At embodiment 4006, module 3910 includes one or more microfluidic chips configured to facilitate detection of the one or more pathogen indicators associated with one or more gases. In some embodiments, a system may include one or more microfluidic chips 108 that are configured facilitate detection of the one or more pathogen indicators 106 associated with one or more gases. In some embodiments, pathogen indicators that are associated with one or more gases include pathogen indicators 106 that are airborne. Examples of such airborne pathogen indicators 106 include, but are not limited to, fungal spores, mold spores, viruses, bacterial spores, and the like. In some embodiments, one or more gases that are being analyzed may be passed through one or more microfluidic chips 108. In some embodiments, gas may be pumped through a microfluidic chip 108. In some embodiments, gas may be drawn through a microfluidic chip 108 through use of a vacuum. In some embodiments, gas may be passed through a filter on which suspected pathogen indicators 106 may be collected for analysis. In some embodiments, gas may be passed through a bubble chamber in which pathogen indicators 106 may be collected for analysis. Accordingly, large volumes of gas may be analyzed.

At embodiment 4008, module 3910 includes one or more microfluidic chips configured to facilitate detection of the one or more pathogen indicators associated with one or more airborne pathogens. In some embodiments, a system may include one or more microfluidic chips 108 that are configured to facilitate detection of the one or more pathogen indicators 106 associated with one or more airborne pathogens 104. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more microfluidic chips 108 may be configured to analyze and/or detect severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

FIG. 41 illustrates alternative embodiments of system 3900 of FIG. 39. FIG. 41 illustrates example embodiments of module 3910. Additional embodiments may include an embodiment 4102, an embodiment 4104, an embodiment 4106, and/or an embodiment 4108.

At embodiment 4102, module 3910 includes one or more microfluidic chips configured to facilitate detection of the one or more pathogen indicators associated with one or more food products. In some embodiments, a system may include one or more microfluidic chips 108 that are configured to facilitate detection of the one or more pathogen indicators 106 associated with one or more food products. Examples of such food associated pathogens 104 include, but are not limited to, microbes, viruses, bacteria, worms, eggs, cysts, prions, protozoans, single-celled organisms, fungi, algaes, pathogenic proteins, and the like. Numerous food associated pathogens 104 are known and have been described. In some embodiments, one or more microfluidic chips 108 may be configured to analyze one or more polynucleotides, one or more polypeptides, one or more portions of one or more polynucleotides, and/or one or more portions of one or more polypeptides that have a nucleic acid sequence and/or an amino acid sequence that corresponds to one or more pathogens 104. The amino acid and/or nucleic acid sequences of numerous pathogens 104 are known and have been reported (e.g., Giardia genome project, Influenza genome sequencing project, Entamoeba histolytica genome project, and the like). Accordingly, one or more microfluidic chips 108 may be configured to process numerous types of food products to facilitate detection of numerous types of pathogen indicators 106.

At embodiment 4104, module 3910 includes one or more microfluidic chips configured to facilitate detection of the one or more pathogen indicators associated with one or more biological products. In some embodiments, a system may include one or more microfluidic chips 108 that are configured to facilitate detection of the one or more pathogen indicators 106 associated with one or more biological samples. Examples of biological samples 102 include, but are not limited to, blood, cerebrospinal fluid, mucus, breath, urine, fecal material, skin, tissue, tears, hair, and the like.

At embodiment 4106, module 3910 includes one or more microfluidic chips configured to facilitate detection of the one or more pathogen indicators through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay. In some embodiments, a system may include one or more microfluidic chips 108 that are configured to facilitate detection of the one or more pathogen indicators 106 through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, competition assay, or substantially any combination thereof.

In some embodiments, one or more microfluidic chips 108 may be configured to analyze one or more pathogen indicators 106 through use of polynucleotide interaction. Numerous methods based on polynucleotide interaction may be used. Examples of such methods include, but are not limited to, those based on polynucleotide hybridization, polynucleotide ligation, polynucleotide amplification, polynucleotide degradation, and the like. Methods that utilize intercalation dyes, FRET analysis, capacitive DNA detection, and nucleic acid amplification have been described (e.g., U.S. Patent Nos.: 7,118,910 and 6,960,437; herein incorporated by reference). In some embodiments, fluorescence resonance energy transfer, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube are combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Methods to utilize carbon nanotubes as probes for nucleic acid interaction have been described (e.g., U.S. Patent No.: 6,821,730; herein incorporated by reference).

In some embodiments, one or more microfluidic chips 108 may be configured to analyze one or more pathogen indicators 106 through use of protein interaction. Numerous methods based on protein interaction may be used. In some embodiments, protein interaction may be used to immobilize one or more pathogen indicators 106. In some embodiments, protein interaction may be used to separate one or more pathogen indicators 106 from one or more samples 102. Examples of such methods include, but are not limited to, those based on ligand binding, protein-protein binding, protein cross-linking, use of green fluorescent protein, phage display, the two-hybrid system, protein arrays, fiber optic evanescent wave sensors, chromatographic techniques, fluorescence resonance energy transfer, regulation of pH to control protein assembly and/or oligomerization, and the like. Methods that may be used to construct protein arrays have been described (e.g., Warren et al., Anal. Chem., 76:4082-4092 (2004) and Walter et al., Trends Mol. Med., 8:250-253 (2002), U.S. Patent No.: 6,780,582; herein incorporated by reference).

In some embodiments, one or more microfluidic chips 108 may be configured to analyze one or more samples 102 through use of peptide interaction. Peptides are generally described as being polypeptides that include less than one hundred amino acids. For example, peptides include dipeptides, tripeptides, and the like. In some embodiments, peptides may include from two to one hundred amino acids. In some embodiments, peptides may include from two to fifty amino acids. In some embodiments, peptides may include from two to one twenty amino acids. In some embodiments, peptides may include from ten to one hundred amino acids. In some embodiments, peptides may include from ten to fifty amino acids. Accordingly, peptides can include numerous numbers of amino acids. Numerous methods based on peptide interaction may be used. In some embodiments, peptide interaction may be used to immobilize one or more pathogen indicators 106. In some embodiments, peptide interaction may be used to separate one or more pathogen indicators 106 from one or more samples 102. Examples of such methods include, but are not limited to, those based on ligand binding, peptide-protein binding, peptide-peptide binding, peptide-polynucleotide binding, peptide cross-linking, use of green fluorescent protein, phage display, the two-hybrid system, protein arrays, peptide arrays, fiber optic evanescent wave sensors, chromatographic techniques, fluorescence resonance energy transfer, regulation of pH to control peptide and/or protein assembly and/or oligomerization, and the like. Accordingly, virtually any technique that may be used to analyze proteins may be utilized for the analysis of peptides. In some embodiments, high-speed capillary electrophoresis may be used to detect binding through use of fluorescently labeled phosphopeptides as affinity probes (Yang et al., Anal. Chem., 10.1021/ac061936e (2006)). Methods to immobilize proteins and peptides have been reported (Taylor, Protein Immobilization: Fundamentals and Applications, Marcel Dekker, Inc., New York (1991)).

In some embodiments, one or more microfluidic chips 108 may be configured to analyze one or more samples 102 through use of antibody interaction. Antibodies may be raised that will bind to numerous pathogen indicators 106 through use of known methods (e.g., Harlow and Lane, Antibodies: A Laboratory Manual, Cold Spring Harbor Laboratory Press, Cold Spring Harbor, New York (1988)). Antibodies may be configured in numerous ways within one or more microfluidic chips 108 to process one or more pathogen indicators 106. For example, in some embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. A labeled detector antibody that binds to the pathogen indicator 106 (or the antibody-pathogen indicator 106 complex) may then be passed over the one or more antibody-pathogen indicator 106 complexes such that the labeled detector antibody will label the pathogen indicator 106 (or the antibody-pathogen indicator 106 complex). Numerous labels may be used that include, but are not limited to, enzymes, fluorescent molecules, radioactive labels, spin labels, redox labels, and the like. In other embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. Such binding provides for detection of the antibody-pathogen indicator 106 complex through use of methods that include, but are not limited to, surface plasmon resonance, conductivity, and the like (e.g., U.S. Patent No.: 7,030,989; herein incorporated by reference). In some embodiments, antibodies may be coupled to a substrate within a microfluidic chip 108 to provide for a competition assay. One or more samples 102 may be mixed with one or more reagent mixtures that include one or more labeled pathogen indicators 106. The mixture may then be passed over the antibodies to facilitate binding of pathogen indicators 106 in the sample 102 and labeled pathogen indicators 106 in the reagent mixture to the antibodies. The unlabeled pathogen indicators 106 in the sample 102 will compete with the labeled pathogen indicators 106 in the reagent mixture for binding to the antibodies. Accordingly, the amount of label bound to the antibodies will vary in accordance with the concentration of unlabeled pathogen indicators 106 in the sample 102. In some embodiments, antibody interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more antibodies may be used in conjunction with one or more aptamers to process one or more samples 102. Accordingly, in some embodiments, aptamers and antibodies may be used interchangeably to process one or more samples 102.

In some embodiments, one or more microfluidic chips 108 may be configured to analyze one or more samples 102 through use of chemical interaction. In some embodiments, one or more microfluidic chips 108 may be configured to utilize chemical extraction to process one or more samples 102. For example, in some embodiments, one or more samples 102 may be mixed with a reagent mixture that includes one or more solvents in which the one or more pathogen indicators 106 are soluble. Accordingly, the solvent phase containing the one or more pathogen indicators 106 may be separated from the sample phase to provide for detection of the one or more pathogen indicators 106. In some embodiments, one or more samples 102 may be mixed with a reagent mixture that includes one or more chemicals that cause precipitation of one or more pathogen indicators 106. Accordingly, the sample phase may be washed away from the one or more precipitated pathogen indicators 106 to provide for detection of the one or more pathogen indicators 106. Accordingly, reagent mixtures that include numerous types of chemicals that interact with one or more pathogen indicators 106 may be used. In some embodiments, one or more microfluidic chips 108 may be configured to analyze one or more samples 102 through use of diffusion. In some embodiments, one or more microfluidic chips 108 may be configured to process one or more fluid samples 102 through use of an H-filter. For example, a microfluidic chip 108 may be configured to include a channel through which a fluid sample 102 and a second fluid flow such that the fluid sample 102 and the second fluid undergo substantially parallel flow through the channel without significant mixing of the sample fluid and the second fluid. As the fluid sample 102 and the second fluid flow through the channel, one or more pathogen indicators 106 in the fluid sample 102 may diffuse through the fluid sample 102 into the second fluid. Accordingly, such diffusion provides for the separation of the one or more pathogen indicators 106 from the sample 102. Methods to construct H-filters have been described (e.g., U.S. Patent Nos.: 6,742,661; 6,409,832; 6,007,775; 5,974,867; 5,971,158; 5,948,684; 5,932,100; 5,716,852; herein incorporated by reference). In some embodiments, diffusion based methods may be combined with immunoassay based methods to process and detect one or more pathogen indicators 106. Methods to conduct microscale diffusion immunoassays have been described (e.g., U.S. Patent No.: 6,541,213; herein incorporated by reference). Accordingly, microfluidic chips 108 may be configured in numerous ways to process one or more pathogen indicators 106 through use of diffusion.

In some embodiments, one or more microfluidic chips 108 may be configured to analyze one or more samples 102 through use of filtration. In some embodiments, one or more microfluidic chips 108 may be configured to include one or more filters that have a molecular weight cut-off. For example, a filter may allow molecules of low molecular weight to pass through the filter while disallowing molecules of high molecular weight to pass through the filter. Accordingly, one or more pathogen indicators 106 that are contained within a sample 102 may be allowed to pass through a filter while larger molecules contained within the sample 102 are disallowed from passing through the filter. Accordingly, in some embodiments, a microfluidic chip 108 may include two or more filters that selectively retain, or allow passage, of one or more pathogen indicators 106 through the filters. Such configurations provide for selective separation of one or more pathogen indicators 106 from one or more samples 102. Membranes and filters having numerous molecular weight cut-offs are commercially available (e.g., Millipore, Billerica, MA). In some embodiments, one or more microfluidic chips 108 may be configured to provide for dialysis of one or more samples 102. For example, in some embodiments, a microfluidic chip 108 may be configured to contain one or more samples 102 in one or more sample chambers that are separated from one or more dialysis chambers by a semi-permeable membrane. Accordingly, in some embodiments, one or more pathogen indicators 106 that are able to pass through the semi-permeable membrane may be collected in the dialysis chamber. In other embodiments, one or more pathogen indicators 106 may be retained in the one or more sample chambers while other sample 102 components may be separated from the one or more pathogen indicators 106 by their passage through the semi-permeable membrane into the dialysis chamber. Accordingly, one or more microfluidic chips 108 may be configured to include two or more dialysis chambers for selective separation of one or more pathogen indicators 106 from one or more samples 102. Semi-permeable membranes and dialysis tubing is available from numerous commercial sources (e.g., Millipore, Billerica, MA; Pierce, Rockford, IL; Sigma-Aldrich, St. Louis, MO). Methods that may be used for microfiltration have been described (e.g., U.S. Patent No.: 5,922,210; herein incorporated by reference).

In some embodiments, one or more microfluidic chips 108 may be configured to analyze one or more samples 102 through use of chromatography. Numerous chromatographic methods may be used to process one or more samples 102. Examples of such chromatographic methods include, but are not limited to, ionexchange chromatography, affinity chromatography, gel filtration chromatography, hydroxyapatite chromatography, gas chromatography, reverse phase chromatography, thin layer chromatography, capillary chromatography, size exclusion chromatography, hydrophobic interaction media, and the like. In some embodiments, a microfluidic chip 108 may be configured to process one or more samples 102 through use of one or more chromatographic methods. In some embodiments, chromatographic methods may be used to process one or more samples 102 for one or more pathogen indicators 106 that include one or more polynucleotides. For example, in some embodiments, one or more samples 102 may be applied to a chromatographic media to which the one or more polynucleotides bind. The remaining components of the sample 102 may be washed from the chromatographic media. The one or more polynucleotides may then be eluted from chromatographic media in a more purified state. Similar methods may be used to process one or more samples 102 for one or more pathogen indicators 106 that include one or more proteins or polypeptides (e.g., Mondal and Gupta, Biomol. Eng., 23:59-76 (2006)). Chromatography media able to separate numerous types of molecules is commercially available (e.g., Bio-Rad, Hercules, CA; Qiagen, Valencia, CA; Pfizer, New York, NY; Millipore, Billerica, MA; GE Healthcare BioSciences Corp., Piscataway, NJ).

In some embodiments, one or more microfluidic chips 108 may be configured to analyze one or more samples 102 through use of aptamer interaction. In some embodiments, one or more aptamers may include polynucleotides (e.g., deoxyribonucleic acid; ribonucleic acid; and derivatives of polynucleotides that may include polynucleotides that include modified bases, polynucleotides in which the phosphodiester bond is replaced by a different type of bond, or many other types of modified polynucleotides). In some embodiments, one or more aptamers may include peptide aptamers. Methods to prepare and use aptamers have been described (e.g., Collett et al., Methods, 37:4-15 (2005); Collet et al., Anal. Biochem., 338:113-123 (2005); Cox et al., Nucleic Acids Res., 30:20 e108 (2002); Kirby et al., Anal. Chem., 76:4066-4075 (2004); Ulrich, Handb. Exp. Pharmacol., 173:305-326 (2006); Baines and Colas, Drug Discovery Today, 11:334-341 (2006); Guthrie et al., Methods, 38:324-330 (2006); Geyer et al., Chapter 13: Selection of Genetic Agents from Random Peptide Aptamer Expression Libraries, Methods in Enzymology, Academic Press, pg. 171-208 (2000); U.S. Patent No.: 6,569,630; herein incorporated by reference). Aptamers may be configured in numerous ways within one or more microfluidic chips 108 to process one or more pathogen indicators 106. For example, in some embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Labeled detector antibodies and/or aptamers that bind to the pathogen indicator 106 (or the aptamer-pathogen indicator 106 complex) may then be passed over the one or more aptamer-pathogen indicator 106 complexes such that the labeled detector antibodies and/or aptamers will label the pathogen indicator 106 (or the aptamer-pathogen indicator 106 complex). Numerous labels may be used that include, but are not limited to, enzymes, fluorescent molecules, radioactive labels, spin labels, redox labels, and the like. In other embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Such binding provides for detection of the aptamer-pathogen indicator 106 complex through use of methods that include, but are not limited to, surface plasmon resonance, conductivity, and the like (e.g., U.S. Patent No.: 7,030,989; herein incorporated by reference). In some embodiments, aptamers may be coupled to a substrate within a microfluidic chip 108 to provide for a competition assay. One or more samples 102 may be mixed with one or more reagent mixtures that include one or more labeled pathogen indicators 106. The mixture may then be passed over the aptamers to facilitate binding of pathogen indicators 106 in the sample 102 and labeled pathogen indicators 106 in the reagent mixture to the aptamers. The unlabeled pathogen indicators 106 in the sample 102 will compete with the labeled pathogen indicators 106 in the reagent mixture for binding to the aptamers. Accordingly, the amount of label bound to the aptamers will vary in accordance with the concentration of unlabeled pathogen indicators 106 in the sample 102. In some embodiments, aptamer interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more aptamers may be used in conjunction with one or more antibodies to process one or more samples 102. In some embodiments, aptamers and antibodies may be used interchangeably to process one or more samples 102. Accordingly, in some embodiments, methods and/or systems for processing and/or detecting pathogen indicators 106 may utilize antibodies and aptamers interchangeably and/or in combination.

In some embodiments, one or more microfluidic chips 108 may be configured to analyze one or more samples 102 through use of electrical conductivity. In some embodiments, one or more samples 102 may be processed through use of magnetism. For example, in some embodiments, one or more samples 102 may be combined with one or more tagged polynucleotides that are tagged with a ferrous material, such as a ferrous bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed over an electromagnet to immobilize the hybridized complexes. Other components in the sample 102 may then be washed away from the hybridized complexes. In some embodiments, a chamber containing the magnetically immobilized hybridized complexes may be heated to release the sample polynucleotides from the magnetically immobilized tagged polynucleotides. The sample polynucleotides may then be collected in a more purified state. In other embodiments, similar methods may be used in conjunction with antibodies, aptamers, peptides, ligands, and the like. Accordingly, one or more microfluidic chips 108 may be configured in numerous ways to utilize magnetism to process one or more samples 102. In some embodiments, one or more samples 102 may be processed through use of eddy currents. Eddy current separation uses the principles of electromagnetic induction in conducting materials to separate non-ferrous metals by their different electric conductivities. An electrical charge is induced into a conductor by changes in magnetic flux cutting through it. Moving permanent magnets passing a conductor generates the change in magnetic flux. Accordingly, in some embodiments, one or more microfluidic chips 108 may be configured to include a magnetic rotor such that when conducting particles move through the changing flux of the magnetic rotor, a spiraling current and resulting magnetic field are induced. The magnetic field of the conducting particles may interact with the magnetic field of the magnetic rotor to impart kinetic energy to the conducting particles. The kinetic energy imparted to the conducting particles may then be used to direct movement of the conducting particles. Accordingly, non-ferrous particles, such as metallic beads, may be utilized to process one or more samples 102. For example, in some embodiments, one or more samples 102 may be combined with one or more tagged polynucleotides that are tagged with a non-ferrous material, such as an aluminum bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed through a magnetic field to impart kinetic energy to the non-ferrous bead. This kinetic energy may then be used to separate the hybridized complex. In other embodiments, similar methods may be used in conjunction with antibodies, aptamers, peptides, ligands, and the like. Accordingly, one or more microfluidic chips 108 may be configured in numerous ways to utilize eddy currents to process one or more samples 102. One or more microfluidic chips 108 may be configured in numerous ways to utilize electrical conductivity to process one or more samples 102.

In some embodiments, one or more microfluidic chips 108 may be configured to analyze one or more samples 102 through use of isoelectric focusing. Methods have been described that may be used to construct capillary isoelectric focusing systems (e.g., Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). Such systems may be modified to provide for the processing of one or more samples 102.

In some embodiments, one or more microfluidic chips 108 may be configured to analyze one or more samples 102 through use of electrophoresis. In some embodiments, one or more microfluidic chips 108 may be configured to process one or more samples 102 through use of one-dimensional electrophoresis. In some embodiments, one or more microfluidic chips 108 may be configured to process one or more samples 102 through use of two-dimensional electrophoresis. In some embodiments, one or more microfluidic chips 108 may be configured to process one or more samples 102 through use of gradient gel electrophoresis. In some embodiments, one or more microfluidic chips 108 may be configured to process one or more samples 102 through use of electrophoresis under denaturing conditions. In some embodiments, one or more microfluidic chips 108 may be configured to process one or more samples 102 through use of electrophoresis under native conditions. One or more microfluidic chips 108 may be configured to utilize numerous electrophoretic methods.

In some embodiments, one or more microfluidic chips 108 may be configured to analyze one or more samples 102 through use of immunoassay. In some embodiments, one or more microfluidic chips 108 may be configured to process one or more samples 102 through use of enzyme linked immunosorbant assay (ELISA). In some embodiments, one or more microfluidic chips 108 may be configured to process one or more samples 102 through use of radioimmuno assay (RIA). In some embodiments, one or more microfluidic chips 108 may be configured to process one or more samples 102 through use of enzyme immunoassay (EIA). In some embodiments, such methods may utilize antibodies (e.g., monoclonal antibodies, polyclonal antibodies, antibody fragments, single-chain antibodies, and the like), aptamers, or substantially any combination thereof. In some embodiments, a labeled antibody and/or aptamer may be used within an immunoassay. In some embodiments, a labeled ligand to which the antibody and/or aptamer binds may be used within an immunoassay. Numerous types of labels may be utilized. Examples of such labels include, but are not limited to, radioactive labels, fluorescent labels, enzyme labels, spin labels, magnetic labels, gold labels, colorimetric labels, redox labels, and the like. Numerous immunoassays are known and may be configured for processing one or more samples 102.

In some embodiments, one or more microfluidic chips 108 may be configured to analyze one or more samples 102 through use of one or more competition assays. In some embodiments, one or more microfluidic chips 108 may be configured to process one or more samples 102 through use of one or more polynucleotide based competition assays. One or more microfluidic chips 108 may be configured to include one or more polynucleotides coupled to a substrate, such as a polynucleotide array. The one or more microfluidic chips 108 may be further configured so that a sample 102 and/or substantially purified polynucleotides obtained from one or more samples 102, may be mixed with one or more reagent mixtures that include one or more labeled polynucleotides to form an analysis mixture. This analysis mixture is then passed over the substrate such that the labeled polynucleotides and the sample polynucleotides are allowed to hybridize to the polynucleotides that are immobilized on the substrate. The sample polynucleotides and the labeled polynucleotides will compete for binding to the polynucleotides that are coupled on the substrate. Accordingly, the presence and/or concentration of the polynucleotides in the sample 102 can be determined through detection of the label (e.g., the concentration of the polynucleotides in the sample 102 will be inversely related to the amount of label that is bound to the substrate). Numerous labels may be used that include, but are not limited to, enzymes, fluorescent molecules, radioactive labels, spin labels, redox labels, and the like. In some embodiments, one or more microfluidic chips 108 may be configured to include one or more antibodies, proteins, peptides, and/or aptamers that are coupled to a substrate. The one or more microfluidic chips 108 may be further configured so that a sample 102 and/or substantially purified sample polypeptides and/or sample peptides obtained from one or more samples 102, may be mixed with one or more reagent mixtures that include one or more labeled polypeptides and/or labeled peptides to form an analysis mixture. This analysis mixture can then be passed over the substrate such that the labeled polypeptides and/or labeled peptides and the sample polypeptides and/or sample peptides are allowed to bind to the antibodies, proteins, peptides, and/or aptamers that are immobilized on the substrate. The sample polypeptides and/or sample peptides and the labeled polypeptides and/or sample peptides will compete for binding to the antibodies, proteins, peptides, and/or aptamers that are coupled on the substrate. Accordingly, the presence and/or concentration of the sample polypeptides and/or sample peptides in the sample 102 can be determined through detection of the label (e.g., the concentration of the sample polypeptides and/or sample peptides in the sample 102 will be inversely related to the amount of label that is bound to the substrate). Numerous labels may be used that include, but are not limited to, enzymes, fluorescent molecules, radioactive labels, spin labels, redox labels, and the like. Microfluidic chips 108 may be configured to utilize numerous types of competition assays.

In some embodiments, one or more microfluidic chips 108 may be configured to utilize numerous analysis methods.

At embodiment 4108, module 3910 includes one or more microfluidic chips configured for detachable connection to the one or more detection units. In some embodiments, a system may include one or more microfluidic chips 108 configured for detachable connection to the one or more detection units 122. In some embodiments, a system may include one or more detection units 122 that are configured to detachably connect with microfluidic chips 108 that are configured to process and/or analyze different types of pathogen indicators 106. For example, a system may include a detection unit 122 that may detachably connect to a first microfluidic chip 108 that is configured to analyze airborne pathogen indicators 106 and detachably connect to a second microfluidic chip 108 that is configured to analyze food associated pathogen indicators 106. Accordingly, in some embodiments, a system may include a single detection unit 122 that may be utilized to detect numerous types of pathogen indicators 106 through use of microfluidic chips 108 that are configured to process and/or analyze numerous types of pathogen indicators 106. Such configurations may be configured for field use. For example, in some embodiments, a system may include one or more detection units 122 that are configured to associate with microfluidic chips 108 that are designed for single use. In some embodiments, such systems provide for the detection of specific pathogen indicators 106 through use of a common detection unit 122 that is configured to detachably connect with microfluidic chips 108 that are configured to process and/or analyze the specific pathogen indicators 106. The one or more detection units 122 may be configured to utilize numerous methods to detect one or more pathogen indicators 106. Examples of such methods include, but are not limited to, surface plasmon resonance, spectroscopy, radioassay, electrical conductivity, and the like.

FIG. 42 illustrates alternative embodiments of system 3900 of FIG. 39. FIG. 42 illustrates example embodiments of module 3920. Additional embodiments may include an embodiment 4202, an embodiment 4204, and/or an embodiment 4206.

At embodiment 4202, module 3920 may include one or more detection units configured to detect the one or more pathogen indicators that are associated with one or more pathogens that are airborne. In some embodiments, a system may include one or more detection units 122 configured to detect the one or more pathogen indicators 106 that are associated with one or more pathogens 104 that are airborne. Accordingly, in some embodiments, one or more detection units 122 may be configured to operably associate with the one or more microfluidic chips 108 and to detect one or more airborne pathogens 104. For example, in some embodiments, one or more microfluidic chips 108 may be configured to allow one or more air samples 102 to contact the one or more microfluidic chips 108 such that one or more pathogen indicators 106 included within the one or more air samples 102 are retained by the one or more microfluidic chips 108. In some embodiments, the one or more air samples 102 may be passed through a filter on which one or more airborne pathogen indicators 106 are collected. The collected airborne pathogen indicators 106 may then be washed from the filter and caused to pass over an antibody array to which the one or more airborne pathogen indicators 106 become immobilized. The immobilized airborne pathogen indicators 106 may then be detected through numerous methods that include, but are not limited to, electrical conductivity, immunoassay based methods, and the like. Accordingly, one or more detection units 122 may be configured to detect the one or more airborne pathogen indicators 106. In some embodiments, one or more detection units 122 may be configured to operably associate with one or more microfluidic chips 108 such that the one or more detection units 122 facilitate air flow through the one or more microfluidic chips 108 to provide for air sampling. For example, in some embodiments, one or more detection units 122 may include one or more fans to push and/or pull air through one or more operably associated microfluidic chips 108. In some embodiments, one or more detection units 122 may include one or more bellows to push and/or pull air through one or more operably associated microfluidic chips 108. Detection units 122 may be configured in numerous ways to provide for detection of one or more airborne pathogen indicators 106.

At embodiment 4204, module 3920 may include one or more detection units configured to detect the one or more pathogen indicators that are associated with one or more food products. In some embodiments, a system may include one or more detection units 122 configured to detect the one or more pathogen indicators 106 that are associated with one or more food products. Accordingly, in some embodiments, one or more detection units 122 may be configured to operably associate with the one or more microfluidic chips 108 and to detect one or more pathogen indicators 106 that are associated with one or more food products. Examples of such food associated pathogens include, but are not limited to, microbes, viruses, bacteria, worms, eggs, cysts, prions, protozoans, single-celled organisms, fungi, algaes, pathogenic proteins, and the like. Numerous food associated pathogens 104 are known and have been described. In some embodiments, one or more detection units 122 may be configured to detect one or more polynucleotides, one or more polypeptides, one or more portions of one or more polynucleotides, and/or one or more portions of one or more polypeptides that have a nucleic acid sequence and/or an amino acid sequence that corresponds to one or more pathogens 104. The amino acid and/or nucleic acid sequences of numerous pathogens 104 are known and have been reported (e.g., Giardia genome project, Influenza genome sequencing project, Entamoeba histolytica genome project, and the like).

At embodiment 4206, module 3920 may include one or more detection units that are configured to detect one or more pathogens that include at least one virus, bacterium, prion, worm, egg, cyst, protozoan, single-celled organism, fungus, algae, pathogenic protein, or microbe. In some embodiments, a system may include one or more detection units 122 configured to detect one or more pathogens 104 that include at least one virus, bacterium, prion, worm, egg, cyst, protozoan, single-celled organism, fungus, algae, protein, microbe, or substantially any combination thereof. A detection unit 122 may be configured to utilize numerous types of techniques, and combinations of techniques, to detect one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

FIG. 43 illustrates alternative embodiments of system 3900 of FIG. 39. FIG. 43 illustrates example embodiments of module 3920. Additional embodiments may include an embodiment 4302, and/or an embodiment 4304.

At embodiment 4302, module 3920 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, a system may include one or more detection units 122 configured to detect the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection units 122 may be configured to detect one or more pathogen indicators 106 that have been processed by one or more microfluidic chips 108 and/or analyzed by one or more analysis units 120. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108.

In some embodiments, one or more detection units 122 may be configured to detect one or more pathogen indicators 106 through use of surface plasmon resonance. In some embodiments, one or more detection units 122 may be configured to operably associate with one or more microfluidic chips 108 may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more microfluidic chips 108 to facilitate detection of one or more pathogen indicators 106 through use of surface plasmon resonance.

In some embodiments, one or more detection units 122 may be configured to detect one or more pathogen indicators 106 through use of nuclear magnetic resonance (NMR). In some embodiments, one or more detection units 122 may be configured to operably associate with one or more microfluidic chips 108 that include a nuclear magnetic resonance (NMR) probe. Accordingly, in some embodiments, one or more pathogen indicators 106 may be analyzed and detected with one or more microfluidic chips and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, one or more analysis units 120 may be configured to facilitate analysis of one or more pathogen indicators 106 and configured to facilitate fluorescent detection of the one or more pathogen indicators 106 with one or more detection units 122.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122 that is configured to operably associate with the one or more microfluidic chips 108.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more micro fluidic chips 108 may be configured to provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleic acid associated with one or more samples 102 may be combined with one or more sets of paired primers such that use of an amplification protocol, such as a polymerase chain reaction, will produce an amplification product corresponding to pathogen associated polynucleic acid that was contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such asa cyst, egg, pathogen, spore, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors may be configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be processed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Microfluidic chips 108 and detection units 122 may be configured in numerous ways to facilitate detection of one or more pathogen indicators 106 through use of isoelectric focusing.

In some embodiments, one or more pathogen indicators 106 may be detected through use of chromatographic methodology alone or in combination with additional detection methods. In some embodiments, one or more microfluidic chips 108 may be configured to provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection units 122 may be configured to detect one or more pathogen indicators 106 through use of immunoprecipitation. In some embodiments, immunoprecipitation may be utilized in combination with additional detection methods to detect one or more pathogen indicators 106. In some embodiments, one or more microfluidic chips 108 may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more detection units 122 may be configured to detect one or more pathogen indicators 106 through use of immunoseparation. In some embodiments, immunoseparation may be utilized in combination with additional detection methods to detect one or more pathogen indicators 106. In some embodiments, one or more microfluidic chips 108 may be configured to facilitate detection of one or more pathogen indicators 106 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection units 122 may be configured to detect one or more pathogen indicators 106 through use of aptamer binding. In some embodiments, aptamer binding may be utilized in combination with additional methods to detect one or more pathogen indicators 106. In some embodiments, one or more microfluidic chips 108 may be configured to facilitate detection of one or more pathogen indicators 106 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to facilitate detection of one or more pathogen indicators 106 through use of electrophoresis. In some embodiments, such microfluidic chips 108 may be configured to operably associate with one or more detection units 122. Accordingly, in some embodiments, one or more detection units 122 may be configured to operably associate with one or more microfluidic chips 108 and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to provide for detection of one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to facilitate detection of one or more pathogen indicators 106 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

At embodiment 4304, module 3920 may include one or more detection units that are configured for detachable connection to the one or more microfluidic chips. In some embodiments, one or more detection units 122 may be configured for detachable connection to the one or more microfluidic chips 108. In some embodiments, the one or more detection units 122 may be connected to the one or more microfluidic chips 108 through use of fasteners. Examples of such fasteners include, but are not limited to, hooks, screws, bolts, pins, grooves, adhesives, and the like. In some embodiments, the one or more detection units 122 may be connected to the one or more microfluidic chips 108 through use of magnets.

FIG. 44 illustrates alternative embodiments of system 3900 of FIG. 39. FIG. 44 illustrates example embodiments of module 3930. Additional embodiments may include an embodiment 4402, an embodiment 4404, an embodiment 4406, an embodiment 4408, and/or an embodiment 4410.

At embodiment 4402, module 3930 may include one or more display units that include one or more passive display units. In some embodiments, one or more display units 124 may display results of the detecting with one or more display units 124 that are passive display units 124. In some embodiments, one or display units 124 may include one or more liquid crystal displays (LCD). Methods to construct passive displays have been described (e.g., U.S. Patent Nos.: 4,807,967; 4,729,636, 4,436,378; 4,257,041; herein incorporated by reference).

At embodiment 4404, module 3930 may include one or more display units that include one or more active display units. In some embodiments, one or more display units 124 may display results of the detecting with one or more display units 124 that are active display units 124. Numerous active display units 124 are known and included, but are not limited to, quarter-video graphics array (QVGA), video graphics array (VGA), super video graphics array (SVGA), extended graphics array (XGA), wide extended graphics array (WXGA), super extended graphics array (SXGA), ultra extended graphics array (LTXGA), wide super extended graphics array (WSXGA), wide ultra extended graphics array (WUXGA).

At embodiment 4406, module 3930 may include one or more display units that indicate a presence or an absence of one or more pathogens within the one or more samples. In some embodiments, one or more display units 124 may indicate a presence or an absence of one or more pathogens 104 within the one or more samples 102. In some embodiments, one or more display units 124 may use a colorimetric message to indicate a presence or an absence of one or more pathogens 104 within one or more samples 102. For example, in some embodiments, one or more display units 124 may display a green light if one or more pathogens 104 are not found within one or more samples 102 and a red light if one or more pathogens 104 are found within one or more samples 102. In some embodiments, one or more display units 124 may use a pictographic message to indicate a presence or an absence of one or more pathogens 104 within one or more samples 102. For example, in some embodiments, one or more display units 124 may display a smiley face if one or more pathogens 104 are not found within one or more samples 102 and a frowny face if one or more pathogens 104 are found within one or more samples 102. In some embodiments, one or more display units 124 may use a typographical message to indicate a presence or an absence of one or more pathogens 104 within one or more samples 102. For example, in some embodiments, one or more display units 124 may display a "Pathogen Not Present" message if one or more pathogens 104 are not found within one or more samples 102 and a "Pathogen Present" message if one or more pathogens 104 are found within one or more samples 102. Such messages may be displayed in numerous languages. In some embodiments, one or more display units 124 may display one or more messages in multiple formats. For example, in some embodiments, one or more messages may be displayed in colored text.

At embodiment 4408, module 3930 may include one or more display units that indicate an identity of one or more pathogens present within the one or more samples. In some embodiments, one or more display units 124 may indicate an identity of one or more pathogens 104 present within the one or more samples 102. In some embodiments, one or more display units 124 may be operably associated with one or more microfluidic chips 108. Accordingly, in some embodiments, one or more display units 124 may be configured to display the identity of one or more pathogens 104 that are present and/or absent from one or more samples 102. For example, in some embodiments, a display unit 124 may be configured to indicate a presence or an absence of Salmonella in a food product.

At embodiment 4410, module 3930 may include one or more display units that indicate one or more concentrations of one or more pathogens within the one or more samples. In some embodiments, one or more display units 124 may indicate one or more concentrations of one or more pathogens 104 within the one or more samples 102. Concentration may be displayed in numerous formats. For example, in some embodiments, concentration may be expressed numerically. In some embodiments, concentration may be expressed graphically. For example, in some embodiments, one or more display units 124 may include a display having a gray scale on which the concentration of one or more pathogen indicators 106 and/or pathogens 104 that are present within one or more samples 102 may be indicated (e.g., higher concentrations of one or more pathogens 104 may be displayed as dark gray while lower concentrations of one or more pathogens 104 may be displayed as light gray). In some embodiments, one or more display units 124 may include a display having a color scale on which the concentration of one or more pathogens 104 that are present within one or more samples 102 may be indicated (e.g., low concentrations of one or more pathogen indicators 106 may be indicated by a green light, intermediate concentrations of one or more pathogen indicators 106 may be indicated by a yellow light, high concentrations of one or more pathogen indicators 106 may be indicated by a red light). In some embodiments, one or more display units 124 may be calibrated to an individual. For example, in some embodiments, a display unit 124 may be calibrated relative to a person who is immune compromised. Accordingly, in some embodiments, an individual may obtain an indication from a display that indicates if a food product contains a dangerous level of one or more pathogens 104.

FIG. 45 illustrates alternative embodiments of system 3900 of FIG. 39. FIG. 45 illustrates example embodiments of module 3940. Additional embodiments may include an embodiment 4502, an embodiment 4504, an embodiment 4506, an embodiment 4508, and/or an embodiment 4510.

At embodiment 4502, module 3940 may include one or more reagent delivery units configured for detachable connection to the one or more microfluidic chips. In some embodiments, a system may include one or more reagent delivery units 116 configured for detachable connection to the one or more microfluidic chips 108. Reagent delivery units 116 may be configured to deliver one or more types of reagents to one or more microfluidic chips 108. In some embodiments, such reagents may be utilized to analyze and/or process one or more samples 102. In some embodiments, such reagents may be utilized to facilitate detection of one or more pathogen indicators 106. Examples of such reagents include, but are not limited to, solvents, water, tags, labels, antibodies, aptamers, polynucleotides, and the like. In some embodiments, one or more reagent delivery units 116 may include connectors that may be coupled to one or more microfluidic chips 108 to provide for delivery of one or more reagents to the one or more microfluidic chips 108. Examples of such connectors include, but are not limited to, leur lock fittings, needles, fluid connectors, and the like. In some embodiments, a reagent delivery unit 116 may include one or more pumps. In some embodiments, a reagent delivery unit 116 may include numerous reservoirs that may include numerous types of reagents. Accordingly, in some embodiments, a reagent delivery unit 116 may be configured to detachably connect with numerous types of microfluidic chips 108 that are configured to facilitate analysis and/or detection of numerous types of pathogens 104 and/or pathogen indicators 106.

At embodiment 4504, module 3940 may include one or more reagent reservoirs. In some embodiments, a system may include one or more reagent reservoirs. In some embodiments, the one or more reagent reservoirs may be configured to contain reagents that may be used to facilitate analysis and/or detection of a single type of pathogen 104 and/or pathogen indicator 106. In some embodiments, the one or more reagent reservoirs may be configured to contain reagents that may be used to facilitate analysis and/or detection of multiple types of pathogens 104 and/or pathogen indicators 106.

At embodiment 4506, module 3940 may include one or more waste reservoirs. In some embodiments, a system may include one or more waste reservoirs. Such waste reservoirs may be configured in numerous ways. For example such waste reservoirs may be configured for containing reagents, samples 102, and the like. In some embodiments, waste reservoirs may be configured to containing liquids, solids, gels, and substantially any combination thereof.

At embodiment 4508, module 3940 may include one or more reagent delivery units physically coupled to the one or more microfluidic chips. In some embodiments, a system may include one or more reagent delivery units 116 physically coupled to the one or more microfluidic chips 108. For example, in some embodiments, one or more reagent delivery units 116 may be included within a microfluidic chip 108 (e.g., as opposed to being separate from a microfluidic chip 108). In some embodiments, such microfluidic chips 108 may be configured for single use to facilitate analysis and/or detection of one or more pathogen indicators 106 that may be present within one or more samples 102. The reagent delivery units 116 may contain numerous types of reagents that may provide for analysis of one or more samples 102.

For example, in some embodiments, a microfluidic chip 108 may be configured for extraction and/or analysis of polynucleotides that may be included within one or more samples 102. In some embodiments, such a microfluidic chip 108 may include: a first reagent delivery unit 116 that includes an alkaline lysis buffer (e.g., sodium hydroxide/sodium dodecyl sulfate), a second reagent delivery unit 116 that includes an agent that precipitates the sodium dodecyl sulfate (e.g., potassium acetate), a third reagent delivery unit 116 that includes an extraction agent (e.g., phenol/chloroform), and a fourth reagent delivery unit 116 that includes a precipitation agent for precipitating any polynucleotides that may be present with the one or more samples 102. Accordingly, in some embodiments, a system may include one or more microfluidic chips 108 that are configured to include all of the reagents necessary to facilitate analysis of one or more samples 102 for one or more pathogen indicators 106. In some embodiments, such microfluidic chips 108 may be configured for single use. In some embodiments, such microfluidic chips 108 may be configured for repeated use. In some embodiments, such microfluidic chips 108 may be configured to detachably connect to one or more detection units 122 such that the same detection unit 122 may be used repeatedly through association with a new microfluidic chip 108.

At embodiment 4510, module 3940 may include one or more reagent delivery units that include one or more pumps. In some embodiments, a system may include one or more reagent delivery units 116 that include one or more pumps. Numerous types of pumps may be associated with one or more reagent delivery units 116.

FIG. 46 illustrates alternative embodiments of system 3900 of FIG. 39. FIG. 44 illustrates example embodiments of module 3950. Additional embodiments may include an embodiment 4602, an embodiment 4604, an embodiment 4606, and/or an embodiment 4608.

At embodiment 4602, module 3950 may include one or more centrifugation units configured to centrifuge the one or more microfluidic chips that are operably associated with the one or more centrifugation units. In some embodiments, a system may include one or more centrifugation units 118 configured to centrifuge the one or more microfluidic chips 108 that are operably associated with the one or more centrifugation units 118. In some embodiments, one or more centrifugation units 118 may be configured to detachably associate with one or more microfluidic chips 108. For example, in some embodiments, a centrifugation unit 118 may include one or more centrifuge drives that are configured to detachably associate with one or more centrifuge rotors that are included within one or more microfluidic chips 108. In some embodiments, such centrifuge drives may magnetically couple with the one or more centrifuge rotors. In some embodiments, such centrifuge drives may physically couple with the one or more centrifuge rotors. In some embodiments, one or more centrifugation units 118 may be configured to centrifuge an entire microfluidic chip 108. For example, in some embodiments, a microfluidic chip 108 may be configured to associate with one or more centrifugation units 118 such that the microfluidic chip 108 is subjected to centrifugal force. In some embodiments, such a microfluidic chip 108 may be configured in a manner that resembles a compact disc. Accordingly, in some embodiments, a centrifugation unit 118 may be configured in a manner that resembles a compact disc player. In some embodiments, one or more centrifugation units 118 may be configured to centrifuge one or more samples 102 through a series of mesh filters to concentrate parasite eggs and/or larvae (e.g., U.S. Patent No.: 4081356; herein incorporated by reference).

At embodiment 4604, module 3950 may include one or more centrifugation units configured to provide for chromatographic separation. In some embodiments, a system may include one or more centrifugation units 118 configured to provide for chromatographic separation. For example, in some embodiments, one or more centrifugation units 118 may be configured to centrifuge one or more samples 102 through one or more chromatographic columns that are associated with one or more microfluidic chips 108. In some embodiments, such microfluidic chips 108 may be coupled to one or more reagent reservoirs such that one or more fluids may be passed through one or more chromatographic columns through use of centrifugation. For example, in some embodiments, chromatographic separation may be used to separate one or more polynucleotides from one or more samples 102 through use of chromatographic media that is configured as a spin column.

At embodiment 4606, module 3950 may include one or more centrifugation units configured for polynucleotide extraction from the one or more samples. In some embodiments, a system may include one or more centrifugation units 118 configured for polynucleotide extraction from the one or more samples 102. For example, a microfluidic chip 108 may be configured to utilize alkaline lysis (e.g., miniprep procedure) to extract polynucleotides from one or more samples 102. Such methods have been described. In some embodiments, alkaline lysis may be combined with additional methods, such as chromatography, to facilitate extraction of polynucleotides from one or more samples 102.

At embodiment 4608, module 3950 may include one or more centrifugation units configured to provide for gradient centrifugation. In some embodiments, a system may include one or more centrifugation units 118 configured to provide for gradient centrifugation. In some embodiments, one or more centrifugation units 118 may be configured to provide for density gradient centrifugation. In some embodiments, one or more centrifugation units 118 may be configured to provide for velocity gradient centrifugation. In some embodiments, gradient centrifugation may be used to concentrate viral particles.

FIG. 47 illustrates alternative embodiments of system 3900 of FIG. 39. FIG. 47 illustrates example embodiments of module 3960. Additional embodiments may include an embodiment 4702, and/or an embodiment 4704.

At embodiment 4702, module 3960 may include one or more reservoirs that are configured for containing the one or more reagents. In some embodiments, a system may include one or more reservoirs that are configured for containing one or more reagents. Reservoirs may be configured to contain and/or deliver numerous types of reagents. Examples of such reagents include, but are not limited to, phenol, chloroform, alcohol, salt solutions, detergent solutions, solvents, reagents used for polynucleotide precipitation, reagents used for polypeptide precipitation, reagents used for polynucleotide extraction, reagents used for polypeptide extraction, reagents used for chemical extractions, and the like. Accordingly, reservoirs may be configured to contain and/or deliver virtually any reagent that may be used for the analysis of one or more pathogens 104 and/or pathogen indicators 106.

At embodiment 4704, module 3960 may include one or more reservoirs that are configured as one or more waste reservoirs. In some embodiments, a system may include one or more reservoirs that are configured as waste reservoirs. Such waste reservoirs may be configured in numerous ways. For example such waste reservoirs may be configured for containing reagents, samples 102, and the like. In some embodiments, waste reservoirs may be configured to containing liquids, solids, gels, and substantially any combination thereof.

FIG. 48 illustrates a system 4800 representing examples of modules that may be used to perform a method for analysis of one or more pathogens 104. In FIG. 48, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The system 4800 includes module 4810 that includes one or more microfluidic chips that are configured to allow one or more magnetically active pathogen indicator binding agents to bind to one or more pathogen indicators associated with one or more samples to form one or more magnetically active pathogen indicator complexes and separate the one or more magnetically active pathogen indicator complexes from the one or more samples through use of one or more magnetic fields and one or more separation fluids that are in substantially parallel flow with the one or more samples. In some embodiments, module 4810 may include one or more magnetic separation fluids. In some embodiments, module 4810 may include one or more attractive magnetic fields. In some embodiments, module 4810 may include one or more repulsive magnetic fields.

The system 4800 may optionally include module 4820 that includes one or more detection units configured to detect the one or more pathogen indicators associated with the one or more samples. In some embodiments, module 4820 may include one or more detection units configured to detect the one or more pathogen indicators that are associated with one or more pathogens that are airborne. In some embodiments, module 4820 may include one or more detection units configured to detect the one or more pathogen indicators that are associated with one or more food products. In some embodiments, module 4820 may include one or more detection units that are configured to detect one or more pathogens that include at least one virus, bacterium, prion, worm, egg, cyst, protozoan, single-celled organism, fungus, algae, pathogenic protein, or microbe. In some embodiments, module 4820 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, module 4820 may include one or more detection units that are configured for detachable connection to the one or more microfluidic chips.

The system 4800 may optionally include module 4830 that includes one or more display units operably associated with the one or more detection units. In some embodiments, module 4830 may include one or more display units that include one or more passive display units. In some embodiments, module 4830 may include one or more display units that include one or more active display units. In some embodiments, module 4830 may include one or more display units that indicate a presence or an absence of one or more pathogens within the one or more samples. In some embodiments, module 4830 may include one or more display units that indicate an identity of one or more pathogens present within the one or more samples. In some embodiments, module 4830 may include one or more display units that indicate one or more concentrations of one or more pathogens within the one or more samples.

The system 4800 may optionally include module 4840 that includes one or more reagent delivery units configured to deliver one or more reagents to the one or more microfluidic chips. In some embodiments, module 4840 may include one or more reagent delivery units configured for detachable connection to the one or more microfluidic chips. In some embodiments, module 4840 may include one or more reagent reservoirs. In some embodiments, module 4840 may include one or more waste reservoirs. In some embodiments, module 4840 may include one or more reagent delivery units physically coupled to the one or more microfluidic chips. In some embodiments, module 4840 may include one or more reagent delivery units that include one or more pumps.

The system 4800 may optionally include module 4850 that includes one or more centrifugation units. In some embodiments, module 4850 may include one or more centrifugation units configured to centrifuge the one or more microfluidic chips that are operably associated with the one or more centrifugation units. In some embodiments, module 4850 may include one or more centrifugation units configured to provide for chromatographic separation. In some embodiments, module 4850 may include one or more centrifugation units configured for polynucleotide extraction from the one or more samples. In some embodiments, module 4850 may include one or more centrifugation units configured to provide for gradient centrifugation.

The system 4800 may optionally include module 4860 that includes one or more reservoir units. In some embodiments, module 4860 may include one or more reservoirs that are configured for containing the one or more reagents. In some embodiments, module 4860 may include one or more reservoirs that are configured as one or more waste reservoirs.

FIG. 49 illustrates alternative embodiments of system 4800 of FIG. 48. FIG. 49 illustrates example embodiments of module 4810. Additional embodiments may include an embodiment 4902, an embodiment 4904, and/or an embodiment 4906.

At embodiment 4902, module 4810 may include one or more magnetic separation fluids. In some embodiments, one or more microfluidic chips 108 may include one or more magnetic separation fluids. In some embodiments, the one or more magnetic separation fluids may include one or more fluids that include suspended magnetic particles. In some embodiments, the one or more magnetic separation fluids may include one or more ferrofluids. In some embodiments, a ferromagnetic separation fluid may be a suspension of magnetically active particles in a liquid carrier. In some embodiments, a ferrofluid may be a stable colloidal suspension of magnetic particles in a liquid carrier. In some embodiments, the magnetic particles may be nano particles. In some embodiments, the particles may be coated with a stabilizing dispersing agent (surfactant) which prevents particle agglomeration. In some embodiments, a ferrofluid may include particles, such as iron and/or iron containing particles, to which a magnet is attracted.

At embodiment 4904, module 4810 may include one or more attractive magnetic fields. In some embodiments, one or more microfluidic chips 108 may include one or more attractive magnetic fields. For example, in some embodiments, one or more magnets may be positioned within a microfluidic chip 108 such that a magnetically active pathogen indicator complex is attracted to the magnetic field. In some embodiments, such attraction may be used to separate one or more magnetically active pathogen indicator complexes from one or more samples 102. For example, in some embodiments, one or more magnetically active pathogen indicator complexes may be held in place while the remaining components of one or more samples 102 are washed away. In some embodiments, magnetically active pathogen indicator complexes may be attracted into a separation fluid and thereby separated from one or more samples 102. In some embodiments, the one or more magnetic fields are produced with one or more electromagnets, one or more permanent magnets, or substantially any combination thereof.

At embodiment 4906, module 4810 may include one or more repulsive magnetic fields. In some embodiments, one or more microfluidic chips 108 may include one or more repulsive magnetic fields. For example, in some embodiments, one or more magnets may be positioned within a microfluidic chip 108 such that one or more magnetically active pathogen indicator complexes are repelled from the magnetic field. In some embodiments, such repulsion may be used to separate one or more magnetically active pathogen indicator complexes from one or more samples 102. For example, in some embodiments, one or more magnetically active pathogen indicator complexes may be repelled from one or more magnetic fields and thereby translocated into a separation fluid where the one or more magnetically active pathogen indicator complexes are separated from one or more samples 102. In some embodiments, the one or more magnetic fields are produced with one or more electromagnets, one or more permanent magnets, or substantially any combination thereof.

FIG. 50 illustrates alternative embodiments of system 4800 of FIG. 48. FIG. 50 illustrates example embodiments of module 4820. Additional embodiments may include an embodiment 5002, and/or an embodiment 5004.

At embodiment 5002, module 4820 may include one or more detection units configured to detect the one or more pathogen indicators that are associated with one or more pathogens that are airborne. In some embodiments, a system may include one or more detection units 122 that are configured to detect the one or more pathogen indicators 106 that are associated with one or more pathogens 104 that are airborne. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more microfluidic chips 108 may be configured to analyze and/or detect severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 5004, module 4820 may include one or more detection units configured to detect the one or more pathogen indicators that are associated with one or more food products. In some embodiments, one or more detection units 122 may be configured to detect the one or more pathogen indicators 106 that are associated with one or more food products. In some embodiments, one or more detection units 122 may be configured to detect one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

FIG. 51 illustrates alternative embodiments of system 4800 of FIG. 48. FIG. 51 illustrates example embodiments of module 4820. Additional embodiments may include an embodiment 5102.

At embodiment 5102, module 4820 may include one or more detection units that are configured to detect one or more pathogens that include at least one virus, bacterium, prion, worm, egg, cyst, protozoan, single-celled organism, fungus, algae, pathogenic protein, or microbe. In some embodiments, one or more detection units 122 may be configured to detect one or more pathogens 104 that include at least one virus, bacterium, prion, worm, egg, cyst, protozoan, single-celled organism, fungus, algae, pathogenic protein, microbe, or substantially any combination thereof. A detection unit 122 may be configured to utilize numerous types of techniques, and combinations of techniques, to detect one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

FIG. 52 illustrates alternative embodiments of system 4800 of FIG. 48. FIG. 52 illustrates example embodiments of module 4820. Additional embodiments may include an embodiment 5202.

At embodiment 5202, module 4820 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more detection units 122 may be configured to detect the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection units 122 may be configured to detect one or more pathogen indicators 106 that have been processed by one or more microfluidic chips 108 and/or analyzed by one or more analysis units 120. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108.

In some embodiments, one or more detection units 122 may be configured to detect one or more pathogen indicators 106 through use of surface plasmon resonance. In some embodiments, one or more detection units 122 may be configured to operably associate with one or more microfluidic chips 108 may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more microfluidic chips 108 to facilitate detection of one or more pathogen indicators 106 through use of surface plasmon resonance.

In some embodiments, one or more detection units 122 may be configured to detect one or more pathogen indicators 106 through use of nuclear magnetic resonance (NMR). In some embodiments, one or more detection units 122 may be configured to operably associate with one or more microfluidic chips 108 that include a nuclear magnetic resonance (NMR) probe. Accordingly, in some embodiments, one or more pathogen indicators 106 may be analyzed and detected with one or more microfluidic chips 108 and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical, Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, one or more analysis units 120 may be configured to facilitate analysis of one or more pathogen indicators 106 and configured to facilitate fluorescent detection of the one or more pathogen indicators 106 with one or more detection units 122.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122 that is configured to operably associate with the one or more microfluidic chips 108.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more microfluidic chips 108 may be configured to provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleic acid associated with one or more samples 102 may be combined with one or more sets of paired primers such that use of an amplification protocol, such as a polymerase chain reaction, will produce an amplification product corresponding to pathogen associated polynucleic acid that was contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such asa cyst, egg, pathogen, spore, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors may be configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be processed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Microfluidic chips 108 and detection units 122 may be configured in numerous ways to facilitate detection of one or more pathogen indicators 106 through use of isoelectric focusing.

In some embodiments, one or more pathogen indicators 106 may be detected through use of chromatographic methodology alone or in combination with additional detection methods. In some embodiments, one or more microfluidic chips 108 may be configured to provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection units 122 may be configured to detect one or more pathogen indicators 106 through use of immunoprecipitation. In some embodiments, immunoprecipitation may be utilized in combination with additional detection methods to detect one or more pathogen indicators 106. In some embodiments, one or more microfluidic chips 108 may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.
In some embodiments, one or more detection units 122 may be configured to detect one or more pathogen indicators 106 through use of immunoseparation. In some embodiments, immunoseparation may be utilized in combination with additional detection methods to detect one or more pathogen indicators 106. In some embodiments, one or more microfluidic chips 108 may be configured to facilitate detection of one or more pathogen indicators 106 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection units 122 may be configured to detect one or more pathogen indicators 106 through use of aptamer binding. In some embodiments, aptamer binding may be utilized in combination with additional methods to detect one or more pathogen indicators 106. In some embodiments, one or more microfluidic chips 108 may be configured to facilitate detection of one or more pathogen indicators 106 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to facilitate detection of one or more pathogen indicators 106 through use of electrophoresis. In some embodiments, such micro fluidic chips 108 may be configured to operably associate with one or more detection units 122. Accordingly, in some embodiments, one or more detection units 122 may be configured to operably associate with one or more microfluidic chips 108 and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to provide for detection of one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to facilitate detection of one or more pathogen indicators 106 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 53 illustrates alternative embodiments of system 4800 of FIG. 48. FIG. 53 illustrates example embodiments of module 4820. Additional embodiments may include an embodiment 5302.

At embodiment 5302, module 4820 may include one or more detection units that are configured for detachable connection to the one or more microfluidic chips. In some embodiments, one or more detection units 122 may be configured for detachable connection to the one or more microfluidic chips 108. In some embodiments, the one or more detection units 122 may be connected to the one or more microfluidic chips 108 through use of fasteners. Examples of such fasteners include, but are not limited to, hooks, screws, bolts, pins, grooves, adhesives, and the like. In some embodiments, the one or more detection units may be connected to the one or more microfluidic chips 108 through use of magnets.

FIG. 54 illustrates alternative embodiments of system 4800 of FIG. 48. FIG. 54 illustrates example embodiments of module 4830. Additional embodiments may include an embodiment 5402, an embodiment 5404, and/or an embodiment 5406.

At embodiment 5402, module 4830 may include one or more display units that include one or more passive display units. In some embodiments, a system may include one or more display units 124 that include one or more passive display units 124. In some embodiments, one or display units 124 may include one or more liquid crystal displays (LCD). Methods to construct passive displays have been described (e.g., U.S. Patent Nos.: 4,807,967; 4,729,636, 4,436,378; 4,257,041; herein incorporated by reference).

At embodiment 5404, module 4830 may include one or more display units that include one or more active display units. In some embodiments, a system may include one or more display units 124 that include one or more active display units 124. Numerous active display units 124 are known and include, but are not limited to, quarter-video graphics array (QVGA), video graphics array (VGA), super video graphics array (SVGA), extended graphics array (XGA), wide extended graphics array (WXGA), super extended graphics array (SXGA), ultra extended graphics array (UXGA), wide super extended graphics array (WSXGA), wide ultra extended graphics array (WUXGA).

At embodiment 5406, module 4830 may include one or more display units that indicate a presence or an absence of one or more pathogens within the one or more samples. In some embodiments, a system may include one or more display units 124 that indicate a presence or an absence of one or more pathogens 104 within the one or more samples 102. In some embodiments, one or more display units 124 may use a colorimetric message to indicate a presence or an absence of one or more pathogens 104 within one or more samples 102. For example, in some embodiments, one or more display units 124 may display a green light if one or more pathogens 104 are not found within one or more samples 102 and a red light if one or more pathogens 104 are found within one or more samples 102. In some embodiments, one or more display units 124 may use a pictographic message to indicate a presence or an absence of one or more pathogens 104 within one or more samples 102. For example, in some embodiments, one or more display units 124 may display a smiley face if one or more pathogens 104 are not found within one or more samples 102 and a frowny face if one or more pathogens 104 are found within one or more samples 102. In some embodiments, one or more display units 124 may use a typographical message to indicate a presence or an absence of one or more pathogens 104 within one or more samples 102. For example, in some embodiments, one or more display units 124 may display a "Pathogen Not Present" message if one or more pathogens 104 are not found within one or more samples 102 and a "Pathogen Present" message if one or more pathogens 104 are found within one or more samples 102. Such messages may be displayed in numerous languages. In some embodiments, one or more display units 124 may display one or more messages in multiple formats. For example, in some embodiments, one or more messages may be displayed in colored text.

FIG. 55 illustrates alternative embodiments of system 4800 of FIG. 48. FIG. 55 illustrates example embodiments of module 4830. Additional embodiments may include an embodiment 5502, and/or an embodiment 5504.

At embodiment 5502, module 4830 may include one or more display units that indicate an identity of one or more pathogens present within the one or more samples. In some embodiments, a system may include one or more display units 124 that indicate an identity of one or more pathogens 104 present within the one or more samples 102. In some embodiments, one or more display units 124 may be operably associated with one or more microfluidic chips 108. Accordingly, in some embodiments, one or more display units 124 may be configured to display the identity of one or more pathogens 104 that are present and/or absent from one or more samples 102. For example, in some embodiments, a display unit 124 may be configured to indicate a presence or an absence of Salmonella in a food product.

At embodiment 5504, module 4830 may include one or more display units that indicate one or more concentrations of one or more pathogens within the one or more samples. In some embodiments, a system may include one or more display units 124 that indicate one or more concentrations of one or more pathogens 104 within the one or more samples 102. Concentration may be displayed in numerous formats. For example, in some embodiments, concentration may be expressed numerically. In some embodiments, concentration may be expressed graphically. For example, in some embodiments, one or more display units 124 may include a display having a gray scale on which the concentration of one or more pathogen indicators 106 and/or pathogens 104 that are present within one or more samples 102 may be indicated (e.g., higher concentrations of one or more pathogens 104 may be displayed as dark gray while lower concentrations of one or more pathogens 104 may be displayed as light gray). In some embodiments, one or more display units 124 may include a display having a color scale on which the concentration of one or more pathogens 104 that are present within one or more samples 102 may be indicated (e.g., low concentrations of one or more pathogen indicators 106 may be indicated by a green light, intermediate concentrations of one or more pathogen indicators 106 may be indicated by a yellow light, high concentrations of one or more pathogen indicators 106 may be indicated by a red light). In some embodiments, one or more display units 124 may be calibrated to an individual. For example, in some embodiments, a display unit 124 may be calibrated relative to a person who is immune compromised. Accordingly, in some embodiments, an individual may obtain an indication from a display that indicates if a food product contains a dangerous level of one or more pathogens 104.

FIG. 56 illustrates alternative embodiments of system 4800 of FIG. 48. FIG. 56 illustrates example embodiments of module 4840. Additional embodiments may include an embodiment 5602, an embodiment 5604, an embodiment 5606, an embodiment 5608, and/or an embodiment 5610.

At embodiment 5602, module 4840 may include one or more reagent delivery units configured for detachable connection to the one or more microfluidic chips. In some embodiments, a system may include one or more reagent delivery units 116 configured for detachable connection to the one or more microfluidic chips 108. Reagent delivery units 116 may be configured to deliver one or more types of reagents to one or more microfluidic chips 108. In some embodiments, such reagents may be utilized to analyze and/or process one or more samples 102. In some embodiments, such reagents may be utilized to facilitate detection of one or more pathogen indicators 106. Examples of such reagents include, but are not limited to, solvents, water, tags, labels, antibodies, aptamers, polynucleotides, and the like. In some embodiments, one or more reagent delivery units 116 may include connectors that may be coupled to one or more microfluidic chips 108 to provide for delivery of one or more reagents to the one or more microfluidic chips 108. Examples of such connectors include, but are not limited to, leur lock fittings, needles, fluid connectors, and the like. In some embodiments, a reagent delivery unit 116 may include one or more pumps. In some embodiments, a reagent delivery unit 116 may include numerous reservoirs that may include numerous types of reagents. Accordingly, in some embodiments, a reagent delivery unit 116 may be configured to detachably connect with numerous types of microfluidic chips 108 that are configured to facilitate analysis and/or detection of numerous types of pathogens 104 and/or pathogen indicators 106.

At embodiment 5604, module 4840 may include one or more reagent reservoirs. In some embodiments, a system may include one or more reagent reservoirs. In some embodiments, the one or more reagent reservoirs may be configured to contain reagents that may be used to facilitate analysis and/or detection of a single type of pathogen 104 and/or pathogen indicator 106. In some embodiments, the one or more reagent reservoirs may be configured to contain reagents that may be used to facilitate analysis and/or detection of multiple types of pathogens 104 and/or pathogen indicators 106.

At embodiment 5606, module 4840 may include one or more waste reservoirs. In some embodiments, a system may include one or more waste reservoirs. Such waste reservoirs may be configured in numerous ways. For example such waste reservoirs may be configured for containing reagents, samples 102, and the like. In some embodiments, waste reservoirs may be configured to contain liquids, solids, gels, and substantially any combination thereof.

At embodiment 5608, module 4840 may include one or more reagent delivery units physically coupled to the one or more microfluidic chips. In some embodiments, a system may include one or more reagent delivery units 116 physically coupled to the one or more microfluidic chips 108. For example, in some embodiments, one or more reagent delivery units 116 may be included within a microfluidic chip 108 (e.g., as opposed to being separate from a microfluidic chip 108). In some embodiments, such microfluidic chips 108 may be configured for single use to facilitate analysis and/or detection of one or more pathogen indicators 106 that may be present within one or more samples 102. The reagent delivery units 116 may contain numerous types of reagents that may provide for analysis of one or more samples 102.

For example, in some embodiments, a microfluidic chip 108 may be configured for extraction and/or analysis of polynucleotides that may be included within one or more samples 102. In some embodiments, such a microfluidic chip 108 may include: a first reagent delivery unit 116 that includes an alkaline lysis buffer (e.g., sodium hydroxide/sodium dodecyl sulfate), a second reagent delivery unit 116 that includes an agent that precipitates the sodium dodecyl sulfate (e.g., potassium acetate), a third reagent delivery unit 116 that includes an extraction agent (e.g., phenol/chloroform), and a fourth reagent delivery unit 116 that includes a precipitation agent for precipitating any polynucleotides that may be present within the one or more samples 102. Accordingly, in some embodiments, a system may include one or more microfluidic chips 108 that are configured to include all of the reagents necessary to facilitate analysis of one or more samples 102 for one or more pathogen indicators 106. In some embodiments, such microfluidic chips 108 may be configured for single use. In some embodiments, such microfluidic chips 108 may be configured for repeated use. In some embodiments, such microfluidic chips 108 may be configured to detachably connect to one or more detection units 122 such that the same detection unit 122 may be used repeatedly through association with a new microfluidic chip 108.

At embodiment 5610, module 4840 may include one or more reagent delivery units that include one or more pumps. In some embodiments, a system may include one or more reagent delivery units 116 that include one or more pumps. Numerous types of pumps may be associated with one or more reagent delivery units 116.

FIG. 57 illustrates alternative embodiments of system 4800 of FIG. 48. FIG. 57 illustrates example embodiments of module 4850. Additional embodiments may include an embodiment 5702, and/or an embodiment 5704.

At embodiment 5702, module 4850 may include one or more centrifugation units configured to centrifuge the one or more microfluidic chips that are operably associated with the one or more centrifugation units. In some embodiments, a system may include one or more centrifugation units 118 configured to centrifuge the one or more microfluidic chips 108 that are operably associated with the one or more centrifugation units 118. In some embodiments, one or more centrifugation units 118 may be configured to detachably associate with one or more microfluidic chips 108. For example, in some embodiments, a centrifugation unit 118 may include one or more centrifuge drives that are configured to detachably associate with one or more centrifuge rotors that are included within one or more microfluidic chips 108. In some embodiments, such centrifuge drives may magnetically couple with the one or more centrifuge rotors. In some embodiments, such centrifuge drives may physically couple with the one or more centrifuge rotors. In some embodiments, one or more centrifugation units 118 may be configured to centrifuge an entire microfluidic chip 108. For example, in some embodiments, a microfluidic chip 108 may be configured to associate with one or more centrifugation units 118 such that the microfluidic chip 108 is subjected to centrifugal force. In some embodiments, such a microfluidic chip 108 may be configured in a manner that resembles a compact disc. Accordingly, in some embodiments, a centrifugation unit 118 may be configured in a manner that resembles a compact disc player. In some embodiments, one or more centrifugation units 118 may be configured to centrifuge one or more samples 102 through a series of mesh filters to concentrate parasite eggs and/or larvae (e.g., U.S. Patent No.: 4081356; herein incorporated by reference).

At embodiment 5704, module 4850 may include one or more centrifugation units configured to provide for chromatographic separation. In some embodiments, a system may include one or more centrifugation units 118 configured to provide for chromatographic separation. For example, in some embodiments, one or more centrifugation units 118 may be configured to centrifuge one or more samples 102 through one or more chromatographic columns that are associated with one or more microfluidic chips 108. In some embodiments, such microfluidic chips 108 may be coupled to one or more reagent reservoirs such that one or more fluids may be passed through one or more chromatographic columns through use of centrifugation. For example, in some embodiments, chromatographic separation may be used to separate one or more polynucleotides from one or more samples 102 through use of chromatographic media that is configured as a spin column.

FIG. 58 illustrates alternative embodiments of system 4800 of FIG. 48. FIG. 58 illustrates example embodiments of module 4850. Additional embodiments may include an embodiment 5802, and/or an embodiment 5804.

At embodiment 5802, module 4850 may include one or more centrifugation units configured for polynucleotide extraction from the one or more samples. In some embodiments, a system may include one or more centrifugation units 118 configured for polynucleotide extraction from the one or more samples 102. For example, a microfluidic chip 108 may be configured to utilize alkaline lysis (e.g., miniprep procedure) to extract polynucleotides from one or more samples 102. Such methods have been described. In some embodiments, alkaline lysis may be combined with additional methods, such as chromatography, to facilitate extraction of polynucleotides from one or more samples 102.

At embodiment 5804, module 4850 may include one or more centrifugation units configured to provide for gradient centrifugation. In some embodiments, a system may include one or more centrifugation units 118 configured to provide for gradient centrifugation. In some embodiments, one or more centrifugation units 118 may be configured to provide for density gradient centrifugation. In some embodiments, one or more centrifugation units 118 may be configured to provide for velocity gradient centrifugation. In some embodiments, gradient centrifugation may be used to concentrate viral particles.

FIG. 59 illustrates alternative embodiments of system 4800 of FIG. 48. FIG. 59 illustrates example embodiments of module 4860. Additional embodiments may include an embodiment 5902, and/or an embodiment 5904.

At embodiment 5902, module 4860 may include one or more reservoirs that are configured for containing the one or more reagents. In some embodiments, a system may include one or more reservoirs that are configured for containing one or more reagents. Reservoirs may be configured to contain and/or deliver numerous types of reagents. Examples of such reagents include, but are not limited to, phenol, chloroform, alcohol, salt solutions, detergent solutions, solvents, reagents used for polynucleotide precipitation, reagents used for polypeptide precipitation, reagents used for polynucleotide extraction, reagents used for polypeptide extraction, reagents used for chemical extractions, and the like. Accordingly, reservoirs may be configured to contain and/or deliver virtually any reagent that may be used for the analysis of one or more pathogens 104 and/or pathogen indicators 106.

At embodiment 5904, module 4860 may include one or more reservoirs that are configured as one or more waste reservoirs. In some embodiments, a system may include one or more reservoirs that are configured as waste reservoirs. Such waste reservoirs may be configured in numerous ways. For example such waste reservoirs may be configured for containing reagents, samples 102, and the like. In some embodiments, waste reservoirs may be configured to contain liquids, solids, gels, and substantially any combination thereof.

FIG. 60 illustrates a system 6000 representing examples of modules that may be used to perform a method for analysis of one or more pathogens 104. In FIG. 60, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The system 6000 includes module 6010 that includes one or more microfluidic chips that are configured to allow one or more magnetically active pathogen indicator binding agents to bind to one or more pathogen indicators associated with one or more samples to form one or more magnetically active pathogen indicator complexes and separate the one or more magnetically active pathogen indicator complexes from the one or more samples through use of one or more magnetic fields and one or more separation fluids that are in substantially antiparallel flow with the one or more samples. In some embodiments, module 6010 may include one or more magnetic separation fluids. In some embodiments, module 6010 may include one or more attractive magnetic fields. In some embodiments, module 6010 may include one or more repulsive magnetic fields.

The system 6000 may optionally include module 6020 that includes one or more detection units configured to detect the one or more pathogen indicators associated with the one or more samples. In some embodiments, module 6020 may include one or more detection units configured to detect the one or more pathogen indicators that are associated with one or more pathogens that are airborne. In some embodiments, module 6020 may include one or more detection units configured to detect the one or more pathogen indicators that are associated with one or more food products. In some embodiments, module 6020 may include one or more detection units that are configured to detect one or more pathogens that include at least one virus, bacterium, prion, worm, egg, cyst, protozoan, single-celled organism, fungus, algae, pathogenic protein, or microbe. In some embodiments, module 6020 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, module 6020 may include one or more detection units that are configured for detachable connection to the one or more microfluidic chips.

The system 6000 may optionally include module 6030 that includes one or more display units operably associated with the one or more detection units. In some embodiments, module 6030 may include one or more display units that include one or more passive display units. In some embodiments, module 6030 may include one or more display units that include one or more active display units. In some embodiments, module 6030 may include one or more display units that indicate a presence or an absence of one or more pathogens within the one or more samples. In some embodiments, module 6030 may include one or more display units that indicate an identity of one or more pathogens present within the one or more samples. In some embodiments, module 6030 may include one or more display units that indicate one or more concentrations of one or more pathogens within the one or more samples.

The system 6000 may optionally include module 6040 that includes one or more reagent delivery units configured to deliver one or more reagents to the one or more microfluidic chips. In some embodiments, module 6040 may include one or more reagent delivery units configured for detachable connection to the one or more microfluidic chips. In some embodiments, module 6040 may include one or more reagent reservoirs. In some embodiments, module 6040 may include one or more waste reservoirs. In some embodiments, module 6040 may include one or more reagent delivery units physically coupled to the one or more microfluidic chips. In some embodiments, module 6040 may include one or more reagent delivery units that include one or more pumps.

The system 6000 may optionally include module 6050 that includes one or more centrifugation units. In some embodiments, module 6050 may include one or more centrifugation units configured to centrifuge the one or more microfluidic chips that are operably associated with the one or more centrifugation units. In some embodiments, module 6050 may include one or more centrifugation units configured to provide for chromatographic separation. In some embodiments, module 6050 may include one or more centrifugation units configured for polynucleotide extraction from the one or more samples. In some embodiments, module 6050 may include one or more centrifugation units configured to provide for gradient centrifugation.

The system 6000 may optionally include module 6060 that includes one or more reservoir units. In some embodiments, module 6060 may include one or more reservoirs that are configured for containing the one or more reagents. In some embodiments, module 6060 may include one or more reservoirs that are configured as one or more waste reservoirs.

FIG. 61 illustrates alternative embodiments of system 6000 of FIG. 60. FIG. 61 illustrates example embodiments of module 6010. Additional embodiments may include an embodiment 6102, an embodiment 6104, and/or an embodiment 6106.

At embodiment 6102, module 6010 may include one or more magnetic separation fluids. In some embodiments, one or more microfluidic chips 108 may include one or more magnetic separation fluids. In some embodiments, the one or more magnetic separation fluids may include one or more fluids that include suspended magnetic particles. In some embodiments, the one or more magnetic separation fluids may include one or more ferrofluids. In some embodiments, a ferromagnetic separation fluid may be a suspension of magnetically active particles in a liquid carrier. In some embodiments, a ferrofluid may be a stable colloidal suspension of magnetic particles in a liquid carrier. In some embodiments, the magnetic particles may be nano particles. In some embodiments, the particles may be coated with a stabilizing dispersing agent (surfactant) which prevents particle agglomeration. In some embodiments, a ferrofluid may include particles, such as iron and/or iron containing particles, to which a magnet is attracted.

At embodiment 6104, module 6010 may include one or more attractive magnetic fields. In some embodiments, one or more microfluidic chips 108 may include one or more attractive magnetic fields. For example, in some embodiments, one or more magnets may be positioned within a microfluidic chip 108 such that a magnetically active pathogen indicator complex is attracted to the magnetic field. In some embodiments, such attraction may be used to separate one or more magnetically active pathogen indicator complexes from one or more samples 102. For example, in some embodiments, one or more magnetically active pathogen indicator complexes may be held in place while the remaining components of one or more samples 102 are washed away. In some embodiments, magnetically active pathogen indicator complexes may be attracted into a separation fluid and thereby separated from one or more samples 102. In some embodiments, the one or more magnetic fields are produced with one or more electromagnets, one or more permanent magnets, or substantially any combination thereof.

At embodiment 6106, module 6010 may include one or more repulsive magnetic fields. In some embodiments, one or more microfluidic chips 108 may include one or more repulsive magnetic fields. For example, in some embodiments, one or more magnets may be positioned within a microfluidic chip 108 such that one or more magnetically active pathogen indicator complexes are repelled from the magnetic field. In some embodiments, such repulsion may be used to separate one or more magnetically active pathogen indicator complexes from one or more samples. For example, in some embodiments, one or more magnetically active pathogen indicator complexes may be repelled from one or more magnetic fields and thereby translocated into a separation fluid where the one or more magnetically active pathogen indicator complexes are separated from one or more samples 102. In some embodiments, the one or more magnetic fields are produced with one or more electromagnets, one or more permanent magnets, or substantially any combination thereof.

FIG. 62 illustrates alternative embodiments of system 6000 of FIG. 60. FIG. 62 illustrates example embodiments of module 6020. Additional embodiments may include an embodiment 6202, and/or an embodiment 6204.

At embodiment 6202, module 6020 may include one or more detection units configured to detect the one or more pathogen indicators that are associated with one or more pathogens that are airborne. In some embodiments, a system may include one or more detection units 122 that are configured to detect the one or more pathogen indicators 106 that are associated with one or more pathogens 104 that are airborne. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more microfluidic chips 108 may be configured to analyze and/or detect severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 6204, module 6020 may include one or more detection units configured to detect the one or more pathogen indicators that are associated with one or more food products. In some embodiments, one or more detection units 122 may be configured to detect the one or more pathogen indicators 106 that are associated with one or more food products. In some embodiments, one or more detection units 122 may be configured to detect one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

FIG. 63 illustrates alternative embodiments of system 6000 of FIG. 60. FIG. 63 illustrates example embodiments of module 6020. Additional embodiments may include an embodiment 6302.

At embodiment 6302, module 6020 may include one or more detection units that are configured to detect one or more pathogens that include at least one virus, bacterium, prion, worm, egg, cyst, protozoan, single-celled organism, fungus, algae, pathogenic protein, or microbe. In some embodiments, one or more detection units 122 may be configured to detect one or more pathogens 104 that include at least one virus, bacterium, prion, worm, egg, cyst, protozoan, single-celled organism, fungus, algae, pathogenic protein, microbe, or substantially any combination thereof. A detection unit may be configured to utilize numerous types of techniques, and combinations of techniques, to detect one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

FIG. 64 illustrates alternative embodiments of system 6000 of FIG. 60. FIG. 64 illustrates example embodiments of module 6020. Additional embodiments may include an embodiment 6402.

At embodiment 6402, module 6020 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more detection units 122 may be configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection units 122 may be configured to detect one or more pathogen indicators 106 that have been processed by one or more microfluidic chips 108 and/or analyzed by one or more analysis units 120. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108.

In some embodiments, one or more detection units 122 may be configured to detect one or more pathogen indicators 106 through use of surface plasmon resonance. In some embodiments, one or more detection units 122 may be configured to operably associate with one or more microfluidic chips 108 that may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more microfluidic chips 108 to facilitate detection of one or more pathogen indicators 106 through use of surface plasmon resonance.

In some embodiments, one or more detection units 122 may be configured to detect one or more pathogen indicators 106 through use of nuclear magnetic resonance (NMR). In some embodiments, one or more detection units 122 may be configured to operably associate with one or more microfluidic chips 108 that include a nuclear magnetic resonance (NMR) probe. Accordingly, in some embodiments, one or more pathogen indicators 106 may be analyzed and detected with one or more microfluidic chips and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, one or more analysis units 120 may be configured to facilitate analysis of one or more pathogen indicators 106 and configured to facilitate fluorescent detection of the one or more pathogen indicators 106 with one or more detection units 122.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122 that is configured to operably associate with the one or more microfluidic chips 108.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more microfluidic chips 108 may be configured to provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleic acid associated with one or more samples 102 may be combined with one or more sets of paired primers such that use of an amplification protocol, such as a polymerase chain reaction, will produce an amplification product corresponding to pathogen associated polynucleic acid that was contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a cyst, egg, pathogen 104, spore, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors may be configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be processed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Microfluidic chips 108 and detection units 122 may be configured in numerous ways to facilitate detection of one or more pathogen indicators 106 through use of isoelectric focusing.

In some embodiments, one or more pathogen indicators 106 may be detected through use of chromatographic methodology alone or in combination with additional detection methods. In some embodiments, one or more microfluidic chips 108 may be configured to provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection units 122 may be configured to detect one or more pathogen indicators 106 through use of immunoprecipitation. In some embodiments, immunoprecipitation may be utilized in combination with additional detection methods to detect one or more pathogen indicators 106. In some embodiments, one or more microfluidic chips 108 may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more detection units 122 may be configured to detect one or more pathogen indicators 106 through use of immunoseparation. In some embodiments, immunoseparation may be utilized in combination with additional detection methods to detect one or more pathogen indicators 106. In some embodiments, one or more microfluidic chips 108 may be configured to facilitate detection of one or more pathogen indicators 106 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection units 122 may be configured to detect one or more pathogen indicators 106 through use of aptamer binding. In some embodiments, aptamer binding may be utilized in combination with additional methods to detect one or more pathogen indicators 106. In some embodiments, one or more microfluidic chips 108 may be configured to facilitate detection of one or more pathogen indicators 106 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to facilitate detection of one or more pathogen indicators 106 through use of electrophoresis. In some embodiments, such microfluidic chips 108 may be configured to operably associate with one or more detection units 122. Accordingly, in some embodiments, one or more detection units 122 may be configured to operably associate with one or more microfluidic chips 108 and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to provide for detection of one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to facilitate detection of one or more pathogen indicators 106 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 65 illustrates alternative embodiments of system 6000 of FIG. 60. FIG. 65 illustrates example embodiments of module 6020. Additional embodiments may include an embodiment 6502.

At embodiment 6502, module 6020 may include one or more detection units that are configured for detachable connection to the one or more microfluidic chips. In some embodiments, one or more detection units 122 may be configured for detachable connection to the one or more microfluidic chips 108. In some embodiments, the one or more detection units 122 may be connected to the one or more microfluidic chips 108 through use of fasteners. Examples of such fasteners include, but are not limited to, hooks, screws, bolts, pins, grooves, adhesives, and the like. In some embodiments, the one or more detection units 122 may be connected to the one or more microfluidic chips 108 through use of magnets.

FIG. 66 illustrates alternative embodiments of system 6000 of FIG. 60. FIG. 66 illustrates example embodiments of module 6030. Additional embodiments may include an embodiment 6602, an embodiment 6604, and/or an embodiment 6606.

At embodiment 6602, module 6030 may include one or more display units that include one or more passive display units. In some embodiments, a system may include one or more display units 124 that include one or more passive display units 124. In some embodiments, one or display units 124 may include one or more liquid crystal displays (LCD). Methods to construct passive displays have been described (e.g., U.S. Patent Nos.: 4,807,967; 4,729,636, 4,436,378; 4,257,041; herein incorporated by reference).

At embodiment 6604, module 6030 may include one or more display units that include one or more active display units. In some embodiments, a system may include one or more display units 124 that include one or more active display units 124. Numerous active display units 124 are known and include, but are not limited to, quarter-video graphics array (QVGA), video graphics array (VGA), super video graphics array (SVGA), extended graphics array (XGA), wide extended graphics array (WXGA), super extended graphics array (SXGA), ultra extended graphics array (UXGA), wide super extended graphics array (WSXGA), wide ultra extended graphics array (WUXGA).

At embodiment 6606, module 6030 may include one or more display units that indicate a presence or an absence of one or more pathogens within the one or more samples. In some embodiments, a system may include one or more display units 124 that indicate a presence or an absence of one or more pathogens 104 within the one or more samples 102. In some embodiments, one or more display units 124 may use a colorimetric message to indicate a presence or an absence of one or more pathogens 104 within one or more samples 102. For example, in some embodiments, one or more display units 124 may display a green light if one or more pathogens 104 are not found within one or more samples 102 and a red light if one or more pathogens 104 are found within one or more samples 102. In some embodiments, one or more display units 124 may use a pictographic message to indicate a presence or an absence of one or more pathogens 104 within one or more samples 102. For example, in some embodiments, one or more display units 124 may display a smiley face if one or more pathogens 104 are not found within one or more samples 102 and a frowny face if one or more pathogens 104 are found within one or more samples 102. In some embodiments, one or more display units 124 may use a typographical message to indicate a presence or an absence of one or more pathogens 104 within one or more samples 102. For example, in some embodiments, one or more display units 124 may display a "Pathogen Not Present" message if one or more pathogens 104 are not found within one or more samples 102 and a "Pathogen Present" message if one or more pathogens 104 are found within one or more samples 102. Such messages may be displayed in numerous languages. In some embodiments, one or more display units 124 may display one or more messages in multiple formats. For example, in some embodiments, one or more messages may be displayed in colored text.

FIG. 67 illustrates alternative embodiments of system 6000 of FIG. 60. FIG. 67 illustrates example embodiments of module 6030. Additional embodiments may include an embodiment 6702, and/or an embodiment 6704.

At embodiment 6702, module 6030 may include one or more display units that indicate an identity of one or more pathogens present within the one or more samples. In some embodiments, a system may include one or more display units 124 that indicate an identity of one or more pathogens 104 present within the one or more samples 102. In some embodiments, one or more display units 124 may be operably associated with one or more microfluidic chips 108. Accordingly, in some embodiments, one or more display units 124 may be configured to display the identity of one or more pathogens 104 that are present and/or absent from one or more samples 102. For example, in some embodiments, a display unit 124 may be configured to indicate a presence or an absence of Salmonella in a food product.

At embodiment 6704, module 6030 may include one or more display units that indicate one or more concentrations of one or more pathogens within the one or more samples. In some embodiments, a system may include one or more display units 124 that indicate one or more concentrations of one or more pathogens 104 within the one or more samples 102. Concentration may be displayed in numerous formats. For example, in some embodiments, concentration may be expressed numerically. In some embodiments, concentration may be expressed graphically. For example, in some embodiments, one or more display units 124 may include a display having a gray scale on which the concentration of one or more pathogen indicators 106 and/or pathogens 104 that are present within one or more samples 102 may be indicated (e.g., higher concentrations of one or more pathogens 104 may be displayed as dark gray while lower concentrations of one or more pathogens 104 may be displayed as light gray). In some embodiments, one or more display units 124 may include a display having a color scale on which the concentration of one or more pathogens 104 that are present within one or more samples 102 may be indicated (e.g., low concentrations of one or more pathogen indicators 106 may be indicated by a green light, intermediate concentrations of one or more pathogen indicators 106 may be indicated by a yellow light, high concentrations of one or more pathogen indicators 106 may be indicated by a red light). In some embodiments, one or more display units 124 may be calibrated to an individual. For example, in some embodiments, a display unit 124 may be calibrated relative to a person who is immune compromised. Accordingly, in some embodiments, an individual may obtain an indication from a display that indicates if a food product contains a dangerous level of one or more pathogens 104.

FIG. 68 illustrates alternative embodiments of system 6000 of FIG. 60. FIG. 68 illustrates example embodiments of module 6040. Additional embodiments may include an embodiment 6802, an embodiment 6804, an embodiment 6806, an embodiment 6808, and/or an embodiment 6810.

At embodiment 6802, module 6040 may include one or more reagent delivery units configured for detachable connection to the one or more microfluidic chips. In some embodiments, a system may include one or more reagent delivery units 116 configured for detachable connection to the one or more microfluidic chips 108. Reagent delivery units 116 may be configured to deliver one or more types of reagents to one or more microfluidic chips 108. In some embodiments, such reagents may be utilized to analyze and/or process one or more samples 102. In some embodiments, such reagents may be utilized to facilitate detection of one or more pathogen indicators 106. Examples of such reagents include, but are not limited to, solvents, water, tags, labels, antibodies, aptamers, polynucleotides, and the like. In some embodiments, one or more reagent delivery units 116 may include connectors that may be coupled to one or more microfluidic chips 108 to provide for delivery of one or more reagents to the one or more microfluidic chips 108. Examples of such connectors include, but are not limited to, leur lock fittings, needles, fluid connectors, and the like. In some embodiments, a reagent delivery unit 116 may include one or more pumps. In some embodiments, a reagent delivery unit 116 may include numerous reservoirs that may include numerous types of reagents. Accordingly, in some embodiments, a reagent delivery unit 116 may be configured to detachably connect with numerous types of microfluidic chips 108 that are configured to facilitate analysis and/or detection of numerous types of pathogens 104 and/or pathogen indicators 106.

At embodiment 6804, module 6040 may include one or more reagent reservoirs. In some embodiments, a system may include one or more reagent reservoirs. In some embodiments, the one or more reagent reservoirs may be configured to contain reagents that may be used to facilitate analysis and/or detection of a single type of pathogen 104 and/or pathogen indicator 106. In some embodiments, the one or more reagent reservoirs may be configured to contain reagents that may be used to facilitate analysis and/or detection of multiple types of pathogens 104 and/or pathogen indicators 106.

At embodiment 6806, module 6040 may include one or more waste reservoirs. In some embodiments, a system may include one or more waste reservoirs. Such waste reservoirs may be configured in numerous ways. For example such waste reservoirs may be configured for containing reagents, samples 102, and the like. In some embodiments, waste reservoirs may be configured to contain liquids, solids, gels, and substantially any combination thereof.

At embodiment 6808, module 6040 may include one or more reagent delivery units physically coupled to the one or more microfluidic chips. In some embodiments, a system may include one or more reagent delivery units 116 physically coupled to the one or more microfluidic chips 108. For example, in some embodiments, one or more reagent delivery units 116 may be included within a microfluidic chip 108 (e.g., as opposed to being separate from a microfluidic chip 108). In some embodiments, such microfluidic chips 108 may be configured for single use to facilitate analysis and/or detection of one or more pathogen indicators 106 that may be present within one or more samples 102. The reagent delivery units 116 may contain numerous types of reagents that may provide for analysis of one or more samples 102.

For example, in some embodiments, a microfluidic chip 108 may be configured for extraction and/or analysis of polynucleotides that may be included within one or more samples 102. In some embodiments, such a microfluidic chip 108 may include: a first reagent delivery unit 116 that includes an alkaline lysis buffer (e.g., sodium hydroxide/sodium dodecyl sulfate), a second reagent delivery unit 116 that includes an agent that precipitates the sodium dodecyl sulfate (e.g., potassium acetate), a third reagent delivery unit 116 that includes an extraction agent (e.g., phenol/chloroform), and a fourth reagent delivery unit 116 that includes a precipitation agent for precipitating any polynucleotides that may be present within the one or more samples 102. Accordingly, in some embodiments, a system may include one or more microfluidic chips 108 that are configured to include all of the reagents necessary to facilitate analysis of one or more samples 102 for one or more pathogen indicators 106. In some embodiments, such microfluidic chips 108 may be configured for single use. In some embodiments, such microfluidic chips 108 may be configured for repeated use. In some embodiments, such microfluidic chips 108 may be configured to detachably connect to one or more detection units 122 such that the same detection unit 122 may be used repeatedly through association with a new microfluidic chip 108.

At embodiment 6810, module 6040 may include one or more reagent delivery units that include one or more pumps. In some embodiments, a system may include one or more reagent delivery units 116 that include one or more pumps. Numerous types of pumps may be associated with one or more reagent delivery units 116.

FIG. 69 illustrates alternative embodiments of system 6000 of FIG. 60. FIG. 69 illustrates example embodiments of module 6050. Additional embodiments may include an embodiment 6902, and/or an embodiment 6904.

At embodiment 6902, module 6050 may include one or more centrifugation units configured to centrifuge the one or more microfluidic chips that are operably associated with the one or more centrifugation units. In some embodiments, a system may include one or more centrifugation units 118 configured to centrifuge the one or more microfluidic chips 108 that are operably associated with the one or more centrifugation units 118. In some embodiments, one or more centrifugation units 118 may be configured to detachably associate with one or more microfluidic chips 108. For example, in some embodiments, a centrifugation unit 118 may include one or more centrifuge drives that are configured to detachably associate with one or more centrifuge rotors that are included within one or more microfluidic chips 108. In some embodiments, such centrifuge drives may magnetically couple with the one or more centrifuge rotors. In some embodiments, such centrifuge drives may physically couple with the one or more centrifuge rotors. In some embodiments, one or more centrifugation units 118 may be configured to centrifuge an entire microfluidic chip 108. For example, in some embodiments, a microfluidic chip 108 may be configured to associate with one or more centrifugation units 118 such that the microfluidic chip 108 is subjected to centrifugal force. In some embodiments, such a microfluidic chip 108 may be configured in a manner that resembles a compact disc. Accordingly, in some embodiments, a centrifugation unit 118 may be configured in a manner that resembles a compact disc player. In some embodiments, one or more centrifugation units may be configured to centrifuge one or more samples 102 through a series of mesh filters to concentrate parasite eggs and/or larvae (e.g., U.S. Patent No.: 4081356; herein incorporated by reference).

At embodiment 6904, module 6050 may include one or more centrifugation units configured to provide for chromatographic separation. In some embodiments, a system may include one or more centrifugation units 118 configured to provide for chromatographic separation. For example, in some embodiments, one or more centrifugation units 118 may be configured to centrifuge one or more samples 102 through one or more chromatographic columns that are associated with one or more microfluidic chips 108. In some embodiments, such microfluidic chips 108 may be coupled to one or more reagent reservoirs such that one or more fluids may be passed through one or more chromatographic columns through use of centrifugation. For example, in some embodiments, chromatographic separation may be used to separate one or more polynucleotides from one or more samples 102 through use of chromatographic media that is configured as a spin column.

FIG. 70 illustrates alternative embodiments of system 6000 of FIG. 60. FIG. 70 illustrates example embodiments of module 6050. Additional embodiments may include an embodiment 7002, and/or an embodiment 7004.

At embodiment 7002, module 6050 may include one or more centrifugation units configured for polynucleotide extraction from the one or more samples. In some embodiments, a system may include one or more centrifugation units 118 configured for polynucleotide extraction from the one or more samples 102. For example, a microfluidic chip 108 may be configured to utilize alkaline lysis (e.g., miniprep procedure) to extract polynucleotides from one or more samples 102. Such methods have been described. In some embodiments, alkaline lysis may be combined with additional methods, such as chromatography, to facilitate extraction of polynucleotides from one or more samples 102.

At embodiment 7004, module 6050 may include one or more centrifugation units configured to provide for gradient centrifugation. In some embodiments, a system may include one or more centrifugation units 118 configured to provide for gradient centrifugation. In some embodiments, one or more centrifugation units 118 may be configured to provide for density gradient centrifugation. In some embodiments, one or more centrifugation units 118 may be configured to provide for velocity gradient centrifugation. In some embodiments, gradient centrifugation may be used to concentrate viral particles.

FIG. 71 illustrates alternative embodiments of system 6000 of FIG. 60. FIG. 71 illustrates example embodiments of module 6060. Additional embodiments may include an embodiment 7102, and/or an embodiment 7104.

At embodiment 7102, module 6060 may include one or more reservoirs that are configured for containing the one or more reagents. In some embodiments, a system may include one or more reservoirs that are configured for containing one or more reagents. Reservoirs may be configured to contain and/or deliver numerous types of reagents. Examples of such reagents include, but are not limited to, phenol, chloroform, alcohol, salt solutions, detergent solutions, solvents, reagents used for polynucleotide precipitation, reagents used for polypeptide precipitation, reagents used for polynucleotide extraction, reagents used for polypeptide extraction, reagents used for chemical extractions, and the like. Accordingly, reservoirs may be configured to contain and/or deliver virtually any reagent that may be used for the analysis of one or more pathogens 104 and/or pathogen indicators 106.

At embodiment 7104, module 6060 may include one or more reservoirs that are configured as one or more waste reservoirs. In some embodiments, a system may include one or more reservoirs that are configured as waste reservoirs. Such waste reservoirs may be configured in numerous ways. For example such waste reservoirs may be configured for containing reagents, samples 102, and the like. In some embodiments, waste reservoirs may be configured to contain liquids, solids, gels, and substantially any combination thereof.

### III. DEVICES FOR ANALYSIS OF ONE OR MORE PATHOGENS

FIG. 72 illustrates a device 7200 representing examples of modules that may be used to perform a method for analysis of one or more pathogens 104. In FIG. 72, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The device 7200 includes module 7210 that includes one or more detection units configured to detachably connect to one or more microfluidic chips and configured to detect one or more pathogen indicators that are associated with one or more samples. In some embodiments, module 7210 may include one or more detection units configured to detect the one or more pathogen indicators that are associated with one or more airborne pathogens. In some embodiments, module 7210 may include one or more detection units configured to detect the one or more pathogen indicators that are associated with one or more food products. In some embodiments, module 7210 may include one or more detection units that are configured to detect one or more pathogens that include at least one virus, bacterium, prion, worm, egg, cyst, protozoan, single-celled organism, fungus, algae, pathogenic protein, or microbe. In some embodiments, module 7210 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, module 7210 may include one or more detection units that are configured for detachable connection to the one or more microfluidic chips.

The device 7200 may optionally include module 7220 that includes one or more reagent delivery units that are configured to deliver one or more reagents to the one or more microfluidic chips. In some embodiments, module 7220 may include one or more reagent delivery units configured for detachable connection to the one or more microfluidic chips. In some embodiments, module 7220 may include one or more reagent reservoirs. In some embodiments, module 7220 may include one or more waste reservoirs. In some embodiments, module 7220 may include one or more reagent delivery units physically coupled to the one or more microfluidic chips. In some embodiments, module 7220 may include one or more reagent delivery units that include one or more pumps.

The device 7200 may optionally include module 7230 that includes one or more controllable magnets that are configured to facilitate movement of a magnetically active plug that is included within the one or more microfluidic chips. In some embodiments, module 7230 may include one or more electromagnets. In some embodiments, module 7230 may include one or more ferromagnets. In some embodiments, module 7230 may include one or more ferrofluids.

FIG. 73 illustrates alternative embodiments of device 7200 of FIG. 72. FIG. 73 illustrates example embodiments of module 7210. Additional embodiments may include an embodiment 7302, an embodiment 7304, an embodiment 7306, an embodiment 7308, and/or an embodiment 7310.

At embodiment 7302, module 7210 may include one or more detection units configured to detect the one or more pathogen indicators that are associated with one or more airborne pathogens. In some embodiments, a system may include one or more detection units 122 that are configured to detect the one or more pathogen indicators 106 that are associated with one or more pathogens 104 that are airborne. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more microfluidic chips 108 may be configured to analyze and/or detect severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 7304, module 7210 may include one or more detection units configured to detect the one or more pathogen indicators that are associated with one or more food products. In some embodiments, one or more detection units 122 may be configured to detect the one or more pathogen indicators 106 that are associated with one or more food products. In some embodiments, one or more detection units 122 may be configured to detect one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

At embodiment 7306, module 7210 may include one or more detection units that are configured to detect one or more pathogens that include at least one virus, bacterium, prion, worm, egg, cyst, protozoan, single-celled organism, fungus, algae, pathogenic protein, or microbe. In some embodiments, one or more detection units may be configured to detect one or more pathogens that include at least one virus, bacterium, prion, worm, egg, cyst, protozoan, single-celled organism, fungus, algae, pathogenic protein, microbe, or substantially any combination thereof. A detection unit may be configured to utilize numerous types of techniques, and combinations of techniques, to detect one or more pathogens. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At embodiment 7308, module 7210 may include one or more detection units that are configured to detect the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, one or more detection units 122 may be configured to detect the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection units 122 may be configured to detect one or more pathogen indicators 106 that have been processed by one or more microfluidic chips 108 and/or analyzed by one or more analysis units 120. For example, in some embodiments, one or more microfluidic chips 108 may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108.

In some embodiments, one or more detection units 122 may be configured to detect one or more pathogen indicators 106 through use of surface plasmon resonance. In some embodiments, one or more detection units 122 may be configured to operably associate with one or more microfluidic chips 108 may include one or more antibodies, aptamers, proteins, peptides, polynucleotides, and the like, that are bound to a substrate (e.g., a metal film) within the one or more microfluidic chips 108. In some embodiments, such microfluidic chips 108 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more microfluidic chips 108 to facilitate detection of one or more pathogen indicators 106 through use of surface plasmon resonance.

In some embodiments, one or more detection units 122 may be configured to detect one or more pathogen indicators 106 through use of nuclear magnetic resonance (NMR). In some embodiments, one or more detection units 122 may be configured to operably associate with one or more microfluidic chips 108 that include a nuclear magnetic resonance (NMR) probe. Accordingly, in some embodiments, one or more pathogen indicators 106 may be analyzed and detected with one or more microfluidic chips and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection units 122 may be configured to detect one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, one or more analysis units 120 may be configured to facilitate analysis of one or more pathogen indicators 106 and configured to facilitate fluorescent detection of the one or more pathogen indicators 106 with one or more detection units 122.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more microfluidic chips 108 that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more microfluidic chips 108 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more microfluidic chips 108 may be configured to utilize numerous electron transfer based assays to provide for detection of one or more pathogen indicators 106 by a detection unit 122 that is configured to operably associate with the one or more microfluidic chips 108.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more microfluidic chips 108 may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more pathogen indicators 106 may be detected through use of electrical conductivity. In some embodiments, one or more microfluidic chips 108 may be configured to provide for detection of one or more pathogen indicators 106 through use of electrical conductivity. In some embodiments, such microfluidic chips 108 may be configured to operably associate with one or more detection units 122 such that the one or more detection units 122 can detect one or more pathogen indicators 106 through use of electrical conductivity. In some embodiments, one or more microfluidic chips 108 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleic acid associated with one or more samples 102 may be combined with one or more sets of paired primers such that use of an amplification protocol, such as a polymerase chain reaction, will produce an amplification product corresponding to pathogen associated polynucleic acid that was contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a cyst, egg, pathogen 104, spore, and the like, will complete an electrical circuit and facilitate the production of a detectable electrical current. Accordingly, in some embodiments, one or more microfluidic chips 108 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more microfluidic chips 108 with the one or more detection units 122. In some embodiments, the one or more detectors may be configured for detachable connection to one or more microfluidic chips 108. Microfluidic chips 108 and detection units 122 may be configured in numerous ways to process one or more samples 102 and detect one or more pathogen indicators 106.

In some embodiments, one or more pathogen indicators 106 may be detected through use of isoelectric focusing. In some embodiments, one or more microfluidic chips 108 may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more microfluidic chips 108 such that the one or more detection units 122 can be used to detect one or more pathogen indicators 106 that have been focused within one or more microfluidic channels of the one or more microfluidic chips 108. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more microfluidic chips 108 may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be processed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Microfluidic chips 108 and detection units 122 may be configured in numerous ways to facilitate detection of one or more pathogen indicators 106 through use of isoelectric focusing.

In some embodiments, one or more pathogen indicators 106 may be detected through use of chromatographic methodology alone or in combination with additional detection methods. In some embodiments, one or more microfluidic chips 108 may be configured to provide for detection of one or more pathogen indicators 106 through use of chromatographic methods. Accordingly, in some embodiments, one or more detection units 122 may be configured to operably associate with the one or more microfluidic chips 108 and detect one or more pathogen indicators 106. In some embodiments, the one or more detection units 122 may be configured to operably associate with one or more microfluidic chips 108 and supply solvents and other reagents to the one or more microfluidic chips 108. For example, in some embodiments, one or more detection units 122 may include pumps and solvent/buffer reservoirs that are configured to supply solvent/buffer flow through chromatographic media (e.g., a chromatographic column) that is operably associated with one or more microfluidic chips 108. In some embodiments, one or more detection units 122 may be configured to operably associate with one or more microfluidic chips 108 and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more microfluidic chips 108 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more microfluidic chips 108 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection units 122 may be configured to detect one or more pathogen indicators 106 through use of immunoprecipitation. In some embodiments, immunoprecipitation may be utilized in combination with additional detection methods to detect one or more pathogen indicators 106. In some embodiments, one or more microfluidic chips 108 may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more microfluidic chips 108 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more detection units 122 may be configured to detect one or more pathogen indicators 106 through use of immunoseparation. In some embodiments, immunoseparation may be utilized in combination with additional detection methods to detect one or more pathogen indicators 106. In some embodiments, one or more microfluidic chips 108 may be configured to facilitate detection of one or more pathogen indicators 106 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection units 122 may be configured to detect one or more pathogen indicators 106 through use of aptamer binding. In some embodiments, aptamer binding may be utilized in combination with additional methods to detect one or more pathogen indicators 106. In some embodiments, one or more microfluidic chips 108 may be configured to facilitate detection of one or more pathogen indicators 106 through use of aptamer binding. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more pathogen indicators 106 may be detected through use of electrophoresis. In some embodiments, one or more microfluidic chips 108 may be configured to facilitate detection of one or more pathogen indicators 106 through use of electrophoresis. In some embodiments, such microfluidic chips 108 may be configured to operably associate with one or more detection units 122. Accordingly, in some embodiments, one or more detection units 122 may be configured to operably associate with one or more microfluidic chips 108 and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Microfluidic chips 108 and detection units 122 may be configured in numerous ways to provide for detection of one or more pathogen indicators 106 through use of electrophoresis.

In some embodiments, one or more pathogen indicators 106 may be detected through use of one or more charge-coupled device (CCD) cameras. In some embodiments, one or more detection units 122 that include one or more CCD cameras may be configured to operably associate with one or more microfluidic chips 108. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more microfluidic chips 108 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be detected through use of immunoassay. In some embodiments, one or more microfluidic chips 108 may be configured to facilitate detection of one or more pathogen indicators 106 through use of immunoassay. In some embodiments, one or more detection units 122 may be configured to operably associate with one or more such microfluidic chips 108 and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

At embodiment 7310, module 7210 may include one or more detection units that are configured for detachable connection to the one or more microfluidic chips. In some embodiments, one or more detection units 122 may be configured for detachable connection to the one or more microfluidic chips 108. In some embodiments, the one or more detection units 122 may be connected to the one or more microfluidic chips 108 through use of fasteners. Examples of such fasteners include, but are not limited to, hooks, screws, bolts, pins, grooves, adhesives, and the like. In some embodiments, the one or more detection units may be connected to the one or more microfluidic chips 108 through use of magnets.

FIG. 74 illustrates alternative embodiments of device 7200 of FIG. 72. FIG. 74 illustrates example embodiments of module 7220. Additional embodiments may include an embodiment 7402, an embodiment 7404, an embodiment 7406, an embodiment 7408, and/or an embodiment 7410.

At embodiment 7402, module 7220 may include one or more reagent delivery units configured for detachable connection to the one or more microfluidic chips. In some embodiments, a system may include one or more reagent delivery units 116 configured for detachable connection to the one or more microfluidic chips 108. Reagent delivery units 116 may be configured to deliver one or more types of reagents to one or more microfluidic chips 108. In some embodiments, such reagents may be utilized to analyze and/or process one or more samples 102. In some embodiments, such reagents may be utilized to facilitate detection of one or more pathogen indicators 106. Examples of such reagents include, but are not limited to, solvents, water, tags, labels, antibodies, aptamers, polynucleotides, and the like. In some embodiments, one or more reagent delivery units 116 may include connectors that may be coupled to one or more microfluidic chips 108 to provide for delivery of one or more reagents to the one or more microfluidic chips 108. Examples of such connectors include, but are not limited to, leur lock fittings, needles, fluid connectors, and the like. In some embodiments, a reagent delivery unit 116 may include one or more pumps. In some embodiments, a reagent delivery unit 116 may include numerous reservoirs that may include numerous types of reagents. Accordingly, in some embodiments, a reagent delivery unit 116 may be configured to detachably connect with numerous types of microfluidic chips 108 that are configured to facilitate analysis and/or detection of numerous types of pathogens 104 and/or pathogen indicators 106.

At embodiment 7404, module 7220 may include one or more reagent reservoirs. In some embodiments, a system may include one or more reagent reservoirs. In some embodiments, the one or more reagent reservoirs may be configured to contain reagents that may be used to facilitate analysis and/or detection of a single type of pathogen 104 and/or pathogen indicator 106. In some embodiments, the one or more reagent reservoirs may be configured to contain reagents that may be used to facilitate analysis and/or detection of multiple types of pathogens 104 and/or pathogen indicators 106.

At embodiment 7406, module 7220 may include one or more waste reservoirs. In some embodiments, a system may include one or more waste reservoirs. Such waste reservoirs may be configured in numerous ways. For example such waste reservoirs may be configured for containing reagents, samples 102, and the like. In some embodiments, waste reservoirs may be configured to contain liquids, solids, gels, and substantially any combination thereof.

At embodiment 7408, module 7220 may include one or more reagent delivery units physically coupled to the one or more microfluidic chips. In some embodiments, a system may include one or more reagent delivery units 116 physically coupled to the one or more microfluidic chips 108. For example, in some embodiments, one or more reagent delivery units 116 may be included within a microfluidic chip 108 (e.g., as opposed to being separate from a microfluidic chip 108). In some embodiments, such microfluidic chips 108 may be configured for single use to facilitate analysis and/or detection of one or more pathogen indicators 106 that may be present within one or more samples 102. The reagent delivery units 116 may contain numerous types of reagents that may provide for analysis of one or more samples 102.

For example, in some embodiments, a microfluidic chip 108 may be configured for extraction and/or analysis of polynucleotides that may be included within one or more samples 102. In some embodiments, such a microfluidic chip 108 may include: a first reagent delivery unit 116 that includes an alkaline lysis buffer (e.g., sodium hydroxide/sodium dodecyl sulfate), a second reagent delivery unit 116 that includes an agent that precipitates the sodium dodecyl sulfate (e.g., potassium acetate), a third reagent delivery unit 116 that includes an extraction agent (e.g., phenol/chloroform), and a fourth reagent delivery unit 116 that includes a precipitation agent for precipitating any polynucleotides that may be present within the one or more samples 102. Accordingly, in some embodiments, a system may include one or more micro fluidic chips 108 that are configured to include all of the reagents necessary to facilitate analysis of one or more samples 102 for one or more pathogen indicators 106. In some embodiments, such microfluidic chips 108 may be configured for single use. In some embodiments, such microfluidic chips 108 may be configured for repeated use. In some embodiments, such microfluidic chips 108 may be configured to detachably connect to one or more detection units 122 such that the same detection unit 122 may be used repeatedly through association with a new microfluidic chip 108.

At embodiment 7410, module 7220 may include one or more reagent delivery units that include one or more pumps. In some embodiments, a system may include one or more reagent delivery units 116 that include one or more pumps. Numerous types of pumps may be associated with one or more reagent delivery units 116.

FIG. 75 illustrates alternative embodiments of device 7200 of FIG. 72. FIG. 75 illustrates example embodiments of module 7230. Additional embodiments may include an embodiment 7502, an embodiment 7504, and/or an embodiment 7506.

At embodiment 7502, module 7230 may include one or more electromagnets. In some embodiments, a system may include one or more electromagnets. In some embodiments, the one or more electromagnets may be configured to facilitate movement of a magnetically active plug relative to one or more microfluidic chips 108. For example, in some embodiments, a magnetically active plug may be movably positioned within one or more channels of one or more microfluidic chips 108. In some embodiments, movement of fluids with the one or more magnetically active plugs may be facilitated by one or more electromagnets. One or more electromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more electromagnets may be used to create an attractive magnetic field (e.g., a magnetic field that attracts a ferrous material). In some embodiments, the one or more electromagnets may be used to create a repulsive magnetic field (e.g., a magnetic field that repulses a ferrous material). In some embodiments, the one or more electromagnets may be used to create one or more eddy currents. In some embodiments, one or more electromagnets may be moved from position to position. In some embodiments, two or more electromagnets may be selectively activated. In some embodiments, the movement of one or more magnetic plugs may be selectively facilitated through selective activation of one or more electromagnets.

At embodiment 7504, module 7230 may include one or more ferromagnets. In some embodiments, a system may include one or more ferromagnets. In some embodiments, the one or more ferromagnets may be configured to facilitate movement of a magnetically active plug relative to one or more microfluidic chips. For example, in some embodiments, a magnetically active plug may be movably positioned within one or more channels of one or more microfluidic chips. In some embodiments, movement of fluids with the one or more magnetically active plugs may be facilitated by one or more ferromagnets. One or more ferromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more ferromagnets may be used to create an attractive magnetic field (e.g., a magnetic field that attracts a ferrous material). In some embodiments, the one or more ferromagnets may be used to create a repulsive magnetic field (e.g., a magnetic field that repulses a ferrous material). In some embodiments, the one or more ferromagnets may be used to create one or more eddy currents. In some embodiments, one or more ferromagnets may be moved from position to position.

At embodiment 7506, module 7230 may include one or more ferrofluids. In some embodiments, a system may include one or more ferrofluids. In some embodiments, the one or more ferromagnets may be configured to facilitate movement of one or more samples 102, such as fluids, relative to one or more microfluidic chips 108. For example, in some embodiments, a ferrofluid plug may be movably positioned within one or more channels of one or more microfluidic chips 108. In some embodiments, movement of one or more ferrofluid plugs may be facilitated by one or more ferromagnets, one or more electromagnets, or substantially any combination thereof.

FIG. 76 illustrates a device 7600 representing examples of modules that may be used to perform a method for analysis of one or more pathogens 104. In FIG. 76, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The device 7600 includes module 7610 that includes one or more fasteners adapted to detachably associate with one or more microfluidic chips that include one or more separation channels that are configured to allow one or more samples that include one or more magnetically active pathogen indicator complexes to flow in a substantially parallel manner with one or more separation fluids. In some embodiments, module 7610 may include one or more mechanical fasteners. In some embodiments, module 7610 may include one or more magnetic fasteners.

The device 7600 includes module 7620 that includes one or more magnets that facilitate movement of the one or more magnetically active pathogen indicator complexes associated with the one or more samples into the one or more separation fluids. In some embodiments, module 7620 may include one or more electromagnets. In some embodiments, module 7620 may include one or more ferromagnets.

FIG. 77 illustrates alternative embodiments of device 7600 of FIG. 76. FIG. 77 illustrates example embodiments of module 7610. Additional embodiments may include an embodiment 7702 and/or an embodiment 7704.

At embodiment 7702, module 7610 may include one or more mechanical fasteners. In some embodiments, a device may include one or more mechanical fasteners. Numerous types of mechanical fasteners may be used to detachably associate a device with one or more microfluidic chips 108. Examples of such fasteners include, but are not limited to, screws, clips, adhesives, pins, brackets, and the like.

At embodiment 7704, module 7610 may include one or more magnetic fasteners. In some embodiments, a device may include one or more magnetic fasteners. Magnetic fasteners may be configured in numerous ways to detachably associate a device with one or more microfluidic chips 108. In some embodiments, one or more magnets may be configured to associate one or more devices with one or more microfluidic chips 108 through direct magnetic attraction. In some embodiments, one or more devices may be associated with one or more microfluidic chips 108 through use of magnets that control fasteners. For example, in some embodiments, one or more magnets may be used to attach a metal pin that serves to fasten one or more microfluidic chips 108 to one or more devices.

FIG. 78 illustrates alternative embodiments of device 7600 of FIG. 76. FIG. 78 illustrates example embodiments of module 7620. Additional embodiments may include an embodiment 7802 and/or an embodiment 7804.

At embodiment 7802, module 7620 may include one or more electromagnets. In some embodiments, a device may include one or more electromagnets. In some embodiments, the one or more electromagnets may be configured to facilitate movement of a magnetically active plug relative to one or more microfluidic chips 108. For example, in some embodiments, a magnetically active plug may be movably positioned within one or more channels of one or more microfluidic chips 108. In some embodiments, movement of fluids with the one or more magnetically active plugs may be facilitated by one or more electromagnets. One or more electromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more electromagnets may be used to create an attractive magnetic field (e.g., a magnetic field that attracts a ferrous material). In some embodiments, the one or more electromagnets may be used to create a repulsive magnetic field (e.g., a magnetic field that repulses a ferrous material). In some embodiments, the one or more electromagnets may be used to create one or more eddy currents. In some embodiments, one or more electromagnets may be moved from position to position. In some embodiments, two or more electromagnets may be selectively activated. In some embodiments, the movement of one or more magnetic plugs may be selectively facilitated through selective activation of one or more electromagnets.

At embodiment 7804, module 7620 may include one or more ferromagnets. In some embodiments, a system may include one or more ferromagnets. In some embodiments, the one or more ferromagnets may be configured to facilitate movement of a magnetically active plug relative to one or more microfluidic chips 108. For example, in some embodiments, a magnetically active plug may be movably positioned within one or more channels of one or more microfluidic chips 108. In some embodiments, movement of fluids with the one or more magnetically active plugs may be facilitated by one or more ferromagnets. One or more ferromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more ferromagnets may be used to create an attractive magnetic field (e.g., a magnetic field that attracts a ferrous material). In some embodiments, the one or more ferromagnets may be used to create a repulsive magnetic field (e.g., a magnetic field that repulses a ferrous material). In some embodiments, the one or more ferromagnets may be used to create one or more eddy currents. In some embodiments, one or more ferromagnets may be moved from position to position.

FIG. 79 illustrates a device 7900 representing examples of modules that may be used to perform a method for analysis of one or more pathogens 104. In FIG. 79, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The device 7900 includes module 7910 that includes one or more fasteners adapted to detachably associate with one or more microfluidic chips that include one or more separation channels that are configured to allow one or more samples that include one or more magnetically active pathogen indicator complexes to flow in a substantially antiparallel manner with one or more separation fluids. In some embodiments, module 7910 may include one or more mechanical fasteners. In some embodiments, module 7910 may include one or more magnetic fasteners.

The device 7900 includes module 7920 that includes one or more magnets that facilitate movement of the one or more magnetically active pathogen indicator complexes associated with the one or more samples into the one or more separation fluids. In some embodiments, module 7920 may include one or more electromagnets. In some embodiments, module 7920 may include one or more ferromagnets.

FIG. 80 illustrates alternative embodiments of device 7900 of FIG. 79. FIG. 80 illustrates example embodiments of module 7910. Additional embodiments may include an embodiment 8002 and/or an embodiment 8004.

At embodiment 8002, module 7910 may include one or more mechanical fasteners. In some embodiments, a device may include one or more mechanical fasteners. Numerous types of mechanical fasteners may be used to detachably associate a device with one or more microfluidic chips 108. Examples of such fasteners include, but are not limited to, screws, clips, adhesives, pins, brackets, and the like.

At embodiment 8004, module 7910 may include one or more magnetic fasteners. In some embodiments, a device may include one or more magnetic fasteners. In some embodiments, a device may include one or more magnetic fasteners. Magnetic fasteners may be configured in numerous ways to detachably associate a device with one or more microfluidic chips 108. In some embodiments, one or more magnets may be configured to associate one or more devices with one or more microfluidic chips 108 through direct magnetic attraction. In some embodiments, one or more devices may be associated with one or more microfluidic chips 108 through use of magnets that control fasteners. For example, in some embodiments, one or more magnets may be used to attach a metal pin that serves to fasten one or more microfluidic chips 108 to one or more devices.

FIG. 81 illustrates alternative embodiments of device 7900 of FIG. 79. FIG. 81 illustrates example embodiments of module 7920. Additional embodiments may include an embodiment 8102 and/or an embodiment 8104.

At embodiment 8102, module 7920 may include one or more electromagnets. In some embodiments, a device may include one or more electromagnets. In some embodiments, the one or more electromagnets may be configured to facilitate movement of a magnetically active plug relative to one or more microfluidic chips 108. For example, in some embodiments, a magnetically active plug may be movably positioned within one or more channels of one or more microfluidic chips 108. In some embodiments, movement of fluids with the one or more magnetically active plugs may be facilitated by one or more electromagnets. One or more electromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more electromagnets may be used to create an attractive magnetic field (e.g., a magnetic field that attracts a ferrous material). In some embodiments, the one or more electromagnets may be used to create a repulsive magnetic field (e.g., a magnetic field that repulses a ferrous material). In some embodiments, the one or more electromagnets may be used to create one or more eddy currents. In some embodiments, one or more electromagnets may be moved from position to position. In some embodiments, two or more electromagnets may be selectively activated. In some embodiments, the movement of one or more magnetic plugs may be selectively facilitated through selective activation of one or more electromagnets.

At embodiment 8104, module 7920 may include one or more ferromagnets. In some embodiments, a device may include one or more ferromagnets. In some embodiments, the one or more ferromagnets may be configured to facilitate movement of a magnetically active plug relative to one or more microfluidic chips 108. For example, in some embodiments, a magnetically active plug may be movably positioned within one or more channels of one or more microfluidic chips 108. In some embodiments, movement of fluids with the one or more magnetically active plugs may be facilitated by one or more ferromagnets. One or more ferromagnets may be used to facilitate movement of numerous types of magnetically active plugs. Examples of such plugs include, but are not limited to, plugs that include ferromagnetic materials, plugs that include non-ferrous metals, ferrofluids, and the like. In some embodiments, the one or more ferromagnets may be used to create an attractive magnetic field (e.g., a magnetic field that attracts a ferrous material). In some embodiments, the one or more ferromagnets may be used to create a repulsive magnetic field (e.g., a magnetic field that repulses a ferrous material). In some embodiments, the one or more ferromagnets may be used to create one or more eddy currents. In some embodiments, one or more ferromagnets may be moved from position to position.

### IV. MICROFLUIDIC CHIPS FOR ANALYSIS OF ONE OR MORE PATHOGENS

FIG. 82 illustrates a microfluidic chip 8200 representing examples of modules that may be used to perform a method for analysis of one or more pathogens 104. In FIG. 82, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The microfluidic chip 8200 includes module 8210 that includes one or more accepting units configured to accept one or more samples. In some embodiments, module 8210 may include one or more accepting units configured to accept the one or more samples that include one or more liquids. In some embodiments, module 8210 may include one or more accepting units configured to accept the one or more samples that include one or more solids. In some embodiments, module 8210 may include one or more accepting units configured to accept the one or more samples that include one or more gases. In some embodiments, module 8210 may include one or more accepting units configured to accept the one or more samples that include one or more food products. In some embodiments, module 8210 may include one or more accepting units configured to accept the one or more samples that include one or more biological samples.

The microfluidic chip 8200 includes module 8220 that includes one or more processing units configured to process the one or more samples for one or more pathogen indicators associated with the one or more samples. In some embodiments, module 8220 may include one or more processing units configured to process the one or more samples through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay.

The microfluidic chip 8200 may optionally include module 8230 that includes one or more analysis units configured for analysis of the one or more pathogen indicators associated with the one or more samples. In some embodiments, module 8230 may include one or more analysis units configured for analysis of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

The microfluidic chip 8200 may optionally include module 8240 that includes one or more detection chambers configured to facilitate detection of the one or more pathogen indicators associated with the one or more samples. In some embodiments, module 8240 may include one or more detection chambers configured to facilitate detection of the one or more pathogen indicators that are associated with one or more airborne pathogens. In some embodiments, module 8240 may include one or more detection chambers configured to facilitate detection of the one or more pathogen indicators that are associated with one or more food products. In some embodiments, module 8240 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, module 8240 may include one or more detection chambers configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

FIG. 83 illustrates alternative embodiments of microfluidic chip 8200 of FIG. 82. FIG. 83 illustrates example embodiments of module 8210. Additional embodiments may include an embodiment 8302, an embodiment 8304, an embodiment 8306, an embodiment 8308, and/or an embodiment 8310.

At embodiment 8302, module 8210 may include one or more accepting units configured to accept the one or more samples that include one or more liquids. In some embodiments, one or more microfluidic chips 108 may include one or more accepting units 110 configured to accept one or more samples 102 that include one or more liquids. In some embodiments, one or more microfluidic chips 108 may include one or more lancets. Such lancets may be configured to provide for collection of one or more samples 102 that include a fluid. In some embodiments, a microfluidic chip 108 may include one or more septa through which a needle may be passed to deliver a fluid sample 102 to the microfluidic chip 108. In some embodiments, a microfluidic chip 108 may include one or more leur lock connectors to which one or more syringes may be coupled to deliver one or more fluid samples 102 to the microfluidic chip 108. In some embodiments, a microfluidic chip 108 may be configured to operably associate with one or more detection units 122 that are configured to deliver one or more liquid samples 102 to the microfluidic chip 108. In some embodiments, an accepting unit 110 may be configured to extract liquids from one or more samples 102. For example, in some embodiments, an accepting unit 110 may include a space into which a sample 102 may be crushed such that the liquid portion of the sample 102 is available for processing by the microfluidic chip 108. In some embodiments, an accepting unit 110 may include one or more sonicators that facilitate release of the liquid portion from a sample 102 to make it available to a microfluidic chip 108. Microfluidic chips 108 may be configured to accept numerous types of liquids. Examples of such liquids include, but are not limited to, beverages, water, food products, solvents, and the like. Accordingly, microfluidic chips 108 may be configured in numerous ways such that they may accept one or more samples 102 that include a liquid.

At embodiment 8304, module 8210 may include one or more accepting units configured to accept the one or more samples that include one or more solids. In some embodiments, one or more microfluidic chips 108 may include one or more accepting units 110 configured to accept one or more samples 102 that include one or more solids. In some embodiments, such accepting units 110 may be configured to suspend a solid sample 102 in a fluid. In some embodiments, such accepting units 110 may be configured to crush a sample 102 into smaller particles. For example, in some embodiments, an accepting unit 110 may accept a solid sample 102 that may be ground into smaller particles to facilitate detection of one or more pathogen indicators 106 that may be present within the sample 102. In some embodiments, an accepting unit 110 may include one or more sonicators that break the sample 102 into smaller particles to facilitate detection of one or more pathogen indicators 106 that may be present within the sample 102. For example, in some embodiments, solid spores, eggs, and/or cysts may be broken into smaller particles to provide for detection of one or more polynucleotides that are associated with the spores. Accordingly, microfluidic chips 108 may be configured in numerous ways such that they may accept one or more samples 102 that include a liquid.

At embodiment 8306, module 8210 may include one or more accepting units configured to accept the one or more samples that include one or more gases. In some embodiments, one or more microfluidic chips 108 may include one or more accepting units 110 configured to accept one or more samples 102 that include one or more gases. For example, in some embodiments, a microfluidic chip 108 may include one or more fans that blow and/or draw gas into the microfluidic chip 108. In some embodiments, a microfluidic chip 108 may include one or more bubble chambers through which one or more gases pass. In some embodiments, such bubble chambers may be configured to include one or more fluids (e.g., solvents) that may be used to selectively retain (e.g., extract) one or more pathogen indicators 106 from one or more gas samples 102. In some embodiments, a microfluidic chip 108 may include one or more electrostatic filters through which one or more gases pass. Such electrostatic filters may be configured to capture numerous types of pathogens 104 and/or pathogen indicators 106. Examples of such pathogens 104 and/or pathogen indicators 106 include, but are not limited to, viruses, fungus, spores, and the like. In some embodiments, a microfluidic chip 108 may include one or more filters through which one or more gases pass. Such filters may be configured to capture pathogen indicators 106 according to numerous properties, such as size, hydrophobicity, charge, and the like.

At embodiment 8308, module 8210 may include one or more accepting units configured to accept the one or more samples that include one or more food products. In some embodiments, one or more microfluidic chips 108 may include one or more accepting units 110 configured to accept one or more samples 102 that include one or more food products. In some embodiments, one or more accepting units 110 may be configured to accept one or more food samples 102 that are liquid, such as water, beverages, soups, sauces, and the like. For example, in some embodiments, one or more accepting units 110 may include one or more lancets that may be inserted into the food product to withdraw one or more samples 102. In some embodiments, one or more accepting units 110 may include one or more septa that may be configured to operably associate with a syringe or the like. In some embodiments, one or more accepting units 110 may be configured to accept one or more food samples 102 that are solids, such as meats, cheeses, nuts, vegetables, fruits, and the like. In some embodiments, one or more accepting units 110 may include one or more mechanisms that can facilitate processing of the one or more samples 102. Examples of such mechanisms include, but are not limited to, grinders, sonicators, treatment of the one or more samples 102 with degredative enzymes (e.g., protease, nuclease, lipase, collagenase, and the like), strainers, filters, centrifugation chambers, and the like.

At embodiment 8310, module 8210 may include one or more accepting units configured to accept the one or more samples that include one or more biological samples. In some embodiments, one or more microfluidic chips 108 may include one or more accepting units 110 configured to accept one or more samples 102 that include one or more biological samples 102. Examples of biological samples 102 include, but are not limited to, blood, cerebrospinal fluid, mucus, breath, urine, fecal material, skin, tissue, tears, hair, and the like.

FIG. 84 illustrates alternative embodiments of microfluidic chip 8200 of FIG. 82. FIG. 84 illustrates example embodiments of module 8220. Additional embodiments may include an embodiment 8402.

At embodiment 8402, module 8220 may include one or more processing units configured to process the one or more samples through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, or competition assay. In some embodiments, one or more microfluidic chips 108 may include one or more processing units that are configured to process the one or more samples 102 through use of polynucleotide interaction, protein interaction, peptide interaction, antibody interaction, chemical interaction, diffusion, filtration, chromatography, aptamer interaction, magnetism, electrical conductivity, isoelectric focusing, electrophoresis, immunoassay, competition assay, or substantially any combination thereof. In some embodiments, pathogen indicators 106 may be separated from other materials included within one or more samples 102 through processing. In some embodiments, pathogen indicators 106 may be immobilized through processing to facilitate detection and/or identification of the one or more pathogen indicators 106.

In some embodiments, one or more microfluidic chips 108 may be configured to process one or more samples 102 through use of polynucleotide interaction. Numerous methods based on polynucleotide interaction may be used. Examples of such methods include, but are not limited to, those based on polynucleotide hybridization, polynucleotide ligation, polynucleotide amplification, polynucleotide degradation, and the like. Methods that utilize intercalation dyes, FRET analysis, capacitive DNA detection, and nucleic acid amplification have been described (e.g., U.S. Patent Nos.: 7,118,910 and 6,960,437; herein incorporated by reference). In some embodiments, fluorescence resonance energy transfer, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube are combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Methods to utilize carbon nanotubes as probes for nucleic acid interaction have been described (e.g., U.S. Patent No.: 6,821,730; herein incorporated by reference).

In some embodiments, one or more microfluidic chips 108 may be configured to process one or more samples 102 through use of protein interaction. Numerous methods based on protein interaction may be used. In some embodiments, protein interaction may be used to immobilize one or more pathogen indicators 106. In some embodiments, protein interaction may be used to separate one or more pathogen indicators 106 from one or more samples 102. Examples of such methods include, but are not limited to, those based on ligand binding, protein-protein binding, protein cross-linking, use of green fluorescent protein, phage display, the two-hybrid system, protein arrays, fiber optic evanescent wave sensors, chromatographic techniques, fluorescence resonance energy transfer, regulation of pH to control protein assembly and/or oligomerization, and the like. Methods that may be used to construct protein arrays have been described (e.g., Warren et al., Anal. Chem., 76:4082-4092 (2004) and Walter et al., Trends Mol. Med., 8:250-253 (2002), U.S. Patent No.: 6,780,582; herein incorporated by reference).

In some embodiments, one or more microfluidic chips 108 may be configured to process one or more samples 102 through use of peptide interaction. Peptides are generally described as being polypeptides that include less than one hundred amino acids. For example, peptides include dipeptides, tripeptides, and the like. In some embodiments, peptides may include from two to one hundred amino acids. In some embodiments, peptides may include from two to fifty amino acids. In some embodiments, peptides may include from two to one twenty amino acids. In some embodiments, peptides may include from ten to one hundred amino acids. In some embodiments, peptides may include from ten to fifty amino acids. Accordingly, peptides can include numerous numbers of amino acids. Numerous methods based on peptide interaction may be used. In some embodiments, peptide interaction may be used to immobilize one or more pathogen indicators 106. In some embodiments, peptide interaction may be used to separate one or more pathogen indicators 106 from one or more samples 102. Examples of such methods include, but are not limited to, those based on ligand binding, peptide-protein binding, peptide-peptide binding, peptide-polynucleotide binding, peptide cross-linking, use of green fluorescent protein, phage display, the two-hybrid system, protein arrays, peptide arrays, fiber optic evanescent wave sensors, chromatographic techniques, fluorescence resonance energy transfer, regulation of pH to control peptide and/or protein assembly and/or oligomerization, and the like. Accordingly, virtually any technique that may be used to analyze proteins may be utilized for the analysis of peptides. In some embodiments, high-speed capillary electrophoresis may be used to detect binding through use of fluorescently labeled phosphopeptides as affinity probes (Yang et al., Anal. Chem., 10.1021/ac061936e (2006)). Methods to immobilize proteins and peptides have been reported (Taylor, Protein Immobilization: Fundamentals and Applications, Marcel Dekker, Inc., New York (1991)).

In some embodiments, one or more microfluidic chips 108 may be configured to process one or more samples 102 through use of antibody interaction. Antibodies may be raised that will bind to numerous pathogen indicators 106 through use of known methods (e.g., Harlow and Lane, Antibodies: A Laboratory Manual, Cold Spring Harbor Laboratory Press, Cold Spring Harbor, New York (1988)). Antibodies may be configured in numerous ways within one or more microfluidic chips 108 to process one or more pathogen indicators 106. For example, in some embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. A labeled detector antibody that binds to the pathogen indicator 106 (or the antibody-pathogen indicator 106 complex) may then be passed over the one or more antibody-pathogen indicator 106 complexes such that the labeled detector antibody will label the pathogen indicator 106 (or the antibody-pathogen indicator 106 complex). Numerous labels may be used that include, but are not limited to, enzymes, fluorescent molecules (e.g., quantum dots), radioactive labels, spin labels, redox labels, and the like. In other embodiments, antibodies may be coupled to a substrate within a microfluidic chip 108. One or more samples 102 may be passed over the antibodies to facilitate binding of one or more pathogen indicators 106 to the one or more antibodies to form one or more antibody-pathogen indicator 106 complexes. Such binding provides for detection of the antibody-pathogen indicator 106 complex through use of methods that include, but are not limited to, surface plasmon resonance, conductivity, and the like (e.g., U.S. Patent No.: 7,030,989; herein incorporated by reference). In some embodiments, antibodies may be coupled to a substrate within a microfluidic chip 108 to provide for a competition assay. One or more samples 102 may be mixed with one or more reagent mixtures that include one or more labeled pathogen indicators 106. The mixture may then be passed over the antibodies to facilitate binding of pathogen indicators 106 in the sample 102 and labeled pathogen indicators 106 in the reagent mixture to the antibodies. The unlabeled pathogen indicators 106 in the sample 102 will compete with the labeled pathogen indicators 106 in the reagent mixture for binding to the antibodies. Accordingly, the amount of label bound to the antibodies will vary in accordance with the concentration of unlabeled pathogen indicators 106 in the sample 102. In some embodiments, antibody interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more antibodies may be used in conjunction with one or more aptamers to process one or more samples 102. Accordingly, in some embodiments, aptamers and antibodies may be used interchangeably to process one or more samples 102.

In some embodiments, one or more microfluidic chips 108 may be configured to process one or more samples 102 through use of chemical interaction. In some embodiments, one or more microfluidic chips 108 may be configured to utilize chemical extraction to process one or more samples 102. For example, in some embodiments, one or more samples 102 may be mixed with a reagent mixture that includes one or more solvents in which the one or more pathogen indicators 106 are soluble. Accordingly, the solvent phase containing the one or more pathogen indicators 106 may be separated from the sample phase to provide for detection of the one or more pathogen indicators 106. In some embodiments, one or more samples 102 may be mixed with a reagent mixture that includes one or more chemicals that cause precipitation of one or more pathogen indicators 106. Accordingly, the sample phase may be washed away from the one or more precipitated pathogen indicators 106 to provide for detection of the one or more pathogen indicators 106. Accordingly, reagent mixtures that include numerous types of chemicals that interact with one or more pathogen indicators 106 may be used.

In some embodiments, one or more microfluidic chips 108 may be configured to process one or more samples 102 through use of diffusion. In some embodiments, one or more microfluidic chips 108 may be configured to process one or more fluid samples 102 through use of an H-filter. For example, a microfluidic chip 108 may be configured to include a channel through which a fluid sample 102 and a second fluid flow such that the fluid sample 102 and the second fluid undergo substantially parallel flow through the channel without significant mixing of the sample fluid and the second fluid. As the fluid sample 102 and the second fluid flow through the channel, one or more pathogen indicators 106 in the fluid sample 102 may diffuse through the fluid sample 102 into the second fluid. Accordingly, such diffusion provides for the separation of the one or more pathogen indicators 106 from the sample 102. Methods to construct H-filters have been described (e.g., U.S. Patent Nos.: 6,742,661; 6,409,832; 6,007,775; 5,974,867; 5,971,158; 5,948,684; 5,932,100; 5,716,852; herein incorporated by reference). In some embodiments, diffusion based methods may be combined with immunoassay based methods to process and detect one or more pathogen indicators 106. Methods to conduct microscale diffusion immunoassays have been described (e.g., U.S. Patent No.: 6,541,213; herein incorporated by reference). Accordingly, microfluidic chips 108 may be configured in numerous ways to process one or more pathogen indicators 106 through use of diffusion.

In some embodiments, one or more microfluidic chips 108 may be configured to process one or more samples 102 through use of filtration. In some embodiments, one or more microfluidic chips 108 may be configured to include one or more filters that have a molecular weight cut-off. For example, a filter may allow molecules of low molecular weight to pass through the filter while disallowing molecules of high molecular weight to pass through the filter. Accordingly, one or more pathogen indicators 106 that are contained within a sample 102 may be allowed to pass through a filter while larger molecules contained within the sample 102 are disallowed from passing through the filter. Accordingly, in some embodiments, a microfluidic chip 108 may include two or more filters that selectively retain, or allow passage, of one or more pathogen indicators 106 through the filters. Such configurations provide for selective separation of one or more pathogen indicators 106 from one or more samples 102. Examples of such pathogen indicators 106 include, but are not limited to, eggs, cysts, body segments, and the like. In some embodiments, pathogen indicators 106 may be separated from fecal samples to detect infection of an animal and/or individual with one or more pathogens 104. Membranes and filters having numerous molecular weight cut-offs are commercially available (e.g., Millipore, Billerica, MA). In some embodiments, one or more microfluidic chips 108 may be configured to provide for dialysis of one or more samples 102. For example, in some embodiments, a microfluidic chip 108 may be configured to contain one or more samples 102 in one or more sample chambers that are separated from one or more dialysis chambers by a semi-permeable membrane. Accordingly, in some embodiments, one or more pathogen indicators 106 that are able to pass through the semi-permeable membrane may be collected in the dialysis chamber. In other embodiments, one or more pathogen indicators 106 may be retained in the one or more sample chambers while other sample 102 components may be separated from the one or more pathogen indicators 106 by their passage through the semi-permeable membrane into the dialysis chamber. Accordingly, one or more microfluidic chips 108 may be configured to include two or more dialysis chambers for selective separation of one or more pathogen indicators 106 from one or more samples 102. Semi-permeable membranes and dialysis tubing is available from numerous commercial sources (e.g., Millipore, Billerica, MA; Pierce, Rockford, IL; Sigma-Aldrich, St. Louis, MO). Methods that may be used for microfiltration have been described (e.g., U.S. Patent No.: 5,922,210; herein incorporated by reference).

In some embodiments, one or more microfluidic chips 108 may be configured to process one or more samples 102 through use of chromatography. Numerous chromatographic methods may be used to process one or more samples 102. Examples of such chromatographic methods include, but are not limited to, ionexchange chromatography, affinity chromatography, gel filtration chromatography, hydroxyapatite chromatography, gas chromatography, reverse phase chromatography, thin layer chromatography, capillary chromatography, size exclusion chromatography, hydrophobic interaction media, and the like. In some embodiments, a microfluidic chip 108 may be configured to process one or more samples 102 through use of one or more chromatographic methods. In some embodiments, chromatographic methods may be used to process one or more samples 102 for one or more pathogen indicators 106 that include one or more polynucleotides. For example, in some embodiments, one or more samples 102 may be applied to a chromatographic media to which the one or more polynucleotides bind. The remaining components of the sample 102 may be washed from the chromatographic media. The one or more polynucleotides may then be eluted from chromatographic media in a more purified state. Similar methods may be used to process one or more samples 102 for one or more pathogen indicators 106 that include one or more proteins or polypeptides (e.g., Mondal and Gupta, Biomol. Eng., 23:59-76 (2006)). Chromatography media able to separate numerous types of molecules is commercially available (e.g., Bio-Rad, Hercules, CA; Qiagen, Valencia, CA; Pfizer, New York, NY; Millipore, Billerica, MA; GE Healthcare BioSciences Corp., Piscataway, NJ).

In some embodiments, one or more microfluidic chips 108 may be configured to process one or more samples 102 through use of aptamer interaction. In some embodiments, one or more aptamers may include polynucleotides (e.g., deoxyribonucleic acid; ribonucleic acid; and derivatives of polynucleotides that may include polynucleotides that include modified bases, polynucleotides in which the phosphodiester bond is replaced by a different type of bond, or many other types of modified polynucleotides). In some embodiments, one or more aptamers may include peptide aptamers. Methods to prepare and use aptamers have been described (e.g., Collett et al., Methods, 37:4-15 (2005); Collet et al., Anal. Biochem., 338:113-123 (2005); Cox et al., Nucleic Acids Res., 30:20 e108 (2002); Kirby et al., Anal. Chem., 76:4066-4075 (2004); Ulrich, Handb. Exp. Pharmacol., 173:305-326 (2006); Baines and Colas, Drug Discovery Today, 11:334-341 (2006); Guthrie et al., Methods, 38:324-330 (2006); Geyer et al., Chapter 13: Selection of Genetic Agents from Random Peptide Aptamer Expression Libraries, Methods in Enzymology, Academic Press, pg. 171-208 (2000); U.S. Patent No.: 6,569,630; herein incorporated by reference). Aptamers may be configured in numerous ways within one or more microfluidic chips 108 to process one or more pathogen indicators 106. For example, in some embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Labeled detector antibodies and/or aptamers that bind to the pathogen indicator 106 (or the aptamer-pathogen indicator 106 complex) may then be passed over the one or more aptamer-pathogen indicator 106 complexes such that the labeled detector antibodies and/or aptamers will label the pathogen indicator 106 (or the aptamer-pathogen indicator 106 complex). Numerous labels may be used that include, but are not limited to, enzymes, fluorescent molecules, radioactive labels, spin labels, redox labels, and the like. In other embodiments, aptamers may be coupled to a substrate within a microfluidic chip 108. One or more samples 102 may be passed over the aptamers to facilitate binding of one or more pathogen indicators 106 to the one or more aptamers to form one or more aptamer-pathogen indicator 106 complexes. Such binding provides for detection of the aptamer-pathogen indicator 106 complex through use of methods that include, but are not limited to, surface plasmon resonance, conductivity, and the like (e.g., U.S. Patent No.: 7,030,989; herein incorporated by reference). In some embodiments, aptamers may be coupled to a substrate within a microfluidic chip 108 to provide for a competition assay. One or more samples 102 may be mixed with one or more reagent mixtures that include one or more labeled pathogen indicators 106. The mixture may then be passed over the aptamers to facilitate binding of pathogen indicators 106 in the sample 102 and labeled pathogen indicators 106 in the reagent mixture to the aptamers. The unlabeled pathogen indicators 106 in the sample 102 will compete with the labeled pathogen indicators 106 in the reagent mixture for binding to the aptamers. Accordingly, the amount of label bound to the aptamers will vary in accordance with the concentration of unlabeled pathogen indicators 106 in the sample 102. In some embodiments, aptamer interaction may be used in association with microcantilevers to process one or more pathogen indicators 106. Methods to construct microcantilevers are known (e.g., U.S. Patent Nos.: 7,141,385; 6,935,165; 6,926,864; 6,763,705; 6,523,392; 6,325,904; herein incorporated by reference). In some embodiments, one or more aptamers may be used in conjunction with one or more antibodies to process one or more samples 102. In some embodiments, aptamers and antibodies may be used interchangeably to process one or more samples 102. Accordingly, in some embodiments, methods and/or systems for processing and/or detecting pathogen indicators 106 may utilize antibodies and aptamers interchangeably and/or in combination.

In some embodiments, one or more microfluidic chips 108 may be configured to process one or more samples 102 through use of electrical conductivity. In some embodiments, one or more samples 102 may be processed through use of magnetism. For example, in some embodiments, one or more samples 102 may be combined with one or more tagged polynucleotides that are tagged with a ferrous material, such as a ferrous bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed over an electromagnet to immobilize the hybridized complexes. Other components in the sample 102 may then be washed away from the hybridized complexes. In some embodiments, a chamber containing the magnetically immobilized hybridized complexes may be heated and/or chemically treated to release the sample polynucleotides from the magnetically immobilized tagged polynucleotides. The sample polynucleotides may then be collected in a more purified state. In other embodiments, similar methods may be used in conjunction with antibodies, aptamers, peptides, ligands, and the like. Accordingly, one or more microfluidic chips 108 may be configured in numerous ways to utilize magnetism to process one or more samples 102. In some embodiments, one or more samples 102 may be processed through use of eddy currents. Eddy current separation uses the principles of electromagnetic induction in conducting materials to separate non-ferrous metals by their different electric conductivities. An electrical charge is induced into a conductor by changes in magnetic flux cutting through it. Moving permanent magnets passing a conductor generates the change in magnetic flux. Accordingly, in some embodiments, one or more microfluidic chips 108 may be configured to include a magnetic rotor such that when conducting particles move through the changing flux of the magnetic rotor, a spiraling current and resulting magnetic field are induced. The magnetic field of the conducting particles may interact with the magnetic field of the magnetic rotor to impart kinetic energy to the conducting particles. The kinetic energy imparted to the conducting particles may then be used to direct movement of the conducting particles. Accordingly, non-ferrous particles, such as metallic beads, may be utilized to process one or more samples 102. For example, in some embodiments, one or more samples 102 may be combined with one or more tagged polynucleotides that are tagged with a non-ferrous material, such as an aluminum bead. The tagged polynucleotides and the polynucleotides in the one or more samples 102 may be incubated to provide hybridized complexes of the tagged polynucleotides and the sample polynucleotides. Hybridization will serve to couple one or more ferrous beads to the polynucleotides in the sample 102 that hybridize with the tagged polynucleotides. Accordingly, the mixture may be passed through a magnetic field to impart kinetic energy to the non-ferrous bead. This kinetic energy may then be used to separate the hybridized complex. In other embodiments, similar methods may be used in conjunction with antibodies, aptamers, peptides, ligands, and the like. Accordingly, one or more microfluidic chips 108 may be configured in numerous ways to utilize eddy currents to process one or more samples 102. One or more microfluidic chips 108 may be configured in numerous ways to utilize electrical conductivity to process one or more samples 102.

In some embodiments, one or more microfluidic chips 108 may be configured to process one or more samples 102 through use of isoelectric focusing. Methods have been described that may be used to construct capillary isoelectric focusing systems (e.g., Herr et al., Investigation of a miniaturized capillary isoelectric focusing (clEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). Such systems may be modified to provide for the processing of one or more samples 102.

In some embodiments, one or more microfluidic chips 108 may be configured to process one or more samples 102 through use of electrophoresis. In some embodiments, one or more microfluidic chips 108 may be configured to process one or more samples 102 through use of one-dimensional electrophoresis. In some embodiments, one or more microfluidic chips 108 may be configured to process one or more samples 102 through use of two-dimensional electrophoresis. In some embodiments, one or more microfluidic chips 108 may be configured to process one or more samples 102 through use of gradient gel electrophoresis. In some embodiments, one or more microfluidic chips 108 may be configured to process one or more samples 102 through use of electrophoresis under denaturing conditions. In some embodiments, one or more microfluidic chips 108 may be configured to process one or more samples 102 through use of electrophoresis under native conditions. One or more microfluidic chips 108 may be configured to utilize numerous electrophoretic methods.

In some embodiments, one or more microfluidic chips 108 may be configured to process one or more samples 102 through use of immunoassay. In some embodiments, one or more microfluidic chips 108 may be configured to process one or more samples 102 through use of enzyme linked immunosorbant assay (ELISA). In some embodiments, one or more microfluidic chips 108 may be configured to process one or more samples 102 through use of radioimmuno assay (RIA). In some embodiments, one or more microfluidic chips 108 may be configured to process one or more samples 102 through use of enzyme immunoassay (EIA). In some embodiments, such methods may utilize antibodies (e.g., monoclonal antibodies, polyclonal antibodies, antibody fragments, single-chain antibodies, and the like), aptamers, or substantially any combination thereof. In some embodiments, a labeled antibody and/or aptamer may be used within an immunoassay. In some embodiments, a labeled ligand to which the antibody and/or aptamer binds may be used within an immunoassay. Numerous types of labels may be utilized. Examples of such labels include, but are not limited to, radioactive labels, fluorescent labels, enzyme labels, spin labels, magnetic labels, gold labels, colorimetric labels, redox labels, and the like. Numerous immunoassays are known and may be configured for processing one or more samples 102.

In some embodiments, one or more microfluidic chips 108 may be configured to process one or more samples 102 through use of one or more competition assays. In some embodiments, one or more microfluidic chips 108 may be configured to process one or more samples 102 through use of one or more polynucleotide based competition assays. One or more microfluidic chips 108 may be configured to include one or more polynucleotides coupled to a substrate, such as a polynucleotide array. The one or more microfluidic chips 108 may be further configured so that a sample 102 and/or substantially purified polynucleotides obtained from one or more samples 102, may be mixed with one or more reagent mixtures that include one or more labeled polynucleotides to form an analysis mixture. This analysis mixture is then passed over the substrate such that the labeled polynucleotides and the sample polynucleotides are allowed to hybridize to the polynucleotides that are immobilized on the substrate. The sample polynucleotides and the labeled polynucleotides will compete for binding to the polynucleotides that are coupled on the substrate. Accordingly, the presence and/or concentration of the polynucleotides in the sample 102 can be determined through detection of the label (e.g., the concentration of the polynucleotides in the sample 102 will be inversely related to the amount of label that is bound to the substrate). Numerous labels may be used that include, but are not limited to, enzymes, fluorescent molecules, radioactive labels, spin labels, redox labels, and the like. In some embodiments, one or more microfluidic chips 108 may be configured to include one or more antibodies, proteins, peptides, and/or aptamers that are coupled to a substrate. The one or more microfluidic chips 108 may be further configured so that a sample 102 and/or substantially purified sample polynucleotides and/or sample peptides obtained from one or more samples 102, may be mixed with one or more reagent mixtures that include one or more labeled polypeptides and/or labeled peptides to form an analysis mixture. This analysis mixture can then be passed over the substrate such that the labeled polypeptides and/or labeled peptides and the sample polynucleotides and/or sample peptides are allowed to bind to the antibodies, proteins, peptides, and/or aptamers that are immobilized on the substrate. The sample polypeptides and/or sample peptides and the labeled polypeptides and/or sample peptides will compete for binding to the antibodies, proteins, peptides, and/or aptamers that are coupled on the substrate. Accordingly, the presence and/or concentration of the sample polypeptides and/or sample peptides in the sample 102 can be determined through detection of the label (e.g., the concentration of the sample polypeptides and/or sample peptides in the sample 102 will be inversely related to the amount of label that is bound to the substrate). Numerous labels may be used that include, but are not limited to, enzymes, fluorescent molecules, radioactive labels, spin labels, redox labels, and the like. Microfluidic chips 108 may be configured to utilize numerous types of competition assays.

In some embodiments, one or more microfluidic chips 108 may be configured to utilize numerous processing methods. For example, in some embodiments, one or more pathogen indicators 106 may be precipitated with salt, dialyzed, and then applied to a chromatographic column.

FIG. 85 illustrates alternative embodiments of microfluidic chip 8200 of FIG. 82. FIG. 85 illustrates example embodiments of module 8230. Additional embodiments may include an embodiment 8502.

At embodiment 8502, module 8230 may include one or more analysis units configured for analysis of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, a microfluidic chip 108 may include one or more analysis units 102 configured for analysis of the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, the one or more analysis units 120 may be configured to facilitate detection of one or more pathogen indicators 106 with one or more detection units 122. For example, in some embodiments, one or more analysis units 120 may include a window (e.g., a quartz window, a cuvette analog, and/or the like) through which one or more detection units 122 may determine if one or more pathogen indicators 106 are present and/or determine the concentration of one or more pathogen indicators 106. In such embodiments, one or more analysis units 120 may be configured to provide for numerous techniques that may be used to detect the one or more pathogen indicators 106, such as visible light spectroscopy, ultraviolet light spectroscopy, infrared spectroscopy, fluorescence spectroscopy, and the like.

In some embodiments, one or more analysis units 120 may be configured to analyze one or more samples 102 through use of surface plasmon resonance. In some embodiments, the one or more analysis units 120 may include one or more antibodies, aptamers, proteins, peptides, polynucleotides, and the like, that are bound to a substrate (e.g., a metal film) within the one or more analysis units 120. In some embodiments, such analysis units 120 may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more analysis units 120 may include an exposed substrate surface that is configured to operably associate with one or more prisms that are included within one or more detection units 122. In some embodiments, one or more analysis units 120 may include a nuclear magnetic resonance (NMR) probe. In such embodiments, the analysis units 120 may be configured to associate with one or more detection units 122 that accept the NMR probe and are configured to detect one or more pathogen indicators 106 through use of NMR spectroscopy. Accordingly, analysis units 120 and detection units 122 may be configured in numerous ways to associate with each other to provide for detection of one or more pathogen indicators 106.

In some embodiments, one or more pathogen indicators 106 may be analyzed through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be analyzed through use of electrochemical detection. In some embodiments, one or more polynucleotides may be analyzed through use of electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). Such methods may be used to analyze numerous polynucleotides, such as messenger ribonucleic acid, genomic deoxyribonucleic acid, fragments thereof, and the like.

In some embodiments, one or more pathogen indicators 106 may be analyzed through use of polynucleotide analysis. In some embodiments, one or more analysis units 120 may be configured to analyze one or more pathogen indicators 106 through use of polynucleotide analysis. Numerous methods may be used to analyze one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for analysis of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). Numerous other methods based on polynucleotide analysis may be used to analyze one or more pathogen indicators 106.

In some embodiments, one or more pathogen indicators 106 may be analyzed through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described.

In some embodiments, one or more pathogen indicators 106 may be analyzed through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to analyze one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to facilitate detection and/or the determination of the concentration of one or more pathogen indicators 106 in one or more samples 102. Numerous combinations of fluorescent donors and fluorescent acceptors may be used to analyze one or more pathogen indicators 106. Accordingly, one or more analysis units 120 may be configured to operably associate with one or more detection units 122 that emit one or more wavelength of light to excite a fluorescent donor and detect one or more wavelengths of light emitted by the fluorescent acceptor. Accordingly, in some embodiments, one or more analysis units 120 may be configured to include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to analyze and/or detect one or more pathogen indicators 106.

In some embodiments, one or more pathogen indicators 106 may be analyzed through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to analyze one or more pathogen indicators 106. For example, in some embodiments, one or more analysis units 120 may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more analysis units 120 may be configured to utilize numerous electron transfer based assays to provide for detection of one or more pathogen indicators 106 by a detection unit 122.

In some embodiments, one or more pathogen indicators 106 may be analyzed through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more analysis units 120 may be configured to facilitate detection of fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more analysis units 120 may be configured to include a substrate to which is coupled one or more antibodies, aptamers, peptides, proteins, polynucleotides, ligands, and the like, that will interact with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more analysis units 120 may be configured to provide for detection of one or more products of enzyme catalysis to provide for detection of one or more pathogen indicators 106.

In some embodiments, one or more pathogen indicators 106 may be analyzed through use of electrical conductivity. In some embodiments, one or more analysis units 120 may be configured to provide for detection of one or more pathogen indicators 106 through use of electrical conductivity. In some embodiments, such analysis units 120 may be configured to operably associate with one or more detection units 122 such that the one or more detection units 122 can detect one or more pathogen indicators 106 through use of electrical conductivity. In some embodiments, one or more analysis units 120 may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a spore, a bacterium, a virus, an egg, a worm, a cyst, a microbe, a prion, a protozoan, a single-celled organism, a fungus, an algae, a protein, a microbe, and the like, will complete an electrical circuit and facilitate the production of a detectable electrical current. Accordingly, in some embodiments, one or more analysis units 120 may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more analysis units 120 with the one or more detection units 122. In some embodiments, the one or more detection units 122 are configured for detachable connection to one or more analysis units 120. Analysis units 120 and detection units 122 may be configured in numerous ways to facilitate detection of one or more pathogen indicators 106.

In some embodiments, one or more pathogen indicators 106 may be analyzed through use of isoelectric focusing. In some embodiments, one or more analysis units 120 may be configured to provide for detection of one or more pathogen indicators 106 through use of isoelectric focusing. In some embodiments, native isoelectric focusing may be utilized to analyze one or more pathogen indicators 106. In some embodiments, denaturing isoelectric focusing may be utilized to analyze one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more analysis units 120 may be configured to operably associate with one or more detection units 122 that can be used to detect one or more pathogen indicators 106. In some embodiments, one or more detection units 122 may be configured to include one or more CCD cameras that can be used to detect one or more pathogen indicators 106 that are analyzed through isoelectric focusing. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106.

In some embodiments, one or more analysis units 120 may be configured to combine one or more samples 102 and/or portions of one or more samples 102 with one or more reagent mixtures that include one or more pathogen indicator binding agents that bind to one or more pathogen indicators 106 that may be present with the one or more samples 102 to form a pathogen indicator-pathogen indicator binding agent complex. Examples of such pathogen indicator binding agents that bind to one or more pathogen indicators 106 include, but are not limited to, antibodies, aptamers, peptides, proteins, polynucleotides, and the like. In some embodiments, a pathogen indicator- pathogen indicator binding agent complex may be analyzed through use of isoelectric focusing and then detected with one or more detection units 122. In some embodiments, one or more pathogen indicator binding agents may include a label. Numerous labels may be used and include, but are not limited to, radioactive labels, fluorescent labels, colorimetric labels, spin labels, and the like. Accordingly, in some embodiments, a pathogen indicator- pathogen indicator binding agent complex (labeled) may be analyzed through use of isoelectric focusing and then detected with one or more detection units 122 that are configured to detect the one or more labels. Analysis units 120 and detection units 122 may be configured in numerous ways to analyze one or more samples 102 and detect one or more pathogen indicators 106 through use of pathogen indicator binding agents.

In some embodiments, one or more pathogen indicators 106 may be analyzed through use of chromatographic methodology alone or in combination with additional analysis and/or detection methods. In some embodiments, one or more analysis units 120 may be configured to analyze one or more samples 102 and provide for detection of one or more pathogen indicators 106 through use of chromatographic methods. Accordingly, in some embodiments, one or more detection units 122 may be configured to operably associate with the one or more analysis units 120 and detect one or more pathogen indicators 106 that were analyzed through use of chromatographic methods. In some embodiments, the one or more detection units 122 may be configured to operably associate with one or more analysis units and supply solvents and other reagents to the one or more analysis units 120. For example, in some embodiments, one or more detection units 122 may include pumps and solvent/buffer reservoirs that are configured to supply solvent/buffer flow through chromatographic media (e.g., a chromatographic column) that is operably associated with analysis units 120. In some embodiments, one or more detection units 122 may be configured to operably associate with one or more analysis units 120 and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more analysis units 120 may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more analysis units 120 may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to process and detect one or more pathogen indicators 106. Numerous detection methods may be used in combination with numerous types of chromatographic methods. Accordingly, one or more detection units 122 may be configured to utilize numerous detection methods to detect one or more pathogen indicators 106 that are analyzed through use of one or more chromatographic methods. Examples of such detection methods include, but are not limited to, conductivity detection, use of ion-specific electrodes, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column. Accordingly, chromatographic methods may be used in combination with additional methods and in combination with numerous types of detection methods.

In some embodiments, one or more pathogen indicators 106 may be analyzed through use of immunoprecipitation. In some embodiments, one or more analysis units 120 may be configured to provide for detection of one or more pathogen indicators 106 through use of immunoprecipitation. In some embodiments, immunoprecipitation may be utilized in combination with additional analysis and/or detection methods to analyze and/or detect one or more pathogen indicators 106. In some embodiments, one or more analysis units 120 may be configured to analyze one or more samples 102 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more analysis units 120 that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more pathogen indicators 106 may be analyzed through use of immunoseparation. In some embodiments, one or more analysis units 120 may be configured to analyze one or more pathogen indicators 106 through use of immunoseparation. In some embodiments, immunoseparation may be utilized in combination with additional analysis and/or detection methods to detect one or more pathogen indicators 106. In some embodiments, one or more analysis units 120 may be configured to analyze one or more samples 102 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more analysis units 120 may be configured to analyze one or more pathogen indicators 106 through use of numerous analysis methods in combination with immunoseparation based methods. In some embodiments, aptamers (polypeptide and/or polynucleotide) may be used in combination with antibodies or in place of antibodies.

In some embodiments, one or more pathogen indicators 106 may be analyzed through use of aptamer binding. In some embodiments, one or more analysis units 120 may be configured to analyze one or more pathogen indicators 106 through use of aptamer binding. In some embodiments, aptamer binding may be utilized in combination with additional analysis and/or detection methods to detect one or more pathogen indicators 106. For example, in some embodiments, one or more samples 102 may be combined with one or more aptamers that bind to one or more pathogen indicators 106 to form one or more aptamer-pathogen indicator 106 complexes. Such complexes may be detected through use of numerous methods that include, but are not limited to, fluorescence resonance energy transfer, fluorescence quenching, surface plasmon resonance, and the like. In some embodiments, aptamer binding constituents may be added that bind to the aptamer-pathogen complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to analyze and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to analyze one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 in combination with numerous analysis methods. In some embodiments, antibodies may be used in combination with aptamers and/or in place of aptamers.

In some embodiments, one or more pathogen indicators 106 may be analyzed through use of electrophoresis. In some embodiments, one or more analysis units 120 may be configured to analyze one or more samples 102 through use of electrophoresis. In some embodiments, such analysis units 120 may be configured to operably associate with one or more detection units 122. Accordingly, in some embodiments, one or more detection units 122 may be configured to operably associate with one or more analysis units 120 and detect one or more pathogen indicators 106 that were analyzed through use of electrophoresis. Numerous electrophoretic methods may be utilized to analyze and detect one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In some embodiments, refraction, absorbance, and/or fluorescence may be used to determine the position of sample components within a gel. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Analysis units 120 and detection units 122 may be configured in numerous ways to analyze and detect one or more pathogen indicators **106** through use of electrophoresis.

In some embodiments, one or more pathogen indicators 106 may be analyzed through use of one or more charge-coupled device (CCD) cameras. In some embodiments, one or more detection units 122 that include one or more CCD cameras may be configured to operably associate with one or more analysis units 120. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more analysis units 120 may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more pathogen indicators 106 may be analyzed through use of immunoassay. In some embodiments, one or more analysis units 120 may be configured to analyze one or more samples 102 through use of immunoassay. In some embodiments, one or more detection units 122 may be configured to operably associate with one or more such analysis units 120 to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 86 illustrates alternative embodiments of microfluidic chip 8200 of FIG. 82. FIG. 86 illustrates example embodiments of module 8240. Additional embodiments may include an embodiment 8602, an embodiment 8604, an embodiment 8606, and/or an embodiment 8608.

At embodiment 8602, module 8240 may include one or more detection chambers configured to facilitate detection of the one or more pathogen indicators that are associated with one or more airborne pathogens. In some embodiments, a microfluidic chip 108 may include one or more detection chambers configured to facilitate detection of the one or more pathogen indicators 106 that are associated with one or more pathogens 104 that are airborne. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more microfluidic chips 108 may be configured to analyze and/or detect severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 8604, module 8240 may include one or more detection chambers configured to facilitate detection of the one or more pathogen indicators that are associated with one or more food products. In some embodiments, a microfluidic chip 108 may include one or more detection chambers configured to facilitate detection of the one or more pathogen indicators 106 that are associated with one or more food products. In some embodiments, one or more detection chambers may be configured to facilitate detection of one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

At embodiment 8606, module 8240 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, a microfluidic chip 108 may include one or more detection chambers configured to facilitate detection of the one or more pathogens 104 that include at least one virus, bacterium, prion, worm, egg, cyst, protozoan, single-celled organism, fungus, algae, pathogenic protein, microbe, or substantially any combination thereof. A detection chamber may be configured to utilize numerous types of techniques, and combinations of techniques, to facilitate detection of one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007))..

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At embodiment 8608, module 8240 may include one or more detection chambers configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, a microfluidic chip 108 may include one or more detection chambers configured to facilitate detection of the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection chambers may be configured to facilitate detection of one or more pathogen indicators 106 that have been processed by one or more microfluidic chips 108 and/or analyzed by one or more analysis units 120. For example, in some embodiments, one or more detection chambers may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection units 122 may include circuitry and/or electro-mechanical mechanisms to detect one or more pathogen indicators 106 present within one or more microfluidic chips 108 through a window in the one or more microfluidic chips 108.

In some embodiments, one or more detection chambers may be configured to facilitate detection of one or more pathogen indicators 106 through use of surface plasmon resonance. In some embodiments, one or more detection chambers may be configured to include one or more antibodies, aptamers, proteins, peptides, polynucleotides, and the like, that are bound to a substrate (e.g., a metal film) within the one or more detection chambers. In some embodiments, such detection chambers may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more detection chambers to facilitate detection of one or more pathogen indicators 106 through use of surface plasmon resonance.

In some embodiments, one or more detection chambers may be configured to facilitate detection of one or more pathogen indicators 106 through use of nuclear magnetic resonance (NMR). In some embodiments, one or more detection units 122 may be configured to operably associate with one or more detection chambers that include a nuclear magnetic resonance (NMR) probe. Accordingly, in some embodiments, one or more pathogen indicators 106 may be analyzed and detected through use of one or more detection chambers and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, one or more pathogen indicators 106 may be detected through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, one or more pathogen indicators 106 may be detected through use of polynucleotide detection. In some embodiments, one or more detection chambers may be configured to facilitate detection of one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more detection chambers may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, one or more pathogen indicators 106 may be detected through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, one or more detection chambers may be configured to facilitate detection of one or more pathogen indicators 106 and configured to facilitate fluorescent detection of the one or more pathogen indicators 106 with one or more detection units 122.

In some embodiments, one or more pathogen indicators 106 may be detected through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to operably associate with one or more detection chambers that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, one or more pathogen indicators 106 may be detected through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more detection chambers may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. One or more detection chambers may be configured to utilize numerous electron transfer based assays to facilitate detection of one or more pathogen indicators 106 by a detection unit 122 that is configured to operably associate with the one or more detection chambers.

In some embodiments, one or more pathogen indicators 106 may be detected through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection units 122 may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more detection chambers may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection chambers may be configured to facilitate detection of one or more products of enzyme catalysis to provide for detection of one or more pathogen indicators 106.

In some embodiments, one or more pathogen indicators 106 may be detected through use of electrical conductivity. In some embodiments, one or more detection chambers may be configured to provide for detection of one or more pathogen indicators 106 through use of electrical conductivity. In some embodiments, such detection chambers may be configured to operably associate with one or more detection units 122 such that the one or more detection units 122 can detect one or more pathogen indicators 106 through use of electrical conductivity. In some embodiments, one or more detection chambers may be configured to include two or more electrodes that are each coupled to one or more detector polynucleotides. Interaction of a pathogen 104 associated polynucleotide, such as hybridization, with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleic acid associated with one or more samples 102 may be combined with one or more sets of paired primers such that use of an amplification protocol, such as a polymerase chain reaction, will produce an amplification product corresponding to pathogen associated polynucleic acid that was contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such asa cyst, egg, pathogen 104, spore, and the like, will complete an electrical circuit and facilitate the production of a detectable electrical current. Accordingly, in some embodiments, one or more detection chambers may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more detection chambers with the one or more detection units 122. In some embodiments, the one or more detectors may be configured for detachable connection to one or more detection chambers. Detection chambers and detection units 122 may be configured in numerous ways to facilitate detection of one or more pathogen indicators 106.

In some embodiments, one or more pathogen indicators 106 may be detected through use of isoelectric focusing. In some embodiments, one or more detection chambers may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more detection chambers such that the one or more detection units 122 can be used to detect one or more pathogen indicators 106 that have been focused within one or more microfluidic channels of the one or more detection chambers. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more detection chambers may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-bindi.ng agent complex may be subjected to isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Detection chambers and detection units 122 may be configured in numerous ways to facilitate detection of one or more pathogen indicators 106 through use of isoelectric focusing.

In some embodiments, one or more pathogen indicators 106 may be detected through use of chromatographic methodology alone or in combination with additional detection methods. In some embodiments, one or more detection chambers may be configured to provide for detection of one or more pathogen indicators 106 through use of chromatographic methods. Accordingly, in some embodiments, one or more detection units 122 may be configured to operably associate with the one or more detection chambers and detect one or more pathogen indicators 106. In some embodiments, the one or more detection units 122 may be configured to operably associate with one or more detection chambers and supply solvents and other reagents to the one or more detection chambers. For example, in some embodiments, one or more detection units 122 may include pumps and solvent/buffer reservoirs that are configured to supply solvent/buffer flow through chromatographic media (e.g., a chromatographic column) that is operably associated with one or more detection chambers. In some embodiments, one or more detection units 122 may be configured to operably associate with one or more detection chambers and be configured to utilize one or more methods to detect one or more pathogen indicators 106. Numerous types of chromatographic methods and media may be used to process one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more detection chambers may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more detection chambers may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. One or more detection units 122 that are operably associated with the chromatographic column may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection chambers may be configured to facilitate detection of one or more pathogen indicators 106 through use of immunoprecipitation. In some embodiments, immunoprecipitation may be utilized in combination with additional detection methods to detect one or more pathogen indicators 106. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample 102 components may be washed away from the precipitated antibody-pathogen indicator 106 complexes. In some embodiments, one or more detection chambers that are configured for immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection chambers may be configured to facilitate detection of one or more pathogen indicators 106 through use of numerous detection methods in combination with immunoprecipitation based methods.

In some embodiments, one or more detection chambers may be configured to facilitate detection of one or more pathogen indicators 106 through use of immunoseparation. In some embodiments, immunoseparation may be utilized in combination with additional detection methods to detect one or more pathogen indicators 106. In some embodiments, one or detection chambers may be configured to facilitate detection of one or more pathogen indicators 106 through use of immunoseparation. For example, in some embodiments, one or more samples 102 may be combined with one or more antibodies that bind to one or more pathogen indicators 106 to form one or more antibody-pathogen indicator 106 complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator 106 complex such that the antibody binding constituent binds to the antibody-pathogen indicator 106 complex and provides for separation of the antibody-pathogen indicator 106 complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator 106 complexes such that the first antibody binding constituent and the second antibody binding constituent bind to antibody-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator 106 complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection chambers may be configured to facilitate detection of one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection chambers may be configured to facilitate detection of one or more pathogen indicators 106 through use of aptamer binding. In some embodiments, aptamer binding may be utilized in combination with additional methods to detect one or more pathogen indicators 106. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection chambers may be configured to facilitate detection of one or more pathogen indicators 106 through use of numerous detection methods in combination with aptamer binding based methods. In some embodiments, antibodies may be used in combination with aptamers or in place of aptamers.

In some embodiments, one or more detection chambers may be configured to facilitate detection of one or more pathogen indicators 106 through use of electrophoresis. In some embodiments, such detection chambers may be configured to operably associate with one or more detection units 122. Accordingly, in some embodiments, one or more detection units 122 may be configured to operably associate with one or more detection chambers and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Detection chambers and detection units 122 may be configured in numerous ways to provide for detection of one or more pathogen indicators 106 through use of electrophoresis.

In some embodiments, one or more detection units 122 that include one or more CCD cameras may be configured to operably associate with one or more detection chambers. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more detection chambers may be configured to facilitate detection of one or more pathogen indicators 106 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. Detection chambers and detection units 122 may be configured in numerous ways to utilize one or more CCD cameras to detect one or more pathogen indicators 106.

In some embodiments, one or more pathogen indicators 106 may be detected through use of immunoassay. In some embodiments, one or more detection chambers may be configured to facilitate detection of one or more pathogen indicators 106 through use of immunoassay. In some embodiments, one or more detection units 122 may be configured to operably associate with one or more such detection chambers and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 87 illustrates a microfluidic chip 8700 representing examples of modules that may be used to perform a method for analysis of one or more pathogens 104. In FIG. 87, discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The microfluidic chip 8700 includes module 8710 that includes one or more separation channels that are configured to allow one or more samples that include one or more magnetically active pathogen indicator complexes to flow in a substantially parallel manner with one or more separation fluids. In some embodiments, module 8710 may include one or more channels that are configured to allow the one or more samples and the one or more separation fluids to flow in a substantially horizontal position. In some embodiments, module 8710 may include one or more channels that are configured to allow the one or more samples and the one or more separation fluids to flow in a substantially vertical position.

The microfluidic chip 8700 includes module 8720 that includes one or more magnetic fields that facilitate movement of the one or more magnetically active pathogen indicator complexes associated with the one or more samples into the one or more separation fluids. In some embodiments, module 8720 may include one or more electromagnets. In some embodiments, module 8720 may include one or more ferromagnets. In some embodiments, module 8720 may include one or more ferrofluids.

The microfluidic chip 8700 may optionally include module 8730 that includes one or more mixing chambers that are configured to allow one or more magnetically active pathogen indicator binding agents to bind to one or more pathogen indicators associated with the one or more samples to form one or more magnetically active pathogen indicator complexes. In some embodiments, module 8730 may include one or more mixing members. In some embodiments, module 8730 may include one or more sonicators.

The microfluidic chip 8700 optionally includes module 8740 that includes one or more detection chambers configured to facilitate detection of the one or more pathogen indicators associated with the one or more samples. In some embodiments, module 8740 may include one or more detection chambers configured to facilitate detection of the one or more pathogen indicators that are associated with one or more airborne pathogens. In some embodiments, module 8740 may include one or more detection chambers configured to facilitate detection of the one or more pathogen indicators that are associated with one or more waterborne pathogens. In some embodiments, module 8740 may include one or more detection chambers configured to facilitate detection of the one or more pathogen indicators that are associated with one or more soilborne pathogens. In some embodiments, module 8740 may include one or more detection chambers configured to facilitate detection of the one or more pathogen indicators that are associated with one or more food products. In some embodiments, module 8740 may include one or more detection chambers configured to facilitate detection of the one or more pathogen indicators that are associated with one or more biological samples. In some embodiments, module 8740 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, module 8740 may include one or more detection chambers that are configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

FIG. 88 illustrates alternative embodiments of microfluidic chip 8700 of FIG. 87. FIG. 88 illustrates example embodiments of module 8710. Additional embodiments may include an embodiment 8802 and/or an embodiment 8804.

At embodiment 8802, module 8710 may include one or more channels that are configured to allow the one or more samples and the one or more separation fluids to flow in a substantially horizontal position. In some embodiments, one or more microfluidic chips 108 may include one or more channels that are configured to allow the one or more samples 102 and the one or more separation fluids to flow in a substantially horizontal position. For example, in some embodiments, the one or more samples 102 and the one or more separation fluids may be configured to flow in a substantially side-by-side manner in a substantially horizontal position. In some embodiments, one or more samples 102 and one or more separation fluids may be selected that are immiscible. In such embodiments, mixing of the one or more samples 102 and the one or more separation fluids may be substantially reduced.

At embodiment 8804, module 8710 may include one or more channels that are configured to allow the one or more samples and the one or more separation fluids to flow in a substantially vertical position. In some embodiments, one or more microfluidic chips 108 may include one or more channels that are configured to allow the one or more samples 102 and the one or more separation fluids to flow in a substantially vertical position. In some embodiments, the one or more samples 102 and the one or more separation fluids may be configured to flow with the one or more samples 102 flowing in a position that is above the flow of the one or more separation fluids. In some embodiments, the one or more samples 102 and the one or more separation fluids may be configured to flow with the one or more samples 102 flowing in a position that is below the flow of the one or more separation fluids. In some embodiments, the positional flow of one or more samples 102, and/or the positional flow of one or more separation fluids may be controlled through modulation of viscosity, density, immiscibility, or substantially any combination thereof. For example, in some embodiments, one or more separation fluids having greater density than the one or more samples 102 may be used to position the one or more separation fluids below the one or more samples 102. In some embodiments, one or more separation fluids that are less dense than the one or more samples 102 may be used to position the one or more separation fluids above the one or more samples 102. In some embodiments, one or more samples 102 and one or more separation fluids may be selected that are immiscible. In such embodiments, mixing of the one or more samples 102 and the one or more separation fluids may be substantially reduced.

FIG. 89 illustrates alternative embodiments of microfluidic chip 8700 of FIG. 87. FIG. 89 illustrates example embodiments of module 8720. Additional embodiments may include an embodiment 8902, an embodiment 8904, and/or an embodiment 8906.

At embodiment 8902, module 8720 may include one or more electromagnets. In some embodiments, a microfluidic chip 108 may include one or more electromagnets. In some embodiments, one or more electromagnets may be used to move one or more magnetic plugs relative to one or more microfluidic chips 108. For example, in some embodiments, a magnetic plug may be used to propel fluid through one or more channels of a microfluidic chip 108 through use of magnetic attraction and/or magnetic repulsion. Accordingly, in some embodiments, electromagnets may be used to selectively create magnetic fields which may be used to selectively move a magnetic plug through one or more channels of a microfluidic chip 108. In some embodiments, one or more electromagnets may be used to separate one or more pathogen indicators 106 from one or more samples 102. In some embodiments, one or more electromagnets may be used to operably associate one or more microfluidic chips 108 to one or more detection units 122, one or more reagent delivery units 116, one or more centrifugation units 118, and the like, in substantially any combination.

At embodiment 8904, module 8720 may include one or more ferromagnets. In some embodiments, a microfluidic chip 108 may include one or more ferromagnets. In some embodiments, one or more ferromagnets may be used to move one or more magnetic plugs relative to one or more microfluidic chips. For example, in some embodiments, a magnetic plug may be used to propel fluid through one or more channels of a microfluidic chip 108 through use of magnetic attraction and/or magnetic repulsion. In some embodiments, one or more ferromagnets may be attached to one or more guides (e.g., rails, channels, cords, and the like) such that the ferromagnet may be selectively positioned relative to one or more microfluidic chips 108. Accordingly, in some embodiments, ferromagnets may be used to selectively create magnetic fields which may be used to selectively move a magnetic plug through one or more channels of a microfluidic chip 108. In some embodiments, one or more ferromagnets may be used to separate one or more pathogen indicators 106 from one or more samples 102. In some embodiments, ferromagnets may be used to create eddy currents. In some embodiments, one or more ferromagnets may be used to operably associate one or more microfluidic chips 108 to one or more detection units 122, one or more reagent delivery units 116, one or more centrifugation units 118, and the like, in substantially any combination.

At embodiment 8906, module 8720 may include one or more ferrofluids. In some embodiments, a microfluidic chip 108 may include one or more ferrofluids. In some embodiments, ferrofluids may be configured to facilitate one or more pathogen indicators 106 from one or more samples 102. In some embodiments, a ferrofluid may be used to selectively position a magnetic plug relative to one or more microfluidic chips 108. For example, in some embodiments, a ferrofluid may be used to selectively position one or more magnetic plugs to facilitate movement of one or more fluids through one or more channels of a microfluidic chip 108.

FIG. 90 illustrates alternative embodiments of microfluidic chip 8700 of FIG. 87. FIG. 90 illustrates example embodiments of module 8730. Additional embodiments may include an embodiment 9002 and/or an embodiment 9004.

At embodiment 9002, module 8730 may include one or more mixing members. In some embodiments, a microfluidic chip 108 may include one or more mixing members. Mixing members may be positioned in numerous chambers of a microfluidic chip 108. Examples of such chambers include, but are not limited to, reaction chambers, mixing chambers, detection chambers, reservoirs, and the like, in substantially any combination. In some embodiments, one or more mixing members may be magnetically active such that the mixing members may be moved through use of one or more magnetic fields. In some embodiments, one or more mixing members may be physically coupled to a drive such that the drive causes movement of the mixing member.

At embodiment 9004, module 8730 may include one or more sonicators. In some embodiments, a microfluidic chip 108 may include one or more sonicators. In some embodiments, a microfluidic chip 108 may include one or more sonication probes. Such probes may be configured such that are able to operably associate with one or more vibration sources in a detachable manner. Accordingly, in some embodiments, one or more microfluidic chips 108 that include one or more probes may be configured to detachably connect with one or more vibration sources that produce a vibration that can be coupled to the one or more probes.

FIG. 91 illustrates alternative embodiments of microfluidic chip 8700 of FIG. 87. FIG. 91 illustrates example embodiments of module 8740. Additional embodiments may include an embodiment 9102, an embodiment 9104, an embodiment 9106, and/or an embodiment 9108.

At embodiment 9102, module 8740 may include one or more detection chambers configured to facilitate detection of the one or more pathogen indicators that are associated with one or more airborne pathogens. In some embodiments, a microfluidic chip 108 may include one or more detection chambers configured to facilitate detection of the one or more pathogen indicators 106 that are associated with one or more airborne pathogens 104. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more detection chambers may be configured to facilitate detection of severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 9104, module 8740 may include one or more detection chambers configured to facilitate detection of the one or more pathogen indicators that are associated with one or more waterborne pathogens. In some embodiments, a microfluidic chip 108 may include one or more detection chambers configured to facilitate detection of the one or more pathogen indicators 106 that are associated with one or more waterborne pathogens 104. A detection chamber may be configured to facilitate detection of numerous types of waterborne pathogens 104. Examples of such waterborne pathogens 104 include, but are not limited to, bacteria (e.g., E. coli O157:H7, Salmonella, Shigella, Clostridium botulinum, Vibrio cholerae, and Campylobacter), protozoa (e.g., Toxoplasma gondii, Giardia, Cryptosporidium, Entamoeba histolytica amoeba), viruses (e.g., Norwalk, Polioviruses, and Hepatitis A), and substantially any combination thereof.

At embodiment 9106, module 8740 may include one or more detection chambers configured to facilitate detection of the one or more pathogen indicators that are associated with one or more soilborne pathogens. In some embodiments, a microfluidic chip 108 may include one or more detection chambers configured to facilitate detection of the one or more pathogen indicators 106 that are associated with one or more soilborne pathogens 104. A detection chamber may be configured to facilitate detection of numerous types of soilborne pathogens 104. Examples of such soilborne pathogens 104 include, but are not limited to, Bacillus anthracis, Botryotinia fuckeliana, Erysiphe graminis, Mycosphaerella fijiensis, Penicillium spp., Phytophthora infestans, Plasmopara viticola, Pseudoperonospora cubensis, Pyricularia spp., Sphaerotheca fuliginea, Venturia spp., Bremia lactucae, Cercospora spp., Gibberella fujikuori, Monilinia spp., Mycosphaerella graminicola, Mycosphaerella musicola, Peronospora spp., Phytophthora infestans, Pyrenophora teres, Rhynchosporium secalis, Sclerotinia spp., Tapesia spp., Uncinula, Alternaria spp., Colletotrichum spp., Fusarium, Hemileia vastatrix, Leptosphaera, Phytophthora spp., Podosphaera leucotricha, Puccinia Pythium spp., Rhizoctonia spp., Sclerotium spp., Tilletia spp., Ustilago spp., and the like.

At embodiment 9108, module 8740 may include one or more detection chambers configured to facilitate detection of the one or more pathogen indicators that are associated with one or more food products. In some embodiments, a microfluidic chip 108 may include one or more detection chambers configured to facilitate detection of the one or more pathogen indicators 106 that are associated with one or more food products. In some embodiments, one or more detection chambers may be configured to facilitate detection of one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

FIG. 92 illustrates alternative embodiments of microfluidic chip 8700 of FIG. 87. FIG. 92 illustrates example embodiments of module 8740. Additional embodiments may include an embodiment 9202, an embodiment 9204, and/or an embodiment 9206.

At embodiment 9202, module 8740 may include one or more detection chambers configured to facilitate detection of the one or more pathogen indicators that are associated with one or more biological samples. In some embodiments, a microfluidic chip 108 may include one or more detection chambers configured to facilitate detection of the one or more pathogen indicators 106 that are associated with one or more biological samples 102. Examples of biological samples 102 include, but are not limited to, blood, cerebrospinal fluid, mucus, breath, urine, fecal material, skin, tissue, tears, hair, and the like.

At embodiment 9204, module 8740 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, a microfluidic chip 108 may include one or more detection chambers configured to facilitate detection of one or more pathogens 104 that include at least one virus, bacterium, prion, worm, egg, cyst, protozoan, single-celled organism, fungus, algae, pathogenic protein, or microbe, or substantially any combination thereof. A detection chamber may be configured to utilize numerous types of techniques, and combinations of techniques, to detect one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At embodiment 9206, module 8740 may include one or more detection chambers that are configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, a microfluidic chip 108 may include one or more detection chambers that are configured to facilitate detection of the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection chambers may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection chambers may include circuitry and/or electro-mechanical mechanisms to facilitate detection of one or more pathogen indicators 106 through a window in the one or more detection chambers.

In some embodiments, one or more detection chambers may be configured to facilitate detection of one or more pathogen indicators 106 through use of surface plasmon resonance. In some embodiments, one or more detection chambers may be configured to operably associate with one or more detection units 122. In some embodiments, one or more detection chambers may include one or more antibodies, aptamers, proteins, peptides, polynucleotides, and the like, that are bound to a substrate (e.g., a metal film) within the one or more detection chambers. In some embodiments, such detection chambers may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more detection chambers to facilitate detection of one or more pathogen indicators 106 through use of surface plasmon resonance.

In some embodiments, one or more detection chambers may be configured to facilitate detection of one or more pathogen indicators 106 through use of nuclear magnetic resonance (NMR). In some embodiments, one or more detection chambers may be configured to operably associate with one or more detection units 122. In some embodiments, the one or more detection chambers may include a nuclear magnetic resonance (NMR) probe. Accordingly, in some embodiments, one or more pathogen indicators 106 may be analyzed and detected through use of one or more detection chambers and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, a detection chamber may be configured to facilitate detection of one or more pathogen indicators 106 through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self-assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chein., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, a detection chamber may be configured to facilitate detection of one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, a detection chamber may be configured to facilitate detection of one or more pathogen indicators 106 through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, a detection chamber may be configured to facilitate detection of one or more pathogen indicators 106 through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more detection chambers that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, a detection chamber may be configured to facilitate detection of one or more pathogen indicators 106 through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more detection chambers may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. In some embodiments, one or more detection chambers may be configured to utilize numerous electron transfer based assays to provide for detection of one or more pathogen indicators 106 by a detection unit 122 that is configured to operably associate with the one or more microfluidic chips 108.

In some embodiments, a detection chamber may be configured to facilitate detection of one or more pathogen indicators 106 through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection chambers may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more detection chambers may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more detection chambers may be configured to provide for detection of one or more pathogen indicators 106 through use of electrical conductivity. In some embodiments, such detection chambers may be configured to operably associate with one or more detection units 122 such that the one or more detection units 122 can detect one or more pathogen indicators 106 through use of electrical conductivity. In some embodiments, one or more detection chambers may be configured to include two or more electrodes that are each coupled to one or more detector polynucleotides. Interaction of one or more pathogen associated polynucleotides (e.g., hybridization) with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleotides associated with one or more samples 102 may be combined with one or more sets of paired primers such that use of an amplification protocol, such as a polymerase chain reaction, will produce an amplification product corresponding to pathogen associated polynucleotides that are contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such asa cyst, egg, pathogen 104, spore, and the like, will complete an electrical circuit and facilitate the production of a detectable electrical current. Accordingly, in some embodiments, one or more detection chambers may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more detection chambers with the one or more detection units 122. Detection chambers and detection units 122 may be configured in numerous ways to facilitate analysis of one or more samples 102 and detect one or more pathogen indicators 106.

In some embodiments, one or more detection chambers may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more detection chambers such that the one or more detection units 122 can be used to detect one or more pathogen indicators 106 that have been focused within one or more microfluidic channels of the one or more detection chambers. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more detection chambers may be configured to combine one or more samples 102 with one or more reagent mixtures that include one or more binding agents that bind to one or more pathogen indicators 106 that may be present within the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be analyzed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Detection chambers and detection units 122 may be configured in numerous ways to facilitate detection of one or more pathogen indicators 106 through use of isoelectric focusing.

In some embodiments, one or more detection chambers may be configured to facilitate detection of one or more pathogen indicators 106 through use of one or more chromatographic methods. Accordingly, in some embodiments, one or more detection units 122 may be configured to operably associate with the one or more detection chambers and detect one or more pathogen indicators 106. In some embodiments, the one or more detection units 122 may be configured to operably associate with one or more detection chambers and supply solvents and other reagents to the one or more detection chambers. For example, in some embodiments, one or more detection units 122 may include pumps and solvent/buffer reservoirs that are configured to supply solvent/buffer flow through chromatographic media (e.g., a chromatographic column) that is operably associated with one or more detection chambers. In some embodiments, one or more detection units 122 may be configured to operably associate with one or more detection chambers and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more detection chambers may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more detection chambers may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. In some embodiments, one or more detection units 122 may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection chambers may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample components may be washed away from the precipitated antibody-pathogen indicator complexes. In some embodiments, one or more detection chambers that are configured to facilitate immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more detection chambers may be configured to facilitate detection of one or more pathogen indicators 106 through use of immunoseparation. In some embodiments, immunoseparation may be utilized in combination with additional detection methods to detect one or more pathogen indicators 106. For example, in some embodiments, one or more samples 102 may be combined with one or more antibodies that bind to one or more pathogen indicators 106 to form one or more antibody-pathogen indicator complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator complex such that the antibody binding constituent binds to the antibody-pathogen indicator 106 complex and provides for separation of the antibody-pathogen indicator complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator complexes such that the first antibody binding constituent and the second antibody binding constituent bind to antibody-pathogen indicator complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection chambers may be configured to facilitate detection of one or more pathogen indicators 106 through use of aptamer binding. In some embodiments, aptamer binding may be utilized in combination with additional methods to detect one or more pathogen indicators 106. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more detection chambers may be configured to facilitate detection of one or more pathogen indicators 106 through use of electrophoresis. In some embodiments, such detection chambers may be configured to operably associate with one or more detection units 122. Accordingly, in some embodiments, one or more detection units 122 may be configured to operably associate with one or more detection chambers and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Detection chambers and detection units 122 may be configured in numerous ways to provide for detection of one or more pathogen indicators 106 through use of electrophoresis.

In some embodiments, one or more detection chambers may be configured to facilitate detection of one or more pathogen indicators 106 through use of one or more charge-coupled device (CCD) cameras. In some embodiments, one or more detection units 122 that include one or more CCD cameras may be configured to operably associate with one or more detection chambers. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more detection chambers may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more detection chambers may be configured to facilitate detection of one or more pathogen indicators 106 through use of immunoassay. In some embodiments, one or more detection units 122 may be configured to operably associate with one or more such detection chambers and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

FIG. 93 illustrates a microfluidic chip 9300 representing examples of modules that may be used to perform a method for analysis of one or more pathogens 104. In FIG. 93 discussion and explanation may be provided with respect to the above-described example of FIG. 1, and/or with respect to other examples and contexts. However, it should be understood that the operations may be executed in a number of other environments and contexts, and/or modified versions of FIG. 1. Also, although the various modules are presented in the sequence(s) illustrated, it should be understood that the various modules may be configured in numerous orientations.

The microfluidic chip 9300 includes module 9310 that includes one or more separation channels that are configured to allow one or more samples that include one or more magnetically active pathogen indicator complexes to flow in a substantially antiparallel manner with one or more separation fluids. In some embodiments, module 9310 may include one or more channels that are configured to allow the one or more samples and the one or more separation fluids to flow in a substantially horizontal position. In some embodiments, module 9310 may include one or more channels that are configured to allow the one or more samples and the one or more separation fluids to flow in a substantially vertical position.

The microfluidic chip 9300 includes module 9320 that includes one or more magnetic fields that facilitate movement of the one or more magnetically active pathogen indicator complexes associated with the one or more samples into the one or more separation fluids. In some embodiments, module 9320 may include one or more electromagnets. In some embodiments, module 9320 may include one or more ferromagnets. In some embodiments, module 9320 may include one or more ferrofluids.

The microfluidic chip 9300 may optionally include module 9330 that includes one or more mixing chambers that are configured to allow one or more magnetically active pathogen indicator binding agents to bind to one or more pathogen indicators associated with the one or more samples to form the one or more magnetically active pathogen indicator complexes. In some embodiments, module 9330 may include one or more mixing members. In some embodiments, module 9330 may include one or more sonicators.

The microfluidic chip 9300 optionally includes module 9340 that includes one or more detection chambers configured to facilitate detection of the one or more pathogen indicators associated with the one or more samples. In some embodiments, module 9340 may include one or more detection chambers configured to facilitate detection of the one or more pathogen indicators that are associated with one or more airborne pathogens. In some embodiments, module 9340 may include one or more detection chambers configured to facilitate detection of the one or more pathogen indicators that are associated with one or more waterborne pathogens. In some embodiments, module 9340 may include one or more detection chambers configured to facilitate detection of the one or more pathogen indicators that are associated with one or more soilborne pathogens. In some embodiments, module 9340 may include one or more detection chambers configured to facilitate detection of the one or more pathogen indicators that are associated with one or more food products. In some embodiments, module 9340 may include one or more detection chambers configured to facilitate detection of the one or more pathogen indicators that are associated with one or more biological samples. In some embodiments, module 9340 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, module 9340 may include one or more detection chambers that are configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

FIG. 94 illustrates alternative embodiments of microfluidic chip 9300 of FIG. 93. FIG. 94 illustrates example embodiments of module 9310. Additional embodiments may include an embodiment 9402 and/or an embodiment 9404.

At embodiment 9402, module 9310 may include one or more channels that are configured to allow the one or more samples and the one or more separation fluids to flow in a substantially horizontal position. In some embodiments, one or more microfluidic chips 108 may include one or more channels that are configured to allow the one or more samples 102 and the one or more separation fluids to flow in a substantially horizontal position. For example, in some embodiments, the one or more samples 102 and the one or more separation fluids may be configured to flow in a substantially side-by-side manner in a substantially horizontal position. In some embodiments, one or more samples 102 and one or more separation fluids may be selected that are immiscible. In such embodiments, mixing of the one or more samples 102 and the one or more separation fluids may be substantially reduced.

At embodiment 9404, module 9310 may include one or more channels that are configured to allow the one or more samples and the one or more separation fluids to flow in a substantially vertical position. In some embodiments, one or more microfluidic chips 108 may include one or more channels that are configured to allow the one or more samples 102 and the one or more separation fluids to flow in a substantially vertical position. In some embodiments, the one or more samples 102 and the one or more separation fluids may be configured to flow with the one or more samples 102 flowing in a position that is above the flow of the one or more separation fluids. In some embodiments, the one or more samples 102 and the one or more separation fluids may be configured to flow with the one or more samples 102 flowing in a position that is below the flow of the one or more separation fluids. In some embodiments, the positional flow of one or more samples 102, and/or the positional flow of one or more separation fluids may be controlled through modulation of viscosity, density, immiscibility, or substantially any combination thereof. For example, in some embodiments, one or more separation fluids having greater density than the one or more samples 102 may be used to position the one or more separation fluids below the one or more samples 102. In some embodiments, one or more separation fluids that are less dense than the one or more samples may be used to position the one or more separation fluids above the one or more samples 102. In some embodiments, one or more samples 102 and one or more separation fluids may be selected that are immiscible. In such embodiments, mixing of the one or more samples 102 and the one or more separation fluids may be substantially reduced.

FIG. 95 illustrates alternative embodiments of microfluidic chip 9300 of FIG. 93. FIG. 95 illustrates example embodiments of module 9320. Additional embodiments may include an embodiment 9502, an embodiment 9504, and/or an embodiment 9506.

At embodiment 9502, module 9320 may include one or more electromagnets. In some embodiments, a microfluidic chip 108 may include one or more electromagnets. In some embodiments, one or more electromagnets may be used to move one or more magnetic plugs relative to one or more microfluidic chips 108. For example, in some embodiments, a magnetic plug may be used to propel fluid through one or more channels of a microfluidic chip 108 through use of magnetic attraction and/or magnetic repulsion. Accordingly, in some embodiments, electromagnets may be used to selectively create magnetic fields which may be used to selectively move a magnetic plug through one or more channels of a microfluidic chip. In some embodiments, one or more electromagnets may be used to separate one or more pathogen indicators 106 from one or more samples 102. In some embodiments, one or more electromagnets may be used to operably associate one or more microfluidic chips to one or more detection units, one or more reagent delivery units, one or more centrifugation units, and the like, in substantially any combination.

At embodiment 9504, module 9320 may include one or more ferromagnets. In some embodiments, a microfluidic chip 108 may include one or more ferromagnets. In some embodiments, one or more ferromagnets may be used to move one or more magnetic plugs relative to one or more microfluidic chips 108. For example, in some embodiments, a magnetic plug may be used to propel fluid through one or more channels of a microfluidic chip 108 through use of magnetic attraction and/or magnetic repulsion. In some embodiments, one or more ferromagnets may be attached to one or more guides (e.g., rails, channels, cords, and the like) such that the ferromagnet may be selectively positioned relative to one or more microfluidic chips 108. Accordingly, in some embodiments, ferromagnets may be used to selectively create magnetic fields which may be used to selectively move a magnetic plug through one or more channels of a microfluidic chip 108. In some embodiments, one or more ferromagnets may be used to separate one or more pathogen indicators 106 from one or more samples 102. In some embodiments, ferromagnets may be used to create eddy currents. In some embodiments, one or more ferromagnets may be used to operably associate one or more microfluidic chips 108 to one or more detection units 122, one or more reagent delivery units 116, one or more centrifugation units 118, and the like, in substantially any combination.

At embodiment 9506, module 9320 may include one or more ferrofluids. In some embodiments, a microfluidic chip 108 may include one or more ferrofluids. In some embodiments, ferrofluids may be configured to facilitate one or more pathogen indicators 106 from one or more samples 102. In some embodiments, a ferrofluid may be used to selectively position a magnetic plug relative to one or more microfluidic chips 108. For example, in some embodiments, a ferrofluid may be used to selectively position one or more magnetic plugs to facilitate movement of one or more fluids through one or more channels of a microfluidic chip 108.

FIG. 96 illustrates alternative embodiments of microfluidic chip 9300 of FIG. 93. FIG. 96 illustrates example embodiments of module 9330. Additional embodiments may include an embodiment 9602 and/or an embodiment 9604.

At embodiment 9602, module 9330 may include one or more mixing members. In some embodiments, a microfluidic chip 108 may include one or more mixing members. Mixing members may be positioned in numerous chambers of a microfluidic chip 108. Examples of such chambers include, but are not limited to, reaction chambers, mixing chambers, detection chambers, reservoirs, and the like, in substantially any combination. In some embodiments, one or more mixing members may be magnetically active such that the mixing members may be moved through use of one or more magnetic fields. In some embodiments, one or more mixing members may be physically coupled to a drive such that the drive causes movement of the mixing member.

At embodiment 9604, module 9330 may include one or more sonicators. In some embodiments, a microfluidic chip 108 may include one or more sonicators. In some embodiments, a microfluidic chip 108 may include one or more sonication probes. Such probes may be configured such that are able to operably associate with one or more vibration sources in a detachable manner. Accordingly, in some embodiments, one or more microfluidic chips 108 that include one or more probes may be configured to detachably connect with one or more vibration sources that produce a vibration that can be coupled to the one or more probes.

FIG. 97 illustrates alternative embodiments of microfluidic chip 9300 of FIG. 93. FIG. 96 illustrates example embodiments of module 9340. Additional embodiments may include an embodiment 9702, an embodiment 9704, an embodiment 9706, and/or an embodiment 9708.

At embodiment 9702, module 9340 may include one or more detection chambers configured to facilitate detection of the one or more pathogen indicators that are associated with one or more airborne pathogens. In some embodiments, a microfluidic chip 108 may include one or more detection chambers 122 configured to facilitate detection of the one or more pathogen indicators 106 that are associated with one or more airborne pathogens 104. Examples of such airborne pathogens 104 include, but are not limited to, fungal spores, mold spores, viruses, bacterial spores, and the like. In some embodiments, the pathogen indicators 106 may be collected within one or more microfluidic chips 108 through filtering air that is passed through the one or more microfluidic chips 108. Such filtering may occur through numerous mechanisms that may include, but are not limited to, use of physical filters, passing air through a fluid bubble chamber, passing the air through an electrostatic filter, and the like. In some embodiments, one or more detection chambers may be configured to facilitate detection of severe acute respiratory syndrome coronavirus (SARS). Polynucleic acid and polypeptide sequences that correspond to SARS have been reported and may be used as pathogen indicators 106 (U.S. Patent Application No.: 20060257852; herein incorporated by reference).

At embodiment 9704, module 9340 may include one or more detection chambers configured to facilitate detection of the one or more pathogen indicators that are associated with one or more waterborne pathogens. In some embodiments, a microfluidic chip 108 may include one or more detection chambers configured to facilitate detection of the one or more pathogen indicators 106 that are associated with one or more waterborne pathogens 104. A detection chamber may be configured to facilitate detection of numerous types of waterborne pathogens 104. Examples of such waterborne pathogens include, but are not limited to, bacteria (e.g., E. coli O157:H7, Salmonella, Shigella, Clostridium botulinum, Vibrio cholerae, and Campylobacter), protozoa (e.g., Toxoplasma gondii, Giardia, Cryptosporidium, Entamoeba histolytica amoeba), viruses (e.g., Norwalk, Polioviruses, and Hepatitis A), and substantially any combination thereof.

At embodiment 9706, module 9340 may include one or more detection chambers configured to facilitate detection of the one or more pathogen indicators that are associated with one or more soilborne pathogens. In some embodiments, a microfluidic chip 108 may include one or more detection chambers 122 configured to facilitate detection of the one or more pathogen indicators 106 that are associated with one or more soilborne pathogens 104. A detection chamber 122 may be configured to facilitate detection of numerous types of soilborne pathogens 104. Examples of such soilborne pathogens 104 include, but are not limited to, Bacillus anthracis, Botryotinia fuckeliana, Erysiphe graminis, Mycosphaerella fijiensis, Penicillium spp., Phytophthora infestans, Plasmopara viticola, Pseudoperonospora cubensis, Pyricularia spp., Sphaerotheca fuliginea, Venturia spp., Bremia lactucae, Cercospora spp., Gibberella fujikuori, Monilinia spp., Mycosphaerella graminicola, Mycosphaerella musicola, Peronospora spp., Phytophthora infestans, Pyrenophora teres, Rhynchosporium secalis, Sclerotinia spp., Tapesia spp., Uncinula, Alternaria spp., Colletotrichum spp., Fusarium, Hemileia vastatrix, Leptosphaera, Phytophthora spp., Podosphaera leucotricha, Puccinia Pythium spp., Rhizoctonia spp., Sclerotium spp., Tilletia spp., Ustilago spp., and the like.

At embodiment 9708, module 9340 may include one or more detection chambers configured to facilitate detection of the one or more pathogen indicators that are associated with one or more food products. In some embodiments, a microfluidic chip 108 may include one or more detection chambers configured to facilitate detection of the one or more pathogen indicators 106 that are associated with one or more food products. In some embodiments, one or more detection chambers may be configured to facilitate detection of one or more pathogen indicators 106 in one or more food samples 102 that are solids, such as meats, cheeses, nuts, vegetables, fruits, and the like, and/or liquids, such as water, juice, milk, and the like. Examples of pathogen indicators 106 include, but are not limited to: microbes such as Salmonella, E. coli, Shigella, amoebas, giardia, and the like; viruses such as avian flu, severe acute respiratory syncytial virus, hepatitis, human immunodeficiency virus, Norwalk virus, rotavirus, and the like; worms such as trichinella, tape worms, liver flukes, nematodes, and the like; eggs and/or cysts of pathogenic organisms; and the like.

FIG. 98 illustrates alternative embodiments of microfluidic chip 9300 of FIG. 93. FIG. 98 illustrates example embodiments of module 9340. Additional embodiments may include an embodiment 9802, an embodiment 9804, and/or an embodiment 9806.

At embodiment 9802, module 9340 may include one or more detection chambers configured to facilitate detection of the one or more pathogen indicators that are associated with one or more biological samples. In some embodiments, a microfluidic chip 108 may include one or more detection chambers configured to facilitate detection of the one or more pathogen indicators 106 that are associated with one or more biological samples 102. Examples of biological samples 102 include, but are not limited to, blood, cerebrospinal fluid, mucus, breath, urine, fecal material, skin, tissue, tears, hair, and the like.

At embodiment 9804, module 9340 may include one or more detection chambers configured to facilitate detection of one or more pathogens that include at least one virus, bacterium, prion, worm, egg, cyst, protozoan, single-celled organism, fungus, algae, pathogenic protein, or microbe. In some embodiments, a microfluidic chip 108 may include one or more detection chambers configured to facilitate detection of one or more pathogens 104 that include at least one virus, bacterium, prion, worm, egg, cyst, protozoan, single-celled organism, fungus, algae, pathogenic protein, microbe, or substantially any combination thereof. A detection chamber 122 may be configured to utilize numerous types of techniques, and combinations of techniques, to detect one or more pathogens 104. Many examples of such techniques are known and are described herein.

Numerous types of viruses may be identified. Such viruses are known and have been described (e.g., U.S. Patent Appl. No.: 20060257852; Field's Virology, Knipe et al, (Fifth Edition) Lippincott Williams & Wilkins, Philadelphia, (2006)). Examples of such viruses include, but are not limited to, hepatitis, influenza, avian influenza, severe acute respiratory syndrome coronavirus (severe acute respiratory syndrome (SARS)), human immunodeficiency virus, herpes viruses, human papilloma virus, rinovirus, rotavirus, West Nile virus, and the like.

Examples of bacteria that may be identified include, but are not limited to, Staphylococcus aureus, Staphylococcus epidermidis, Staphylococcus sp., Streptococcus pneumoniae, Streptococcus agalactiae, Streptococcus pyogenes, Enterococcus sp., Bacillus anthracis, Bacillus cereus, Bifidobacterium bifidum, Lactobacillus sp., Listeria monocytogenes, Nocardia sp., Rhodococcus equi, Erysipelothrix rhusiopathiae, Corynebacterium diptheriae, Propionibacterium acnes, Actinomyces sp., Clostridium botulinum, Clostridium difficile, Clostridium perfringens, Clostridium tetani, Mobiluncus sp., Peptostreptococcus sp., Neisseria gonorrhoeae, Neisseria meningitides, Moraxella catarrhalis, Veillonella sp., Actinobacillus actinomycetemcomitans, Acinetobacter baumannii, Bordetella pertussis, Brucella sp., Campylobacter sp., Capnocytophaga sp., Cardiobacterium hominis, Eikenella corrodens, Francisella tularensis, Haemophilus ducreyi, Haemophilus influenzae, Helicobacter pylori, Kingella kingae, Legionella pneumophila, Pasteurella multocida, Klebsiella granulomatis, Enterobacteriaceae, Citrobacter sp., Enterobacter sp., Escherichia coli, Klebsiella pneumoniae, Proteus sp., Salmonella enteriditis, Salmonella typhi, Shigella sp., Serratia marcescens, Yersinia enterocolitica, Yersinia pestis, Aeromonas sp., Plesiomonas shigelloides, Vibrio cholerae, Vibrio parahaemolyticus, Vibrio vulnificus, Acinetobacter sp., Flavobacterium sp., Pseudomonas aeruginosa, Burkholderia cepacia, Burkholderia pseudomallei, Xanthomonas maltophilia, Stenotrophomonas maltophila, Bacteroides fragilis, Bacteroides sp., Prevotella sp., Fusobacterium sp., Spirillum minus, or substantially any combination thereof.

Numerous prions may be identified. Examples of such prions include, but are not limited to, bovine prion protein, human prion protein, monkey prion protein, dog prion protein, and the like. The amino acid sequences and/or nucleotide sequences of numerous prions are known and have been reported (e.g., Premzl and Gamulin, BMC Genomics, 8:1 (2007)).

Numerous pathogenic worms may be identified. Examples of such worms include, but are not limited to, tapeworms, helminths, whipworms, hookworms, ringworms, roundworms, pinworms, ascarids, filarids, and the like.

In some embodiments, the eggs and/or cysts of pathogens 104 may be identified. Examples of such eggs and/or cysts include, but are not limited to, eggs and/or cysts of: parasitic worms (e.g., Heterodera glycines, Trichinella), amoebe (e.g., Entamoeba histolytica, Acanthamoeba), protozoans (e.g., Giardia, cryptosporidium, Toxoplasma), and the like.

Numerous protozoans may be identified. Examples of protozoans include, but are not limited to, slime molds, flagellates, ciliates, and the like (e.g., cryptosporidium, giardia, naegleria fowleri, acanthamoeba, entamoeba histolytica, cryptosporidium parvum, cyclospora cayetanensis, isospora belli, microsporidia) (Marshall et al., Clin, Micro. Rev., 10:67-85 (1997)).

Examples of pathogenic fungi include, but are not limited to, dimorphic fungi that may assume a mold form but may also adopt a yeast form, histoplasma capsulatum, coccidioides immitis, candida, aspergillus, and the like.

Pathogenic algae include, but are not limited to, Prototheca members, Helicosporidiu members, Chattonella members (e.g., Chattonella marina), and the like.

Numerous types of pathogenic proteins may be identified and include, but are not limited to, toxins (e.g., exotoxing, endotoxins), prions, and the like.

Numerous microbes may be identified. In some embodiments, microbes may be prokaryotes. In some embodiments, microbes may be eukaryotes. Examples of such microbes include, but are not limited to, Giardia, amoeba (e.g., Entamoeba, Naegleria, Acanthamoeba), trypanosomes, Plasmodium (e.g., Plasmodium falciparum, Plasmodium vivax, Plasmodium ovale, Plasmodium malariae, Plasmodium knowlesi), Eimeria, Toxoplasma, Neospora, Mycoplasma, Leishmania, Trichomonas, Cryptosporidium, Isospora, Balantidium, protozoans, Mycoplasma hominis, Ureaplasma urealyticum, and the like.

In some embodiments, a pathogen 104 may be a member of numerous groups of pathogens 104. For example, single-celled organisms may include microbes, protozoans, and the like.

At embodiment 9806, module 9340 may include one or more detection chambers that are configured to facilitate detection of the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay. In some embodiments, a microfluidic chip 108 may include one or more detection chambers that are configured to facilitate detection of the one or more pathogen indicators 106 with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, immunoassay, or substantially any combination thereof.

In some embodiments, one or more detection chambers may include a window (e.g., a quartz window, a cuvette analog, and/or the like) through which one or more detection units 122 may determine if one or more pathogen indicators 106 are present or determine the concentration of one or more pathogen indicators 106. In such embodiments, numerous techniques may be used to detect one or more pathogen indicators 106, such as visible light spectroscopy, ultraviolet light spectroscopy, infrared spectroscopy, fluorescence spectroscopy, and the like. Accordingly, in some embodiments, one or more detection chambers may include circuitry and/or electro-mechanical mechanisms to facilitate detection of one or more pathogen indicators 106 through a window in the one or more detection chambers.

In some embodiments, one or more detection chambers may be configured to facilitate detection of one or more pathogen indicators 106 through use of surface plasmon resonance. In some embodiments, one or more detection chambers may be configured to operably associate with one or more detection units 122. In some embodiments, one or more detection chambers may include one or more antibodies, aptamers, proteins, peptides, polynucleotides, and the like, that are bound to a substrate (e.g., a metal film) within the one or more detection chambers. In some embodiments, such detection chambers may include a prism through which one or more detection units 122 may shine light to detect one or more pathogen indicators 106 that interact with the one or more antibodies, aptamers, proteins, peptides, polynucleotides, and the like, that are bound to a substrate. In some embodiments, one or more detection units 122 may include one or more prisms that are configured to associate with one or more exposed substrate surfaces that are included within one or more detection chambers to facilitate detection of one or more pathogen indicators 106 through use of surface plasmon resonance.

In some embodiments, one or more detection chambers may be configured to facilitate detection of one or more pathogen indicators 106 through use of nuclear magnetic resonance (NMR). In some embodiments, one or more detection chambers may be configured to operably associate with one or more detection units 122. In some embodiments, the one or more detection chambers may include a nuclear magnetic resonance (NMR) probe. Accordingly, in some embodiments, one or more pathogen indicators 106 may be analyzed and detected through use of one or more detection chambers and one or more detection units 122.

In some embodiments, one or more pathogen indicators 106 may be detected through use of spectroscopy. Numerous types of spectroscopic methods may be used. Examples of such methods include, but are not limited to, ultraviolet spectroscopy, visible light spectroscopy, infrared spectroscopy, x-ray spectroscopy, fluorescence spectroscopy, mass spectroscopy, plasmon resonance (e.g., Cherif et al., Clinical Chemistry, 52:255-262 (2006) and U.S. Patent No.: 7,030,989; herein incorporated by reference), nuclear magnetic resonance spectroscopy, Raman spectroscopy, fluorescence quenching, fluorescence resonance energy transfer, intrinsic fluorescence, ligand fluorescence, and the like.

In some embodiments, a detection chamber may be configured to facilitate detection of one or more pathogen indicators 106 through use of electrochemical detection. In some embodiments, one or more polynucleotides may be detected through electrochemical detection. For example, in some embodiments, a polynucleotide that includes a redox label, such as ferrocene is coupled to a gold electrode. The labeled polynucleotide forms a stem-loop structure that can self assemble onto a gold electrode by means of facile gold-thiol chemistry. Hybridization of a sample polynucleotide induces a large conformational change in the surface-confined polynucleotide structure, which in turn alters the electron-transfer tunneling distance between the electrode and the redoxable label. The resulting change in electron transfer efficiency may be measured by cyclic voltammetry (Fan et al., Proc. Natl. Acad. Sci., 100:9134-9137 (2003); Wang et al., Anal. Chem., 75:3941-3945 (2003); Singh-Zocchi et al., Proc. Natl. Acad. Sci., 100:7605-7610 (2003)). In some embodiments, such methods may be used to detect messenger ribonucleic acid, genomic deoxyribonucleic acid, and fragments thereof.

In some embodiments, a detection chamber may be configured to facilitate detection of one or more pathogen indicators 106 through use of polynucleotide detection. Numerous methods may be used to detect one or more polynucleotides. Examples of such methods include, but are not limited to, those based on polynucleotide hybridization, polynucleotide ligation, polynucleotide amplification, polynucleotide degradation, and the like. Methods that utilize intercalation dyes, fluorescence resonance energy transfer, capacitive deoxyribonucleic acid detection, and nucleic acid amplification have been described (e.g., U.S. Patent Nos.: 7,118,910 and 6,960,437; herein incorporated by reference). Such methods may be adapted to provide for detection of one or more pathogen indicators 106. In some embodiments, fluorescence quenching, molecular beacons, electron transfer, electrical conductivity, and the like may be used to analyze polynucleotide interaction. Such methods are known and have been described (e.g., Jarvius, DNA Tools and Microfluidic Systems for Molecular Analysis, Digital Comprehensive Summaries of Uppsala Dissertations from the Faculty of Medicine 161, ACTA UNIVERSITATIS UPSALIENSIS UPPSALA 2006, ISBN: 91-554-6616-8; Singh-Zocchi et al., Proc. Natl. Acad. Sci., 100:7605-7610 (2003); Wang et al., Anal. Chem., 75:3941-3945 (2003); Fan et al., Proc. Natl. Acad. Sci., 100:9134-9137 (2003); U.S. Patent Nos.: 6,958,216; 5,093,268; 6,090,545; herein incorporated by reference). In some embodiments, one or more polynucleotides that include at least one carbon nanotube may be combined with one or more samples 102, and/or one or more partially purified polynucleotides obtained from one or more samples 102. The one or more polynucleotides that include one or more carbon nanotubes are allowed to hybridize with one or more polynucleotides that may be present within the one or more samples 102. The one or more carbon nanotubes may be excited (e.g., with an electron beam and/or an ultraviolet laser) and the emission spectra of the excited nanotubes may be correlated with hybridization of the one or more polynucleotides that include at least one carbon nanotube with one or more polynucleotides that are included within the one or more samples 102. Accordingly, polynucleotides that hybridize to one or more pathogen indicators 106 may include one or more carbon nanotubes. Methods to utilize carbon nanotubes as probes for nucleic acid interaction have been described (e.g., U.S. Patent No.: 6,821,730; herein incorporated by reference). In some embodiments, one or more analysis units 120 may be configured to facilitate hybridization of one or more pathogen indicators 106 and configured to facilitate detection of the one or more pathogen indicators 106 with one or more detection units 122. Numerous other methods based on polynucleotide detection may be used to detect one or more pathogen indicators 106.

In some embodiments, a detection chamber may be configured to facilitate detection of one or more pathogen indicators 106 through use of fluorescence anisotropy. Fluorescence anisotropy is based on measuring the steady state polarization of sample 102 fluorescence imaged in a confocal arrangement. A linearly polarized laser excitation source preferentially excites fluorescent target molecules with transition moments aligned parallel to the incident polarization vector. The resultant fluorescence is collected and directed into two channels that measure the intensity of the fluorescence polarized both parallel and perpendicular to that of the excitation beam. With these two measurements, the fluorescence anisotropy, r, can be determined from the equation: r = (Intensity parallel - Intensity perpendicular)/(Intensity parallel + 2(Intensity perpendicular)) where the I terms indicate intensity measurements parallel and perpendicular to the incident polarization. Fluorescence anisotropy detection of fluorescent molecules has been described. Accordingly, fluorescence anisotropy may be coupled to numerous fluorescent labels as have been described herein and as have been described. In some embodiments, a detection chamber may be configured to facilitate detection of one or more pathogen indicators 106 through use of fluorescence resonance energy transfer (FRET). Fluorescence resonance energy transfer refers to an energy transfer mechanism between two fluorescent molecules. A fluorescent donor is excited at its fluorescence excitation wavelength. This excited state is then nonradiatively transferred to a second molecule, the fluorescent acceptor. Fluorescence resonance energy transfer may be used within numerous configurations to detect one or more pathogen indicators 106. For example, in some embodiments, an antibody may be labeled with a fluorescent donor and one or more pathogen indicators 106 may be labeled with a fluorescent acceptor. Accordingly, such labeled antibodies and pathogen indicators 106 may be used within competition assays to detect the presence and/or concentration of one or more pathogen indicators 106 in one or more samples 102. Numerous combinations of fluorescent donors and fluorescent acceptors may be used to detect one or more pathogen indicators 106. Accordingly, one or more detection units 122 may be configured to emit one or more wavelength of light to excite a fluorescent donor and may be configured to detect one or more wavelength of light emitted by the fluorescent acceptor. Accordingly, in some embodiments, one or more detection units 122 may be configured to accept one or more detection chambers that include a quartz window through which fluorescent light may pass to provide for detection of one or more pathogen indicators 106 through use of fluorescence resonance energy transfer. Accordingly, fluorescence resonance energy transfer may be used in conjunction with competition assays and/or numerous other types of assays to detect one or more pathogen indicators 106.

In some embodiments, a detection chamber may be configured to facilitate detection of one or more pathogen indicators 106 through use of electron transfer. Electron transfer is the process by which an electron moves from an electron donor to an electron acceptor causing the oxidation states of the electron donor and the electron acceptor to change. In some embodiments, electron transfer may occur when an electron is transferred from one or more electron donors to an electrode. In some embodiments, electron transfer may be utilized within competition assays to detect one or more pathogen indicators 106. For example, in some embodiments, one or more detection chambers may include one or more polynucleotides that may be immobilized on one or more electrodes. The immobilized polynucleotides may be incubated with a reagent mixture that includes sample polynucleotides and polynucleotides that are tagged with an electron donor. Hybridization of the tagged polynucleotides to the immobilized polynucleotides allows the electron donor to transfer an electron to the electrode to produce a detectable signal. Accordingly, a decrease in signal due to the presence of one or more polynucleotides that are pathogen indicators 106 in the reagent mixture indicates the presence of a pathogen indicator 106 in the sample 102. Such methods may be used in conjunction with polynucleotides, polypeptides, peptides, antibodies, aptamers, and the like. In some embodiments, one or more detection chambers may be configured to utilize numerous electron transfer based assays to provide for detection of one or more pathogen indicators 106 by a detection unit 122 that is configured to operably associate with the one or more microfluidic chips 108.

In some embodiments, a detection chamber may be configured to facilitate detection of one or more pathogen indicators 106 through use of one or more enzyme assays. Numerous enzyme assays may be used to provide for detection of one or more pathogen indicators 106. Examples of such enzyme assays include, but are not limited to, beta-galactosidase assays, peroxidase assays, catalase assays, alkaline phosphatase assays, and the like. In some embodiments, enzyme assays may be configured such that an enzyme will catalyze a reaction involving an enzyme substrate that produces a fluorescent product. Accordingly, one or more detection chambers may be configured to detect fluorescence resulting from the fluorescent product. Enzymes and fluorescent enzyme substrates are known and are commercially available (e.g., Sigma-Aldrich, St. Louis, MO). In some embodiments, enzyme assays may be configured as binding assays that provide for detection of one or more pathogen indicators 106. For example, in some embodiments, one or more detection chambers may be configured to include a substrate to which is coupled one or more antibodies, aptamers, peptides, proteins, polynucleotides, ligands, and the like, that will interact (e.g., bind) with one or more pathogen indicators 106. One or more samples 102 may be passed across the substrate such that one or more pathogen indicators 106 present within the one or more samples 102 will interact with the one or more antibodies, aptamers, peptides, proteins, polynucleotides, ligands, and the like, and be immobilized on the substrate. One or more antibodies, aptamers, peptides, proteins, polynucleotides, ligands, and the like, that are labeled with an enzyme may then be passed across the substrate such that the one or more labeled antibodies, aptamers, peptides, proteins, polynucleotides, ligands, and the like, will bind to the one or more immobilized pathogen indicators 106. An enzyme substrate may then be introduced to the one or more immobilized enzymes such that the enzymes are able to catalyze a reaction involving the enzyme substrate to produce a fluorescent product. Such assays are often referred to as sandwich assays. Accordingly, one or more detection units 122 may be configured to detect one or more products of enzyme catalysis to provide for detection of one or more pathogen indicators 106.

In some embodiments, one or more detection chambers may be configured to provide for detection of one or more pathogen indicators 106 through use of electrical conductivity. In some embodiments, such detection chambers may be configured to operably associate with one or more detection units 122 such that the one or more detection units 122 can detect one or more pathogen indicators 106 through use of electrical conductivity. In some embodiments, one or more detection chambers may be configured to include two or more electrodes that are each coupled to one or more detector polynucleotides. Interaction of one or more pathogen associated polynucleotides (e.g., hybridization) with two detector polynucleotides that are coupled to two different electrodes will complete an electrical circuit. This completed circuit will provide for the flow of a detectable electrical current between the two electrodes and thereby provide for detection of one or more pathogen associated polynucleotides that are pathogen indicators 106. In some embodiments, one or more pathogen associated polynucleotides may be detected through use of nucleic acid amplification and electrical conductivity. For example, polynucleotides associated with one or more samples 102 may be combined with one or more sets of paired primers such that use of an amplification protocol, such as a polymerase chain reaction, will produce an amplification product corresponding to pathogen associated polynucleotides that are contained within the one or more samples 102. In such embodiments, primers may be used that include a tag that facilitates association of the amplification product with an electrical conductor to complete an electrical circuit. Accordingly, the production of an amplification product incorporates two paired primers into a single amplification product which allows the amplification product to associate with two electrical conductors and complete an electrical circuit to provide for detection of pathogen associated polynucleotides within one or more samples 102. Such a protocol is illustrated in Figure 99. In some embodiments, the paired primers are each coupled to the same type of tag. In some embodiments, the paired primers are each coupled to different types of tags. Numerous types of tags may be used. Examples of such tags include, but are not limited to, biotin, avidin, streptavidin, histidine tags, nickel tags, ferrous tags, non-ferrous tags, and the like. In some embodiments, tags may be bound by an antibody and/or an aptamer. In some embodiments, a tag may be a reactive group that chemically bonds to an electrical conductor. In some embodiments, the electrodes may be carbon nanotubes (e.g., U.S. Patent No.: 6,958,216; herein incorporated by reference). In some embodiments, electrodes may include, but are not limited to, one or more conductive metals, such as gold, copper, iron, silver, platinum, and the like; one or more conductive alloys; one or more conductive ceramics; and the like. In some embodiments, electrodes may be selected and configured according to protocols typically used in the computer industry that include, but are not limited to, photolithography, masking, printing, stamping, and the like. In some embodiments, other molecules and complexes that interact with one or more pathogen indicators 106 may be used to detect the one or more pathogen indicators 106 through use of electrical conductivity. Examples of such molecules and complexes include, but are not limited to, proteins, peptides, antibodies, aptamers, and the like. For example, in some embodiments, two or more antibodies may be immobilized on one or more electrodes such that contact of the two or more antibodies with a pathogen indicator 106, such as a cyst, egg, pathogen 104, spore, and the like, will complete an electrical circuit and facilitate the production of a detectable electrical current. Accordingly, in some embodiments, one or more detection chambers may be configured to include electrical connectors that are able to operably associate with one or more detection units 122 such that the detection units 122 may detect an electrical current that is due to interaction of one or more pathogen indicators 106 with two or more electrodes. In some embodiments, one or more detection units 122 may include electrical connectors that provide for operable association of one or more detection chambers with the one or more detection units 122. Detection chambers and detection units 122 may be configured in numerous ways to facilitate analysis of one or more samples 102 and detect one or more pathogen indicators 106.

In some embodiments, one or more detection chambers may be configured to provide for detection of one or more pathogen indicators 106 through use of isoelectric focusing. In some embodiments, native isoelectric focusing may be utilized to detect one or more pathogen indicators 106. In some embodiments, denaturing isoelectric focusing may be utilized to detect one or more pathogen indicators 106. Methods to construct microfluidic channels that may be used for isoelectric focusing have been reported (e.g., Macounova et al., Anal Chem., 73:1627-1633 (2001); Macounova et al., Anal Chem., 72:3745-3751 (2000); Herr et al., Investigation of a miniaturized capillary isoelectric focusing (cIEF) system using a full-field detection approach, Mechanical Engineering Department, Stanford University, Stanford, CA; Wu and Pawliszyn, Journal of Microcolumn Separations, 4:419-422 (1992); Kilar and Hjerten, Electrophoresis, 10:23-29 (1989); U.S. Patent Nos.: 7,150,813; 7,070,682; 6,730,516; herein incorporated by reference). In some embodiments, one or more detection units 122 may be configured to operably associate with one or more detection chambers such that the one or more detection units 122 can be used to detect one or more pathogen indicators 106 that have been focused within one or more microfluidic channels of the one or more detection chambers. In some embodiments, one or more detection units 122 may be configured to include one or more CCD cameras that can be used to detect one or more pathogen indicators 106. In some embodiments, one or more detection units 122 may be configured to include one or more spectrometers that can be used to detect one or more pathogen indicators 106. Numerous types of spectrometers may be utilized to detect one or more pathogen indicators 106 following isoelectric focusing. In some embodiments, one or more detection units 122 may be configured to utilize refractive index to detect one or more pathogen indicators 106. In some embodiments, one or more detection chambers may be configured to combine one or more samples 102 with one or more reagent mixtures that include one or more binding agents that bind to one or more pathogen indicators 106 that may be present with the one or more samples 102 to form a pathogen indicator-binding agent complex. Examples of such binding agents that bind to one or more pathogen indicators 106 include, but are not limited to, antibodies, aptamers, peptides, proteins, polynucleotides, and the like. In some embodiments, a pathogen indicator-binding agent complex may be analyzed through use of isoelectric focusing and then detected with one or more detection units 122. In some embodiments, one or more binding agents may include a label. Numerous labels may be used and include, but are not limited to, radioactive labels, fluorescent labels, colorimetric labels, spin labels, and the like. Accordingly, in some embodiments, a pathogen indicator-binding agent complex (labeled) may be detected with one or more detection units 122 that are configured to detect the one or more labels. Detection chambers and detection units 122 may be configured in numerous ways to facilitate detection of one or more pathogen indicators 106 through use of isoelectric focusing.

In some embodiments, one or more detection chambers may be configured to facilitate detection of one or more pathogen indicators 106 through use of one or more chromatographic methods. Accordingly, in some embodiments, one or more detection units 122 may be configured to operably associate with the one or more detection chambers and detect one or more pathogen indicators 106. In some embodiments, the one or more detection units 122 may be configured to operably associate with one or more detection chambers and supply solvents and other reagents to the one or more detection chambers. For example, in some embodiments, one or more detection units 122 may include pumps and solvent/buffer reservoirs that are configured to supply solvent/buffer flow through chromatographic media (e.g., a chromatographic column) that is operably associated with one or more detection chambers. In some embodiments, one or more detection units 122 may be configured to operably associate with one or more detection chambers and be configured to utilize one or more methods to detect one or more pathogen indicators 106. Numerous types of chromatographic methods and media may be used to analyze one or more samples 102 and provide for detection of one or more pathogen indicators 106. Chromatographic methods include, but are not limited to, low pressure liquid chromatography, high pressure liquid chromatography (HPLC), microcapillary low pressure liquid chromatography, microcapillary high pressure liquid chromatography, ion exchange chromatography, affinity chromatography, gel filtration chromatography, size exclusion chromatography, thin layer chromatography, paper chromatography, gas chromatography, and the like. In some embodiments, one or more detection chambers may be configured to include one or more high pressure microcapillary columns. Methods that may be used to prepare microcapillary HPLC columns (e.g., columns with a 100 micrometer-500 micrometer inside diameter) have been described (e.g., Davis et al., Methods, A Companion to Methods in Enzymology, 6: Micromethods for Protein Structure Analysis, ed. by John E. Shively, Academic Press, Inc., San Diego, 304-314 (1994); Swiderek et al., Trace Structural Analysis of Proteins. Methods of Enzymology, ed. by Barry L. Karger & William S. Hancock, Spectrum, Publisher Services, 271, Chap. 3, 68-86 (1996); Moritz and Simpson, J. Chromatogr., 599:119-130 (1992)). In some embodiments, one or more detection chambers may be configured to include one or more affinity columns. Methods to prepare affinity columns have been described. Briefly, a biotinylated site may be engineered into a polypeptide, peptide, aptamer, antibody, or the like. The biotinylated protein may then be incubated with avidin coated polystyrene beads and slurried in Tris buffer. The slurry may then be packed into a capillary affinity column through use of high pressure packing. Affinity columns may be prepared that may include one or more molecules and/or complexes that interact with one or more pathogen indicators 106. For example, in some embodiments, one or more aptamers that bind to one or more pathogen indicators 106 may be used to construct an affinity column. Accordingly, numerous chromatographic methods may be used alone, or in combination with additional methods, to facilitate detection of one or more pathogen indicators 106. Numerous detection methods may be used in combination with numerous types of chromatographic methods. Examples of such detection methods include, but are not limited to, conductivity detection, refractive index detection, colorimetric detection, radiological detection, detection by retention time, detection through use of elution conditions, spectroscopy, and the like. For example, in some embodiments, one or more chromatographic markers may be added to one or more samples 102 prior to the samples 102 being applied to a chromatographic column. In some embodiments, one or more detection units 122 may be configured to detect the one or more chromatographic markers and use the elution time and/or position of the chromatographic markers as a calibration tool for use in detecting one or more pathogen indicators 106 if those pathogen indicators 106 are eluted from the chromatographic column.

In some embodiments, one or more detection chambers may be configured to facilitate detection of one or more pathogen indicators 106 through use of immunoprecipitation. For example, in some embodiments, one or more samples 102 may be combined with one or more antibodies that bind to one or more pathogen indicators 106 to form one or more antibody-pathogen indicator 106 complexes. An insoluble form of an antibody binding constituent, such as protein A (e.g., protein A-sepharose bead, protein A-magnetic bead, protein A-ferrous bead, protein A-non-ferrous bead, and the like), Protein G, a second antibody, an aptamer, and the like, may then be mixed with the antibody-pathogen indicator 106 complex such that the insoluble antibody binding constituent binds to the antibody-pathogen indicator 106 complex and provides for precipitation of the antibody-pathogen indicator 106 complex. Such complexes may be separated from other sample 102 components to provide for detection of one or more pathogen indicators 106. For example, in some embodiments, sample components may be washed away from the precipitated antibody-pathogen indicator complexes. In some embodiments, one or more detection chambers that are configured to facilitate immunoprecipitation may be operably associated with one or more centrifugation units 118 to assist in precipitating one or more antibody-pathogen indicator 106 complexes. In some embodiments, aptamers (polypeptide and/or polynucleotide) may be used in combination with antibodies or in place of antibodies. Accordingly, one or more detection units 122 may be configured to detect one or more pathogen indicators 106 through use of numerous detection methods in combination with immunoprecipitation based methods.

In some embodiments, one or more detection chambers may be configured to facilitate detection of one or more pathogen indicators 106 through use of immunoseparation. In some embodiments, immunoseparation may be utilized in combination with additional detection methods to detect one or more pathogen indicators 106. For example, in some embodiments, one or more samples 102 may be combined with one or more antibodies that bind to one or more pathogen indicators 106 to form one or more antibody-pathogen indicator complexes. An antibody binding constituent may be added that binds to the antibody-pathogen complex. Examples of such antibody binding constituents that may be used alone or in combination include, but are not limited to, protein A (e.g., protein A-sepharose bead, protein A-magnetic bead, protein A-ferrous bead, protein A-non-ferrous bead, and the like), Protein G, a second antibody, an aptamer, and the like. Such antibody binding constituents may be mixed with an antibody-pathogen indicator complex such that the antibody binding constituent binds to the antibody-pathogen indicator 106 complex and provides for separation of the antibody-pathogen indicator complex. In some embodiments, the antibody binding constituent may include a tag that allows the antibody binding constituent and complexes that include the antibody binding constituent to be separated from other components in one or more samples 102. In some embodiments, the antibody binding constituent may include a ferrous material. Accordingly, antibody-pathogen indicator 106 complexes may be separated from other sample 102 components through use of a magnet, such as an electromagnet. In some embodiments, an antibody binding constituent may include a non-ferrous metal. Accordingly, antibody-pathogen indicator 106 complexes may be separated from other sample 102 components through use of an eddy current to direct movement of one or more antibody-pathogen indicator 106 complexes. In some embodiments, two or more forms of an antibody binding constituents may be used to detect one or more pathogen indicators 106. For example, in some embodiments, a first antibody binding constituent may be coupled to a ferrous material and a second antibody binding constituent may be coupled to a non-ferrous material. Accordingly, the first antibody binding constituent and the second antibody binding constituent may be mixed with antibody-pathogen indicator complexes such that the first antibody binding constituent and the second antibody binding constituent bind to antibody-pathogen indicator complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more antibodies that bind to one or more pathogen indicators 106 to form one or more antibody-pathogen indicator complexes. In some embodiments, the one or more antibodies may include one or more tags that provide for separation of the antibody-pathogen indicator 106 complexes. For example, in some embodiments, an antibody may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with immunoseparation based methods. In some embodiments, aptamers (polypeptide and/or polynucleotide) may be used in combination with antibodies or in place of antibodies.

In some embodiments, one or more detection chambers may be configured to facilitate detection of one or more pathogen indicators 106 through use of aptamer binding. In some embodiments, aptamer binding may be utilized in combination with additional methods to detect one or more pathogen indicators 106. For example, in some embodiments, one or more samples 102 may be combined with one or more aptamers that bind to one or more pathogen indicators 106 to form one or more aptamer-pathogen indicator 106 complexes. In some embodiments, aptamer binding constituents may be added that bind to the aptamer-pathogen 104 complex. Numerous aptamer binding constituents may be utilized. For example, in some embodiments, one or more aptamers may include one or more tags to which one or more aptamer binding constituents may bind. Examples of such tags include, but are not limited to, biotin, avidin, streptavidin, histidine tags, nickel tags, ferrous tags, non-ferrous tags, and the like. In some embodiments, one or more tags may be conjugated with a label to provide for detection of one or more complexes. Examples of such tag-label conjugates include, but are not limited to, Texas red conjugated avidin, alkaline phosphatase conjugated avidin, CY2 conjugated avidin, CY3 conjugated avidin, CY3.5 conjugated avidin, CY5 conjugated avidin, CY5.5 conjugated avidin, fluorescein conjugated avidin, glucose oxidase conjugated avidin, peroxidase conjugated avidin, rhodamine conjugated avidin, agarose conjugated anti-protein A, alkaline phosphatase conjugated protein A, anti-protein A, fluorescein conjugated protein A, IRDye® 800 conjugated protein A, peroxidase conjugated protein A, sepharose protein A, alkaline phosphatase conjugated streptavidin, AMCA conjugated streptavidin, anti- streptavidin (Streptomyces avidinii) (rabbit) IgG Fraction, beta-galactosidase conjugated streptavidin, CY2 conjugated streptavidin, CY3 conjugated streptavidin, CY3.5 conjugated streptavidin, CY5 conjugated streptavidin, CY5.5 conjugated streptavidin, fluorescein conjugated streptavidin, IRDye® 700DX conjugated streptavidin, IRDye® 800 conjugated streptavidin, IRDye® 800CW conjugated streptavidin, peroxidase conjugated streptavidin, phycoerythrin conjugated streptavidin, rhodamine conjugated streptavidin, Texas red conjugated streptavidin, alkaline phosphatase conjugated biotin, anti-biotin (rabbit) IgG fraction, beta-galactosidase conjugated biotin, glucose oxidase conjugated biotin, peroxidase conjugated biotin, alkaline phosphatase conjugated protein G, anti-protein G (rabbit) Agarose conjugated, anti-protein G (Rabbit) IgG fraction, fluorescein conjugated protein G, IRDye® 800 conjugated protein G, peroxidase conjugated protein G, and the like. Many such labeled tags are commercially available (e.g., Rockland Immunochemicals, Inc., Gilbertsville, PA). Such labels may also be used in association with other methods to process and detect one or more pathogen indicators 106. Aptamer binding constituents may be mixed with an aptamer-pathogen indicator 106 complex such that the aptamer binding constituent binds to the aptamer-pathogen indicator 106 complex and provides for separation of the aptamer-pathogen indicator 106 complex. In some embodiments, the aptamer binding constituent may include a tag that allows the aptamer binding constituent and complexes that include the aptamer binding constituent to be separated from other components in one or more samples 102. In some embodiments, the aptamer binding constituent may include a ferrous material. Accordingly, aptamer-pathogen indicator 106 complexes may be separated from other sample 102 components through use of a magnet, such as an electromagnet. In some embodiments, an aptamer binding constituent may include a non-ferrous metal. Accordingly, aptamer-pathogen indicator 106 complexes may be separated from other sample 102 components through use of an eddy current to direct movement of one or more aptamer-pathogen indicator 106 complexes. In some embodiments, two or more forms of aptamer binding constituents may be used to detect one or more pathogen indicators 106. For example, in some embodiments, a first aptamer binding constituent may be coupled to a ferrous material and a second aptamer binding constituent may be coupled to a non-ferrous material. Accordingly, the first aptamer binding constituent and the second aptamer binding constituent may be mixed with aptamer-pathogen indicator 106 complexes such that the first aptamer binding constituent and the second aptamer binding constituent bind to aptamer-pathogen indicator 106 complexes that include different pathogen indicators 106. Accordingly, in such embodiments, different pathogen indicators 106 from a single sample 102 and/or a combination of samples 102 may be separated through use of direct magnetic separation in combination with eddy current based separation. In some embodiments, one or more samples 102 may be combined with one or more aptamers that bind to one or more pathogen indicators 106 to form one or more aptamer-pathogen indicator 106 complexes. In some embodiments, the one or more aptamers may include one or more tags that provide for separation of the aptamer-pathogen indicator 106 complexes. For example, in some embodiments, an aptamer may include a tag that includes one or more magnetic beads, a ferrous material, a non-ferrous metal, an affinity tag, a size exclusion tag (e.g., a large bead that is excluded from entry into chromatographic media such that antibody-pathogen indicator 106 complexes pass through a chromatographic column in the void volume), and the like. Accordingly, one or more detection units 122 may be configured to detect one or more pathogen indicators 106 through use of numerous detection methods in combination with aptamer binding based methods. In some embodiments, antibodies may be used in combination with aptamers or in place of aptamers.

In some embodiments, one or more detection chambers may be configured to facilitate detection of one or more pathogen indicators 106 through use of electrophoresis. In some embodiments, such detection chambers may be configured to operably associate with one or more detection units 122. Accordingly, in some embodiments, one or more detection units 122 may be configured to operably associate with one or more detection chambers and detect one or more pathogen indicators 106. Numerous electrophoretic methods may be utilized to provide for detection of one or more pathogen indicators 106. Examples of such electrophoretic methods include, but are not limited to, capillary electrophoresis, one-dimensional electrophoresis, two-dimensional electrophoresis, native electrophoresis, denaturing electrophoresis, polyacrylamide gel electrophoresis, agarose gel electrophoresis, and the like. Numerous detection methods may be used in combination with one or more electrophoretic methods to detect one or more pathogen indicators 106. In some embodiments, one or more pathogen indicators 106 may be detected according to the position to which the one or more pathogen indicators 106 migrate within an electrophoretic field (e.g., a capillary and/or a gel). In some embodiments, the position of one or more pathogen indicators 106 may be compared to one or more standards. For example, in some embodiments, one or more samples 102 may be mixed with one or more molecular weight markers prior to gel electrophoresis. The one or more samples 102, that include the one or more molecular weight markers, may be subjected to electrophoresis and then the gel may be stained. In such embodiments, the molecular weight markers may be used as a reference to detect one or more pathogen indicators 106 present within the one or more samples 102. In some embodiments, one or more components that are known to be present within one or more samples 102 may be used as a reference to detect one or more pathogen indicators 106 present within the one or more samples 102. In some embodiments, gel shift assays may be used to detect one or more pathogen indicators 106. For example, in some embodiments, a sample 102 (e.g., a single sample 102 or combination of multiple samples) may be split into a first sample 102 and a second sample 102. The first sample 102 may be mixed with an antibody, aptamer, ligand, or other molecule and/or complex that binds to the one or more pathogen indicators 106. The first and second samples 102 may then be subjected to electrophoresis. The gels corresponding to the first sample 102 and the second sample 102 may then be analyzed to determine if one or more pathogen indicators 106 are present within the one or more samples 102. Detection chambers and detection units 122 may be configured in numerous ways to provide for detection of one or more pathogen indicators 106 through use of electrophoresis.

In some embodiments, one or more detection chambers may be configured to facilitate detection of one or more pathogen indicators 106 through use of one or more charge-coupled device (CCD) cameras. In some embodiments, one or more detection units 122 that include one or more CCD cameras may be configured to operably associate with one or more detection chambers. Such detection units 122 may be utilized in combination with numerous analysis methods. Examples of such methods include, but are not limited to, electrophoresis; competition assays; methods based on polynucleotide interaction, protein interaction, peptide interaction, antibody interaction, aptamer interaction, immunoprecipitation, immunoseparation, and the like. For example, in some embodiments, one or more detection chambers may be configured to analyze one or more samples 102 through use of immunoprecipitation. In some embodiments, one or more antibodies may be conjugated to a fluorescent label such that binding of one or more labeled antibodies to one or more pathogen indicators 106 included within one or more samples 102 will form a fluorescently labeled antibody-pathogen indicator 106 complex. One or more insoluble pathogen indicator 106 binding constituents, such as a sepharose bead that includes an antibody or aptamer that binds to the one or more pathogen indicators 106, may be bound to the fluorescently labeled antibody-pathogen indicator 106 complex and used to precipitate the complex. One or more detection units 122 that include a CCD camera that is configured to detect fluorescent emission from the one or more fluorescent labels may be used to detect the one or more pathogen indicators 106. In some embodiments, one or more CCD cameras may be configured to utilize dark frame subtraction to cancel background and increase sensitivity of the camera. In some embodiments, one or more detection units 122 may include one or more filters to select and/or filter wavelengths of energy that can be detected by one or more CCD cameras (e.g., U.S. Patent No.: 3,971,065; herein incorporated by reference). In some embodiments, one or more detection units 122 may include polarized lenses. One or more detection units 122 may be configured in numerous ways to utilize one or more CCD cameras to detect one or more pathogen indicators 106.

In some embodiments, one or more detection chambers may be configured to facilitate detection of one or more pathogen indicators 106 through use of immunoassay. In some embodiments, one or more detection units 122 may be configured to operably associate with one or more such detection chambers and to detect one or more pathogen indicators 106 associated with the use of immunoassay. Numerous types of detection methods may be used in combination with immunoassay based methods. In some embodiments, a label may be used within one or more immunoassays that may be detected by one or more detection units 122. Examples of such labels include, but are not limited to, fluorescent labels, spin labels, fluorescence resonance energy transfer labels, radiolabels, electrochemiluminescent labels (e.g., U.S. Patent Nos.: 5,093,268; 6,090,545; herein incorporated by reference), and the like. In some embodiments, electrical conductivity may be used in combination with immunoassay based methods.

Figure 99 illustrates a method that may be used to detect a pathogen indicator 106 that may include one or more polynucleotides. In some embodiments, one or more polynucleotides 910 associated with one or more samples 102 may be combined with one or more sets of paired primers 920 such that the primers 920 anneal to the pathogen associated polynucleotide 910 to form one or more primed polynucleotide templates 930. Accordingly, an amplification protocol, such as a polymerase chain reaction, may be used to produce an amplification product 940 corresponding to pathogen associated polynucleic acid 910 that was contained within the one or more samples 102. In such embodiments, primers 920 may be used that include a tag that facilitates association of the amplification product 940 with an electrical conductor to complete an electrical circuit 950. Accordingly, the production of an amplification product 940 incorporates two paired primers 920 into a single amplification product 940 which allows the amplification product 940 to associate with two electrical conductors and complete an electrical circuit 950 to provide for detection of pathogen associated polynucleotides 910 within one or more samples 102.

Figure 100 illustrates an embodiment of a microfluidic chip 1000. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially parallel. In some embodiments, the reagent reservoir 1008 may include a magnetically active separation fluid that may attract one or more magnetically active pathogen indicators 106 that may be contained within the sample fluid. In some embodiments, one or more pathogen indicators 106 that may be contained within one or more samples 102 associated with the sample chamber 1002 may diffuse into the separation fluid. Accordingly, in some embodiments, such a microfluidic chip 1000 may facilitate translocation of one or more pathogen indicators 106 from one or more samples to one or more detection chambers 1014.

Figure 101 illustrates an embodiment of a microfluidic chip 1010. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially parallel. Microfluidic chip 1010 includes a magnet 1016. In some embodiments, the magnet 1016 may include an electromagnet. In some embodiments, the magnet 1016 may include a ferromagnet. In some embodiments, translocation of one or more magnetically active pathogen indicators 106 from the sample fluid into the separation fluid may be facilitated may the magnet 1016. In some embodiments, such translocation may be facilitated through one or more eddy currents. In some embodiments, such translocation may be facilitated through magnetic repulsion. Accordingly, in some embodiments, such a microfluidic chip 1010 may facilitate translocation of one or more pathogen indicators 106 from one or more samples to one or more detection chambers 1014.

Figure 102 illustrates an embodiment of a microfluidic chip 1020. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially parallel. Microfluidic chip 1020 includes a magnet 1016. In some embodiments, the magnet 1016 may include an electromagnet. In some embodiments, the magnet 1016 may include a ferromagnet. In some embodiments, translocation of one or more magnetically active pathogen indicators 106 from the sample fluid into the separation fluid may be facilitated may the magnet 1016. In some embodiments, such translocation may be facilitated through magnetic attraction. Accordingly, in some embodiments, such a microfluidic chip 1020 may facilitate translocation of one or more pathogen indicators 106 from one or more samples 102 to one or more detection chambers 1014.

Figure 103 illustrates an embodiment of a microfluidic chip 1030. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially antiparallel. In some embodiments, the reagent reservoir 1008 may include a magnetically active separation fluid that may attract one or more magnetically active pathogen indicators 106 that may be contained within the sample fluid. In some embodiments, one or more pathogen indicators 106 that may be contained within one or more samples 102 associated with the sample chamber 1002 may diffuse into the separation fluid. Accordingly, in some embodiments, such a microfluidic chip 1000 may facilitate translocation of one or more pathogen indicators 106 from one or more samples 102 to one or more detection chambers 1014.

Figure 104 illustrates an embodiment of a microfluidic chip 1040. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially antiparallel. Microfluidic chip 1040 includes a magnet 1016. In some embodiments, the magnet 1016 may include an electromagnet. In some embodiments, the magnet 1016 may include a ferromagnet. In some embodiments, translocation of one or more magnetically active pathogen indicators 106 from the sample fluid into the separation fluid may be facilitated by the magnet 1016. In some embodiments, such translocation may be facilitated through one or more eddy currents. In some embodiments, such translocation may be facilitated through magnetic repulsion. Accordingly, in some embodiments, such a microfluidic chip 1040 may facilitate translocation of one or more pathogen indicators 106 from one or more samples to one or more detection chambers 1014.

Figure 105 illustrates an embodiment of a microfluidic chip 1050. A sample chamber 1002 and a reagent chamber 1004 are each flowably associated with a mixing chamber 1006 that is flowably associated with an H-filter 1010 and a waste reservoir 1012. Such a configuration facilitates flow of a sample fluid from the sample chamber through the H-filter. A reagent reservoir 1008 is flowably associated with an H-filter 1010, a detection chamber 1014, and a waste reservoir 1012. Such a configuration facilitates flow of a separation fluid from the reagent reservoir 1008 through the H-filter. Flow of the sample fluid and the separation fluid through the H-filter is indicated by the arrows as being substantially antiparallel. Microfluidic chip 1050 includes a magnet 1016. In some embodiments, the magnet 1016 may include an electromagnet. In some embodiments, the magnet 1016 may include a ferromagnet. In some embodiments, translocation of one or more magnetically active pathogen indicators 106 from the sample fluid into the separation fluid may be facilitated by the magnet 1016. In some embodiments, such translocation may be facilitated through magnetic attraction. Accordingly, in some embodiments, such a microfluidic chip 1050 may facilitate translocation of one or more pathogen indicators 106 from one or more samples 102 to one or more detection chambers 1014.

One skilled in the art will recognize that the herein described components (e.g., steps), devices, and objects and the discussion accompanying them are used as examples for the sake of conceptual clarity and that various configuration modifications are within the skill of those in the art. Consequently, as used herein, the specific exemplars set forth and the accompanying discussion are intended to be representative of their more general classes. In general, use of any specific exemplar herein is also intended to be representative of its class, and the non-inclusion of such specific components (e.g., steps), devices, and objects herein should not be taken as indicating that limitation is desired.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. Furthermore, it is to be understood that the invention is defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the an absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

Those having skill in the art will recognize that the state of the art has progressed to the point where there is little distinction left between hardware and software implementations of aspects of systems; the use of hardware or software is generally (but not always, in that in certain contexts the choice between hardware and software can become significant) a design choice representing cost vs. efficiency tradeoffs. Those having skill in the art will appreciate that there are various vehicles by which processes and/or systems and/or other technologies described herein can be effected (e.g., hardware, software, and/or firmware), and that the preferred vehicle will vary with the context in which the processes and/or systems and/or other technologies are deployed. For example, if an implementer determines that speed and accuracy are paramount, the implementer may opt for a mainly hardware and/or firmware vehicle; alternatively, if flexibility is paramount, the implementer may opt for a mainly software implementation; or, yet again alternatively, the implementer may opt for some combination of hardware, software, and/or firmware. Hence, there are several possible vehicles by which the processes and/or devices and/or other technologies described herein may be effected, none of which is inherently superior to the other in that any vehicle to be utilized is a choice dependent upon the context in which the vehicle will be deployed and the specific concerns (e.g., speed, flexibility, or predictability) of the implementer, any of which may vary. Those skilled in the art will recognize that optical aspects of implementations will typically employ optically-oriented hardware, software, and or firmware.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one embodiment, several portions of the subject matter described herein may be implemented via Application Specific Integrated Circuits (ASICs), Field Programmable Gate Arrays (FPGAs), digital signal processors (DSPs), or other integrated formats. However, those skilled in the art will recognize that some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and /or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a recordable type medium such as a floppy disk, a hard disk drive, a Compact Disc (CD), a Digital Video Disk (DVD), a digital tape, a computer memory, etc.; and a transmission type medium such as a digital and/or an analog communication medium (e.g., a fiber optic cable, a waveguide, a wired communications link, a wireless communication link, etc.).

In a general sense, those skilled in the art will recognize that the various embodiments described herein can be implemented, individually and/or collectively, by various types of electro-mechanical systems having a wide range of electrical components such as hardware, software, firmware, or virtually any combination thereof; and a wide range of components that may impart mechanical force or motion such as rigid bodies, spring or torsional bodies, hydraulics, and electro-magnetically actuated devices, or virtually any combination thereof. Consequently, as used herein "electro-mechanical system" includes, but is not limited to, electrical circuitry operably coupled with a transducer (e.g., an actuator, a motor, a piezoelectric crystal, etc.), electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), electrical circuitry forming a communications device (e.g., a modem, communications switch, or optical-electrical equipment), and any non-electrical analog thereto, such as optical or other analogs. Those skilled in the art will also appreciate that examples of electro-mechanical systems include, but are not limited to, a variety of consumer electronics systems, as well as other systems such as motorized transport systems, factory automation systems, security systems, and communication/computing systems. Those skilled in the art will recognize that electro-mechanical as used herein is not necessarily limited to a system that has both electrical and mechanical actuation except as context may dictate otherwise.

In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

Those skilled in the art will recognize that it is common within the art to implement devices and/or processes and/or systems in the fashion(s) set forth herein, and thereafter use engineering and/or business practices to integrate such implemented devices and/or processes and/or systems into more comprehensive devices and/or processes and/or systems. That is, at least a portion of the devices and/or processes and/or systems described herein can be integrated into other devices and/or processes and/or systems via a reasonable amount of experimentation. Those having skill in the art will recognize that examples of such other devices and/or processes and/or systems might include - as appropriate to context and applicationall or part of devices and/or processes and/or systems of (a) an air conveyance (e.g., an airplane, rocket, hovercraft, helicopter, etc.), (b) a ground conveyance (e.g., a car, truck, locomotive, tank, armored personnel carrier, etc.), (c) a building (e.g., a home, warehouse, office, etc.), (d) an appliance (e.g., a refrigerator, a washing machine, a dryer, etc.), (e) a communications system (e.g., a networked system, a telephone system, a voice-over IP system, etc.), (f) a business entity (e.g., an Internet Service Provider (ISP) entity such as Comcast Cable, Quest, Southwestern Bell, etc), or (g) a wired/wireless services entity such as Sprint, Cingular, Nextel, etc.), etc.

Although a user 128 is shown/described herein as a single illustrated figure, those skilled in the art will appreciate that a user 128 may be representative of a human user, a robotic user 128 (e.g., computational entity), and/or substantially any combination thereof (e.g., a user 128 may be assisted by one or more robotic agents). In addition, a user 128 as set forth herein, although shown as a single entity may in fact be composed of two or more entities. Those skilled in the art will appreciate that, in general, the same may be said of "sender" and/or other entity-oriented terms as such terms are used herein.

There have been described various methods.

An example method involves: accepting one or more samples with one or more microfluidic chips; and processing the one or more samples with the one or more microfluidic chips to facilitate analysis of one or more pathogen indicators associated with the one or more samples.

Another example method involves: processing one or more samples with one or more microfluidic chips to facilitate analysis of one or more pathogen indicators associated with the one or more samples; and analyzing the one or more samples with one or more analysis units that are configured to operably associate with the one or more microfluidic chips.

A further example method involves: combining one or more samples with one or more magnetically active pathogen indicator binding agents that can bind to one or more pathogen indicators associated with the one or more samples to form one or more magnetically active pathogen indicator complexes; and separating the one or more magnetically active pathogen indicator complexes from the one or more samples through use of one or more magnetic fields and one or more separation fluids that are in substantially parallel flow with the one or more samples.

Another example method involves: combining one or more samples with one or more magnetically active pathogen indicator binding agents that can bind to one or more pathogen indicators associated with the one or more samples to form one or more magnetically active pathogen indicator complexes; and separating the one or more magnetically active pathogen indicator complexes from the one or more samples through use of one or more magnetic fields and one or more separation fluids that are in substantially antiparallel flow with the one or more samples.

A further example method involves: accepting one or more samples that include one or more magnetically active pathogen indicator binding agents that can bind to one or more pathogen indicators associated with the one or more samples to form one or more magnetically active pathogen indicator complexes; and separating the one or more magnetically active pathogen indicator complexes from the one or more samples through use of one or more magnetic fields and one or more separation fluids that are in substantially parallel flow with the one or more samples.

Another example method involves: accepting one or more samples that include one or more magnetically active pathogen indicator binding agents that can bind to one or more pathogen indicators associated with the one or more samples to form one or more magnetically active pathogen indicator complexes; and separating the one or more magnetically active pathogen indicator complexes from the one or more samples through use of one or more magnetic fields and one or more separation fluids that are in substantially antiparallel flow with the one or more samples.

A further example method involves: separating one or more magnetically active pathogen indicator complexes from one or more samples through use of one or more magnetic fields and one or more separation fluids that are in substantially parallel flow with the one or more samples.

Another example method involves: separating one or more magnetically active pathogen indicator complexes from one or more samples through use of one or more magnetic fields and one or more separation fluids that are in substantially antiparallel flow with the one or more samples.

The herein described subject matter sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include, but are not limited to, physically mateable and/or physically interacting components and/or wirelessly interactable and/or wirelessly interacting components and/or logically interacting and/or logically interactable components.

Any and all of the above patents, patent application publications, patent applications, and non-patent publications referred to in this specification are incorporated herein by reference, in their entireties.

## Claims

1. A method comprising:
separating one or more magnetically active pathogen indicator complexes from the one or more samples through use of one or more magnetic fields and one or more separation fluids that are in substantially parallel flow with the one or more samples or that are in substantially antiparallel flow with the one or more samples.

2. The method of claim 1, wherein said separating comprises:
separating the one or more magnetically active pathogen indicator complexes from the one or more samples through use of one or more magnetic fields and one or more separation fluids that are in substantially parallel flow with the one or more samples.

3. The method of claim 1, wherein said separating comprises:
separating the one or more magnetically active pathogen indicator complexes from the one or more samples through use of one or more magnetic fields and one or more separation fluids that are in substantially antiparallel flow with the one or more samples.

4. The method of any preceding claim, comprising, prior to said separating:
providing one or more samples that include one or more magnetically active pathogen indicator binding agents that can bind to one or more pathogen indicators associated with the one or more samples to form one or more magnetically active pathogen indicator complexes

5. The method of any preceding claim, comprising, prior to said separating:
combining one or more samples with one or more magnetically active pathogen indicator binding agents that can bind to one or more pathogen indicators associated with the one or more samples to form one or more magnetically active pathogen indicator complexes.

6. The method of claim 5, wherein said combining comprises:
combining the one or more samples with at least one magnetically active antibody, aptamer, polynucleotide, or polypeptide.

7. The method of any preceding claim, wherein said separating comprises:
separating the one or more magnetically active pathogen indicator complexes through use of magnetic attraction or magnetic repulsion.

8. The method of any of claims 1 to 6, wherein said separating comprises:
separating the one or more magnetically active pathogen indicator complexes through use of one or more ferrofluids.

9. The method of any preceding claim, further comprising:
detecting the one or more samples with one or more detection units.

10. A method comprising processing one or more samples with one or more microfluidic chips to facilitate analysis of one or more pathogen indicators associated with the one or more samples.

11. The method of claim 10, wherein said processing comprises:
processing the one or more samples through use of polynucleotide interaction, protein interaction, peptide interaction, antibody interaction, chemical interaction, diffusion, filtration, chromatography, aptamer interaction, electrical conductivity, isoelectric focusing, electrophoresis, immunoassay, or competition assay.

12. The method of claim 10 or claim 11, further comprising:
detecting the one or more pathogen indicators with one or more analysis units that are configured to operably associate with the one or more microfluidic chips.

13. The method of claim 9 or claim 12, wherein said detecting comprises:
detecting the one or more pathogen indicators with at least one technique that includes spectroscopy, electrochemical detection, polynucleotide detection, fluorescence anisotropy, fluorescence resonance energy transfer, electron transfer, enzyme assay, magnetism, electrical conductivity, isoelectric focusing, chromatography, immunoprecipitation, immunoseparation, aptamer binding, electrophoresis, use of a CCD camera, or immunoassay.

14. The method of claim 13, further comprising:
identifying one or more pathogens present within the one or more samples.

15. The method of claim 14, wherein said identifying comprises:
identifying the one or more pathogens that include at least one virus, bacterium, prion, worm, egg, cyst, protozoan, single-celled organism, fungus, algae, pathogenic protein, or microbe.

16. The method of claim 14 or claim 15, wherein said identifying comprises:
displaying an identity of the one or more pathogens present within the one or more samples.

17. The method of any preceding claim, wherein the one or more samples includes one or more of:
one or more liquids,
one or more solids;
one or more gases;
one or more food products;
one or more biological samples.

18. A system comprising means for implementing the method of any preceding claim.

19. The system of claim 18, wherein said means comprise means for separating one or more magnetically active pathogen indicator complexes from the one or more samples through use of one or more magnetic fields and one or more separation fluids that are in substantially parallel flow with the one or more samples or that are in substantially antiparallel flow with the one or more samples.

20. The system of claim 18 or claim 19, comprising at least one microfluidic chip.
